(19) Europäisches Patentamt
European Patent Office
Office européen des brevets

(11) EP 2 947 081 B1

# (12) EUROPEAN PATENT SPECIFICATION

(45) Date of publication and mention of the grant of the patent:
**18.10.2017 Bulletin 2017/42**

(21) Application number: **15157476.1**

(22) Date of filing: **24.06.2011**

(51) Int Cl.:
*C07D 471/04* (2006.01) *A61K 31/437* (2006.01)
*A61P 13/12* (2006.01) *A61P 11/00* (2006.01)
*A61P 19/10* (2006.01) *A61P 25/28* (2006.01)
*A61P 35/00* (2006.01)

## (54) 2-Pyridyl substituted imidazoles as therapeutic Alk5 and/or Alk4 inhibitors

2-pyridyl-substituierte Imidazole als therapeutische ALK5- und/oder ALK4-Hemmer

Imidazoles à substitution 2-pyridyle comme inhibiteurs thérapeutiques alk4 et/ou alk5

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.06.2010 US 826338**

(43) Date of publication of application:
**25.11.2015 Bulletin 2015/48**

(62) Document number(s) of the earlier application(s) in accordance with Art. 76 EPC:
**11801087.5 / 2 588 479**

(73) Proprietor: **Ewha University-Industry Collaboration Foundation**
**Seodaemun-gu**
**Seoul, 120-750 (KR)**

(72) Inventors:
- **Kim, Dae Kee**
  **110-901 Seoul (KR)**
- **Sheen, Yhun Yhong**
  **Seocho-gu, Seoul 137-764 (KR)**
- **Jin, Cheng Hua**
  **Seodaemun-gu, Seoul 120-750 (KR)**
- **Park, Chul-Yong**
  **Eunpyeong-gu, Seoul 120-750 (KR)**
- **Sreenu, Domalapally**
  **Seodaemun-gu, Seoul 120-750 (KR)**
- **Rao, Kota Sudhakar**
  **Seodaemun-gu, Seoul 120-750 (KR)**
- **Krishnaiah, Maddeboina**
  **Seodaemun-gu, Seoul 120-750 (KR)**
- **Subrahmanyam, Vura Bala**
  **Seodaemun-gu, Seoul 120-140 (KR)**

(74) Representative: **V.O.**
**P.O. Box 87930**
**2508 DH Den Haag (NL)**

(56) References cited:
**WO-A1-2004/026859 WO-A1-2005/103028**
**WO-A2-2009/150547**

- **KIM D K ET AL: "Synthesis and biological evaluation of benzenesulfonamide-substituted 4-(6-alkylpyridin-2-yl)-5-(quinoxalin-6-yl )imidazoles as transforming growth factor-beta type 1 receptor kinase inhibitors", EUROPEAN JOURNAL OF MEDICINAL CHEMISTRY, EDITIONS SCIENTIFIQUE ELSEVIER, PARIS, FR, vol. 44, no. 2, 1 February 2009 (2009-02-01), pages 568-576, XP025950187, ISSN: 0223-5234, DOI: 10.1016/J.EJMECH.2008.03.024 [retrieved on 2008-04-04]**
- **KIM Y W ET AL: "Pharmacokinetics and tissue distribution of 3-((5-(6-methylpyridin-2-yl)-4-(quinoxalin -6-yl)-1H-imidazol-2- yl)methyl)benzamide; a novel ALK5 inhibitor and a potential anti-fibrosis", XENOBIOTICA, TAYLOR AND FRANCIS, LONDON, GB, vol. 38, no. 3, 1 January 2008 (2008-01-01), pages 325-339, XP008136643, ISSN: 0049-8254, DOI: 10.1080/00498250701781924**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 947 081 B1

## Description

### TECHNICAL FIELD

This invention relates to 2-pyridyl substituted imidazoles which are inhibitors of the transforming growth factor-β (TGF-β) type I receptor (ALK5) and/or the activin type I receptor (ALK4), methods for their preparation, and their use in medicine, specifically in the treatment and prevention of a disease state mediated by these receptors.

### BACKGROUND ART

TGT-β denotes a family of proteins, TGF-β1, TGF-β2 and TGF-β3, which are pleiotropic modulators of cell proliferation and differentiation, wound healing, extracellular matrix production, and immunosuppression. Other members of this superfamily include activins, inhibins, bone morphogenetic proteins, growth and differentiation factors, and Mullerian inhibiting substance.

TGF-β1 transduces signals through two highly conserved single transmembrane serine/threonine kinases, the type I (ALK5) and type II TGF-β receptors. Upon ligand induced oligomerization, the type II receptor hyperphosphorylates serine/threonine residues in the GS region of the ALK5, which leads to activation of the ALK5 by creating a binding site for Smad proteins. The activated ALK5 in turn phosphorylates Smad2 and Smad3 proteins at the C-terminal SSXS-motif thereby causing their dissociation from the receptor and heteromeric complex formation with Smad4. Smad complexes translocate to the nucleus, assemble with specific DNA-binding co-factors and co-modulators to finally activate transcription of extracellular matrix components and inhibitors of matrix-degrading proteases.

Activins transduce signals in a manner similar to TGF-β. Activins bind to serine/thereonine kinase, the activin type II receptor (ActRIIB), and the activated type II receptor hyperphosphorylates serine/threonine residues in the GS region of the ALK4. The activated ALK4 in turn phosphorylates Smad2 and Smad3. The consequent formation of a hetero-Smad complex with Smad4 results in the activin-induced regulation of gene transcription.

Numerous experimental animal studies demonstrate an association between glomerular expression of TGT-β and fibrosis, including the Thy-1 rat model of proliferative glomerulonephritis, anti-GBM glomerulonephritis in rabbits, and the 5/6 nephrectomy rat model of focal segmental glomerulosclerosis, as has been reviewed recently (e.g., Bitzer, M. et al., Kidney Blood Press. Res. 21: 1-12 (1998)). Neutralizing antibody to TGT-β improves glomerular histology in the Thy-1 nephritis model (e.g., Border, W. A. et al., Nature 346: 371-374 (1990)).

Hyperglycemic conditions increase TGT-β mRNA and protein synthesis in both murine proximal tubule cells and human mesangial cells (e.g., Wahab, N. A. et al., Biochem. J. 316: 985-992 (1996); Rocco, M. V. et al., Kidney Int. 41: 107-114 (1992)). Diabetic patients with early kidney disease show increased accumulation of TGT-β mRNA and protein within the glomerulus (e.g., Yoshioka, K. et al., Lab. Invest. 68: 154-163 (1993)). In kidneys with chronic renal interstitial fibrosis, the hallmarks are thickened tubular basement membranes and an expanded interstitial compartment, with interstitial fibrosis characterized by an increase in collagens I, III, V, VII, and fibronectin (e.g., Eddy, A. A., J. Am. Soc. Nephrol. 7: 2495-2508 (1996)).

TGT-β gene expression and protein production are increased in a variety of animal models of pulmonary fibrosis including bleomycin, silica, asbestos, and radiation (e.g., Phan, S. H. and Kunkel, S. L., Exp. Lung Res. 18: 29-43 (1992); Williams, A. O. et al., Am. J. Pathol. 142: 1831-1840 (1993); Rube, C. E. et al., Int. J. Radiat. Oncol. Biol. Phys. 47: 1033-1042 (2000)). Coincident increase in TGF-β1 protein and collagen gene expression in adjacent tissue slices from idiopathic pulmonary fibrosis is observed in human pulmonary fibrotic disease (e.g., Broekelmann, T. J. et al., Proc. Natl. Acad. Sci. USA 88: 6642-6646 (1991)). Increased TGT-β production has been documented in patients with sarcoidosis, pneumoconiosis, asbestosis, and radiation-induced fibrosis (e.g., Khalil, N. et al., Am. J. Respir. Cell. Mol. Biol. 14: 131-138 (1996); Jagirdar, J. et al., Environ. Health Perspect. 105: 1197-1203 (1997)). Anti-TGF-β antibodies and TGF-β-soluble receptors could partially inhibit fibrosis in bleomycin-induced lung fibrosis rodent models (e.g., Giri, S. N. et al., Thorax 48: 959-966 (1993); Wang, Q. et al., Thorax 54: 805-812 (1999)). Tobacco smoke has been implicated as one of the most important factors that can cause small airway disease followed by chronic obstructive pulmonary disease (COPD) (e.g., Wright, J. M. et al., Am. Rev. Respir. Dis. 146: 240-262 (1992)). COPD is a slowly progressive and irreversible disorder characterized by the functional abnormality of airway obstruction. TGT-β has been hypothesized to be involved in airway remodeling found in chronic airway inflammatory disorders such as COPD (e.g., Takizawa, H. Int. J. Mol. Med. 1: 367-378 (1998); Ning, W. et al., Proc. Natl. Acad. Sci. USA 101: 14895-14900 (2004)).

Hepatic stellate cells (HSC) are the major source of extracellular matrix proteins in hepatic fibrosis. Extracellular matrix production by activated hepatic stellate cells is markedly increased through the action of TGF-β1 (e.g., Friedman, S. L., Prog. Liver Dis. 14: 101-130 (1996); Pietrangelo, A., Semin. Liver Dis. 16: 13-30 (1996)). Transgenic mice that overexpress TGF-β1 in the liver develop hepatic fibrosis as well as extrahepatic pathologies such as renal fibrosis (e.g., Sanderson, N. et al., Proc. Natl. Acad. Sci. USA 92: 2572-2576 (1995)).

TGF-β1 and its receptors are overexpressed in injured blood vessels and in fibroproliferative vascular lesions

leading to overproduction of extracellular matrix (e.g., Saltis, J. et al., Clin. Exp. Pharmacol. Physiol. 23: 193-200 (1996); McCaffrey, T. A. et al., J. Clin. Invest. 96: 2667-2675 (1995)).

Anti-TGF-β antibodies reduce scar formation and improve the cytoarchitecture of the neodermis in rats (e.g., Shah, M., J. Cell. Sci. 108: 985-1002 (1995)), improve healing of corneal wounds in rabbits (e.g., Moller-Pedersen, T., Curr. Eye Res. 17: 736-747 (1998)), and accelerate wound healing of gastric ulcers in rats (e.g., Ernst, H., Gut 39: 172-175 (1996)).

Radiation fibrosis is a frequent sequel of therapeutic or accidental radiation overexposure in normal human tissues. TGF-β1 plays a central role in the initiation, development, and persistence of radiation fibrosis, as has been reviewed recently (e.g., Martin, M. et al., Int. J. Radiat. Oncol. Biol. Phys. 47: 277-290 (2000)).

Organ transplantation is complicated in many instances by chronic rejection and for some organs such as the kidney, it is the major forms of graft loss. In human patients, chronic rejection of lung and kidney transplants is associated with increased expression of TGT-β within the tissue (e.g., El-Gamel, A. et al., Eur. J. Cardiothorac. Surg. 13: 424-430 (1998); Shihab, F. S. et al., J. Am. Soc. Nephrol. 6: 286-294 (1995)).

TGT-β is implicated in peritoneal adhesions (e.g., Saed, G. M. et al., Wound Repair Regen. 7: 504-510 (1999)). The peritoneal and sub-dermal fibrotic adhesions could be prevented by inhibitors of ALK5 and/or ALK4.

TGF-β2 levels are increased in nearly half of the eyes with primary open-angle glaucoma (POAG) and in most of the eyes with juvenile glaucoma in the aqueous humor of eyes (e.g., Picht, G. et al., Graefes Arch. Clin. Exp. Ophthalmol. 239: 199-207 (2001)). Both TGF-β1 and TGF-β2 isoforms are reported to increase extracellular matrix production in cultured human Tenon's capsule fibroblasts derived from patients with pseudoexfoliation glaucoma and POAG (e.g., Kottler, U. B. et al., Exp. Eye Res. 80: 121-134 (2005)). US 2007/0142376 A1 discloses treatment of glaucoma and control of intraocular pressure using ALK5 modulating agents, and an ALK5 inhibitor reduces the level of fibronectin in TGF-β2-treated perfused human anterior segments and the levels of fibronectin, plasminogen activator inhibitor-1 (PAI-1), and pro-collagen type I C-peptide in TGF-β2-treated trabecular meshwork cell cultures.

The tumor cells and the stromal cells within the tumors in late stages of various cancers generally overexpress TGT-β. This leads to stimulation of angiogenesis and cell motility, suppression of the immune system, and increased interaction of tumor cells with the extracellular matrix (e.g., Hojo, M. et al., Nature 397: 530-534 (1999)). Consequently, the tumor cells become more invasive and metastasize to distant organs (e.g., Maehara, Y et al., J. Clin. Oncol. 17: 607-614 (1999); Picon, A. et al., Cancer Epidemiol. Biomarkers Prev. 7: 497-504 (1998)).

PAI-1 is the major physiological inhibitor of both tissue-type plasminogen activator and urokinase-type plasminogen activator. Elevated levels of PAI-1 are associated with thrombosis and vascular disease, suggesting that high plasma PAI-1 may promote a hypercoagulable state by disrupting the natural balance between fibrinolysis and coagulation (e.g., Vaughan, D. E., J. Invest. Med. 46: 370-376 (1998)). It is known that TGF-β stimulates the expression of PAI-1 (e.g., Dennler, S. et al., EMBO J. 17: 3091-3100 (1998)). Accordingly, inhibition of the production of PAI-1 with an inhibitor of the TGT-β signaling pathway could produce a novel fibrinolytic therapy.

Activin signaling and overexpression of activin is linked to pathological disorders that involve extracellular matrix accumulation and fibrosis (e.g., Matsuse, T. et al., Am. J. Respir. Cell Mol. Biol. 13: 17-24 (1995); Inoue, S. et al., Biochem. Biophys. Res. Comm. 205: 441-448 (1994); Matsuse, T. et al., Am. J. Pathol. 148: 707-713 (1996); De Bleser et al., Hepatology 26: 905-912 (1997); Pawlowski, J. E., et al., J. Clin. Invest. 100: 639-648 (1997); Sugiyama, M. et al., Gastroenterology 114: 550-558 (1998); Munz, B. et al., EMBO J. 18: 5205-5215 (1999)), inflammatory responses (e.g., Rosendahl, A. et al., Am. J. Respir. Cell Mol. Biol. 25: 60-68 (2001), cachexia or wasting (Matzuk, M. M. et al., Proc. Natl. Acd. Sci. USA 91: 8817-8821 (1994); Coerver, K. A. et al., Mol. Endocrinol. 10: 534-543 (1996); Cipriano, S. C. et al., Endocrinology 141: 2319-2327 (2000)), diseases or pathological responses in the central nervous system (e.g., Logan, A. et al., Eur. J. Neurosci. 11: 2367-2374 (1999); Logan, A. et al., Exp. Neurol. 159: 504-510 (1999); Masliah, E. et al., Neurochem. Int. 39: 393-400 (2001); De Groot, C. J. A. et al., J. Neuropathol. Exp. Neurol. 58: 174-187 (1999); John, G. R. et al., Nat. Med. 8: 1115-1121 (2002)) and hypertension (e.g., Dahly, A. J. et al., Am. J. Physiol. Regul. Integr. Comp. Physiol. 283: R757-767 (2002)). Studies have shown that TGT-β and activin can act synergistically to induce extracellular matrix production (e.g., Sugiyama, M. et al., Gastroenterology 114; 550-558 (1998)).

Therefore, it becomes evident that inhibition of ALK5 and/or ALK4 phosphorylation of Smad2 and Smad3 by the preferred compounds of this invention could treat and prevent disorders involving these signaling pathways.

WO 00/61576 and US 2003/0149277 A1 disclose triarylimidazole derivatives and their use as ALK5 inhibitors. WO 01/62756 A1 discloses pyridinylimidazole derivatives and their use as ALK5 inhibitors. WO 02/055077 A1 discloses use of imidazolyl cyclic acetal derivatives as ALK5 inhibitors. WO 03/087304 A2 discloses tri-substituted heteroaryls and their use as ALK5 and/or ALK4 inhibitors. WO 2005/103028 A1 and US 7,407,958 B2 disclose 2-pyridyl substituted imidazoles as ALK5 and/or ALK4 inhibitors. Especially, one of the representative compounds claimed in WO 2005/103028 A1 and US 7,407,958 B2 , IN-1130, demonstrates its use in several animal models as ALK5 and/or ALK4 inhibitors. IN-1130 effectively suppresses renal fibrosis induced by unilateral ureteral obstruction (UUO) in rats (Moon, J.-A. et al., Kidney Int. 70: 1234-1243 (2006)), ameliorates experimental autoimmune encephalomyelitis (EAE) in SBE-luc and GFAP-luc mice immunized with $MOG_{35-55}$ (Luo, J. et al., J. Clin. Invest. 117: 3306-3315 (2007)), lessens tunical fibrosis

and corrects penile curvature in rats (Ryu, J.-K. et al., J. Sex. Med. 6: 1284-1296 (2009)), and dramatically reduces tumor volume with an enhanced immune response in mice treated with murine prostate cancer cell line Tramp C2 (Lee, G. T. et al., J. Urol. 180: 2660-2667 (2008)). And, also, US 2008/0319012 A1 discloses 2-pyridyl substituted imidazoles as ALK5 and/or ALK4 inhibitors. Especially, one of the representative compounds claimed in US 2008/0319012 A1, IN-1233, demonstrates its use in several animal models as ALK5 and/or ALK4 inhibitors. IN-1233 effectively prevents the development and progression of pulmonary arterial hypertension in the monocrotaline rat model through the inhibition of TGF-β signaling (Long, L. et al., Circulation 119: 566-576 (2009)) and also prevents granulation tissue formation after bare metallic stent placement in a rat urethral model (Kim, J. H. et al., Radiology 255: 75-82 (2010)).

Kim et al. in "synthesis and biological evaluation of benzenesulfonamide-substituted 4-(6-alkylpyridon-2-yl)-5-(quioxalin-6-yl)imidazoles as transforming growth factor-beta type 1 receptor kinase inhibitors" (Eur.J. Med. Chem. vol 44, no. 2, pg 568-576 (2009), describes compounds which do not have a thiazolopyridine ring.

Kim et al. in "pharmacokinetics and tissue distribution of 3-((5-(6-methylpyridin-2-yl)-4-quinoxalin-6-yl)-1H-imidazol-2-yl)methyl)benzamide; a novel AK5 inhibitor and a potential antifibrosis", furthermore describes compounds which do not have a thiazolopyridine ring.

WO2005/103028 and WO 2009/150547 furthermore describe compounds which do not have a thiazolopyridine ring.

## SUMMARY OF THE INVENTION

### Technical Solution

The invention pertains to a pharmaceutical composition, comprising one or more of the compounds

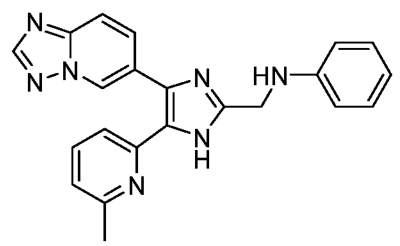
,
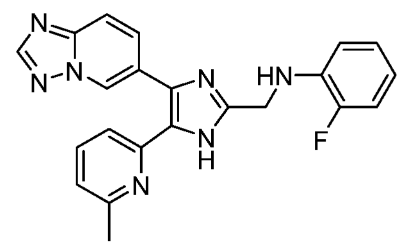
,
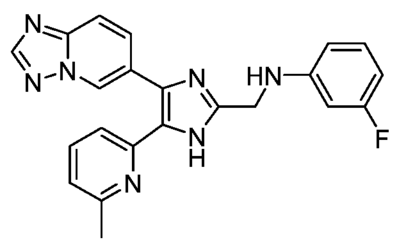
,
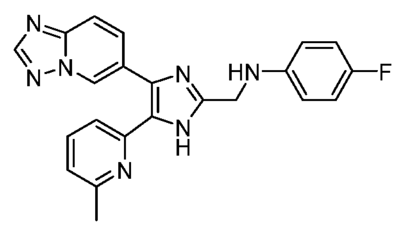
,
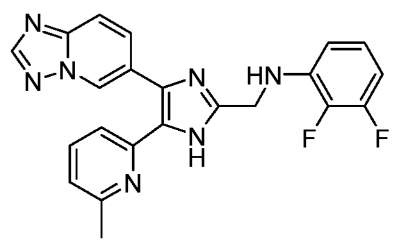
,
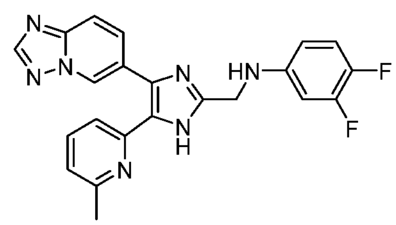
,

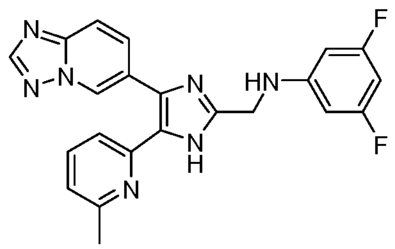

,

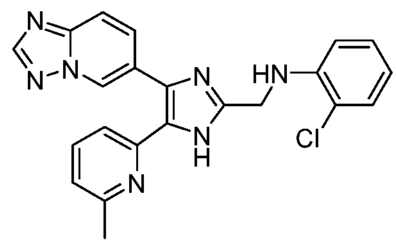

,

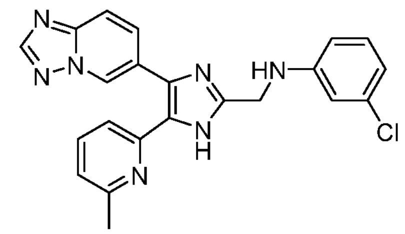

,

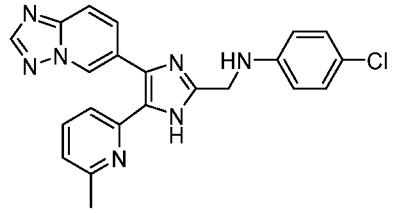

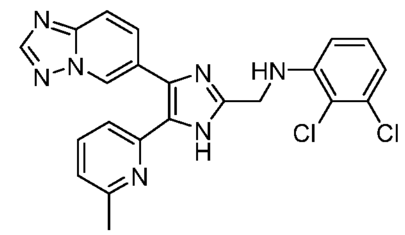

,

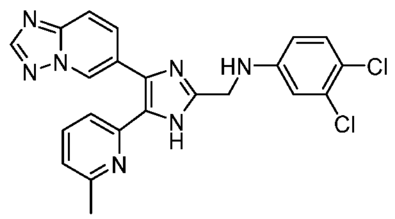

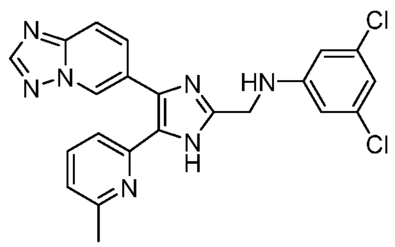

,

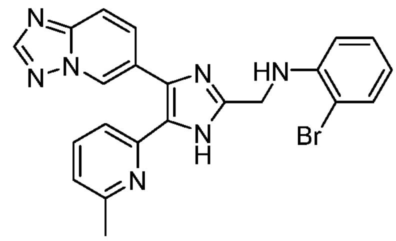

,

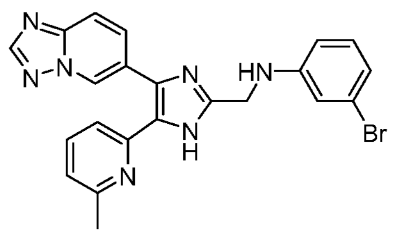

,

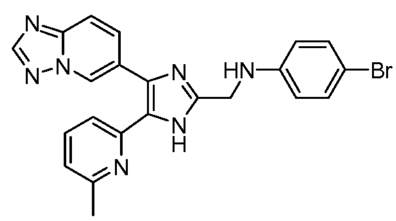

,

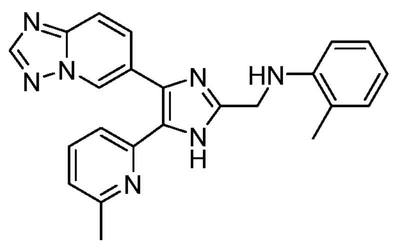

,

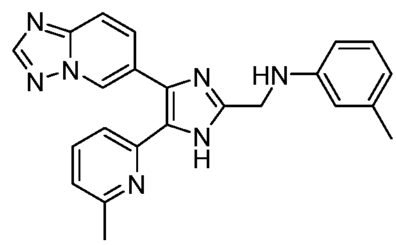

,

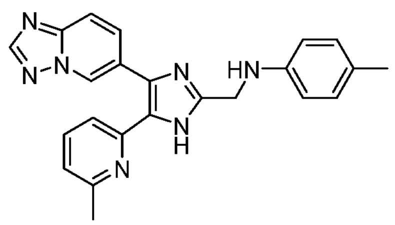
,
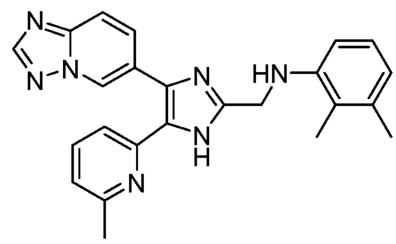
,
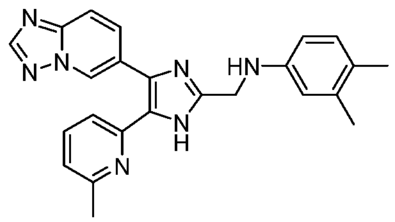
,
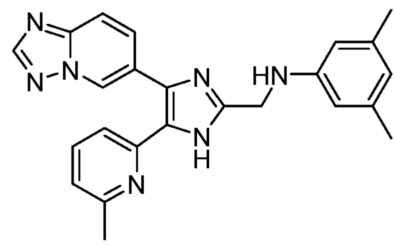
,
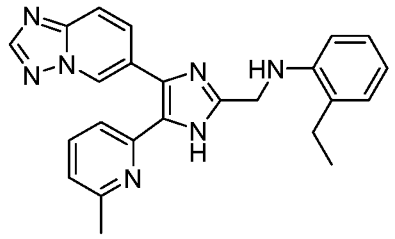
,
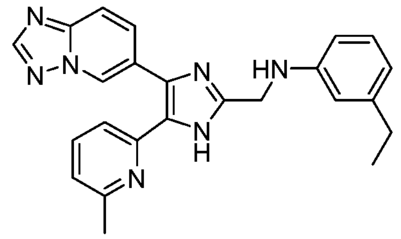
,
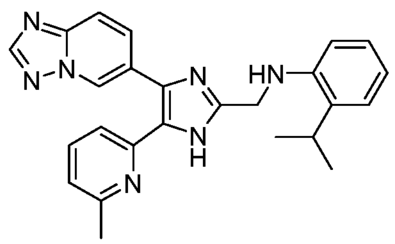
,
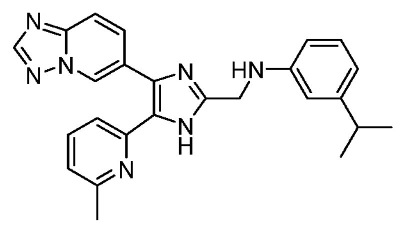
,
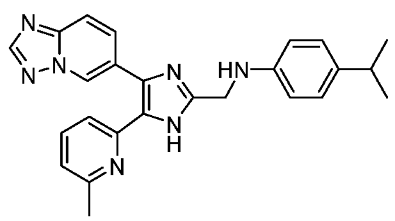
,
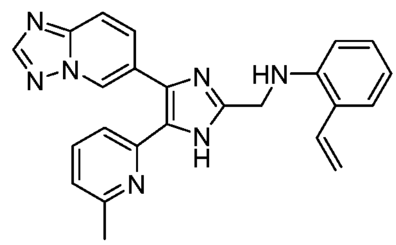
,
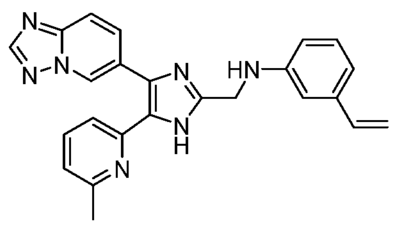
,
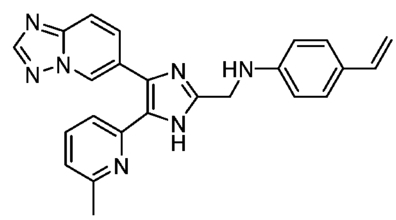
,

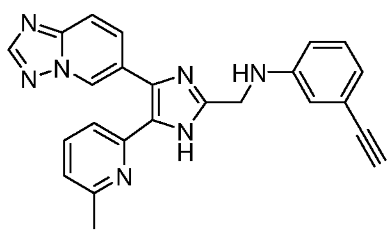
,
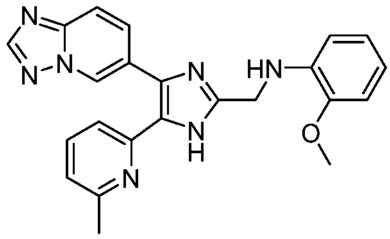
,
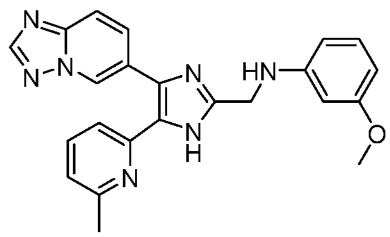
,
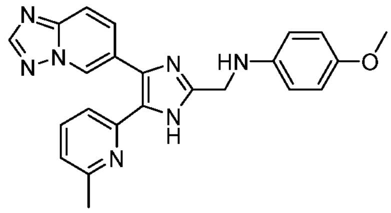
,
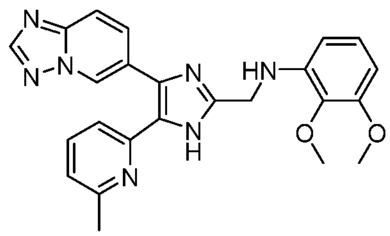
,
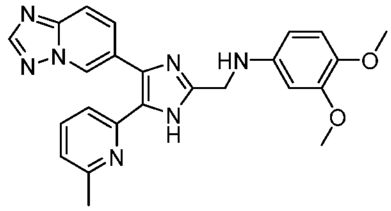
,
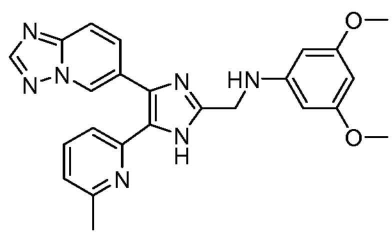
,
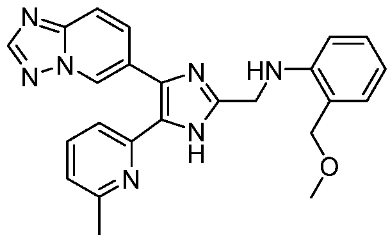
,
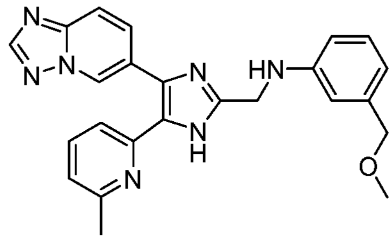
,
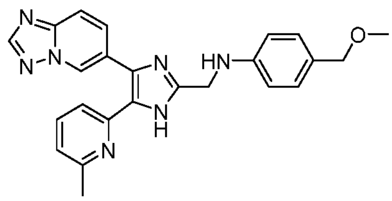
,
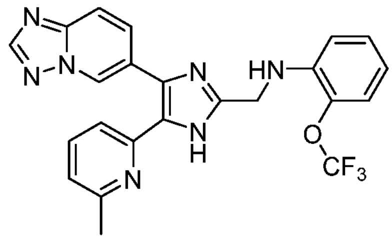
,
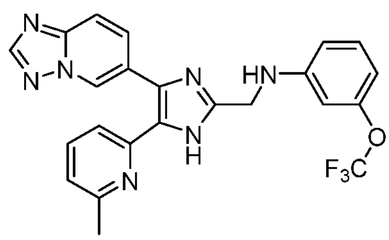
,

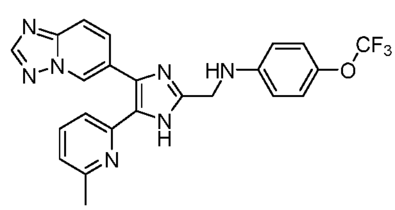 ,

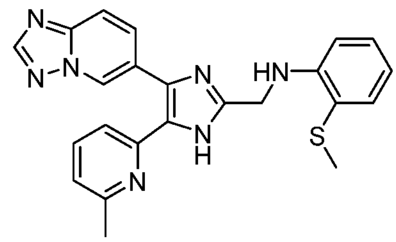 ,

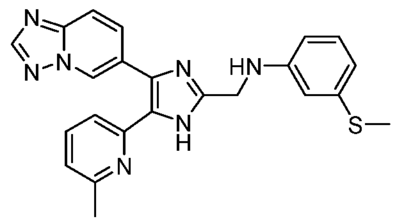 ,

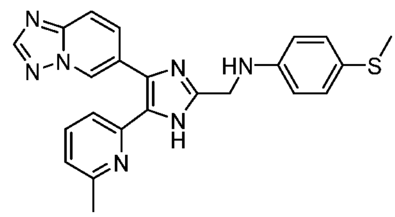 ,

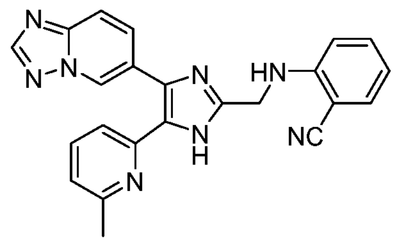 ,

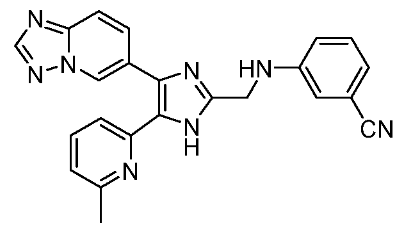 ,

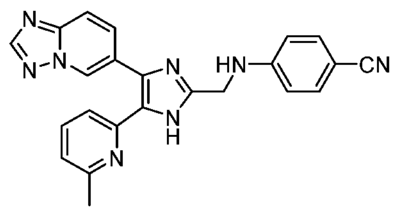 ,

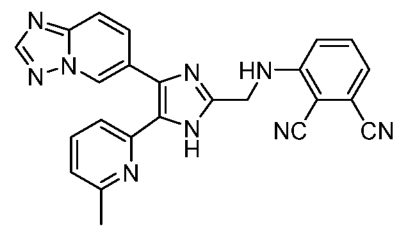 ,

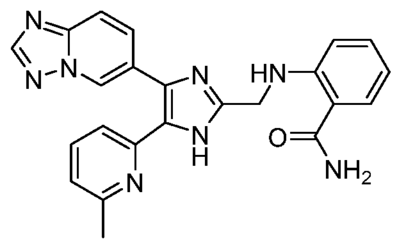 ,

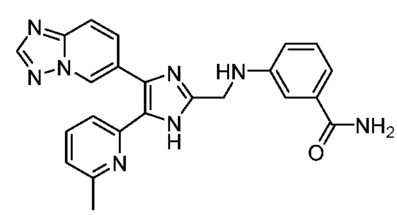 ,

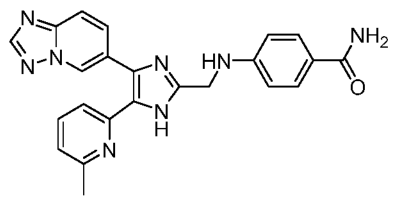 ,

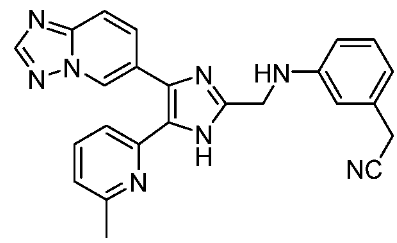 ,

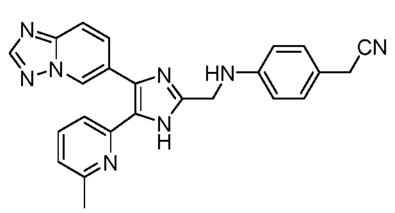
,
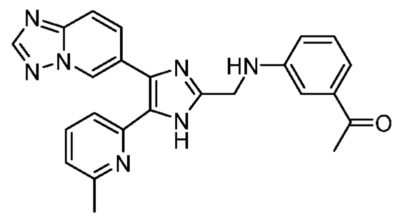
,
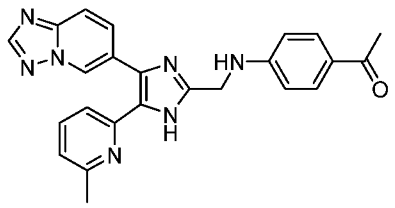
,
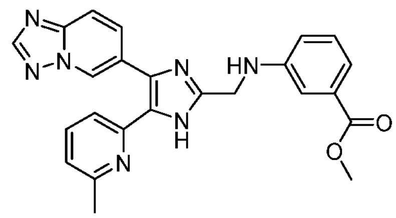
,
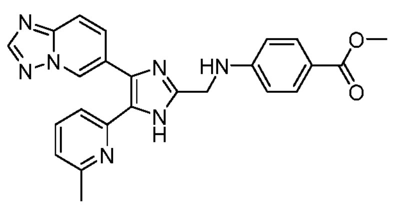
,
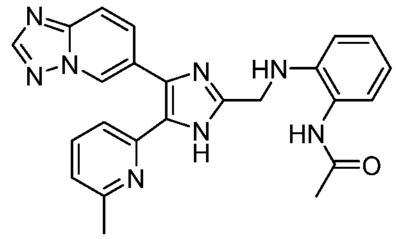
,
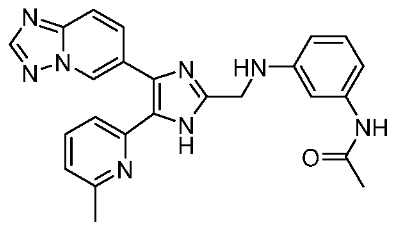
,
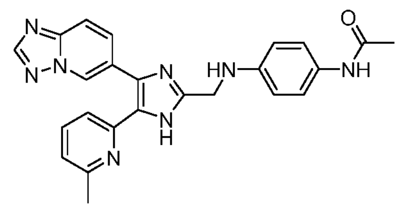
,
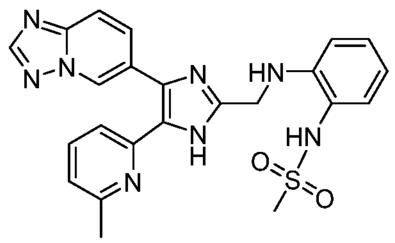
,
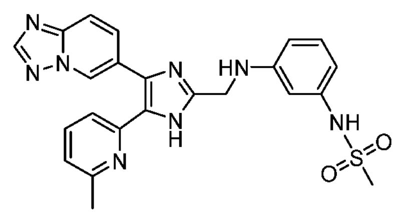
,
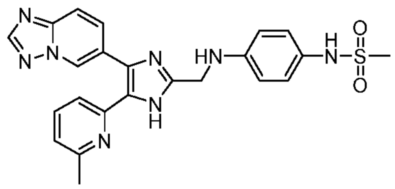
,
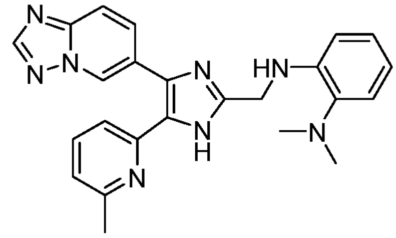
,

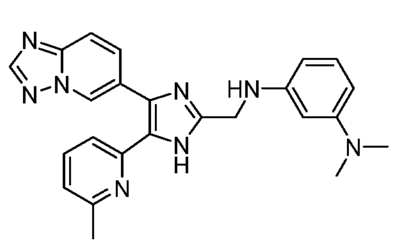

,

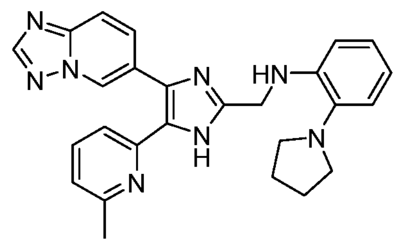

,

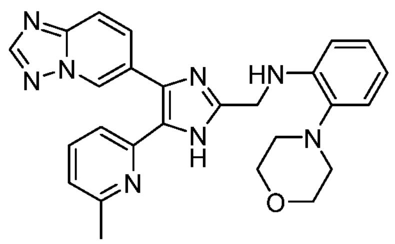

,

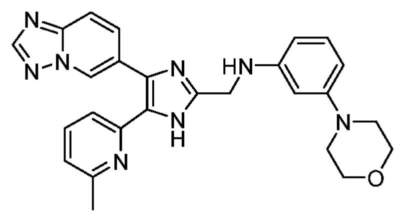

,

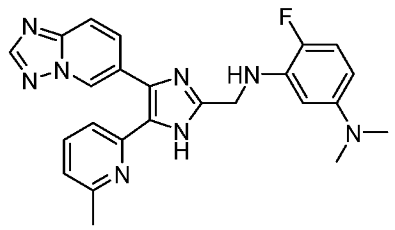

,

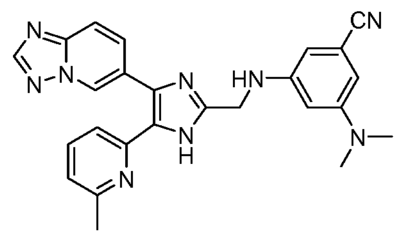

,

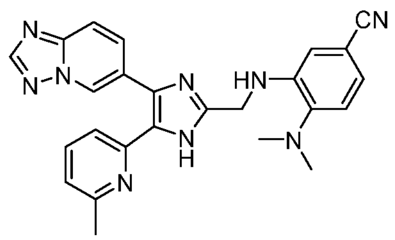

,

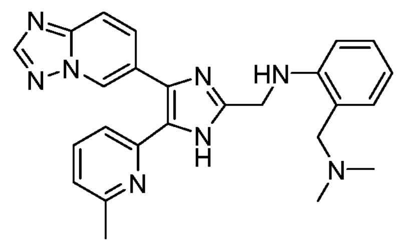

,

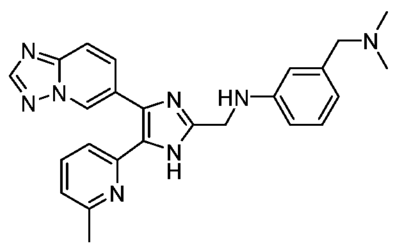

,

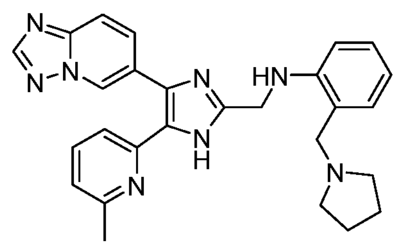

,

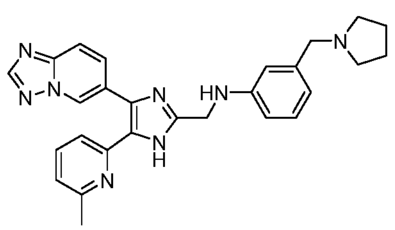

,

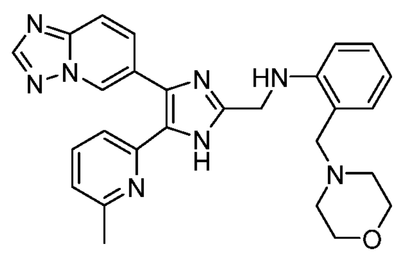

,

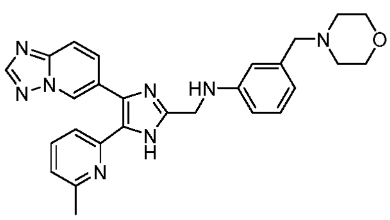
,

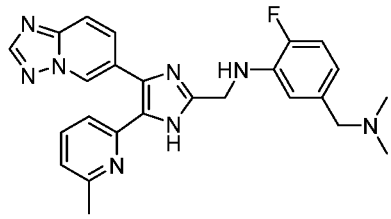
,

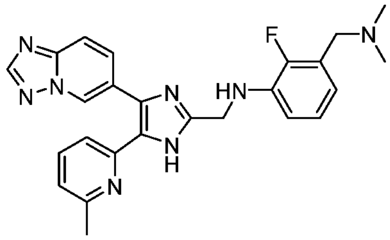
,

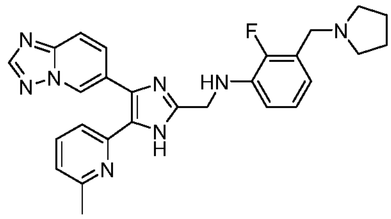
,

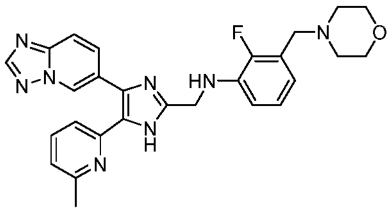
,

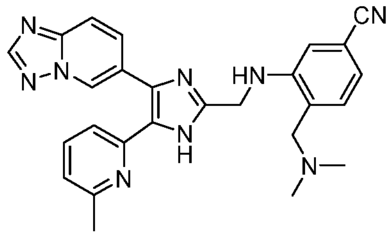
,

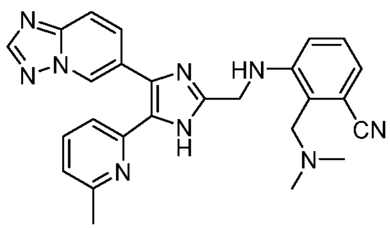
,

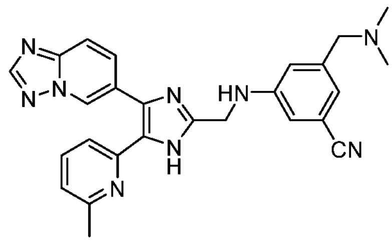
,

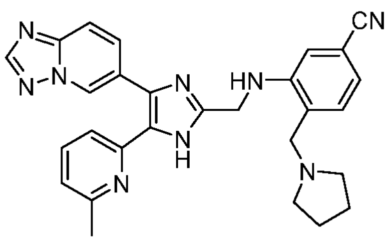
,

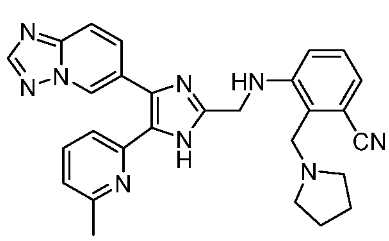
,

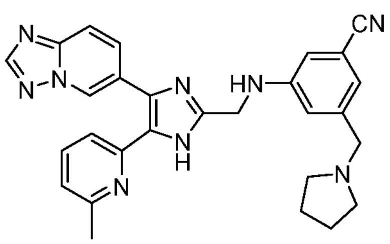
,

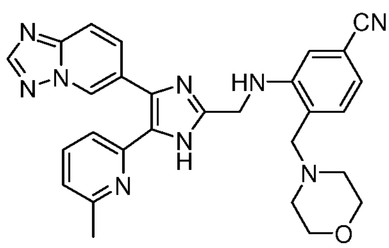
,

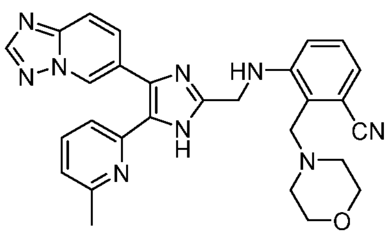
,
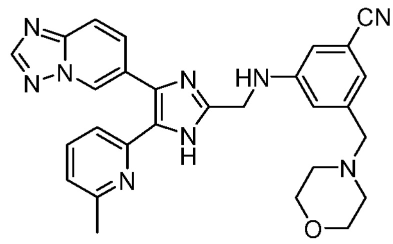
,
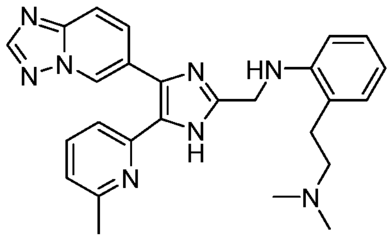
,
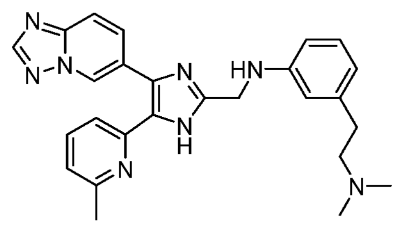
,
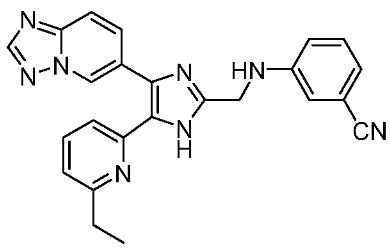
,
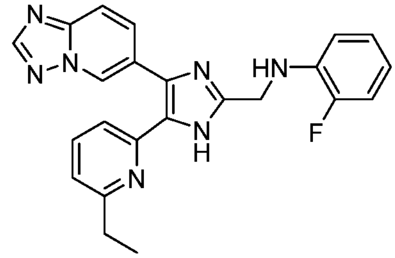
,
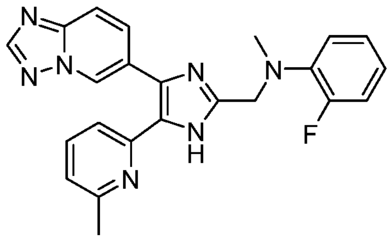
,
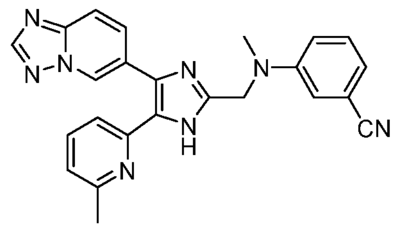
,
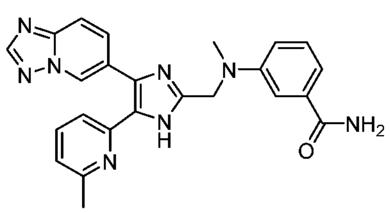
,
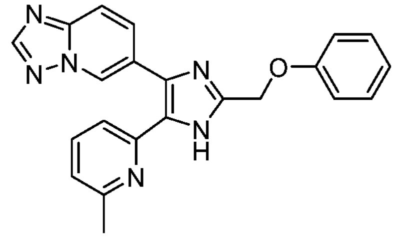
,
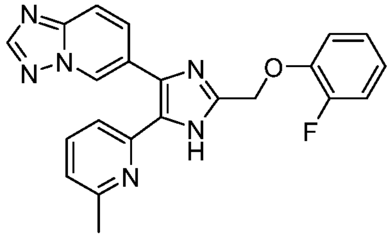
,
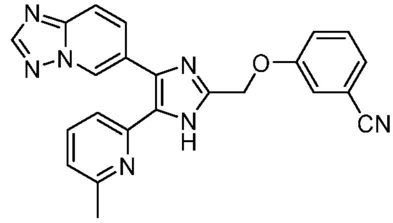
,

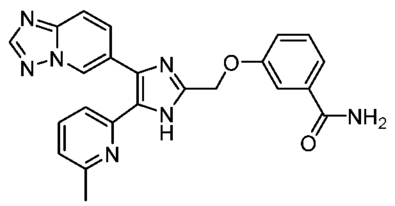

,

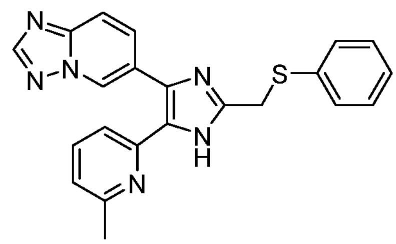

,

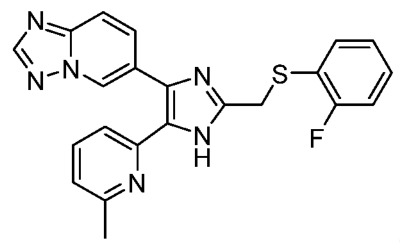

,

or a pharmaceutically acceptable salt or hydrate thereof, wherein

- the pharmaceutical composition is an oral, buccal or sub-lingual dosage form delivering 50-5000 mg of the one or more compounds daily; or
- the pharmaceutical composition is a topical formulation further comprising 1 - 98 % of a carrier; or
- the pharmaceutical composition is a parenteral dosage form comprising 25-250 mg of the one or more compounds per single dose.

Surprisingly, it has now been discovered that these 2-pyridyl substituted imidazoles function as potent and selective inhibitors of ALK5 and/or ALK4 and, therefore, have utility in the treatment, prevention, and reduction of various disease states mediated by ALK5 and/or ALK4, such as glomerulonephritis, diabetic nephropathy, lupus nephritis, hypertension-induced nephropathy, renal interstitial fibrosis, renal fibrosis resulting from complications of drug exposure, HIV-associated nephropathy, transplant nephropathy, liver fibrosis due to all etiologies, hepatic dysfunction attributable to infections, alcohol-induced hepatitis, disorders of the biliary tree, cystic fibrosis, pulmonary fibrosis, interstitial lung disease, acute lung injury, adult respiratory distress syndrome, idiopathic pulmonary fibrosis, chronic obstructive pulmonary disease, pulmonary disease due to infectious or toxic agents, post-infarction cardiac fibrosis, congestive heart failure, dilated cardiomyopathy, myocarditis, intimal thickening, vascular stenosis, hypertension-induced vascular remodeling, pulmonary arterial hypertension, coronary restenosis, peripheral restenosis, carotid restenosis, stent-induced restenosis, atherosclerosis, ocular scarring, corneal scarring, proliferative vitreoretinopathy, glaucoma, intraocular pressure, excessive or hypertrophic scar or keloid formation in the dermis occurring during wound healing resulting from trauma or surgical wounds, peritoneal and sub-dermal adhesion, scleroderma, fibrosclerosis, progressive systemic sclerosis, dermatomyositis, polymyositis, arthritis, osteoporosis, ulcers, impaired neurological function, male erectile dysfunction, Peyronie's disease, Dupuytren's contracture, Alzheimer's disease, Raynaud's syndrome, radiation-induced fibrosis, thrombosis, tumor metastasis growth, multiple myeloma, melanoma, glioma, glioblastomas, leukemia, sarcomas, leiomyomas, mesothelioma, and carcinomas of lung, breast, colon, kidney, ovary, cervix, liver, biliary tract, gastrointestinal tract, pancreas, prostate, head, and neck.

## DESCRIPTION OF DRAWINGS

The aforementioned aspects and other features of the present invention will be explained in the following description, taken in conjunction with the accompanying drawings, wherein:

FIG. 1 shows effect of Examples 2, 60, 86, 92, and 94 on the TGF-β1-induced 3TP-Luc reporter activity in HaCaT-3TP-Luc cells,
FIG. 2 shows effect of Examples 2, 60, 86, 92, and 94 on the TGF-β1-induced 3TP-Luc reporter activity in 4T1-3TP-Luc cells,
FIG. 3 shows effect of Example 2 on the TGF-β1-induced Smad2/3 nuclear translocation in MCF10A cells,
FIG. 4 shows effect of Example 2 on the TGF-β1-induced cell migration in MCF10A cells,
FIGS. 5a and 5b show effect of Example 2 on the TGF-β1-induced cell invasion in 4T1 cells.
(5a). DAPI-stained cells remaining on the bottom surface. (5b). Average cell number per view field obtained from 5

random fields,
FIG. 6 shows effect of Example 2 on the cell growth of 4T1 cells,
FIG. 7 shows effect of Example 2 on the cell growth of MCF10A cells,
FIGS. 8a and 8b show effect of Example 3 on the breast tumor metastasis to the lung in BALB/c 4T1 xenografted mice. Example 3 (13.6 or 27.3 mg/kg) dissolved in water (vehicle) was given to mice orally BID five consecutive days per week for four weeks. (8a). White spots on the lung surface indicate metastastic nodules (white arrows). (8b). Number of metastastic nodules on whole lung surface,
FIGS. 9a and 9b show effect of Example 2 on the breast tumor metastasis to the lung in BALB/c 4T1 xenografted mice. Example 2 (5, 10, 20, or 40 mg/kg) dissolved in artificial gastric fluid formulation (vehicle) was given to mice orally five consecutive days per week for three weeks. (9a). White spots on the lung surface indicate metastastic nodules. (9b). Number of metastastic nodules on surface of left lobe of lung,
FIGS. 10a, 10b, and 10c show effect of Example 2 on the breast tumor metastasis to the lung in BALB/c 4T1 xenografted mice. Example 2 (5, 10, 20, or 40 mg/kg) dissolved in artificial gastric fluid formulation (vehicle) was given to mice orally every other day (three times per week) for 24 days. (10a). White spots on the lung surface indicate metastastic nodules. (10b). Number of metastastic nodules on surface of left lobe of lung. (10c). Effect on the TGF-β1-induced Smad2 phosphorylation in tumor tissues,
FIGS. 11a, 11b, and 11c show effect of Example 61 on the breast tumor metastasis to the lung in BALB/c 4T1 xenografted mice. Example 61 (43.6 mg/kg) dissolved in saline (vehicle) was given to mice intraperitoneally every other day (three times per week) for 2.5 weeks. (11a). White spots on the lung surface indicate metastastic nodules. (11b). Number of metastastic nodules on surface of left lobe of lung. (11c). Volume of primary tumor,
FIGS. 12a, 12b, 12c, and 12d show effect of Example 61 on the breast tumor metastasis to the lung in MMTV/c-Neu mice. Tumor-bearing MMTV/c-Neu mice were treated intraperitoneally with Example 61 (43.6 mg/kg) every other day for three weeks. (12a). Hematoxylin and eosin (H&E) staining of mammary tumor and lung tissues. (12b). Number of histologically detectable metastastic lesions in the lung. (12c). Volume of mammary tumor. (12d). β-Casein mRNA level,
FIGS. 13a and 13b show effect of Example 3 on the breast tumor metastasis to the lung in MMTV/c-Neu mice. Tumor-bearing MMTV/c-Neu mice were treated intraperitoneally with Example 3 (43.6 mg/kg) every other day for ten weeks. (13a). β-Casein mRNA level. (13b). Activity of MMP-9 and MMP-2 in the primary mammary tumor,
FIGS. 14a, 14b, 14c, and 14d show effect of Example 3 on the bile duct-ligated liver fibrosis in rats. Example 3 (21.8 or 43.6 mg/kg) dissolved in saline (vehicle) was given to rats orally three times per week for four weeks starting from BDL surgery. (14a). Activity of serum alanine aminotransferase (ALT) and aspartate aminotransferase (AST). (14b). Level of pSmad3 protein in the liver. (14c). Level of α-SMA, fibronectin, and vimentin proteins in the liver. (14d). Hematoxylin and eosin (H&E) staining of liver tissues,
FIGS. 15a, 15b, and 15c show effect of Example 2 on the bile duct-ligated liver fibrosis in rats. Example 2 (5, 10, or 20 mg/kg) dissolved in artificial gastric fluid formulation (vehicle) was given to rats orally three times per week for four weeks starting from BDL surgery. (15a). Activity of ALT and AST. (15b). Level of α-SMA and fibronectin proteins in the liver. (15c). Hematoxylin and eosin (H&E) staining of liver tissues,
FIGS. 16a and 16b show effect of Example 2 on the bleomycin-induced lung fibrosis in mice. Example 2 (5, 10, or 20 mg/kg) dissolved in artificial gastric fluid formulation (vehicle) was given to mice orally five times per week for two weeks starting from day 7. (16a). Level of α-SMA and fibronectin proteins in the lung. (16b). Hematoxylin and eosin (H&E) staining of lung tissues.

Table 1 shows structures and $^1$H NMR and MS spectral data of Examples 1-139,
Table 2 shows structures and $^1$H NMR and MS spectral data of Examples 140-153,
Table 3 shows $IC_{50}$ values of selected Examples on ALK5 kinase phosphorylation,
Table 4 shows either $IC_{50}$ values or % inhibition of Example 2 on various kinases phosphorylation,
Table 5 shows effect of Example 3 on the body and organ weight changes in BDL rats, and
Table 6 shows effect of Example 2 on the body and organ weight changes in BDL rats.

## BEST MODE

The present invention pertains to a pharmaceutical composition, comprising one or more of the compounds

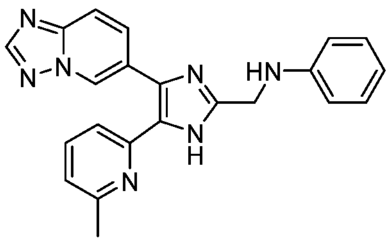
,
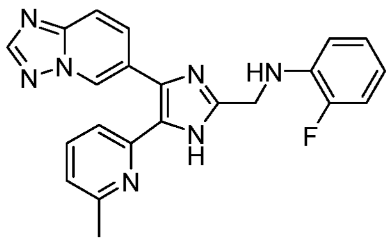
,
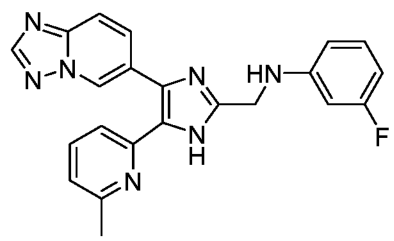
,
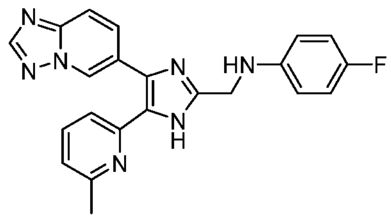
,
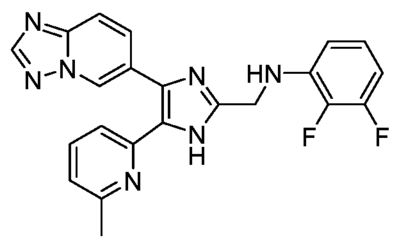
,
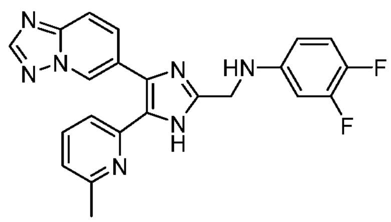
,
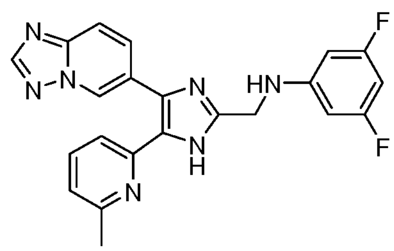
,
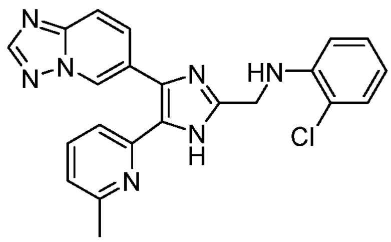
,
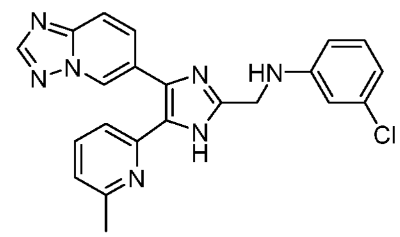
,
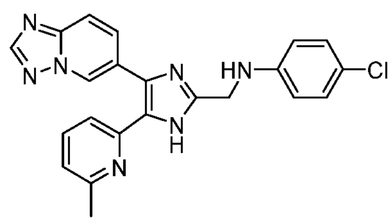

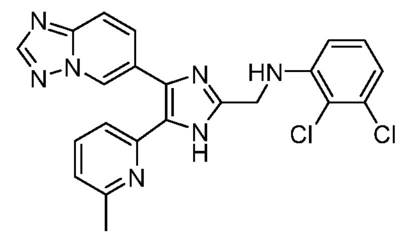
,
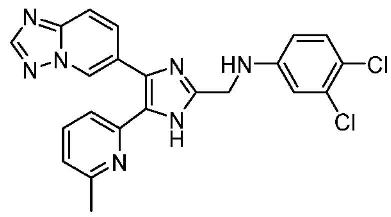

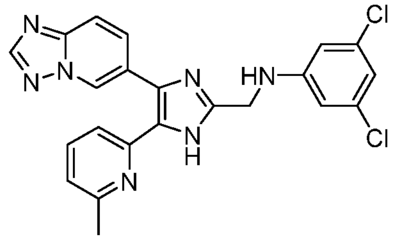

,

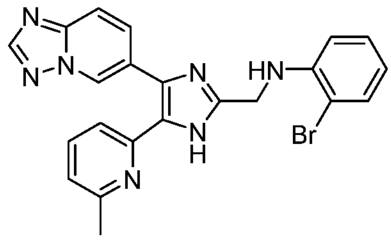

,

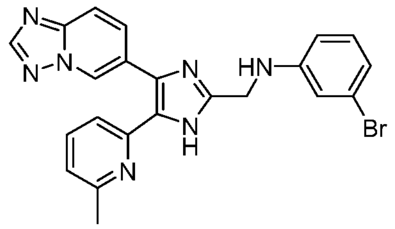

,

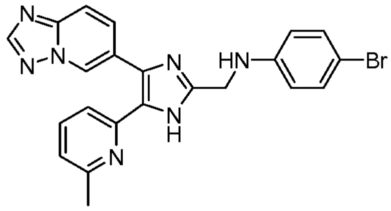

,

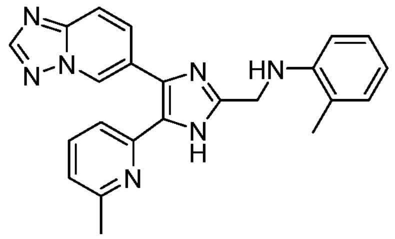

,

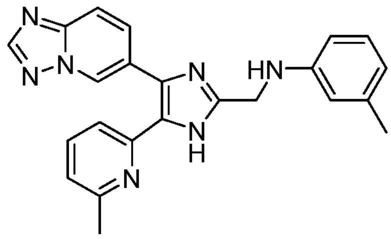

,

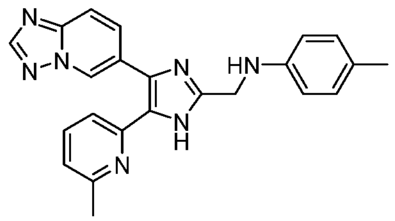

,

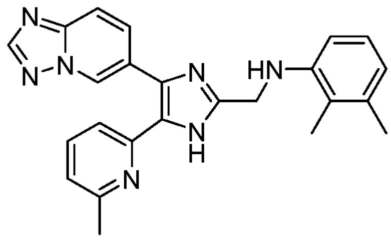

,

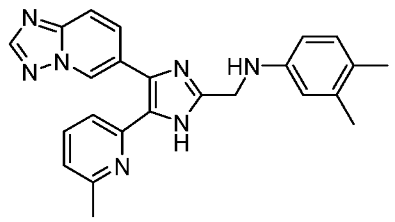

,

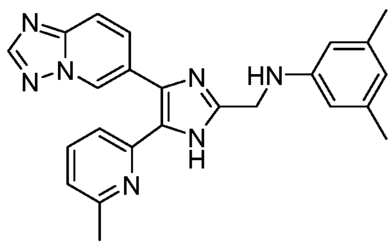

,

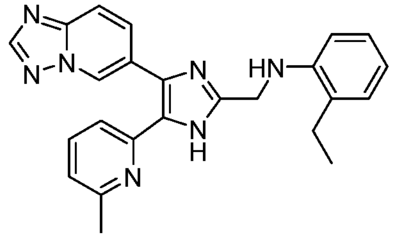

,

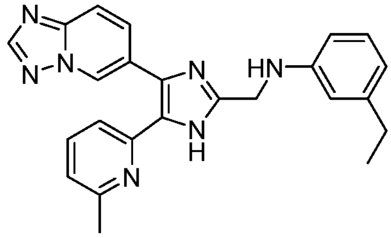

,

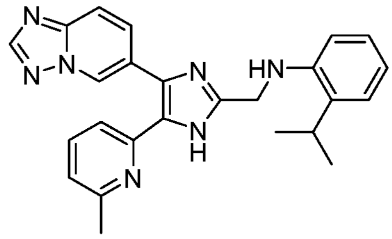
,
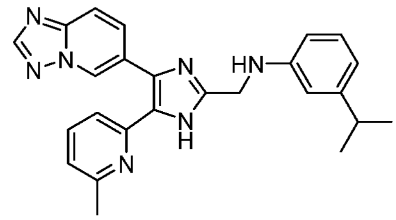
,

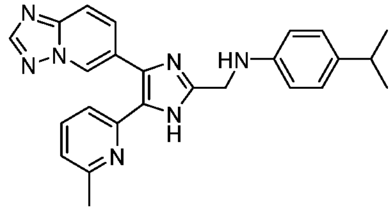
,
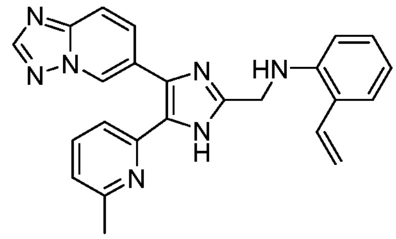
,

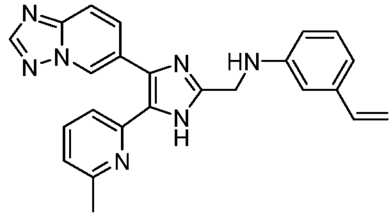
,
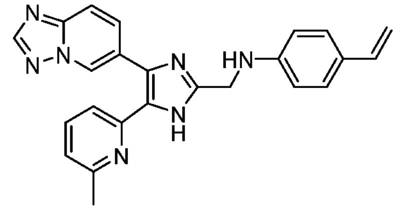
,

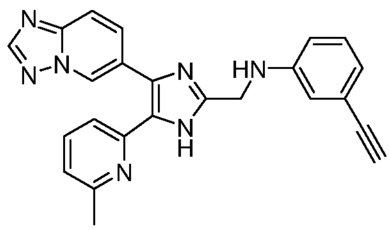
,
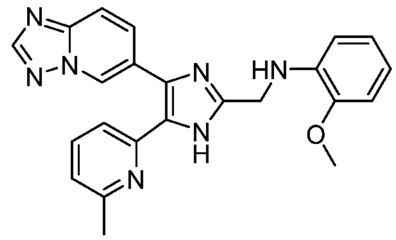
,

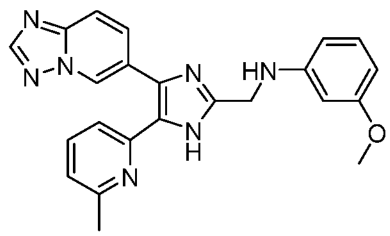
,
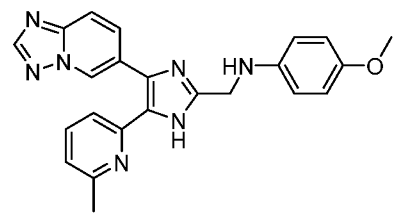
,

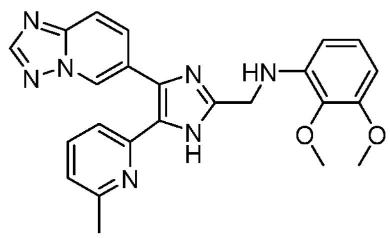
,
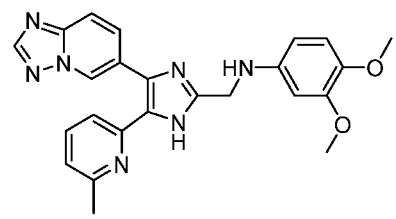
,

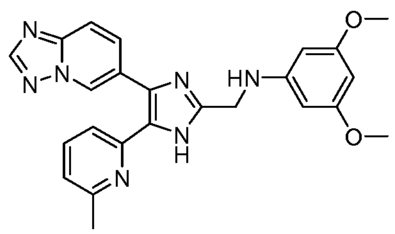 ,

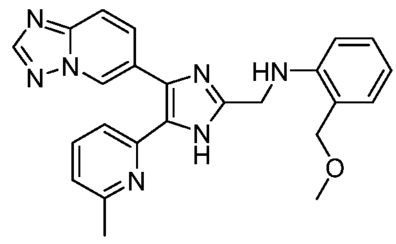 ,

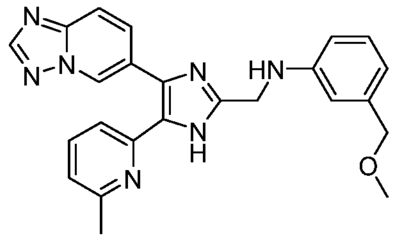 ,

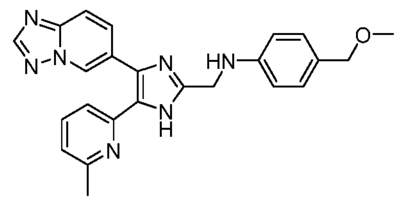 ,

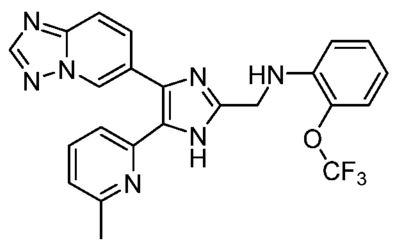 ,

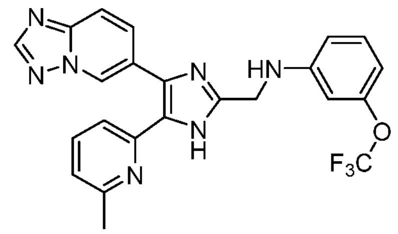 ,

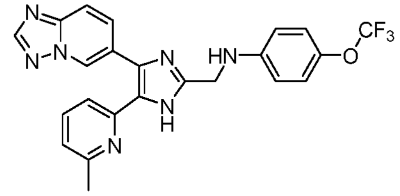 ,

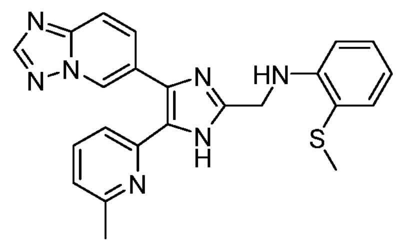 ,

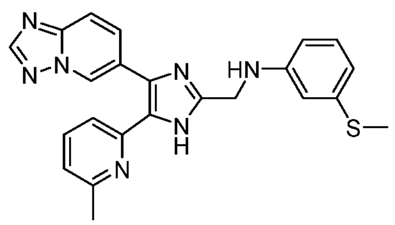 ,

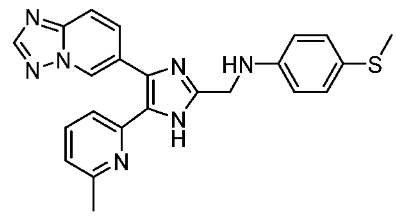 ,

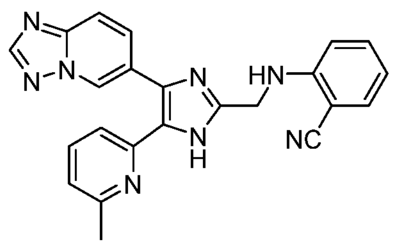 ,

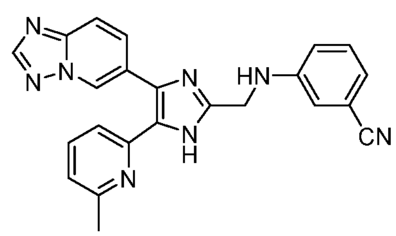 ,

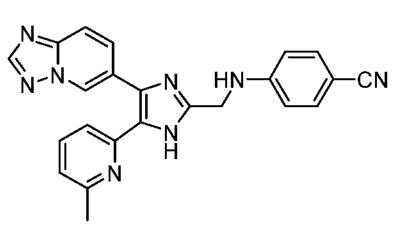
,
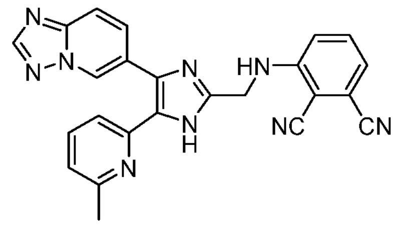
,

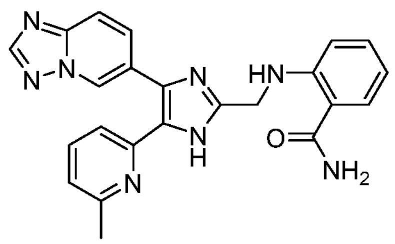
,
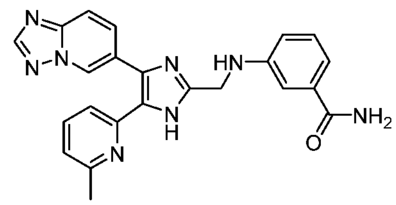
,

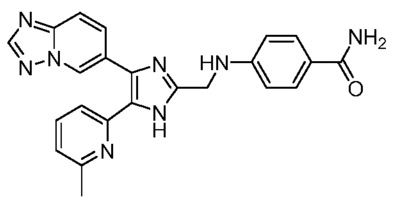
,
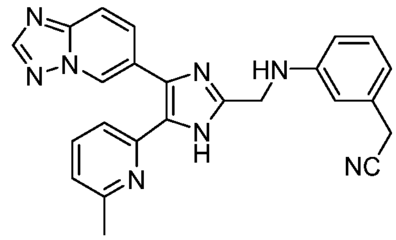
,

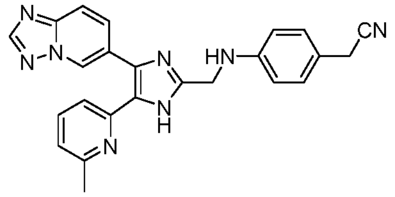
,
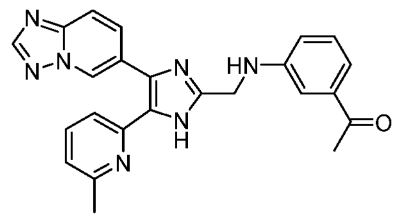
,

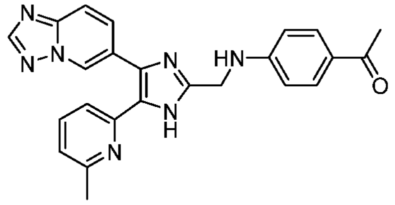
,
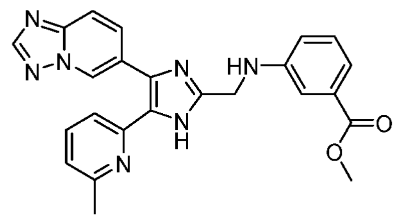
,

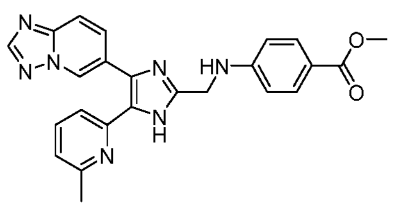
,
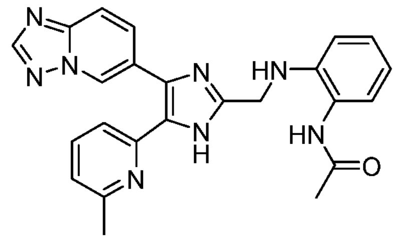
,

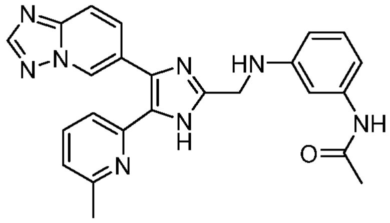

,

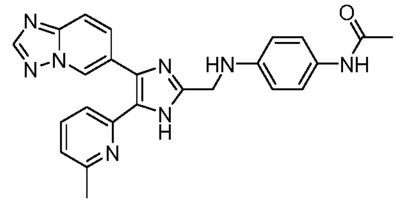

,

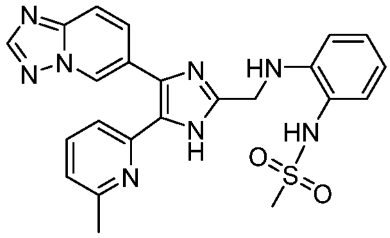

,

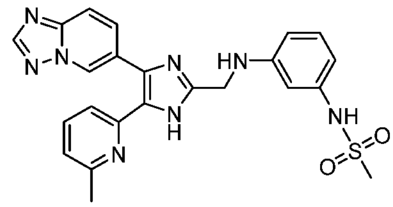

,

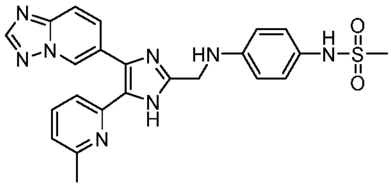

,

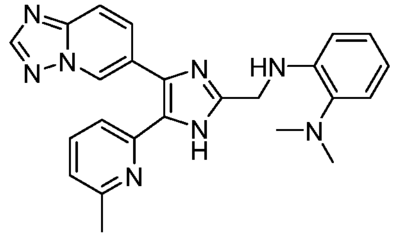

,

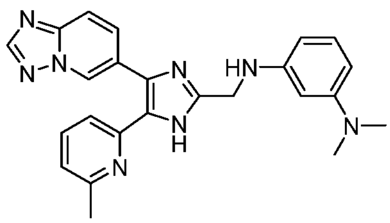

,

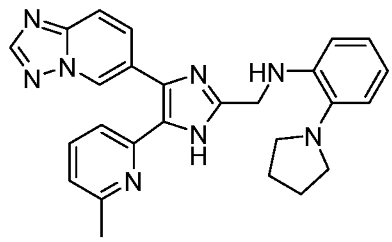

,

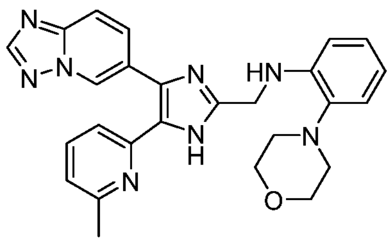

,

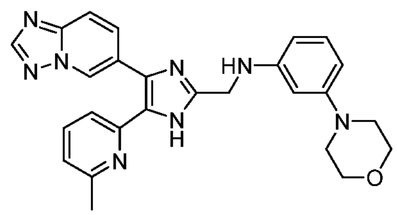

,

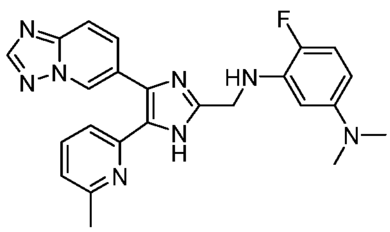

,

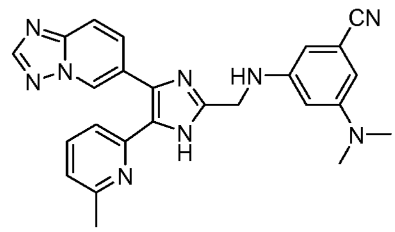

,

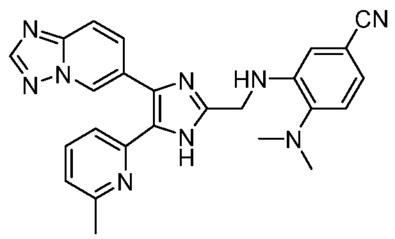

,

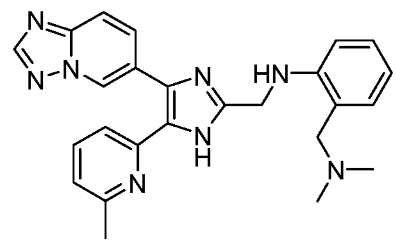

,

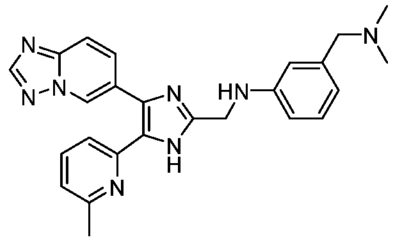

,

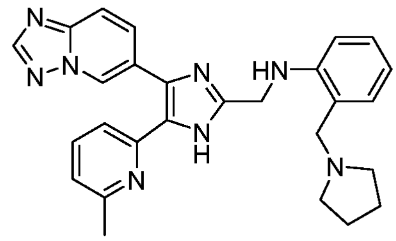

,

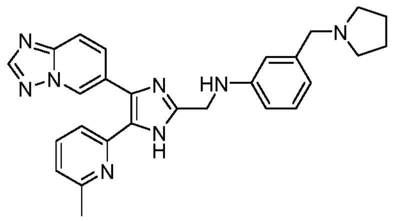

,

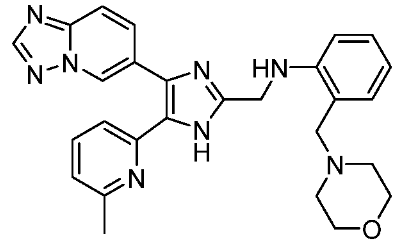

,

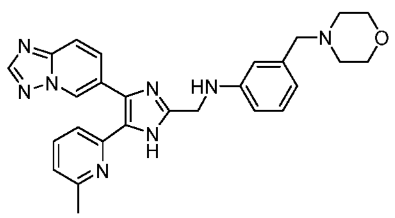

,

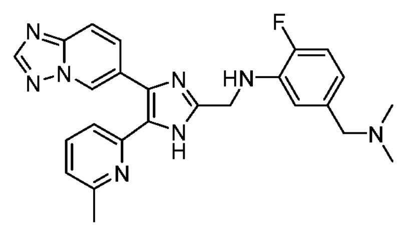

,

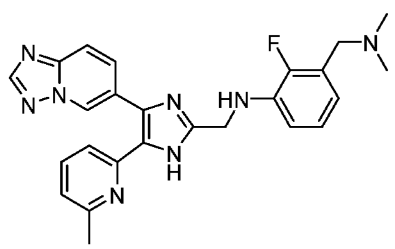

,

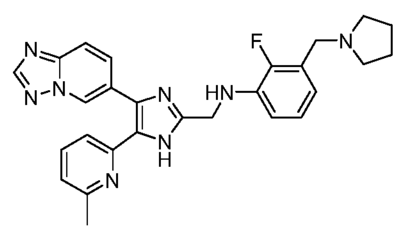

,

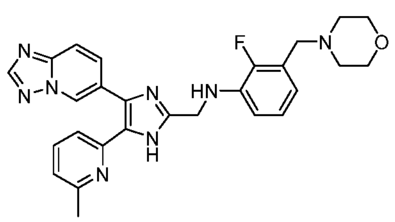

,

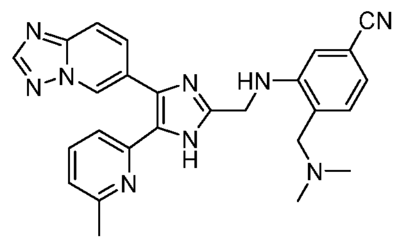

,

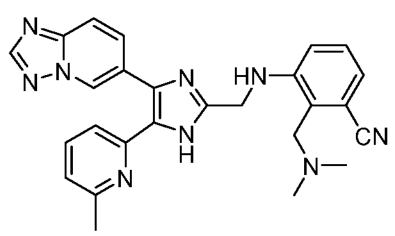

,

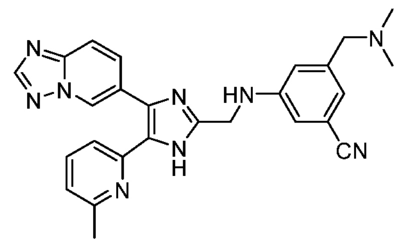

,

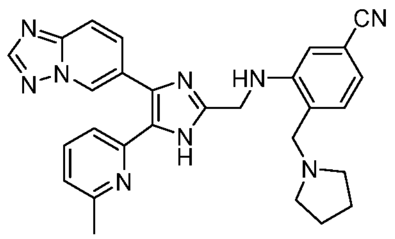

,

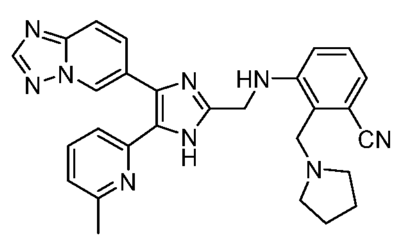

,

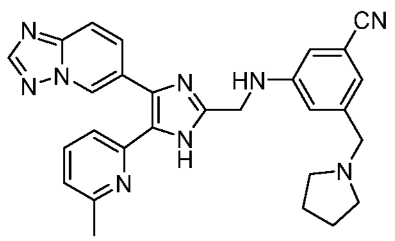

,

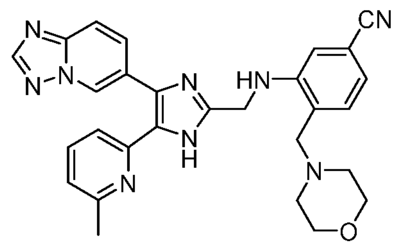

,

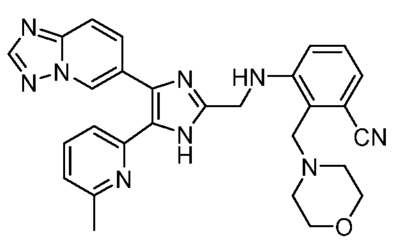

,

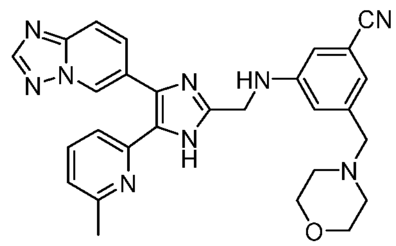

,

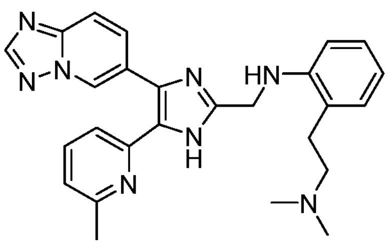

,

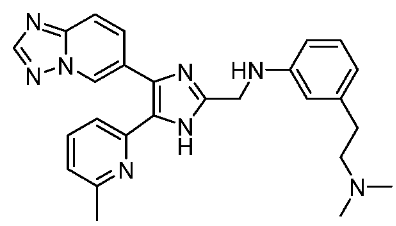

,

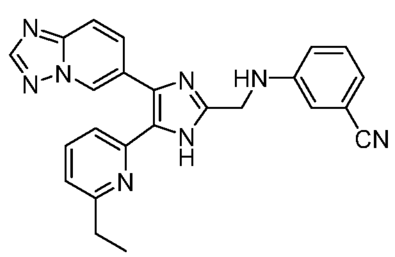

,

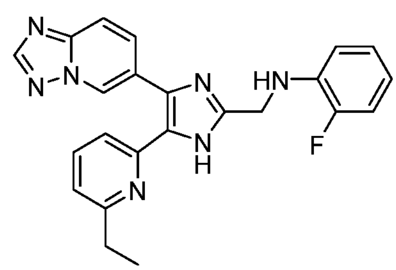

,

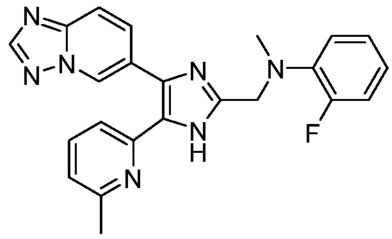,
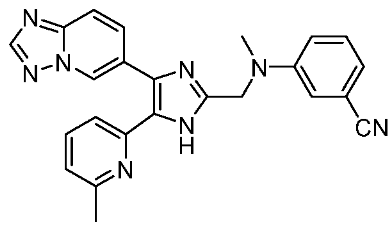,
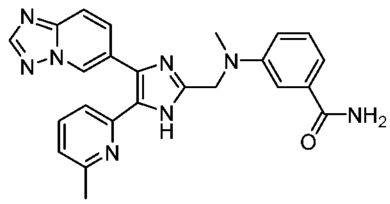,
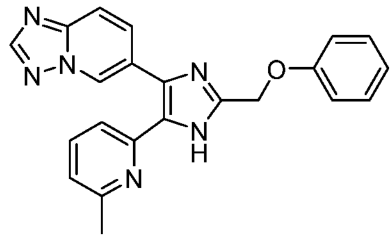,
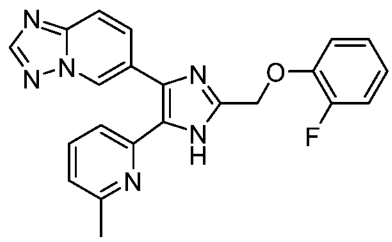,
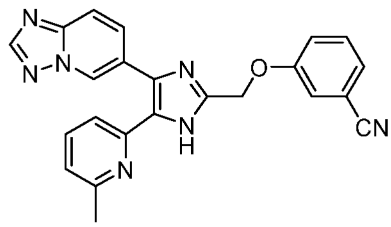,
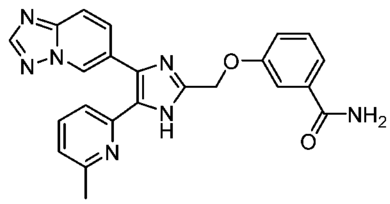,
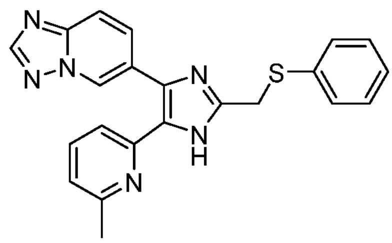,
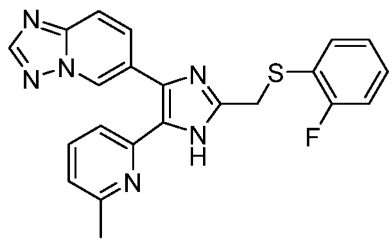,

or a pharmaceutically acceptable salt or hydrate thereof, wherein

- the pharmaceutical composition is an oral, buccal or sub-lingual dosage form delivering 50-5000 mg of the one or more compounds daily; or
- the pharmaceutical composition is a topical formulation further comprising 1 - 98 % of a carrier; or
- the pharmaceutical composition is a parenteral dosage form comprising 25-250 mg of the one or more compounds per single dose.

The one or more compounds comprised in the pharmaceutical composition of the invention can be described as compounds of formula (I) or a pharmaceutically acceptable salt thereof:

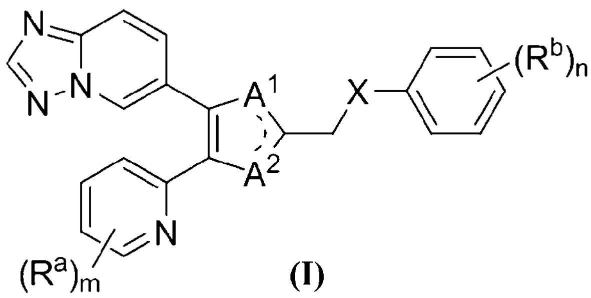

(I)

wherein each $R^a$ is independently H, halo, $C_{1-6}$alkyl, $C_{1-6}$haloalkyl, $C_{3-6}$cycloalkyl, OH, -O-$C_{1-6}$alkyl, -O-$C_{1-6}$haloalkyl, -O-$C_{3-6}$cycloalkyl, $NH_2$, -NH-$C_{1-6}$alkyl, -NH-$C_{1-6}$haloalkyl, -NH-$C_{3-6}$cycloalkyl, -S-$C_{1-6}$alkyl, -S-$C_{1-6}$haloalkyl, -S-$C_{3-6}$cycloalkyl, CN, or $NO_2$;
m is 0, 1, 2, 3, or 4;
one of $A^1$ and $A^2$ is N and the other is $NR^1$, wherein $R^1$ is H, OH, $C_{1-6}$alkyl, $C_{1-6}$ haloalkyl, or $C_{3-6}$cycloalkyl;
X is a bond, $-(CH_2)_p-$, $-NR^2-$, -O-, or -S-, wherein p is 0 or 1, and $R^2$ is H or $C_{1-3}$alkyl;
each $R^b$ is independently H, halo, $C_{1-6}$alkyl, $C_{1-6}$haloalkyl, $C_{3-6}$cycloalkyl, $C_{2-6}$alkenyl, $C_{2-6}$alkynyl, $-(CH_2)_q-OR^3$, $-(CH_2)_q-NR^3R^4$, $-(CH_2)_q-SR^3$, $-(CH_2)_q-NO_2$, $-(CH_2)_q$-CONHOH, $-(CH_2)_q$-CN, $-(CH_2)_q-COR^3$, $-(CH_2)_q-CO_2R^3$, $-(CH_2)_q-CONR^3R^4$, $-(CH_2)_q$-tetrazole, $-(CH_2)_q$-CH=CH-CN, $-(CH_2)_q-CH=CH-CO_2R^3$, $-(CH_2)_q-CH=CH-CONR^3R^4$, $-(CH_2)_q$-CH=CH-tetrazole, $-(CH_2)_q-NHCOR^3$, $-(CH_2)_q-NHCO_2R^3$, $-(CH_2)_q-CONHSO_2R^3$, $-(CH_2)_q-NHSO_2R^3$, $-(CH_2)_q$-C≡C-CN, $-(CH_2)_q-C≡C-CO_2R^3$, $-(CH_2)_q-C≡C-CONR^3R^4$, $-(CH_2)_q$-C≡C-tetrazole, $-(CH_2)_q-SOR^5$, $-(CH_2)_q-SO_2R^5$, or $-(CH_2)_r-(OR^3)_2$, wherein $R^3$ and $R^4$ are independently H, $C_{1-6}$alkyl, $C_{1-6}$haloalkyl, or $C_{3-6}$cycloalkyl; or taken together with the nitrogen atom to which they are attached form a mono-cyclic ring such as imidazole, pyrrolidine, piperidine, morpholine, piperazine and homopiperazine; $R^5$ is $C_{1-6}$alkyl, $C_{1-6}$haloalkyl, or $C_{3-6}$cycloalkyl; q is 0, 1, 2, 3, or 4; and r is 1, 2, 3, or 4; n is 0, 1, 2, 3, 4, or 5.

As used herein, the double bond indicated by the dotted lines of formula (I), represent the possible tautomeric ring forms of the compounds falling within the scope of this invention, the double bond being to the unsubstituted nitrogen. Preferably, $R^a$ is $C_{1-3}$alkyl or halo.

Preferably, m is 1 or 2.

Preferably, one of $A^1$ and $A^2$ is N and the other is $NR^1$, wherein $R^1$ is H.

Preferably, X is $-(CH_2)_p-$ or $-NR^2-$, wherein p is 0 and $R^2$ is H.

Preferably, $R^b$ is halo, $C_{1-3}$alkyl, $C_{1-3}$haloalkyl, $C_{3-4}$cycloalkyl, $C_{2-4}$alkenyl, $C_{2-4}$alkynyl, $-(CH_2)_q-OR^3$, $-(CH_2)_q-NR^3R^4$, $-(CH_2)_q-SR^3$, $-(CH_2)_q$-CN, $-(CH_2)_q-COR^3$, - $(CH_2)_q-CO_2R^3$, $-(CH_2)_q-CONR^3R^4$, $-(CH_2)_q-NHCOR^3$, $-(CH_2)_q-NHSO_2R^3$, $-(CH_2)_q-SOR^5$, or $-(CH_2)_q-SO_2R^5$, wherein $R^3$ and $R^4$ are independently H, $C_{1-3}$alkyl,

$C_{1-3}$haloalkyl, or $C_{3-4}$cycloalkyl; or taken together with the nitrogen atom to which they are attached form a mono-cyclic ring such as imidazole, pyrrolidine, piperidine, morpholine, piperazine and homopiperazine; $R^5$ is methyl; and q is 0, 1, or 2.

Preferably, n is 1, 2, or 3.

Specific compounds of formula (I) which may be mentioned include the following and pharmaceutically acceptable salts thereof:

*N*-((4-([1,2,4]triazolo[1,5-*a*]pyridin-6-yl)-5-(6-methylpyridin-2-yl)-1*H*-imidazol-2-yl)methyl)aniline;
*N*-((4-([1,2,4]triazolo[1,5-*a*]pyridin-6-yl)-5-(6-methylpyridin-2-yl)-1*H*-imidazol-2-yl)methyl)-2-fluoroaniline;
*N*-((4-([1,2,4]triazolo[1,5-*a*]pyridin-6-yl)-5-(6-methylpyridin-2-yl)-1*H*-imidazol-2-yl)methyl)-3-fluoroaniline;
*N*-((4-([1,2,4]triazolo[1,5-*a*]pyridin-6-yl)-5-(6-methylpyridin-2-yl)-1*H*-imidazol-2-yl)methyl)-4-fluoroaniline;
*N*-((4-([1,2,4]triazolo[1,5-*a*]pyridin-6-yl)-5-(6-methylpyridin-2-yl)-1*H*-imidazol-2-yl)methyl)-2,3-difluoroaniline;
*N*-((4-([1,2,4]triazolo[1,5-*a*]pyridin-6-yl)-5-(6-methylpyridin-2-yl)-1*H*-imidazol-2-yl)methyl)-3,4-difluoroaniline;
*N*-((4-([1,2,4]triazolo[1,5-*a*]pyridin-6-yl)-5-(6-methylpyridin-2-yl)-1*H*-imidazol-2-yl)methyl)-3,5-difluoroaniline;
*N*-((4-([1,2,4]triazolo[1,5-*a*]pyridin-6-yl)-5-(6-methylpyridin-2-yl)-1*H*-imidazol-2-yl)methyl)-2-chloroaniline;
*N*-((4-([1,2,4]triazolo[1,5-*a*]pyridin-6-yl)-5-(6-methylpyridin-2-yl)-1*H*-imidazol-2-yl)methyl)-3-chloroaniline;
*N*-((4-([1,2,4]triazolo[1,5-*a*]pyridin-6-yl)-5-(6-methylpyridin-2-yl)-1*H*-imidazol-2-yl)methyl)-4-chloroaniline;
*N*-((4-([1,2,4]triazolo[1,5-*a*]pyridin-6-yl)-5-(6-methylpyridin-2-yl)-1*H*-imidazol-2-yl)methyl)-2,3-dichloroaniline;
*N*-((4-([1,2,4]triazolo[1,5-*a*]pyridin-6-yl)-5-(6-methylpyridin-2-yl)-1*H*-imidazol-2-yl)methyl)-3,4-dichloroaniline;
*N*-((4-([1,2,4]triazolo[1,5-a]pyridin-6-yl)-5-(6-methylpyridin-2-yl)-1*H*-imidazol-2-yl)methyl)-3,5-dichloroaniline;
*N*-((4-([1,2,4]triazolo[1,5-*a*]pyridin-6-yl)-5-(6-methylpyridin-2-yl)-1*H*-imidazol-2-yl)methyl)-2-bromoaniline;
*N*-((4-([1,2,4]triazolo[1,5-*a*]pyridin-6-yl)-5-(6-methylpyridin-2-yl)-1*H*-imidazol-2-yl)methyl)-3-bromoaniline;

*N*-((4-([1,2,4]triazolo[1,5-*a*]pyridin-6-yl)-5-(6-methylpyridin-2-yl)-1*H*-imidazol-2-yl)methyl)-4-bromoaniline;
*N*-((4-([1,2,4]triazolo[1,5-*a*]pyridin-6-yl)-5-(6-methylpyridin-2-yl)-1*H*-imidazol-2-yl)methyl)-2-methylaniline;
*N*-((4-([1,2,4]triazolo[1,5-*a*]pyridin-6-yl)-5-(6-methylpyridin-2-yl)-1*H*-imidazol-2-yl)methyl)-3-methylaniline;
*N*-((4-([1,2,4]triazolo[1,5-*a*]pyridin-6-yl)-5-(6-methylpyridin-2-yl)-1*H*-imidazol-2-yl)methyl)-4-methylaniline;
*N*-((4-([1,2,4]triazolo[1,5-*a*]pyridin-6-yl)-5-(6-methylpyridin-2-yl)-1*H*-imidazol-2-yl)methyl)-2,3-dimethylaniline;
*N*-((4-([1,2,4]triazolo[1,5-*a*]pyridin-6-yl)-5-(6-methylpyridin-2-yl)-1*H*-imidazol-2-yl)methyl)-3,4-dimethylaniline;
*N*-((4-([1,2,4]triazolo[1,5-*a*]pyridin-6-yl)-5-(6-methylpyridin-2-yl)-1*H*-imidazol-2-yl)methyl)-3,5-dimethylaniline;
*N*-((4-([1,2,4]triazolo[1,5-*a*]pyridin-6-yl)-5-(6-methylpyridin-2-yl)-1*H*-imidazol-2-yl)methyl)-2-ethylaniline;
*N*-((4-([1,2,4]triazolo[1,5-*a*]pyridin-6-yl)-5-(6-methylpyridin-2-yl)-1*H*-imidazol-2-yl)methyl)-3-ethylaniline;
*N*-((4-([1,2,4]triazolo[1,5-*a*]pyridin-6-yl)-5-(6-methylpyridin-2-yl)-1*H*-imidazol-2-yl)methyl)-2-isopropylaniline;
*N*-((4-([1,2,4]triazolo[1,5-*a*]pyridin-6-yl)-5-(6-methylpyridin-2-yl)-1*H*-imidazol-2-yl)methyl)-3-isopropylaniline;
*N*-((4-([1,2,4]triazolo[1,5-*a*]pyridin-6-yl)-5-(6-methylpyridin-2-yl)-1*H*-imidazol-2-yl)methyl)-4-isopropylaniline;
*N*-((4-([1,2,4]triazolo[1,5-*a*]pyridin-6-yl)-5-(6-methylpyridin-2-yl)-1*H*-imidazol-2-yl)methyl)-2-vinylaniline;
*N*-((4-([1,2,4]triazolo[1,5-*a*]pyridin-6-yl)-5-(6-methylpyridin-2-yl)-1*H*-imidazol-2-yl)methyl)-3-vinylaniline;
*N*-((4-([1,2,4]triazolo[1,5-*a*]pyridin-6-yl)-5-(6-methylpyridin-2-yl)-1*H*-imidazol-2-yl)methyl)-4-vinylaniline;
*N*-((4-([1,2,4]triazolo[1,5-*a*]pyridin-6-yl)-5-(6-methylpyridin-2-yl)-1*H*-imidazol-2-yl)methyl)-3-ethynylaniline;
*N*-((4-([1,2,4]triazolo[1,5-*a*]pyridin-6-yl)-5-(6-methylpyridin-2-yl)-1*H*-imidazol-2-yl)methyl)-2-methoxyaniline;
*N*-((4-([1,2,4]triazolo[1,5-*a*]pyridin-6-yl)-5-(6-methylpyridin-2-yl)-1*H*-imidazol-2-yl)methyl)-3-methoxyaniline;
*N*-((4-([1,2,4]triazolo[1,5-*a*]pyridin-6-yl)-5-(6-methylpyridin-2-yl)-1*H*-imidazol-2-yl)methyl)-4-methoxyaniline;
*N*-((4-([1,2,4]triazolo[1,5-*a*]pyridin-6-yl)-5-(6-methylpyridin-2-yl)-1*H*-imidazol-2-yl)methyl)-2,3-dimethoxyaniline;
*N*-((4-([1,2,4]triazolo[1,5-*a*]pyridin-6-yl)-5-(6-methylpyridin-2-yl)-1*H*-imidazol-2-yl)methyl)-3,4-dimethoxyaniline;
*N*-((4-([1,2,4]triazolo[1,5-*a*]pyridin-6-yl)-5-(6-methylpyridin-2-yl)-1*H*-imidazol-2-yl)methyl)-3,5-dimethoxyaniline;
*N*-((4-([1,2,4]triazolo[1,5-*a*]pyridin-6-yl)-5-(6-methylpyridin-2-yl)-1*H*-imidazol-2-yl)methyl)-2-(methoxymethyl)aniline;
*N*-((4-([1,2,4]triazolo[1,5-*a*]pyridin-6-yl)-5-(6-methylpyridin-2-yl)-1*H*-imidazol-2-yl)methyl)-3-(methoxymethyl)aniline;
*N*-((4-([1,2,4]triazolo[1,5-*a*]pyridin-6-yl)-5-(6-methylpyridin-2-yl)-1*H*-imidazol-2-yl)methyl)-4-(methoxymethyl)aniline;
*N*-((4-([1,2,4]triazolo[1,5-*a*]pyridin-6-yl)-5-(6-methylpyridin-2-yl)-1*H*-imidazol-2-yl)methyl)-2-(trifluoromethoxy)aniline;
*N*-((4-([1,2,4]triazolo[1,5-*a*]pyridin-6-yl)-5-(6-methylpyridin-2-yl)-1*H*-imidazol-2-yl)methyl)-3-(trifluoromethoxy)aniline;
*N*-((4-([1,2,4]triazolo[1,5-*a*]pyridin-6-yl)-5-(6-methylpyridin-2-yl)-1*H*-imidazol-2-yl)methyl)-4-(trifluoromethoxy)aniline;
*N*-((4-([1,2,4]triazolo[1,5-*a*]pyridin-6-yl)-5-(6-methylpyridin-2-yl)-1*H*-imidazol-2-yl)methyl)-2-(methylthio)aniline;
*N*-((4-([1,2,4]triazolo[1,5-*a*]pyridin-6-yl)-5-(6-methylpyridin-2-yl)-1*H*-imidazol-2-yl)methyl)-3-(methylthio)aniline;
*N*-((4-([1,2,4]triazolo[1,5-*a*]pyridin-6-yl)-5-(6-methylpyridin-2-yl)-1*H*-imidazol-2-yl)methyl)-4-(methylthio)aniline;
2-((4-([1,2,4]triazolo[1,5-*a*]pyridin-6-yl)-5-(6-methylpyridin-2-yl)-1*H*-imidazol-2-yl)methylamino)benzonitrile;
3-((4-([1,2,4]triazolo[1,5-*a*]pyridin-6-yl)-5-(6-methylpyridin-2-yl)-1*H*-imidazol-2-yl)methylamino)benzonitrile;
4-((4-([1,2,4]triazolo[1,5-*a*]pyridin-6-yl)-5-(6-methylpyridin-2-yl)-1*H*-imidazol-2-yl)methylamino)benzonitrile;
3-((4-([1,2,4]triazolo[1,5-*a*]pyridin-6-yl)-5-(6-methylpyridin-2-yl)-1*H*-imidazol-2-yl)methylamino)phthalonitrile;
2-((4-([1,2,4]triazolo[1,5-*a*]pyridin-6-yl)-5-(6-methylpyridin-2-yl)-1*H*-imidazol-2-yl)methylamino)benzamide;
3-((4-([1,2,4]triazolo[1,5-*a*]pyridin-6-yl)-5-(6-methylpyridin-2-yl)-1*H*-imidazol-2-yl)methylamino)benzamide;
4-((4-([1,2,4]triazolo[1,5-*a*]pyridin-6-yl)-5-(6-methylpyridin-2-yl)-1*H*-imidazol-2-yl)methylamino)benzamide;
2-(3-((4-([1,2,4]triazolo[1,5-*a*]pyridin-6-yl)-5-(6-methylpyridin-2-yl)-1*H*-imidazol-2-yl)methylamino)phenyl)acetonitrile;
2-(4-((4-([1,2,4]triazolo[1,5-*a*]pyridin-6-yl)-5-(6-methylpyridin-2-yl)-1*H*-imidazol-2-yl)methylamino)phenyl)acetonitrile;
1-(3-((4-([1,2,4]triazolo[1,5-*a*]pyridin-6-yl)-5-(6-methylpyridin-2-yl)-1*H*-imidazol-2-yl)methylamino)phenyl)ethanone;
1-(4-((4-([1,2,4]triazolo[1,5-*a*]pyridin-6-yl)-5-(6-methylpyridin-2-yl)-1*H*-imidazol-2-yl)methylamino)phenyl)ethanone;
Methyl 3-((4-([1,2,4]triazolo[1,5-*a*]pyridin-6-yl)-5-(6-methylpyridin-2-yl)-1*H*-imidazol-2-yl)methylamino)benzoate;
Methyl 4-((4-([1,2,4]triazolo[1,5-*a*]pyridin-6-yl)-5-(6-methylpyridin-2-yl)-1*H*-imidazol-2-yl)methylamino)benzoate;
*N*-(2-((4-([1,2,4]triazolo[1,5-*a*]pyridin-6-yl)-5-(6-methylpyridin-2-yl)-1*H*-imidazol-2-yl)methylamino)phenyl)acetamide;
*N*-(3-((4-([1,2,4]triazolo[1,5-*a*]pyridin-6-yl)-5-(6-methylpyridin-2-yl)-1*H*-imidazol-2-yl)methylamino)phenyl)acetamide;

*N*-(4-((4-([1,2,4]triazolo[1,5-*a*]pyridin-6-yl)-5-(6-methylpyridin-2-yl)-1*H*-imidazol-2-yl)methylamino)phenyl)acetamide;
*N*-(2-((4-([1,2,4]triazolo[1,5-*a*]pyridin-6-yl)-5-(6-methylpyridin-2-yl)-1*H*-imidazol-2-yl)methylamino)phenyl)methanesulfonamide;
*N*-(3-((4-([1,2,4]triazolo[1,5-*a*]pyridin-6-yl)-5-(6-methylpyridin-2-yl)-1*H*-imidazol-2-yl)methylamino)phenyl)methanesulfonamide;
*N*-(4-((4-([1,2,4]triazolo[1,5-*a*]pyridin-6-yl)-5-(6-methylpyridin-2-yl)-1*H*-imidazol-2-yl)methylamino)phenyl)methanesulfonamide;
$N^1$-((4-([1,2,4]triazolo[1,5-*a*]pyridin-6-yl)-5-(6-methylpyridin-2-yl)-1*H*-imidazol-2-yl)methyl)-$N^2$,$N^2$-dimethylbenzene-1,2-diamine;
$N^1$-((4-([1,2,4]triazolo[1,5-*a*]pyridin-6-yl)-5-(6-methylpyridin-2-yl)-1*H*-imidazol-2-yl)methyl)-$N^3$,$N^3$-dimethylbenzene-1,3-diamine;
*N*-((4-([1,2,4]triazolo[1,5-*a*]pyridin-6-yl)-5-(6-methylpyridin-2-yl)-1*H*-imidazol-2-yl)methyl)-2-(pyrrolidin-1-yl)aniline;
*N*-((4-([1,2,4]triazolo[1,5-*a*]pyridin-6-yl)-5-(6-methylpyridin-2-yl)-1*H*-imidazol-2-yl)methyl)-2-morpholinoaniline;
*N*-((4-([1,2,4]triazolo[1,5-*a*]pyridin-6-yl)-5-(6-methylpyridin-2-yl)-1*H*-imidazol-2-yl)methyl)-3-morpholinoaniline;
$N^3$-((4-([1,2,4]triazolo[1,5-*a*]pyridin-6-yl)-5-(6-methylpyridin-2-yl)-1*H*-imidazol-2-yl)methyl)-4-fluoro-$N^1$,$N^1$-dimethylbenzene-1,3-diamine;
3-((4-([1,2,4]triazolo[1,5-*a*]pyridin-6-yl)-5-(6-methylpyridin-2-yl)-1*H*-imidazol-2-yl)methylamino)-5-(dimethylamino)benzonitrile;
3-((4-([1,2,4]triazolo[1,5-*a*]pyridin-6-yl)-5-(6-methylpyridin-2-yl)-1*H*-imidazol-2-yl)methylamino)-4-(dimethylamino)benzonitrile;
*N*-((4-([1,2,4]triazolo[1,5-*a*]pyridin-6-yl)-5-(6-methylpyridin-2-yl)-1*H*-imidazol-2-yl)methyl)-2-((dimethylamino)methyl)aniline;
*N*-((4-([1,2,4]triazolo[1,5-*a*]pyridin-6-yl)-5-(6-methylpyridin-2-yl)-1*H*-imidazol-2-yl)methyl)-3-((dimethylamino)methyl)aniline;
*N*-((4-([1,2,4]triazolo[1,5-*a*]pyridin-6-yl)-5-(6-methylpyridin-2-yl)-1*H*-imidazol-2-yl)methyl)-2-(pyrrolidin-1-ylmethyl)aniline;
*N*-((4-([1,2,4]triazolo[1,5-*a*]pyridin-6-yl)-5-(6-methylpyridin-2-yl)-1*H*-imidazol-2-yl)methyl)-3-(pyrrolidin-1-ylmethyl)aniline;
*N*-((4-([1,2,4]triazolo[1,5-*a*]pyridin-6-yl)-5-(6-methylpyridin-2-yl)-1*H*-imidazol-2-yl)methyl)-2-(morpholinomethyl)aniline;
*N*-((4-([1,2,4]triazolo[1,5-*a*]pyridin-6-yl)-5-(6-methylpyridin-2-yl)-1*H*-imidazol-2-yl)methyl)-3-(morpholinomethyl)aniline;
*N*-((4-([1,2,4]triazolo[1,5-*a*]pyridin-6-yl)-5-(6-methylpyridin-2-yl)-1*H*-imidazol-2-yl)methyl)-5-((dimethylamino)methyl)-2-fluoroaniline;
*N*-((4-([1,2,4]triazolo[1,5-*a*]pyridin-6-yl)-5-(6-methylpyridin-2-yl)-1*H*-imidazol-2-yl)methyl)-3-((dimethylamino)methyl)-2-fluoroaniline;
*N*-((4-([1,2,4]triazolo[1,5-*a*]pyridin-6-yl)-5-(6-methylpyridin-2-yl)-1*H*-imidazol-2-yl)methyl)-2-fluoro-3-(pyrrolidin-1-ylmethyl)aniline;
*N*-((4-([1,2,4]triazolo[1,5-*a*]pyridin-6-yl)-5-(6-methylpyridin-2-yl)-1*H*-imidazol-2-yl)methyl)-2-fluoro-3-(morpholinomethyl)aniline;
3-((4-([1,2,4]triazolo[1,5-*a*]pyridin-6-yl)-5-(6-methylpyridin-2-yl)-1*H*-imidazol-2-yl)methylamino)-4-((dimethylamino)methyl)benzonitrile;
3-((4-([1,2,4]triazolo[1,5-*a*]pyridin-6-yl)-5-(6-methylpyridin-2-yl)-1*H*-imidazol-2-yl)methylamino)-2-((dimethylamino)methyl)benzonitrile;
3-((4-([1,2,4]triazolo[1,5-*a*]pyridin-6-yl)-5-(6-methylpyridin-2-yl)-1*H*-imidazol-2-yl)methylamino)-5-((dimethylamino)methyl)benzonitrile;
3-((4-([1,2,4]triazolo[1,5-*a*]pyridin-6-yl)-5-(6-methylpyridin-2-yl)-1*H*-imidazol-2-yl)methylamino)-4-(pyrrolidin-1-ylmethyl)benzonitrile;
3-((4-([1,2,4]triazolo[1,5-*a*]pyridin-6-yl)-5-(6-methylpyridin-2-yl)-1*H*-imidazol-2-yl)methylamino)-2-(pyrrolidin-1-ylmethyl)benzonitrile;
3-((4-([1,2,4]triazolo[1,5-*a*]pyridin-6-yl)-5-(6-methylpyridin-2-yl)-1*H*-imidazol-2-yl)methylamino)-5-(pyrrolidin-1-ylmethyl)benzonitrile;
3-((4-([1,2,4]triazolo[1,5-*a*]pyridin-6-yl)-5-(6-methylpyridin-2-yl)-1*H*-imidazol-2-yl)methylamino)-4-(morpholinomethyl)benzonitrile;
3-((4-([1,2,4]triazolo[1,5-*a*]pyridin-6-yl)-5-(6-methylpyridin-2-yl)-1*H*-imidazol-2-yl)methylamino)-2-(morpholinomethyl)benzonitrile;
3-((4-([1,2,4]triazolo[1,5-*a*]pyridin-6-yl)-5-(6-methylpyridin-2-yl)-1*H*-imidazol-2-yl)methylamino)-5-(morpholinomethyl)benzonitrile;
*N*-((4-([1,2,4]triazolo[1,5-*a*]pyridin-6-yl)-5-(6-methylpyridin-2-yl)-1*H*-imidazol-2-yl)methyl)-2-(2-(dimethylamino)ethylaniline;
*N*-((4-([1,2,4]triazolo[1,5-*a*]pyridin-6-yl)-5-(6-methylpyridin-2-yl)-1*H*-imidazol-2-yl)methyl)-3-(2-(dimethylamino)ethylaniline;
3-((4-([1,2,4]triazolo[1,5-*a*]pyridin-6-yl)-5-(6-ethylpyridin-2-yl)-1*H*-imidazol-2-yl)methylamino)benzonitrile;
*N*-((4-([1,2,4]triazolo[1,5-*a*]pyridin-6-yl)-5-(6-ethylpyridin-2-yl)-1*H*-imidazol-2-yl)methyl)-2-fluoroaniline;
*N*-((4-([1,2,4]triazolo[1,5-*a*]pyridin-6-yl)-5-(6-methylpyridin-2-yl)-1*H*-imidazol-2-yl)methyl)-2-fluoro-*N*-methylaniline;
3-((4-([1,2,4]triazolo[1,5-*a*]pyridin-6-yl)-5-(6-methylpyridin-2-yl)-1*H*-imidazol-2-yl)methyl)(methyl)amino)benzonitrile;
3-((4-([1,2,4]triazolo[1,5-*a*]pyridin-6-yl)-5-(6-methylpyridin-2-yl)-1*H*-imidazol-2-yl)methyl)(methyl)amino)benzamide;
6-(2-benzyl-5-(6-methylpyridin-2-yl)-1*H*-imidazol-4-yl)-[1,2,4]triazolo[1,5-a]pyridine;
6-(2-(2-fluorobenzyl)-5-(6-methylpyridin-2-yl)-1*H*-imidazol-4-yl)-[1,2,4]triazolo[1,5-*a*]pyridine;
3-((4-([1,2,4]triazolo[1,5-*a*]pyridin-6-yl)-5-(6-methylpyridin-2-yl)-1*H*-imidazol-2-yl)methyl)benzonitrile;
3-((4-([1,2,4]triazolo[1,5-*a*]pyridin-6-yl)-5-(6-methylpyridin-2-yl)-1*H*-imidazol-2-yl)methyl)benzamide;
6-(5-(6-methylpyridin-2-yl)-2-(phenoxymethyl)-1*H*-imidazol-4-yl)-[1,2,4]triazolo[1,5-*a*]pyridine;
6-(2-((2-fluorophenoxy)methyl)-5-(6-methylpyridin-2-yl)-1*H*-imidazol-4-yl)-[1,2,4]triazolo[1,5-*a*]pyridine;
3-((4-([1,2,4]triazolo[1,5-*a*]pyridin-6-yl)-5-(6-methylpyridin-2-yl)-1*H*-imidazol-2-yl)methoxy)benzonitrile;
3-((4-([1,2,4]triazolo[1,5-*a*]pyridin-6-yl)-5-(6-methylpyridin-2-yl)-1*H*-imidazol-2-yl)methoxy)benzamide;
6-(5-(6-methylpyridin-2-yl)-2-(phenylthiomethyl)-1*H*-imidazol-4-yl)-[1,2,4]triazolo[1,5-*a*]pyridine;
6-(2-((2-fluorophenylthio)methyl)-5-(6-methylpyridin-2-yl)-1*H*-imidazol-4-yl)-[1,2,4]triazolo[1,5-*a*]pyridine.

The compounds of the present composition typically are small organic molecules (non-peptide small molecules), generally less than about 1,000 daltons in size. Preferred non-peptide small molecules have molecular weights of less than about 750 daltons, more preferably less than about 500 daltons.

The compounds comprised in the composition may also be supplied in the form of a "prodrug" which is designed to release the compound when administered to a subject. Prodrug formed designs are well known in the art, and depend on the substituents contained in the compound. For example, a substituent containing hydroxyl could be coupled to a carrier which renders the compound biologically inactive until it is removed by endogenous enzymes or, for example, by enzymes targeted to a particular receptor or location in the subject.

A compound comprised in the composition that is acidic in nature (e.g., having a carboxyl or phenolic hydroxyl group) can form a pharmaceutically acceptable salt such as a sodium, potassium, calcium, or gold salt. Also within the scope of the invention are salts formed with pharmaceutically acceptable amines such as ammonia, alkyl amines, hydroxyalkylamines, and *N*-methylglycamine. A compound comprised in the composition can be treated with an acid to form acid addition salts. Examples of such acids include hydrochloric acid, hydrobromic acid, hydroiodic acid, sulfuric acid, methanesulfonic acid, phosphoric acid, *p*-bromophenylsulfonic acid, carbonic acid, succinic acid, citric acid, benzoic acid, oxalic acid, malonic acid, salicyclic acid, malic acid, fumaric acid, ascorbic acid, maleic acid, acetic acid, and other mineral and organic acids well known to those skilled in the art. The acid addition salts can be prepared by treating a compound comprised in the composition in its free base form with a sufficient amount of an acid (e.g., hydrochloric acid) to produce an acid addition salt (e.g., a hydrochloride salt). The acid addition salt can be converted back to its free base form by treating the salt with a suitable dilute aqueous basic solution (e.g., sodium hydroxide, sodium bicarbonate, potassium carbonate, or ammonia).

Some of the compounds comprised in this composition may be crystallized or recrystallized from solvents such as aqueous and organic solvents. In such cases solvates may be formed. This invention includes within its scope stoichiometric solvates including hydrates as well as compounds containing variable amounts of water that may be produced by processes such as lyophilization.

Compounds comprised in the composition may contain one or more asymmetric centers and thus can exist as enantiomers or diastereomers. It is to be understood that the invention includes both mixtures and separate individual isomers of compounds. Furthermore, certain compounds comprised in the composition which contain alkenyl groups may exist as cis- or trans-isomers. In each instance, the invention includes both mixtures and separate individual isomers.

Compounds comprised in the composition may also exist in tautomeric forms and the invention includes both mixtures and separate individual tautomers thereof.

Also included in the invention are radiolabelled derivatives of compounds comprised in the composition which are suitable for biological studies.

As used herein, the term "alkyl" group refers to a saturated aliphatic hydrocarbon group containing 1-6 carbon atoms. An alkyl group can be straight or branched. Examples of an alkyl group include, but are not limited to, methyl, ethyl, propyl, isopropyl, butyl, isobutyl, *sec*-butyl, *tert*-butyl, *n*-pentyl, and *n*-hexyl. An alkyl group can be optionally

substituted with one or more substituents such as alkoxy, cycloalkoxy, amino, nitro, carboxy, cyano, halo, hydroxyl, sulfo, or mercapto.

As used herein, the term "cycloalkyl" group refers to an aliphatic carbocyclic ring of 3-6 carbon atoms. Examples of cycloalkyl groups include cyclopropyl, cyclobutyl, cyclopentyl, and cyclohexyl.

As used herein, the term "haloalkyl" group refers to an alkyl group containing one or more halogen atoms. Examples of haloalkyl groups include fluoromethyl, chloromethyl, bromomethyl, and trifluoromethyl.

As used herein, the term "halo" group refers to fluorine, chlorine, bromine, or iodine.

As used herein, the term "alkenyl" group refers to an aliphatic carbon group that contains 2-6 carbon atoms and at least one double bond. Like an alkyl group, an alkenyl group can be straight or branched. Examples of an alkenyl group include, but are not limited to, vinyl, allyl, isoprenyl, 2-butenyl, and 2-hexenyl. An alkenyl group can be optionally substituted with one or more substituents such as alkoxy, cycloalkoxy, amino, nitro, carboxy, cyano, halo, hydroxyl, sulfo, or mercapto.

As used herein, the term "alkynyl" group refers to an aliphatic carbon group that contains 2-6 carbon atoms and has at least one triple bond. An alkynyl group can be straight or branched. Examples of an alkynyl group include, but are not limited to, ethynyl, propargyl, and butynyl. An alkynyl group can be optionally substituted with one or more substituents such as alkoxy, cycloalkoxy, amino, nitro, carboxy, cyano, halo, hydroxyl, sulfo, or mercapto.

As used herein, the term "ALK5 and/or ALK4 inhibitor" refers to a compound, other than inhibitory Smads, e.g. Smad6 and Smad7, which selectively inhibits the ALK5 and/or ALK4 receptors preferentially over p38 or type II receptors.

As used herein, the term "ALK5 and/or ALK4-mediated diease state" refers to any disease state which is mediated (or modulated) by ALK5 and/or ALK4, for example, a disease which is modulated by the inhibition of the phosphorylation of Smad2 and Smad3 in the TGT-β and/or activin signaling pathways.

As used herein, the term "ulcers" is used to include, but not to be limited to, diabetic ulcers, chronic ulcers, gastric ulcers, and duodenal ulcers.

Compounds comprised in the composition may be prepared by a number of known methods from commercially available or known starting materials. If the starting materials are unavailable from a commercial source, they can be prepared by procedures known in the art.

Scheme 1

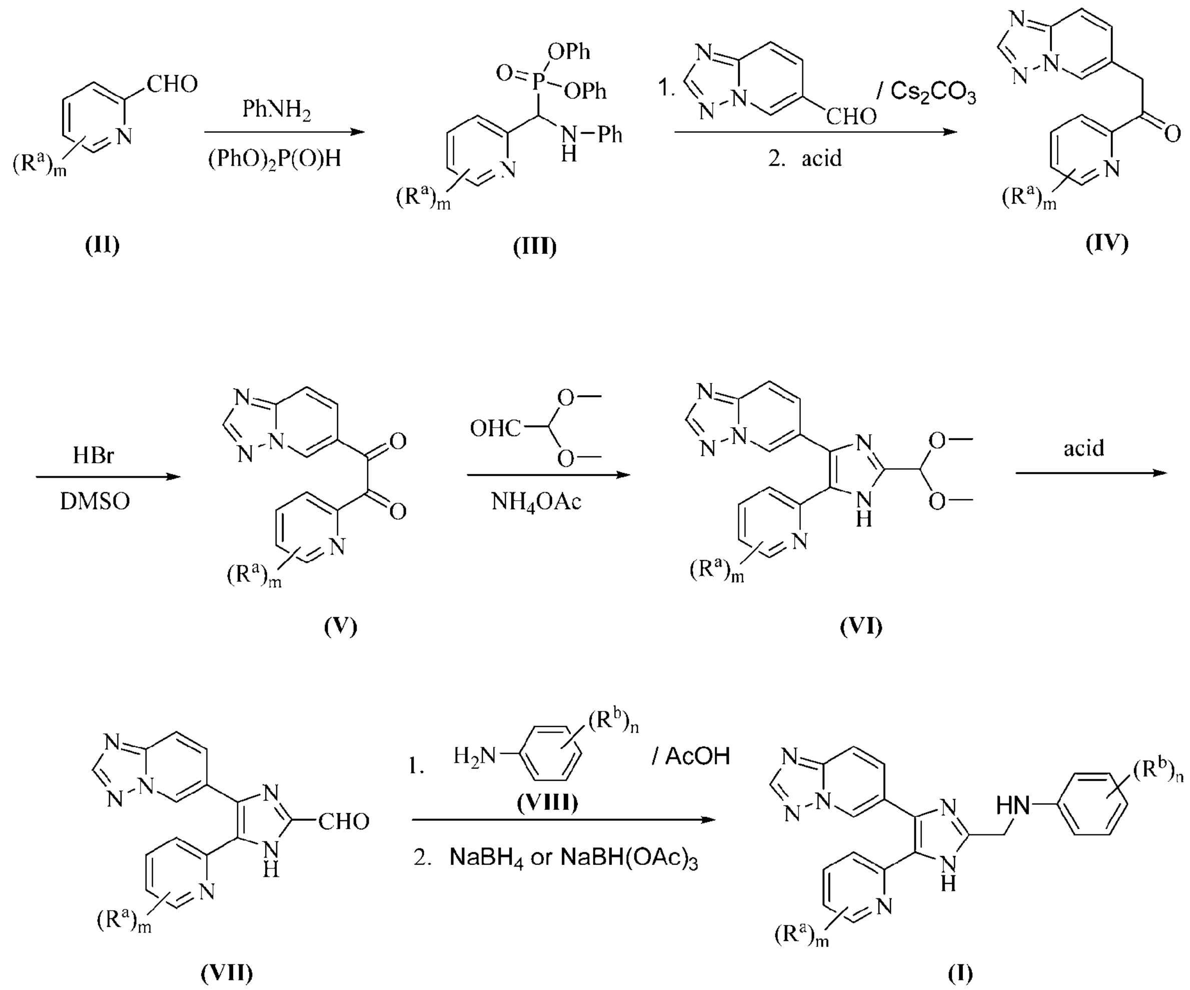


(II) (III) (IV) (V) (VI) (VII) (VIII) (I)

In one method, compounds of formula (I) wherein $A^1$ is N and $A^2$ is NH, or $A^1$ is NH and $A^2$ is N, and X is -NH- are prepared according to Scheme 1. Specifically, $R^a$-substituted pyridine-2-carbaldehyde (II) is reacted with aniline and diphenyl phosphite to give *N*,*P*-acetal (III), which can be further coupled with [1,2,4]triazolo[1,5-a]pyridine-6-carbaldehyde followed by hydrolysis in acidic condition to produce a monoketone (IV). The monoketone (IV) may be oxidized to a diketone (V) with HBr in DMSO. This diketone (V) can then be condensed with 2,2-dimethoxyacetaldehyde in the presence of ammonium acetate to yield an acetal-protected imidazole (VI), which can be hydrolyzed in acidic condition to produce an imidazole-2-carbaldehyde (VII). The imidazole-2-carbaldehyde (VII) can be coupled with $R^b$-substituted aniline (VIII) in the presence of an acid such as acetic acid to generate an immine, which can be further reduced with a reducing agent such as sodium borohydride or sodium triacetoxyborohydride to yield a compound of formula (I). $R^a$, $R^b$, m, and n have been defined as above.

Scheme 2

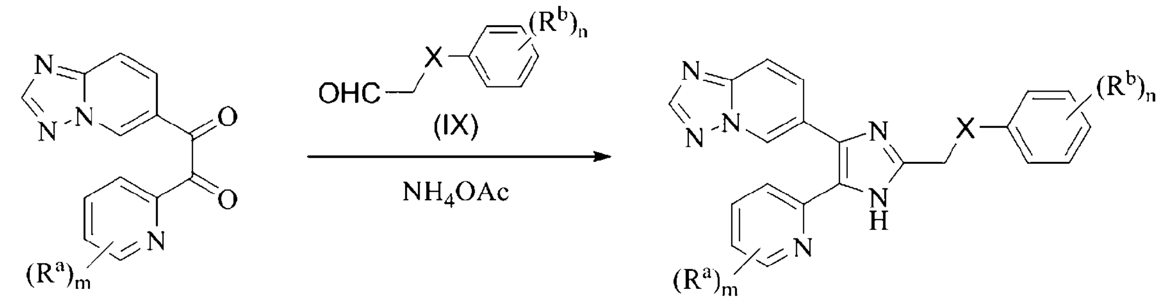


(V) (I)

In another method, compounds of formula (I) wherein $A^1$ is N and $A^2$ is NH, or $A^1$ is NH and $A^2$ is N, and X is $-(CH_2)_p-$, $-NR^2-$, -O-, or -S-, wherein p is 0 or 1, and $R^2$ is

$C_{1-3}$alkyl, are prepared according to Scheme 2. The diketone (V) can be condensed with an appropriate $R^b$-substituted aldehyde (IX) in the presence of ammonium acetate to yield a compound of formula (I). $R^a$, $R^b$, m, and n have been defined as above.

Scheme 3

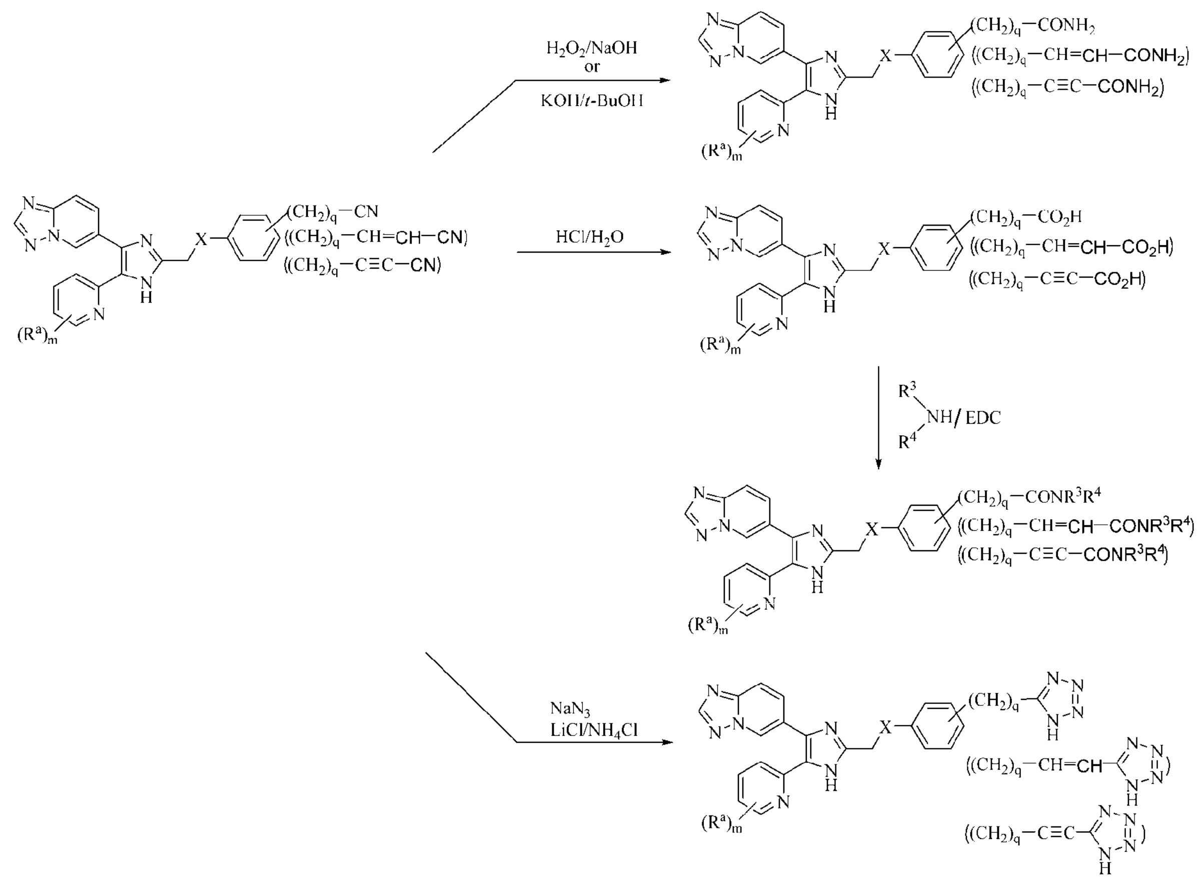


Alternatively, when $R^b$ compounds of formula (I) is $-(CH_2)_q$-CN, $-(CH_2)_q$-CH=CH-CN, or $-(CH_2)_q$-C≡C-CN, it can be further functionalized to form a compound of formula (I) as depicted in Scheme 3. $R^a$, $R^3$, $R^4$, X, m, and q have been defined as above.

The resulting compounds of this invention represented by the formula (I)-(IX) can be separated and purified by appropriate conventional methods such as column chromatography and recrystallization.

Compounds of the invention are administered in the form of

- an oral, buccal or sub-lingual dosage form delivering 50-5000 mg of the one or more compounds daily; or
- the pharmaceutical composition is a topical formulation further comprising 1 - 98 % of a carrier; or
- the pharmaceutical composition is a parenteral dosage form comprising 25-250 mg of the one or more compounds per single dose.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers are present as from about 1% up to about 98% of the formulation. More usually, they will form up to about 80% of the formulation.

For administration to man in the curative or prophylactic treatment of the disorders identified above, oral, buccal or sub-lingual dosages of a compound of formula (I) will be in the range of from 50-5000 mg daily for an average adult patient (70 kg). Thus for a typical adult patient, individual tablets or capsules contain from 25-500 mg of active compound, in a suitable pharmaceutically acceptable vehicle or carrier, for administration in single or multiple doses, once or several times per day. Dosages for parenteral administration are within the range of from 25-250 mg per single dose as required. In practice the physician will determine the actual dosing regimen which will be most suitable for an individual patient and it will vary with the age, weight and response of the particular patient.

For human use, a compound comprised in the composition can be administered alone, but will generally be administered in admixture with a pharmaceutical carrier selected with regard to the intended route of administration and standard pharmaceutical practice. For example, the compound may be administered orally, buccally or sublingually, in the form of tablets containing excipients such as starch or lactose, or in capsules or ovules either alone or in admixture with excipients, or in the form of elixirs or suspensions containing flavoring or coloring agents. Such liquid preparations may be prepared with pharmaceutically acceptable additives such as suspending agent (e.g. methylcellulose, a semi-synthetic glyceride such as witepsol or mixtures of glycerides such as a mixture of apricot kernel oil and PEG-6 esters or mixtures of PEG-8 and caprylic/capric glycerides). A compound may also be injected parenterally, for example intravenously, intramuscularly, subcutaneously or intracoronarily. For parenteral administration, the compound is best used in the form of a sterile aqueous solution which may contain other substances, for example, salts, or monosaccharides such as mannitol or glucose, to make the solution isotonic with blood.

Thus, the invention provides in a further aspect a pharmaceutical composition comprising a compound as recited in claim 1, or a pharmaceutically acceptable salt or solvate thereof, together with a pharmaceutically acceptable diluent or carrier therefor.

The invention also provides a pharmaceutical composition comprising a compound as recited in claim 1, or a pharmaceutically acceptable salt or solvate thereof, for use in therapy.

The invention further provides a pharmaceutical composition comprising a compound as recited in claim 1, or a pharmaceutically acceptable salt or solvate thereof, for use in the treatment of a disease, mediated by the ALK5 and/or ALK4 receptors in mammals.

ALK5- and/or ALK4-mediated disease states include, but are not limited to glomerulonephritis, diabetic nephropathy, lupus nephritis, hypertension-induced nephropathy, renal interstitial fibrosis, renal fibrosis resulting from complications of drug exposure, HIV-associated nephropathy, transplant nephropathy, liver fibrosis due to all etiologies, hepatic dysfunction attributable to infections, alcohol-induced hepatitis, disorders of the biliary tree, cystic fibrosis, pulmonary fibrosis, interstitial lung disease, acute lung injury, adult respiratory distress syndrome, idiopathic pulmonary fibrosis, chronic obstructive pulmonary disease, pulmonary disease due to infectious or toxic agents, post-infarction cardiac fibrosis, congestive heart failure, dilated cardiomyopathy, myocarditis, intimal thickening, vascular stenosis, hypertension-induced vascular remodeling, pulmonary arterial hypertension, coronary restenosis, peripheral restenosis, carotid restenosis, stent-induced restenosis, atherosclerosis, ocular scarring, corneal scarring, proliferative vitreoretinopathy, glaucoma, intraocular pressure, excessive or hypertrophic scar or keloid formation in the dermis occurring during wound healing resulting from trauma or surgical wounds, peritoneal and sub-dermal adhesion, scleroderma, fibrosclerosis, progressive systemic sclerosis, dermatomyositis, polymyositis, arthritis, osteoporosis, ulcers, impaired neurological function, male erectile dysfunction, Peyronie's disease, Dupuytren's contracture, Alzheimer's disease, Raynaud's syndrome, radiation-induced fibrosis, thrombosis, tumor metastasis growth, multiple myeloma, melanoma, glioma, glioblastomas, leukemia, sarcomas, leiomyomas, mesothelioma, and carcinomas of lung, breast, colon, kidney, ovary, cervix, liver, biliary tract, gastrointestinal tract, pancreas, prostate, head, and neck.

The invention further provides a composition for use in inhibiting the TGF-β and/or activin signaling pathways

in human, for example, inhibiting the phosphorylation of Smad2 or Smad3 by ALK5 and/or ALK4.

The invention further provides a composition for use in reducing the accumulation of excess extracellular matrix in human by inhibiting the TGF-β and/or activin signaling pathways, for example, inhibiting the phosphorylation of Smad2 or Smad3 by ALK5 and/or ALK4.

The invention further provides a composition for use in treating, preventing, or reducing metastasis of tumor cells in human by inhibiting the TGT-β signaling pathway.

The invention further provides a composition for use in treating, preventing, or reducing carcinomas mediated by an overexpression of TGT-β in human by inhibiting the TGT-β signaling pathway.

The invention further provides a composition for use in treating, preventing, or reducing vascular injuries in human by inhibiting the TGT-β signaling pathway.

The present invention is further illustrated in the following Examples, which should not be taken to limit the scope of the invention described in the claims. In the Examples, electrospray ionization mass spectra (ESI-MS) were obtained on a Q-Tof2 mass spectrometer (Micromass, Manchester, UK).

## EXAMPLES

### Preparative Example 1

Preparation of diphenyl (6-methylpyridin-2-yl)(phenylamino)methylphosphonate (a compound of the formula (**III**) wherein $R^a = CH_3$)

A mixture of 6-methylpyridine-2-carboxaldehyde (2.12 g, 17.50 mmol), aniline (1.63 g, 17.50 mmol), diphenyl phosphite (4.92 g, 21.00 mmol), and zirconyl chloride octahydrate (0.56 g, 1.75 mmol) was stirred at room temperature 1 h. The reaction mixture was extracted with $CH_2Cl_2$ (3 × 50 mL), and the $CH_2Cl_2$ solution was washed with water (2 × 20 mL), dried over anhydrous $Na_2SO_4$, filtered, and evaporated to dryness under reduced pressure. The residue was purified by MPLC on silica gel using a mixture of EtOAc and hexane as eluent to give the titled compound (6.96 g, 92%) as a white solid. $^1H$ NMR (400 MHz, $CDCl_3$): $\delta$ 7.51 (t, 1 H, *J* = 7.8 Hz), 7.38 (dd, 1 H, *J* = 7.6, 2.0 Hz), 7.27-7.22 (m, 4 H), 7.19-7.15 (m, 2 H), 7.14-7.07 (m, 4 H), 7.05-7.02 (m, 3 H), 6.80-6.74 (m, 3 H), 5.53 (pseudo t, 1 H, *J* = 7.4 Hz), 5.36 (dd, 1 H, *J* = 21.0, 8.2 Hz), 2.54 (s, 3 H).

### Preparative Example 2

Preparation of diphenyl (6-ethylpyridin-2-yl)(phenylamino)methylphosphonate (a compound of the formula (**III**) wherein $R^a = CH_2CH_3$)

The titled compound was prepared as described in Preparative Example 1 by using 6-ethylpyridine-2- carboxaldehyde in place of 6-methylpyridine-2-carboxaldehyde. Yield: 81%; $^1H$ NMR (400 MHz, $CDCl_3$): $\delta$ 7.55 (t, 1 H, *J* = 7.6 Hz), 7.38 (dd, 1 H, *J* = 7.6, 2.0 Hz), 7.26-7.09 (m, 8 H), 7.07-7.00 (m, 5 H), 5.59 (pseudo t, 1 H, *J* = 7.0 Hz), 5.34 (dd, 1 H, *J* = 20.8, 8.0 Hz), 2.82 (q, 2 H, *J* = 7.6 Hz), 1.28 (t, 3 H, *J* = 7.6 Hz).

### Preparative Example 3

Preparation of 2-([1,2,4]triazolo[1,5-*a*]pyridin-6-yl)-1-(6-methylpyridin-2-yl)ethanone (a compound of the formula (**IV**) wherein $R^a = CH_3$)

To a stirred solution of [1,2,4]triazolo[1,5-a]pyridine-6-carbaldehyde (2.50 g, 17.01 mmol) (prepared according to the method described in WO 03/087304 A2) and diphenyl (6-methylpyridin-2-yl)(phenylamino)methylphosphonate (7.32 g, 17.01 mmol) in a mixture of THF (40 mL) and *i*-PrOH (10 mL) was added $Cs_2CO_3$ (7.20 g, 22.11 mmol), and the mixture was stirred at room temperature overnight. A solution of 3 N HCl (25 mL) was added dropwise to the reaction mixture, and the mixture was stirred for 1 h. It was then diluted with *tert*-butyl methyl ether (40 mL) and extracted with 1 N HCl (2 × 35 mL). The aqueous extracts were neutralized with 50% KOH until pH 7-8 was reached. The precipitates were collected by filtration, washed with water, and dried over $P_2O_5$ *in vacuo* to give the titled compound (3.41 g, 80%) as an off-white solid. $^1H$ NMR (400 MHz, $CDCl_3$): $\delta$ 8.61 (d, 1 H, *J* = 0.8 Hz), 8.31 (s, 1 H), 7.88 (dd, 1 H, *J* = 7.6, 1.6 Hz), 7.73 (t, 1 H, overlapped, *J* = 7.6 Hz), 7.71 (dd, 1 H, overlapped, *J* = 9.2, 0.8 Hz), 7.54 (dd, 1 H, *J* = 9.2, 1.6 Hz), 7.37 (dd, 1 H, *J* = 7.6, 1.6 Hz), 4.62 (s, 2 H), 2.67 (s, 3 H).

**Preparative Example 4**

Preparation of 2-([1,2,4]triazolo[1,5-*a*]pyridin-6-yl)-1-(6-ethylpyridin-2-yl)ethanone (a compound of the formula (**IV**) wherein $R^a$ = $CH_2CH_3$)

The titled compound was prepared as described in Preparative Example 3 by using diphenyl (6-ethylpyridin-2-yl)(phenylamino)methylphosphonate in place of diphenyl (6-methylpyridin-2-yl)(phenylamino)methylphosphonate. Yield: 78%; $^1$H NMR (400 MHz, $CDCl_3$): $\delta$ 8.61 (dd, 1 H, *J* = 1.6, 0.8 Hz), 8.29 (s, 1 H), 7.88 (br d, 1 H, *J* = 7.6 Hz), 7.74 (t, 1 H, *J* = 7.6 Hz), 7.70 (dd, 1 H, *J* = 9.2, 0.8 Hz), 7.54 (dd, 1 H, *J* = 9.2, 1.6 Hz), 7.37 (dd, 1 H, *J* = 7.6, 0.8 Hz), 4.62 (s, 2 H), 2.93 (q, 2 H, *J* = 7.6 Hz), 1.39 (t, 3 H, *J* = 7.6 Hz).

**Preparative Example 5**

Preparation of 1-([1,2,4]triazolo[1,5-*a*]pyridin-6-yl)-2-(6-methylpyridin-2-yl)ethane-1,2-dione (a compound of the formula (**V**) wherein $R^a$ = $CH_3$)

To a stirred suspension of 2-([1,2,4]triazolo[1,5-*a*]pyridin-6-yl)-1-(6-methylpyridin-2-yl)ethanone (6.20 g, 24.57 mmol) in DMSO (48 mL) was added dropwise HBr (48 wt. % in water, 5.96 g, 12.4 mL) at 0°C, and the mixture was heated at 60-70°C. After 2 h, the reaction mixture was cooled to 0°C, poured onto ice water (20 mL), and basified to pH 10 with solid $K_2CO_3$. The mixture was extracted with $CHCl_3$ (2 × 250 mL), and the organic phase was washed with water (2 × 100 mL), dried over anhydrous $Na_2SO_4$, filtered, and evaporated to dryness under reduced pressure. The residue was purified by MPLC on silica gel using a mixture of MeOH and $CH_2Cl_2$ as eluent to give the titled compound (6.02 g, 92%) as a light yellow solid. $^1$H NMR (400 MHz, $CDCl_3$): $\delta$ 9.11 (dd, 1 H, *J* = 1.6, 1.2 Hz), 8.47 (s, 1 H), 8.14 (dd, 1 H, *J* = 9.2, 1.6 Hz), 8.04 (br d, 1 H, *J* = 7.6 Hz), 7.88 (dd, 1 H, *J* = 9.2, 1.2 Hz), 7.84 (t, 1 H, *J* = 7.8 Hz), 7.42 (br d, 1 H, *J* = 8.0 Hz), 2.49 (s, 3 H).

**Preparative Example 6**

Preparation of 1-([1,2,4]triazolo[1,5-a]pyridin-6-yl)-2-(6-ethylpyridin-2-yl)ethane-1,2-dione (a compound the of formula (**V**) wherein $R^a$ = $CH_2CH_3$)

The titled compound was prepared as described in Preparative Example 5 by using 2-([1,2,4]triazolo[1,5-*a*]pyridin-6-yl)-1-(6-ethylpyridin-2-yl)ethanone in place of 2-([1,2,4]triazolo[1,5-*a*]pyridin-6-yl)-1-(6-methylpyridin-2-yl)ethanone. Yield: 79%; $^1$H NMR (400 MHz, $CDCl_3$): $\delta$ 9.11 (dd, 1 H, *J* = 1.6, 0.8 Hz), 8.42 (s, 1 H), 8.08 (dd, 1 H, *J* = 9.2, 1.6 Hz), 7.98 (br d, 1 H, *J* = 7.6 Hz), 7.83 (dd, 1 H, overlapped, *J* = 9.2, 0.8 Hz), 7.82 (t, 1 H, overlapped, *J* = 7.6 Hz), 7.38 (br d, 1 H, *J* = 7.6 Hz), 2.71 (q, 2 H, *J* = 7.6 Hz), 1.08 (t, 3 H, *J* = 7.6 Hz).

**Preparative Example 7**

Preparation of 6-(2-(dimethoxymethyl)-5-(6-methylpyridin-2-yl)-1*H*-imidazol-4-yl)-[1,2,4]triazolo[1,5-*a*]pyridine (a compound of the formula (**VI**) wherein $R^a$ = $CH_3$)

A stirred solution of 1-([1,2,4]triazolo[1,5-a]pyridin-6-yl)-2-(6-methylpyridin-2-yl)ethane-1,2-dione (6.00 g, 22.49 mmol) in *tert*-butyl methyl ether (120 mL) was treated with glyoxal dimethyl acetal (60 wt. % solution in water, 7.8 mL, 44.98 mmol). $NH_4OAc$ (4.33 g, 56.2 mmol) in MeOH (60 mL) was added to it, and the resulting mixture was stirred at room temperature for 3 h. The pH of the reaction was adjusted to 8 with saturated aqueous $NaHCO_3$ solution. The reaction mixture was extracted with $CHCl_3$ (2 × 150 mL), and the $CHCl_3$ solution was washed with water (100 mL), dried over anhydrous $Na_2SO_4$, filtered, and evaporated to dryness under reduced pressure. The residue was purified by MPLC on silica gel using a mixture of MeOH and $CH_2Cl_2$ as eluent to give the titled compound (6.13 g, 78%) as a light yellow foam. $^1$H NMR (400 MHz, $CDCl_3$): $\delta$ 10.54 (br s, 1 H), 8.96 (s, 1 H), 8.36 (s, 1 H), 7.82 (dd, 1 H, *J* = 9.2, 1.6 Hz), 7.77 (dd, 1 H, *J* = 9.2, 0.8 Hz), 7.47 (t, 1 H, *J* = 7.8 Hz), 7.23 (d, 1 H, *J* = 7.6 Hz), 7.04 (d, 1 H, *J* = 8. 0 Hz), 5.57 (s, 1 H), 3.48 (s, 6 H), 2.58 (s, 3 H).

**Preparative Example 8**

Preparation of 6-(2-(dimethoxymethyl)-5-(6-ethylpyridin-2-yl)-1*H*-imidazol-4-yl)-[1,2,4]triazolo[1,5-a]pyridine (a compound of the formula (**VI**) wherein $R^a = CH_2CH_3$)

The titled compound was prepared as described in Preparative Example 7 by using 1-([1,2,4]triazolo[1,5-*a*]pyridin-6-yl)-2-(6-ethylpyridin-2-yl)ethane-1,2-dione in place of 1-([1,2,4]triazolo[1,5-a]pyridin-6-yl)-2-(6-methylpyridin-2-yl)ethane-1,2-dione. Yield: 68%; $^1$H NMR (400 MHz, $CDCl_3$): $\delta$ 10.67 (br s, 1 H), 8.97 (br s, 1 H), 8.35 (s, 1 H), 7.83 (dd, 1 H, *J* = 9.2, 1.6 Hz), 7.76 (dd, 1 H, *J* = 9.2, 0.8 Hz), 7.50 (t, 1 H, *J* = 7.8 Hz), 7.25 (br d, 1 H, *J* = 7.6 Hz), 7.05 (d, 1 H, *J* = 8.0 Hz), 5.56 (s, 1 H), 3.46 (s, 6 H), 2.83 (q, 2 H, *J* = 7.6 Hz), 1.31 (t, 3 H, *J* = 7.6 Hz).

**Preparative Example 9**

Preparation of 4-([1,2,4]triazolo[1,5-a]pyridin-6-yl)-5-(6-methylpyridin-2-yl)-1*H*-imidazole-2-carbaldehyde (a compound of the formula (**VII**) wherein $R^a = CH_3$)

6-(2-(Dimethoxymethyl)-5-(6-methylpyridin-2-yl)-1*H*-imidazol-4-yl)-[1,2,4]triazolo[1,5-a]pyridine (6.00 g, 17.12 mmol) was dissolved in 1 N HCl (120 mL), and the mixture was heated at 70°C for 3 h. The reaction mixture was allowed to cool to 0°C, and then it was neutralized with saturated aqueous $NaHCO_3$ solution. The mixture was extracted with 10% MeOH in $CHCl_3$ (3 $\times$ 200 mL), and the organic phase was dried over anhydrous $Na_2SO_4$, filtered, and evaporated to dryness under reduced pressure to give the titled compound (4.69 g, 90%) as a light yellow solid. $^1$H NMR (400 MHz, $CDCl_3$): $\delta$ 9.82 (s, 1 H), 9.01 (br s, 1 H), 8.41 (s, 1 H), 7.85 (dd, 1 H, *J* = 9.2, 0.8 Hz), 7.82 (dd, 1 H, *J* = 9.2, 1.6 Hz), 7.55 (t, 1 H, *J* = 7.8 Hz), 7.33 (br s, 1 H), 7.16 (d, 1 H, *J* = 8. 0 Hz), 2.60 (s, 3 H).

**Preparative Example 10**

Preparation of 4-([1,2,4]triazolo[1,5-a]pyridin-6-yl)-5-(6-ethylpyridin-2-yl)-1 H-imidazole-2-carbaldehyde (a compound of the formula (**VII**) wherein $R^a = CH_2CH_3$)

The titled compound was prepared as described in Preparative Example 9 by using 6-(2-(dimethoxymethyl)-5-(6-ethylpyridin-2-yl)-1*H*-imidazol-4-yl)-[1,2,4]triazolo[1,5-a]pyridine in place of 6-(2-(dimethoxymethyl)-5-(6-methylpyridin-2-yl)-1*H*-imidazol-4-yl)-[1,2,4]triazolo[1,5-*a*]pyridine. Yield: 99%; $^1$H NMR (400 MHz, DMSO-$d_6$) $\delta$ 9.86 (t, 1 H, *J* = 1.2 Hz), 9.59 (s, 1 H), 8.43 (s, 1 H), 8.21 (dd, 1 H, *J* = 9.2, 1.6 Hz), 7.82 (br d, 1 H, *J* = 8.0 Hz), 7.73 (dd, 1 H, *J* = 9.2, 0.8 Hz), 7.69 (t, 1 H, *J* = 7.8 Hz), 7.08 (br d, 1 H, *J* = 7.6 Hz), 2.71 (q, 2 H, *J* = 7.6 Hz), 1.16 (t, 3 H, *J* = 7.6 Hz).

**Preparative Example 11**

Preparation of 3-amino-5-(dimethylamino)benzonitrile (a compound of the formula (**VIII**) wherein $R^b$ = 3-cyano-5-dimethylamino). This compound was prepared by the following 2 steps.

3-Bromo-*N*,*N*-dimethyl-5-nitroaniline (1.73 g, 7.06 mmol) (prepared according to the method described in J. Org. Chem. 60: 5091-5103 (2003)), pyridine (24 mL), and CuCN (1.26 g, 2.14 mmol) were added to a dry sealed tube. The mixture was heated at 220°C with stirring for 3.5 h. The reaction mixture was allowed to cool to 100°C, poured into a flask containing a mixture of aqueous ammonia (100 mL) and water (100 mL), and extracted with EtOAc (2 $\times$ 100 mL). The EtOAc solution was washed with diluted ammonia solution (100 mL), water (100 mL) and brine (100 mL) successively, dried over anhydrous $Na_2SO_4$, filtered, and evaporated to dryness under reduced pressure. The residue was purified by MPLC on silica gel using a mixture of EtOAc and hexane as eluent to give 3-(dimethylamino)-5-nitrobenzonitrile (0.44 g, 33%) as an orange solid. $^1$H NMR (400 MHz, $CDCl_3$): $\delta$ 7.74 (dd, 1 H, *J* = 2.0, 1.2 Hz), 7.65 (t, 1 H, *J* = 2.2 Hz), 7.11 (dd, 1 H, *J* = 2.4, 1.2 Hz), 3.10 (s, 6 H).

The above nitro compound, 3-(dimethylamino)-5-nitrobenzonitrile (0.42 g, 2.22 mmol) in methanol (80 mL) was hydrogenated in the presence of 10% Pd/C (0.04 g) under a hydrogen gas atmosphere overnight. The reaction mixture was filtered through a Celite pad, and the filtrate was evaporated to dryness under reduced pressure. The residue was purified by MPLC on silica gel using a mixture of EtOAc and hexane as eluent to give the titled compound (0.29 g, 80%) as a brown viscous liquid. $^1$H NMR (400 MHz, $CDCl_3$): $\delta$ 6.35 (dd, 1 H, *J* = 2.4, 1.6 Hz), 6.28 (dd, 1 H, *J* = 2.0, 1.6 Hz), 6.14 (t, 1 H, *J* = 2.2 Hz), 3.76 (br s, 2 H), 2.92 (s, 6 H).

**Preparative Example 12**

Preparation of 3-((dimethylamino)methyl)-2-fluroaniline (a compound of the formula (**VIII**) wherein $R^b$ =

3-(dimethylamino)methyl-2-fluoro). This compound was prepared by the following 3 steps started with commercially available 2-fluoro-1-methyl-3-nitrobenzene.

A stirred solution of 2-fluoro-1-methyl-3-nitrobenzene (15.80 g, 101.94 mmol) and *N*-bromosuccinimide (18.14 g, 101.94 mmol) in $CCl_4$ (400 mL) was treated with benzoyl peroxide (0.37 g, 1.52 mmol). The mixture was heated at reflux temperature overnight and then cooled to room temperature. The reaction mixture was filtered, and the filtrate was evaporated to dryness under reduced pressure. The residue was dissolved in $CH_2Cl_2$ (100 mL) and filtered again. The filtrate was evaporated to dryness under reduced pressure, and the residue was purified by MPLC on silica gel using a mixture of EtOAc and hexane as eluent to give 1-(bromomethyl)-2-fluoro-3-nitrobenzene (8.11 g, 34%) as an off-white solid. $^1$H NMR (400 MHz, $CDCl_3$): $\delta$ 8.02 (m, 1 H), 7.71 (m, 1 H), 7.30 (td, 1 H, *J* = 8.4, 1.6Hz), 4.55(d, 2H, *J* = 1.6 Hz).

To a stirred mixture of 1-(bromomethyl)-2-fluoro-3-nitrobenzene (0.70 g, 2.99 mmol) and dimethylamine hydrochloride (0.48 g, 5.98 mmol) in $CH_2Cl_2$ (10 mL) was added triethylamine (0.91 g, 8.97 mmol) dropwise. The mixture was stirred at room temperature for 3 h and evaporated to dryness under reduced pressure. The residue was diluted with water (10 mL) and extracted with EtOAc (2 x 25 mL). The EtOAc solution was washed with water (20 mL), dried over anhydrous $Na_2SO_4$, filtered, and evaporated to dryness under reduced pressure. The residue was purified by MPLC on silica gel using a mixture of EtOAc and hexane as eluent to give 1-(2-fluoro-3-nitrophenyl)-*N*,*N*dimethylmethanamine (0.45 g, 76%) as a yellow viscous liquid. $^1$H NMR (400 MHz, $CD_3OD$): $\delta$ 8.04-7.99 (m, 1 H), 7.78-7.73 (m, 1 H), 7.37 (td, 1 H, *J* = 8.0, 1.0 Hz), 3.64 (d, 2 H, *J* = 2.0 Hz), 2.29 (d, 6 H, *J* = 0.8 Hz).

A mixture of the above nitro compound, 1-(2-fluoro-3-nitrophenyl)-*N*,*N*-dimethylmethanamine (0.45 g, 1.93 mmol), iron powder (1.35 g, 2.41 mmol), 2 N HCl (1 mL), and ethanol (5 mL) was heated at reflux temperature with stirring for 2 h. After cooling to room temperature, the mixture was filtered through a Celite pad. The filtrate was evaporated to dryness under reduced pressure, and the residue was diluted with water (10 mL) and basified with solid $K_2CO_3$ to pH 10. The aqueous mixture was extracted with EtOAc (2 × 25 mL), and the EtOAc solution was dried over anhydrous $Na_2SO_4$, filtered, and evaporated to dryness under reduced pressure. The residue was purified by MPLC on silica gel using a mixture of EtOAc and hexane as eluent to give the titled compound (0.35 g, 91%) as a white solid. $^1$H NMR (400 MHz, $CDCl_3$): $\delta$ 6.88 (td, 1 H, *J* = 7.8, 1.0 Hz), 6.72-6.67 (m, 2 H), 3.71 (br s, 2 H), 3.47 (d, 2 H, *J* = 1.6 Hz), 2.27 (s, 6 H).

**Preparative Example 13**

Preparation of 2-fluoro-3-(pyrrolidin-1-ylmethyl)aniline (a compound of the formula (**VIII**) wherein $R^b$ = 2-fluoro-3-(pyrrolidin-1-ylmethyl))

To a stirred solution of 1-(bromomethyl)-2-fluoro-3-nitrobenzene (2.00 g, 8.54 mmol) and pyrrolidine (0.91 g, 12.82 mmol) in $CH_2Cl_2$ (15 mL) was added triethylamine (1.72 g, 17.08 mmol) dropwise at 0°C. The mixture was stirred at room temperature overnight and then evaporated to dryness under reduced pressure. The residue was diluted with water (15 mL) and extracted with EtOAc (2 × 30 mL). The EtOAc solution was washed with brine (20 mL), dried over anhydrous $Na_2SO_4$, filtered, and evaporated to dryness under reduced pressure. The residue was purified by MPLC on silica gel using a mixture of EtOAc and hexane as eluent to give 1-(2-fluoro-3-nitrobenzyl)pyrrolidine (1.20 g, 63%) as a viscous oil. $^1$H NMR (400 MHz, $CD_3OD$): $\delta$ 8.02-7.97 (m, 1 H), 7.81-7.76 (m, 1 H), 7.36 (td, 1 H, *J* = 8.0, 1.2 Hz), 3.81 (d, 1 H, *J* = 2.0 Hz), 2.62-2.58 (m, 4 H), 1.84-1.80 (m, 4H).

The titled compound was prepared as described in Preparative Example 12 by using 1-(2-fluoro-3-nitrobenzyl)pyrrolidine in place of 1-(2-fluoro-3-nitrophenyl)-*N*,*N*-dimethylmethanamine. Yield: 80%; $^1$H NMR (400 MHz, $CD_3OD$): $\delta$ 6.87 (td, 1 H, *J* = 8.0, 0.8 Hz), 6.77 (td, 1 H, *J* = 8.0, 2.0 Hz), 6.68-6.64 (m, 1 H), 3.67 (d, 2 H, *J* = 1.6 Hz), 2.60-2.57 (m, 4 H), 1.82-1.78 (m, 4 H).

**Preparative Example 14**

Preparation of 2-fluoro-3-(morpholinomethyl)aniline (a compound of the formula (**VIII**) wherein $R^b$ = 2-fluoro-3-(morpholinomethyl))

A stirred solution of 1-(bromomethyl)-2-fluoro-3-nitrobenzene (2.50 g, 10.6 mmol) and morpholine (2.78 g, 32.0 mmol) in toluene (24 mL) was heated at reflux temperature for 2.5 h. The reaction mixture was allowed to cool to room temperature and then washed with 1 N NaOH (2 × 20 mL). The aqueous layer was extracted with EtOAc (2 × 25 mL), and the combined toluene solution and EtOAc extracts were dried over anhydrous $Na_2SO_4$, filtered, and evaporated to dryness under reduced pressure. The residue was purified by MPLC on silica gel using a mixture of EtOAc and hexane as eluent to give 4-(2-fluoro-3-nitrobenzyl)morpholine (1.95 g, 89%) as a light yellow solid. $^1$H NMR (400 MHz, $CD_3OD$): $\delta$ 8.02-7.97 (m, 1 H), 7.83-7.78 (m, 1 H), 7.36 (td, 1 H, *J* = 8.0, 1.0 Hz), 3.70-3.67 (m, 6 H), 2.51 (br t, 4 H, *J* = 4.6 Hz).

The titled compound was prepared as described in Preparative Example 12 by using (4-(2-fluoro-3-nitrobenzyl)morpholine) in place of 1-(2-fluoro-3-nitrophenyl)-*N*,*N*-dimethylmethanamine. Yield: 90%; $^1$H NMR (400 MHz,

$CD_3OD$): $\delta$ 6.87 (td, 1 H, $J$ = 8.0, 0.8 Hz), 6.77 (td, 1 H, $J$ = 8.0, 2.0 Hz), 6.68-6.64 (m, 1 H), 3.68 (br t, 4 H, $J$ = 4.8 Hz), 3.55 (d, 2 H, $J$ = 1.6 Hz), 2.49 (br t, 4 H, $J$ = 4.8 Hz).

**Preparative Example 15**

Preparation of 3-amino-4-((dimethylamino)methyl)benzonitrile (a compound of the formula (**VIII**) wherein $R^b$ = 5-cyano-2-(dimethylamino)methyl)

To a stirred mixture of 4-(bromomethyl)-3-nitrobenzonitrile (5.00 g, 20.74 mmol) (prepared according to the method described in WO 07/024945 A1) and dimethylamine hydrochloride (2.03 g, 24.89 mmol) in $CH_2Cl_2$ (70 mL) was added triethylamine (6.30 g, 62.23 mmol) dropwise at 0°C, and the mixture was stirred at room temperature overnight. The reaction mixture was evaporated to dryness under reduced pressure, and the residue was diluted with water (20 mL) and extracted with $CH_2Cl_2$ (3 × 100 mL). The $CH_2Cl_2$ solution was dried over anhydrous $Na_2SO_4$, filtered, and evaporated to dryness under reduced pressure. The residue was purified by MPLC on silica gel using a mixture of EtOAc and hexane as eluent to give 4-((dimethylamino)methyl)-3-nitrobenzene (3.58 g, 84%) as an orange solid. $^1H$ NMR (400 MHz, $CDCl_3$): $\delta$ 8.15 (br s, 1 H), 7.92 (br s, 1 H), 7.85 (br d, 1 H, $J$ = 7.2 Hz), 3.80 (s, 2 H), 2.27 (s, 6 H).

The titled compound was prepared as described in Preparative Example 11 by using 4-((dimethylamino)methyl)-3-nitrobenzonitrile in place of 3-(dimethylamino)-5-nitrobenzonitrile. Yield: 91%; $^1H$ NMR (400 MHz, $CDCl_3$): $\delta$ 7.02 (dd, 1 H, $J$ = 7.6, 0.4 Hz), 6.91 (dd, 1 H, $J$ = 7.6, 1.6 Hz), 6.84 (d, 1 H, $J$ = 1.6 Hz), 5.05 (br s, 2 H), 3.43 (s, 2 H), 2.18 (s, 6 H).

**Preparative Example 16**

Preparation of 3-amino-2-((dimethylamino)methyl)benzonitrile (a compound of the formula (**VIII**) wherein $R^b$ = 3-cyano-2-(dimethyamino)methyl)

To a stirred mixture of 2-(bromomethyl)-3-nitrobenzonitrile (1.10 g, 4.56 mmol) (prepared according to the method described in Tetrahedron 40: 1863-1868 (1984)) and dimethylamine hydrochloride (0.74 g, 9.13 mmol) in $CH_2Cl_2$ (15 mL) was added triethylamine (1.85 g, 18.25 mmol) dropwise at 0°C. The resulting mixture was stirred at room temperature for 2 h and evaporated to dryness under reduced pressure. The residue was diluted with water (10 mL) and extracted with EtOAc (3 × 50 mL). The EtOAc solution was dried over anhydrous $Na_2SO_4$, filtered, and evaporated to dryness under reduced pressure. The residue was purified by MPLC on silica gel using a mixture of EtOAc and hexane as eluent to give 2-((dimethylamino)methyl)-3-nitrobenzonitrile (0.75 g, 80%) as a yellow oil. $^1H$ NMR (400 MHz, $CDCl_3$): $\delta$ 7.93 (br d, 1 H, $J$ = 8.0 Hz), 7.85 (dd, 1 H, $J$ = 8.0, 1.4 Hz), 7.55 (t, 1 H, $J$ = 8.0 Hz), 3.94 (s, 2 H), 2.24 (s, 6 H).

The titled compound was prepared as described in Preparative Example 11 by using 2-((dimethylamino)methyl)-3-nitrobenzonitrile in place of 3-(dimethylamino)-5-nitrobenzonitrile. Yield: 93%; $^1H$ NMR (400 MHz, $CDCl_3$): $\delta$ 7.15 (t, 1 H, $J$ = 7.6 Hz), 7.00 (dd, 1 H, $J$ = 7.6, 0.8 Hz), 6.83 (d, 1 H, $J$ = 7.6 Hz), 5.06 (br s, 2 H), 3.73 (s, 2 H), 2.29 (s, 6 H).

**Preparative Example 17**

Preparation of 3-amino-5-((dimethylamino)methyl)benzonitrile (a compound of the formula (**VIII**) wherein $R^b$ = 3-cyano-5-(dimethylamino)methyl)

To a stirred mixture of 3-(bromomethyl)-5-nitrobenzonitrile (1.50 g, 6.22 mmol) (prepared according to the method described in J. Org. Chem. 55: 1040-1043 (1990)) and dimethylamine hydrochloride (1.01 g, 12.44 mmol) in $CH_2Cl_2$ (15 mL) was added triethylamine (1.88 g, 18.66 mmol) dropwise at 0°C. The resulting mixture was stirred at room temperature for 3 h and evaporated to dryness under reduced pressure. The residue was diluted with water (15 mL) and extracted with EtOAc (3 x 50 mL). The EtOAc solution was dried over anhydrous $Na_2SO_4$, filtered, and evaporated to dryness under reduced pressure. The residue was purified by MPLC on silica gel using a mixture of EtOAc and hexane as eluent to give 3-((dimethylamino)methyl)-5-nitrobenzonitrile (1.10 g, 87%) as a viscous liquid. $^1H$ NMR (400 MHz, $CDCl_3$): $\delta$ 8.45 (s, 1 H), 8.40 (d, 1 H, $J$ = 1.6 Hz), 8.01 (s, 1 H), 3.59 (s, 2 H), 2.30 (s, 6 H).

The titled compound was prepared as described in Preparative Example 11 by using 3-((dimethylamino)methyl)-5-nitrobenzonitrile in place of 3-(dimethylamino)-5-nitrobenzonitrile. Yield: 98%; $^1H$ NMR (400 MHz, $CDCl_3$): $\delta$ 6.96 (m, 1 H), 6.88 (br d, 1 H, $J$ = 0.8 Hz), 6.80 (dd, 1 H, $J$ = 2.4, 1.6 Hz), 3.86 (br s, 2 H), 3.34 (s, 2 H), 2.24 (s, 6 H).

**Preparative Example 18**

Preparation of 3-amino-4-(pyrrolidin-1-ylmethyl)benzonitrile (a compound of the formula (**VIII**) wherein $R^b$ = 5-cyano-2-(pyrrolidin-1-ylmethyl))

To a stirred solution of 4-(bromomethyl)-3-nitrobenzonitrile (5.12 g, 21.57 mmol) and pyrrolidine (1.84 g, 25.88 mmol) in $CH_2Cl_2$ (72 mL) was added triethylamine (6.54 g, 64.71 mmol) dropwise at 0°C. The mixture was stirred at room temperature for 1.5 h and evaporated to dryness under reduced pressure. The residue was diluted with water (30 mL) and extracted with $CH_2Cl_2$ (3 × 100 mL). The $CH_2Cl_2$ solution was dried over anhydrous $Na_2SO_4$, filtered, and evaporated to dryness under reduced pressure. The residue was purified by MPLC on silica gel using a mixture of EtOAc and hexane as eluent to give 3-nitro-4-(pyrrolidin-1-ylmethyl)benzonitrile (2.24 g, 45%) as a yellow solid. $^1H$ NMR (400 MHz, $CDCl_3$): $\delta$ 8.16 (d, 1 H, *J* = 1.6 Hz), 7.94 (br d, 1 H, *J* = 8.0 Hz), 7.83 (dd, 1 H, *J* = 8.0, 1.6 Hz), 3.99 (s, 2 H), 2.54 (br s, 4 H), 1.79 (m, 4 H).

The titled compound was prepared as described in Preparative Example 11 by using 3-nitro-4-(pyrrolidin-1-ylmethyl)benzonitrile in place of 3-(dimethylamino)-5-nitrobenzonitrile. Yield: 91%; $^1H$ NMR (400 MHz, $CDCl_3$): $\delta$ 7.06 (d, 1 H, *J* = 7.6 Hz), 6.91 (dd, 1 H, *J* = 7.6, 1.6 Hz), 6.84 (d, 1 H, *J* = 1.6 Hz), 5.08 (br s, 2 H), 3.64 (s, 2 H), 2.47 (br s, 4 H), 1.78 (br s, 4 H).

**Preparative Example 19**

Preparation of 3-amino-2-(pyrrolidin-1-ylmethyl)benzonitrile (a compound of the formula (**VIII**) wherein $R^b$ = 3-cyano-2-(pyrrolidin-1-ylmethyl))

To a stirred solution of 2-(bromomethyl)-3-nitrobenzonitrile (1.10 g, 4.56 mmol) and pyrrolidine (0.65 g, 9.13 mmol) in $CH_2Cl_2$ (15 mL) was added triethylamine (1.85 g, 18.25 mmol) dropwise at 0°C. The mixture was stirred at room temperature for 2 h and evaporated to dryness under reduced pressure. The residue was diluted with water (10 mL) and extracted with $CH_2Cl_2$ (3 × 50 mL). The $CH_2Cl_2$ solution was dried over anhydrous $Na_2SO_4$, filtered, and evaporated to dryness under reduced pressure. The residue was purified by MPLC on silica gel using a mixture of EtOAc and hexane as eluent to give 3-nitro-2-(pyrrolidin-1-ylmethyl)benzonitrile (0.96 g, 91%) as a yellow solid. $^1H$ NMR (400 MHz, $CDCl_3$): $\delta$ 7.90 (d, 1 H, *J* = 7.6 Hz), 7.83 (dd, 1 H, *J* = 7.6, 0.8 Hz), 7.52 (t, 1 H, *J* = 7.6 Hz), 4.14 (s, 2 H), 2.52 (br s, 4 H), 1.72 (br s, 4 H).

The titled compound was prepared as described in Preparative Example 11 by using 3-nitro-2-(pyrrolidin-1-ylmethyl)benzonitrile in place of 3-(dimethylamino)-5-nitrobenzonitrile. Yield: 93%; $^1H$ NMR (400 MHz, $CDCl_3$): $\delta$ 7.13 (t, 1 H, *J* = 7.8 Hz), 6.99 (dd, 1 H, *J* = 7.8, 1.2 Hz), 6.82 (d, 1 H, *J* = 8.0 Hz), 5.11 (br s, 2 H), 3.91 (s, 2 H), 2.58 (br s, 4 H), 1.81 (br s, 4 H).

**Preparative Example 20**

Preparation of 3-amino-5-(pyrrolidin-1-ylmethyl)benzonitrile (a compound of the formula (**VIII**) wherein $R^b$ = 3-cyano-5-(pyrrolidin-1-ylmethyl))

To a stirred solution of 3-(bromomethyl)-5-nitrobenzonitrile (1.50 g, 6.22 mmol) and pyrrolidine (0.53 g, 7.46 mmol) in $CH_2Cl_2$ (15 mL) was added triethylamine (1.88 g, 18.68 mmol) dropwise at 0°C, and the mixture was stirred at room temperature overnight. The reaction mixture was evaporated to dryness under reduced pressure, and the residue was diluted with water (15 mL) and extracted with EtOAc (3 × 50 mL). The EtOAc solution was dried over anhydrous $Na_2SO_4$, filtered, and evaporated to dryness under reduced pressure. The residue was purified by MPLC on silica gel using a mixture of EtOAc and hexane as eluent to give 3-nitro-5-(pyrrolidin-1-ylmethyl)benzonitrile (1.30 g, 90%) as a yellow solid. $^1H$ NMR (400 MHz, $CDCl_3$): $\delta$ 8.45 (br s, 1 H), 8.39 (br t, 1 H, *J* = 1.6 Hz), 8.02 (br s, 1 H), 3.78 (s, 2 H), 2.56 (br s, 4 H), 1.84 (br s, 4 H).

The titled compound was prepared as described in Preparative Example 11 by using 3-nitro-5-(pyrrolidin-1-ylmethyl)benzonitrile in place of 3-(dimethylamino)-5-nitrobenzonitrile. Yield: 85%; $^1H$ NMR (400 MHz, $CDCl_3$): $\delta$ 6.99 (pseudo t, 1 H, *J* = 1.6 Hz), 6.94 (pseudo t, 1 H, *J* = 1.6 Hz), 6.79 (dd, 1 H, *J* = 2.4, 1.6 Hz), 3.87 (br s, 2 H), 3.56 (s, 2 H), 2.54 (m, 4 H), 1.81 (m, 4 H).

**Preparative Example 21**

Preparation of 3-amino-4-(morpholinomethyl)benzonitrile (a compound of the formula (**VIII**) wherein $R^b$ = 5-cyano-2-(morpholinomethyl))

To a stirred solution of 4-(bromomethyl)-3-nitrobenzonitrile (7.12 g, 29.55 mmol) and morpholine (3.09 g, 35.45 mmol) in $CH_2Cl_2$ (98 mL) was added triethylamine (8.97 g, 88.64 mmol) dropwise at 0°C. The mixture was stirred at room temperature for 1.5 h and evaporated to dryness under reduced pressure. The residue was diluted with water (40 mL) and extracted with $CH_2Cl_2$ (3 × 100 mL). The $CH_2Cl_2$ solution was washed with water (50 mL), dried over anhydrous $Na_2SO_4$, filtered and evaporated to dryness under reduced pressure. The residue was purified by MPLC on silica gel using a mixture of EtOAc and hexane as eluent to give 4-(morpholinomethyl)-3-nitrobenzonitrile (5.84 g, 80%) as a yellow solid. $^1H$ NMR (400 MHz, $CDCl_3$): $\delta$ 8.12 (s, 1 H), 7.83 (br s, 2 H), 3.84 (s, 2 H), 3.67 (t, 4 H, *J* = 4.6 Hz), 2.45 (t, 4 H, *J* = 4.6 Hz).

The titled compound was prepared as described in Preparative Example 11 by using 4-(morpholinomethyl)-3-nitrobenzonitrile in place of 3-(dimethylamino)-5-nitrobenzonitrile. Yield: 78%; $^1H$ NMR (400 MHz, $CDCl_3$): $\delta$ 7.05 (d, 1 H, *J* = 8.0 Hz), 6.92 (dd, 1 H, *J* = 8.0, 1.6 Hz), 6.86 (d, 1 H, *J* = 1.6 Hz), 5.02 (br s, 2 H), 3.68 (br t, 4 H, *J* = 4.0 Hz), 3.53 (s, 2 H), 2.41 (br s, 4 H).

**Preparative Example 22**

Preparation of 3-amino-2-(morpholinomethyl)benzonitrile (a compound of the formula (**VIII**) wherein $R^b$ = 3-cyano-2-(morpholinomethyl))

To a stirred solution of 2-(bromomethyl)-3-nitrobenzonitrile (1.10 g, 4.56 mmol) and morpholine (0.80 g, 9.13 mmol) in $CH_2Cl_2$ (15 mL) was added triethylamine (1.85 g, 18.25 mmol) dropwise at 0°C. The mixture was stirred at room temperature for 1.5 h and evaporated to dryness under reduced pressure. The residue was diluted with water (10 mL) and extracted with $CH_2Cl_2$ (3 × 50 mL). The $CH_2Cl_2$ solution was dried over anhydrous $Na_2SO_4$, filtered, and evaporated to dryness under reduced pressure. The residue was purified by MPLC on silica gel using a mixture of EtOAc and hexane as eluent to give 2-(morpholinomethyl)-3-nitrobenzonitrile (1.02 g, 90%) as a yellow solid. $^1H$ NMR (400 MHz, $CDCl_3$): $\delta$ 7.90 (br d, 1 H, *J* = 8.0 Hz), 7.84 (dd, 1 H, *J* = 8.0, 1.2 Hz), 7.56 (t, 1 H, *J* = 8.0 Hz), 3.99 (s, 2 H), 3.60 (br t, 4 H, *J* = 4.4 Hz), 2.46 (br s, 4 H).

The titled compound was prepared as described in Preparative Example 11 by using 2-(morpholinomethyl)-3-nitrobenzonitrile in place of 3-(dimethylamino)-5-nitrobenzonitrile. Yield: 82%; $^1H$ NMR (400 MHz, $CDCl_3$): $\delta$ 7.15 (t, 1 H, *J* = 7.6 Hz), 7.01 (d, 1 H, *J* = 7.6 Hz), 6.83 (d, 1 H, *J* = 7.6 Hz), 5.00 (br s, 2 H), 3.78 (s, 2 H), 3.69 (br s, 4 H), 2.50 (br s, 4 H).

**Preparative Example 23**

Preparation of 3-amino-5-(morpholinomethyl)benzonitrile (a compound of the formula (**VIII**) wherein $R^b$ = 3-cyano-5-(morpholinomethyl))

To a stirred solution of 3-(bromomethyl)-5-nitrobenzonitrile (1.50 g, 6.22 mmol) and morpholine (0.65 g, 7.46 mmol) in $CH_2Cl_2$ (15 mL) was added triethylamine (1.88 g, 18.66 mmol) dropwise at 0°C. The mixture was stirred at room temperature overnight and then evaporated to dryness under reduced pressure. The residue was diluted with water (15 mL) and extracted with EtOAc (3 × 50 mL). The EtOAc solution was dried over anhydrous $Na_2SO_4$, filtered and evaporated to dryness under reduced pressure. The residue was purified by MPLC on silica gel using a mixture of EtOAc and hexane as eluent to give 3-(morpholinomethyl)-5-nitrobenzonitrile (0.87 g, 85%) as an off-white solid. $^1H$ NMR (400 MHz, $CDCl_3$): $\delta$ 8.45 (br s, 1 H), 8.41 (br s, 1 H), 8.01 (br s, 1 H), 3.74 (br t, 4 H, *J* = 4.4 Hz), 3.64 (s, 2 H), 2.48 (br t, 4 H, *J* = 4.4 Hz).

The titled compound was prepared as described in Preparative Example 11 by using 3-(morpholinomethyl)-5-nitrobenzonitrile in place of 3-(dimethylamino)-5-nitrobenzonitrile. Yield: 85%; $^1H$ NMR (400 MHz, $CDCl_3$): $\delta$ 7.00 (br t, 1 H, *J* = 1.6 Hz), 6.93 (br s, 1 H), 6.81 (dd, 1 H, *J* = 2.4, 1.6 Hz), 3.88 (br s, 2 H), 3.74 (br t, 4 H, *J*= 4.6 Hz), 3.44 (s, 2 H), 2.47 (br s, 4 H).

**Preparative Example 24**

Preparation of 2-(2-fluorophenoxy)acetaldehyde (a compound of the formula (**IX**) wherein $R^b$ = 2-fluoro, X = O). This compound was prepared by the following 2 steps.

A stirred mixture of 2-fluorophenol (1.00 g, 8.92 mmol), 2-bromo-1,1-diethoxyethane (1.75 g, 8.92 mmol), and

$K_2CO_3$ (1.47 g, 10.7 mmol) in anhydrous DMF (10 mL) was heated at 110°C overnight. The reaction mixture was poured into ice cold water (15 mL) and extracted with EtOAc (2 × 100 mL). The EtOAc solution was washed with water (25 mL) and brine (25 mL), dried over anhydrous $Na_2SO_4$, filtered, and evaporated to dryness under reduced pressure. The residue was purified by MPLC on silica gel using a mixture of EtOAc and hexane as eluent to give 1-(2,2-diethoxyethoxy)-2-fluorobenzene (1.65 g, 81%) as a viscous liquid. $^1$H NMR (400 MHz, $CDCl_3$): $\delta$ 7.09-6.96 (m, 3 H), 6.93-6.87 (m, 1 H), 4.85 (t, 1 H, $J$ = 5.2 Hz), 4.07 (d, 2 H, $J$ = 5.2 Hz), 3.82-3.74 (m, 2 H), 3.69-3.61 (m, 2 H), 1.24 (t, 6 H, $J$ = 7.0 Hz).

To a stirred solution of 1-(2,2-diethoxyethoxy)-2-fluorobenzene (1.65 g, 7.23 mmol) in a mixture of 1,4-dioxane (50 mL) and water (40 mL) at 0°C was added conc. HCl (17.6 mL), and the mixture was stirred at room temperature overnight. The reaction mixture was cooled to 0°C, neutralized with saturated $NaHCO_3$ solution, and extracted with EtOAc (2 × 200 mL). The EtOAc solution was dried over anhydrous $Na_2SO_4$, filtered, and evaporated to dryness under reduced pressure. The residue was purified by MPLC on silica gel using a mixture of EtOAc and hexane as eluent to give the titled compound (0.78 g, 71%) as a viscous liquid. $^1$H NMR (400 MHz, $CDCl_3$): $\delta$ 9.87 (s, 1 H), 7.12 (ddd, 1 H, $J$= 11.4, 8.2, 1.6 Hz), 7.08-7.04 (m, 1 H), 7.01-6.95 (m, 1 H), 6.91 (td. 1 H, $J$ = 8.2, 1.6 Hz), 4.63 (s, 2 H).

**Preparative Example 25**

Preparation of 2-(2-fluorophenylthio)acetaldehyde (a compound of the formula (**IX**) wherein $R^b$ = 2-fluoro, X = S). This compound was prepared by the following 2 steps.

A mixture of 2-fluorothiophenol (1.00 g, 7.80 mmol), bromoacetaldehyde diethyl acetal (1.41 mL, 9.36 mmol), and $Cs_2CO_3$ (3.05 g, 9.36 mmol) in anhydrous DMF (20 mL) was stirred under $N_2$ at room temperature overnight. The reaction mixture was filtered through a sintered funnel, and the filtrate was diluted with water (20 mL). The aqueous mixture was extracted with $Et_2O$ (3 × 100 mL), and the organic phase was dried over anhydrous $MgSO_4$, filtered, and evaporated to dryness under reduced pressure. The residue was purified by MPLC on silica gel using a mixture of EtOAc and hexane as eluent to give (2,2-diethoxyethyl)(2-fluorophenyl)sulfane (1.81 g, 95%) as a viscous liquid. $^1$H NMR (400 MHz, $CDCl_3$): $\delta$ 7.46-7.41 (m, 1 H), 7.24-7.18 (m, 1 H), 7.09-7.02 (m, 1 H), 4.64 (t, 1 H, $J$ = 5.6 Hz), 3.69-3.61 (m, 2 H), 3.56-3.49 (m, 2 H), 3.10 (d, 2 H, $J$ = 5.6 Hz), 1.17 (t, 6 H, $J$ = 7.2 Hz).

To a stirred solution of (2,2-diethoxyethyl)(2-fluorophenyl)sulfane (1.00 g, 4.09 mmol) in a mixture of 1,4-dioxane (30 mL) and water (25 mL) at 0°C was added conc. HCl (9 mL), and the mixture was stirred at room temperature for 2 h. The reaction mixture was cooled to 0°C, neutralized with saturated $NaHCO_3$ solution, and extracted with $CH_2Cl_2$ (3 × 50 mL). The organic phase was washed with water (50 mL), dried over anhydrous $Na_2SO_4$, filtered, and evaporated to dryness under reduced pressure to give the titled compound (0.59 g, 85%) as a viscous liquid, which was immediately used for the next step without further purification. $^1$H NMR (400MHz, $CDCl_3$): $\delta$ 9.56 (td, 1 H, $J$ = 3.2, 1.2 Hz), 7.42-7.38 (m, 1 H), 7.31-7.25 (m, 1 H), 7.12-7.06 (m, 2 H), 3.58 (d, 2 H, $J$ = 3.2 Hz).

**Preparative Example 26**

Preparation of 3-(methyl(2-oxoethyl)amino)benzonitrile (a compound of the formula (**IX**) wherein $R^b$ = 3-cyano, X = NMe). This compound was prepared by the following 3 steps started with commercially available 3-aminobenzonitrile.

To a stirred solution of 3-aminobenzonitrile (2.50 g, 21.10 mmol) in anhydrous DMSO (30 mL) at 0°C was added NaH (0.61 g, 25.39 mmol) portionwise, and the mixture was stirred at room temperature for 20 min and then treated with bromoacetaldehyde diethyl acetal (4.20 g, 21.10 mmol). After 2 h, to it, saturated aqueous $NH_4Cl$ (20 mL) was added slowly at 0°C, and the reaction mixture was extracted with EtOAc (2 × 50 mL). The EtOAc solution was dried over anhydrous $Na_2SO_4$, filtered, and evaporated to dryness under reduced pressure. The residue was purified by MPLC on silica gel using EtOAc and hexane as eluent to give 3-(2,2-diethoxyethylamino)benzonitrile (0.86 g, 17%) as a light orange oil. $^1$H NMR (400 MHz, $CDCl_3$): $\delta$ 7.22 (td, 1 H, $J$= 7.8, 0.6 Hz), 6.97 (m, 1 H), 6.84-6.81 (m, 2 H), 4.67 (t, 1 H, $J$ = 5.4 Hz), 3.77-3.69 (m, 2 H), 3.61-3.53 (m, 2 H), 3.24 (d, 2H, $J$ = 5.6Hz), 1.24 (t, 6 H, $J$ = 7.0 Hz).

To a stirred solution of 3-(2,2-diethoxyethylamino)benzonitrile (0.84 g, 3.59 mmol) in anhydrous DMF (5 mL) at 0°C was added NaH (0.10 g, 4.30 mmol) portionwise. After 20 min, MeI (0.61 g, 4.30 mmol) was added, and the mixture was stirred at room temperature for 6 h. The reaction mixture was cooled to 0°C, and to it, aqueous $NH_4Cl$ (10 mL) solution was added dropwise. The aqueous mixture was extracted with $CHCl_3$ (2 × 30 mL), and the organic phase was dried over anhydrous $Na_2SO_4$, filtered, and evaporated to dryness under reduced pressure. The residue was purified by MPLC on silica gel using a mixture of EtOAc and hexane as eluent to give 3-((2,2-diethoxyethyl)(methyl)amino)benzonitrile (0.65 g, 73%) as a viscous liquid. $^1$H NMR (400 MHz, $CDCl_3$): $\delta$ 7.28-7.24 (m, 1 H), 6.96-6.93 (m, 3 H), 4.60(t, 1 H, $J$ = 5.2 Hz), 3.76-3.69 (m, 2 H), 3.55-3.47 (m, 2 H), 3.46 (d, 2 H, $J$ = 5.2 Hz), 3.02 (s, 3 H), 1.20 (t, 6 H, $J$ = 7.0 Hz).

To a stirred solution of 3-((2,2-diethoxyethyl)(methyl)amino)benzonitrile (0.65 g, 2.61 mmol) in anhydrous dioxane (6 mL) at 0°C was added 1 N HCl (4.30 mmol) dropwise, and the mixture was stirred at room temperature for 1 h. The reaction mixture was cooled to 0°C, neutralized with aqueous $NaHCO_3$ solution, and extracted with $CHCl_3$ (2 × 30 mL). The $CHCl_3$ solution was dried over anhydrous $Na_2SO_4$, filtered, and evaporated to dryness under reduced

pressure to give the titled compound (0.24 g, 52%) as a viscous liquid, which was used for the next step without further purification. $^1$H NMR (400 MHz, $CDCl_3$): δ 9.74 (t, 1 H, *J* = 0.8 Hz), 7.29 (ddd, 1 H, *J* = 8.8, 7.4, 0.8 HZ), 7.02 (ddd, 1 H, *J* = 7.4, 0.8, 1.2 Hz), 6.86 (dd, 1 H, *J* = 2.4, 1.2 Hz), 6.82 (ddd, 1 H, *J* = 8.8, 2.4, 1.2 Hz) 4.14 (d, 2 H, *J* = 0.8 Hz), 3.10 (s, 3 H).

**Preparative Example 27**

Preparation of 2-((2-fluorophenyl)(methyl)amino)acetaldehyde (a compound of the formula (**IX**) wherein $R^b$ = 2-fluoro, X = NMe). This compound was prepared by the following 3 steps started with commercially available 2-fluoroaniline.

A stirred mixture of 2-fluoroaniline (2.00 g, 17.90 mmol), bromoacetaldehyde dimethyl acetal (3.25 mL, 21.40 mmol), and $Cs_2CO_3$ (11.70 g, 35.80 mmol) in anhydrous DMF (10 mL) was heated to 120°C overnight. The reaction mixture was evaporated to dryness under reduced pressure, and the residue was extracted with $Et_2O$ (2 × 150 mL). The $Et_2O$ solution was washed with water (4 × 50 mL) and brine (2 × 50 mL), dried over anhydrous $MgSO_4$, filtered, and evaporated to dryness under reduced pressure. The residue was purified by MPLC on silica gel using a mixture of EtOAc and hexane as eluent to give *N*-(2,2-diethoxyethyl)-2-fluoroaniline (2.00 g, 49%) as a colorless oil. $^1$H NMR (400MHz, $CDCl_3$): δ 7.03-6.95 (m, 2 H), 6.82-6.77 (m, 1 H), 6.71-6.65 (m, 1 H), 4.72 (t, 1 H, *J* = 5.6 Hz), 3.78-3.71 (m, 2 H), 3.62-3.54 (m, 2 H), 3.29 (d, 2 H, *J* = 5.6 Hz), 1.26-1.22 (m, 6 H).

To a stirred solution of *N*-(2,2-diethoxyethyl)-2-fluoroaniline (1.00 g, 4.40 mmol) in anhydrous DMF (10 mL) at 0°C was added NaH (0.16 g, 6.60 mmol) portionwise. After 30 min, MeI (0.5 mL, 8.80 mmol) was added, and the mixture was stirred at room temperature overnight. The reaction mixture was extracted with EtOAc (2 × 100 mL), and the EtOAc solution was washed with water (50 mL) and brine (50 mL), dried over anhydrous $Na_2SO_4$, filtered, and evaporated to dryness under reduced pressure to give *N*-(2,2-diethoxyethyl)-2-fluoro-*N*-methylaniline (0.41 g, 38%) as a colorless oil. $^1$H NMR (400 MHz, $CDCl_3$): δ 7.05-6.96 (m, 3 H), 6.83-6.81 (m, 1 H), 4.69 (t, 1 H, *J* = 5.2 Hz), 3.72-3.64 (m, 2 H), 3.55-3.49 (m, 2 H), 3.33 (dd, 2 H, *J* = 5.2, 1.2 Hz), 2.97 (s, 3 H), 1.19-1.53 (m, 6 H).

To a stirred solution of *N*-(2,2-diethoxyethyl)-2-fluoro-*N*-methylaniline (0.40 g, 1.60 mmol) in 1,4-dioxane (5 mL) at 0°C was added 2.5 N HCl (5 mL) dropwise, and the mixture was stirred at room temperature overnight. The reaction mixture was cooled to 0°C, neutralized with saturated $NaHCO_3$ solution, and extracted with $CHCl_3$ (2 × 50 mL). The $CHCl_3$ solution was washed with water (20 mL) and brine (20 mL), dried over $Na_2SO_4$, filtered, and evaporated to dryness under reduced pressure to give the titled compound (0.27 g, 98%) as a colorless oil, which was used for next step without further purification. $^1$H NMR (400 MHz, $CDCl_3$): δ 9.80 (dd, 1 H, *J* = 2.4, 1.2 Hz), 7.07-6.89 (m, 4 H), 3.91 (dd, 2 H, *J* = 1.8, 0.8 Hz), 2.97 (s, 3 H).

**Practice Example 1**

Preparation of *N*-((4-([1,2,4]triazolo[1,5-*a*]pyridin-6-yl)-5-(6-methylpyridin-2-yl)-1*H*-imidazol-2-yl)methyl)-3-vinylaniline (Example 37)

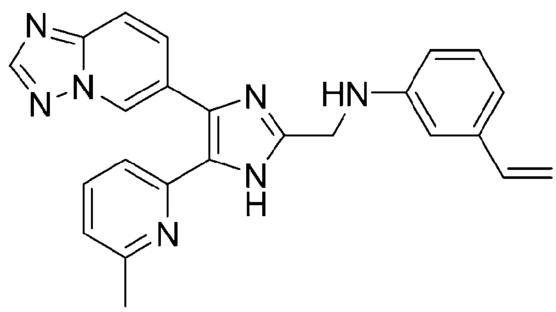

To a stirred solution of 4-([1,2,4]triazolo[1,5-*a*]pyridin-6-yl)-5-(6-methylpyridin-2-yl)-1*H*-imidazole-2-carbaldehyde (4.00 g, 13.14 mmol) in 1,2-dichloroethane (240 mL) were added 3-vinylaniline (2.36 g, 19.71 mmol) and AcOH (0.79 g, 13.14 mmol), and the mixture was heated at 80°C for 2 h. The reaction mixture was cooled to 0°C and, to it, was added $NaBH(OAc)_3$ (5.56 g, 26.20 mmol). The mixture was stirred at 40°C overnight, and then the pH of the reaction mixture was adjusted to 7-8 at 0°C with 10% $K_2CO_3$ solution. The reaction mixture was extracted with 5% MeOH in $CHCl_3$ (2 × 200 mL), and the organic phase was dried over anhydrous $Na_2SO_4$, filtered, and evaporated to dryness under reduced pressure. The residue was purified by MPLC on silica gel using a mixture of MeOH and $CH_2Cl_2$ (1:19 (v/v)) as eluent to give the titled compound (2.89 g, 63%) as a solid. $^1$H NMR (400 MHz, $CDCl_3$) δ 10.59 (br s, 1 H), 8.94

(s, 1 H), 8.37 (s, 1 H), 7.81 (d, 1 H, *J* = 9.2 Hz), 7.77 (d, 1 H, *J* = 9.2 Hz), 7.45 (t, 1 H, *J* = 7.8 Hz), 7.20 (br d, 1 H, overlapped, *J* = 7.6 Hz), 7.15 (t, 1 H, overlapped, *J* = 7.8 Hz), 7.00 (d, 1 H, *J* = 8.0 Hz), 6.86 (d, 1 H, *J* = 7.6 Hz), 6.75 (t, 1 H, *J* = 2.0 Hz), 6.63 (dd, 1 H, overlapped, *J* = 17.6, 10.8 Hz), 6.61 (dd, 1 H, overlapped, *J* = 8.0, 2.0 Hz), 5.69 (dd, 1 H, *J* = 17.6, 0.8 Hz), 5.21 (dd, 1 H, *J* = 10.8, 0.8 Hz), 4.55 (s, 2 H), 4.39 (br s, 1 H), 2.51 (s, 3 H); MS (ESI) *m/z* 408.21 ($MH^+$).

**Practice Example 2**

Preparation of *N*-((4-([1,2,4]triazolo[1,5-*a*]pyridin-6-yl)-5-(6-methylpyridin-2-yl)-1*H*-imidazol-2-yl)methyl)-3-vinylaniline hydrochloride (Example 38)

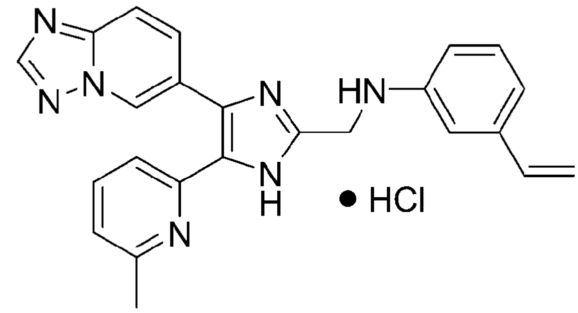

A stirred suspension of *N*-((4-([1,2,4]triazolo[1,5-*a*]pyridin-6-yl)-5-(6-methylpyridin-2-yl)-1*H*-imidazol-2-yl)methyl)-3-vinylaniline (1.00 g, 2.45 mmol) in anhydrous $CHCl_3$ (12 mL) was heated at 50°C to give a clear solution. The $CHCl_3$ solution was cooled to 0°C and, to it, was added 1.0 M HCl in $Et_2O$ (7.36 mL, 7.36 mmol). After 5 min, the precipitates were filtered under $N_2$ and dried thoroughly over $P_2O_5$ *in vacuo* to give the titled compound (1.07 g, 98%) as a yellow powder. $^1$H NMR (400 MHz, DMSO-$d_6$) δ 9.49 (dd, 1 H, *J* = 1.6, 0.8 Hz), 8.65 (s, 1 H), 7.97 (dd, 1 H, *J* = 9.2, 0.8 Hz), 7.86 (dd, 1 H, overlapped, *J* = 9.2, 1.6 Hz), 7.85 (t, 1 H, overlapped, *J* = 7.8 Hz), 7.65 (d, 1 H, *J* = 8.0 Hz), 7.38 (d, 1 H, *J* = 7.6 Hz), 7.12 (t, 1 H, *J* = 7.8 Hz), 6.91 (t, 1 H, *J* = 1.6 Hz), 6.79 (d, 1 H, *J* = 7.6 Hz), 6.71 (dd, 1 H, *J* = 8.0, 1.6 Hz), 6.64 (dd, 1 H, *J* = 17.6, 11.2 Hz), 5.80 (dd, 1 H, *J* = 17.6, 0.8 Hz), 5.20 (dd, 1 H, *J* = 11.2, 0.8 Hz), 4.79 (s, 2 H), 2.51 (s, 3 H).

**Practice Example 3**

Preparation of *N*-((4-([1,2,4]triazolo[1,5-*a*]pyridin-6-yl)-5-(6-methylpyridin-2-yl)-1*H*-imidazol-2-yl)methyl)-3-vinylaniline sulfate (Example 39)

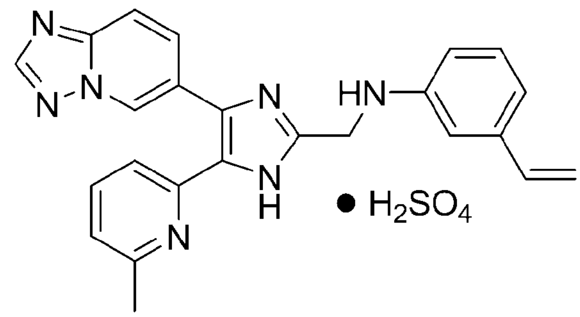

To a stirred suspension of *N*-((4-([1,2,4]triazolo[1,5-*a*]pyridin-6-yl)-5-(6-methylpyridin-2-yl)-1*H*-imidazol-2-yl)methyl)-3-vinylaniline (100 mg, 0.25 mmol) in anhydrous EtOH (2 mL) at 0°C was added 10% $H_2SO_4$ in anhydrous EtOH (0.20 mL, 0.37 mmol). The mixture was allowed to warm to room temperature and stirred for 10 min. The reaction mixture was diluted with anhydrous $Et_2O$ (8 mL) and stirred for an additional 10 min. The precipitates were filtered under $N_2$, washed with anhydrous $Et_2O$ (4 × 4 mL), and then dried thoroughly over $P_2O_5$ *in vacuo* to give the titled compound (79 mg, 64%) as a yellow solid. $^1$H NMR (400 MHz, DMSO-$d_6$) δ 9.40 (dd, 1 H, *J* = 1.6, 0.8 Hz), 8.63 (s, 1 H), 7.98 (dd, 1 H, *J* = 9.2, 0.8 Hz), 7.84 (t, 1 H, *J* = 8.0 Hz), 7.76 (dd, 1 H, *J* = 9.2, 1.6 Hz), 7.43 (d, 1 H, *J* = 7.6 Hz), 7.40 (d, 1 H, *J* =

8.0 Hz), 7.13 (t, 1 H, *J* = 7.8 Hz), 6.80 (br s, 1 H), 6.79 (d, 1 H, overlapped, *J* = 7.6 Hz), 6.64 (dd, 1 H, overlapped, *J* = 17.6, 11.2 Hz), 6.63 (dd, 1 H, overlapped, *J* = 7.6, 2.0 Hz), 5.74 (dd, 1 H, *J* = 17.6, 0.8 Hz), 5.21 (dd, 1 H, *J* = 11.2, 0.8 Hz), 4.68 (s, 2 H), 2.58 (s, 3 H).

**Practice Example 4**

Preparation of 3-((4-([1,2,4]triazolo[1,5-a]pyridin-6-yl)-5-(6-methylpyridin-2-yl)-1*H*-imidazol-2-yl)methylamino)-4-((dimethylamino)methyl)benzonitrile (Example 116)

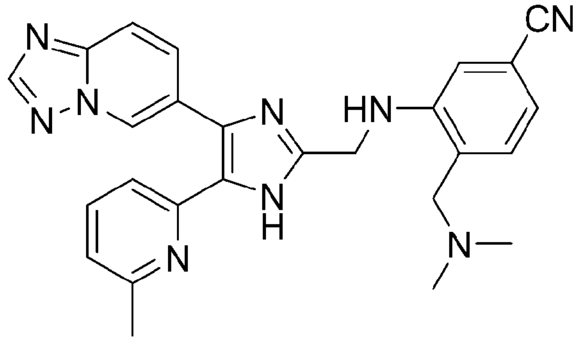

To a stirred solution of 4-((1,2,4)triazolo(1,5-*a*)pyridin-6-yl)-5-(6-methylpyridin-2-yl)-1*H*-imidazole-2-carbaldehyde (0.50 g, 1.64 mmol) in 1,2-dichloroethane (30 mL) were added 3-amino-4-((dimethylamino)methyl)benzonitrile (0.43 g, 2.46 mmol) and AcOH (0.20 g, 3.29 mmol), and the mixture was heated at 80°C overnight. The reaction mixture was concentrated under reduced pressure, and the residue was dissolved in anhydrous MeOH (30 mL). To a methanolic solution at 0°C was added $NaBH_4$ (0.25 g, 6.57 mmol), and then the mixture was allowed to warm to room temperature and stirred for an additional 3 h. The pH of the reaction mixture was adjusted to 7-8 at 0°C with 1 N HCl, and then MeOH was removed under reduced pressure. The aqueous mixture was extracted with $CH_2Cl_2$ (2 × 50 mL), and the $CH_2Cl_2$ solution was dried over anhydrous $Na_2SO_4$, filtered, and evaporated to dryness under reduced pressure. The residue was purified by MPLC on silica gel using a mixture of MeOH and $CH_2Cl_2$ (1:19 (v/v)) as eluent to give the titled compound (0.60 g, 79%) as a white solid. $^1H$ NMR (400 MHz, $CDCl_3$) δ 8.99 (s, 1 H), 8.36 (s, 1 H), 7.86 (dd, 1 H, *J* = 9.2, 1.6 Hz), 7.78 (dd, 1 H, *J* = 9.2, 0.8 Hz), 7.47 (t, 1 H, *J* = 7.6 Hz), 7.23 (br d, 1 H, *J* = 7.6 Hz), 7.09 (d, 1 H, *J* = 7.6 Hz), 7.01 (d, 1 H, *J* = 7.6 Hz), 6.98 (dd, 1 H, *J* = 7.6, 1.6 Hz), 6.93 (br s, 1 H), 4.59 (s, 2 H), 3.63 (s, 2 H), 2.53 (s, 3 H), 2.33 (s, 6 H); MS (ESI) *m/z* 464.23 ($MH^+$).

The compounds listed in the following Table 1 were prepared in an analogous manner to those described in the Practice Examples 1-4 above. The mass spectroscopy data of these compounds are included in the Table 1.

**Table 1**

| **Example** | **Structure** | **$^1$H NMR** (ppm) | **MS (ESI) m/z ($MH^+$)** |
|---|---|---|---|
| **1** | 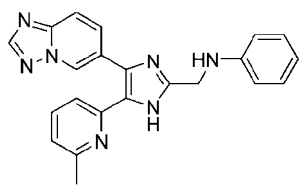 | (400 MHz, $CDCl_3$) δ 10.43 (br s, 1 H), 8.96 (s, 1 H), 8.37 (s, 1 H), 7.82 (dd, 1 H, *J* = 9.2, 1.6 Hz), 7.77 (dd, 1 H, *J* = 9.2, 0.8 Hz), 7.45 (t, 1 H, *J* = 7.6 Hz), 7.25-7.19 (m, 3 H), 7.01 (d, 1 H, *J* = 7.6 Hz), 6.80 (tt, 1 H, *J* = 8.0, 1.2 Hz), 6.74-6.72 (m, 2 H), 4.55 (s, 2 H), 2.53 (s, 3 H) | 382.19 |

(continued)

| Example | Structure | $^1$H NMR (ppm) | MS (ESI) m/z (MH$^+$) |
|---|---|---|---|
| **2** | 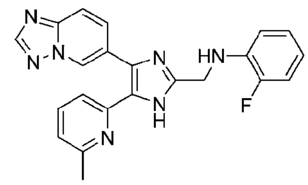 | (400 MHz, $CDCl_3$) δ 11.34 (br s, 1 H), 8.96 (dd, 1 H, *J* = 1.6, 0.8 Hz), 8.35 (s, 1 H), 7.81 (dd, 1 H, *J* = 9.2, 1.6 Hz), 7.74 (dd, 1 H, *J* = 9.2, 0.8 Hz), 7.45 (t, 1 H, *J* = 7.6 Hz), 7.23 (d, 1 H, *J* = 7.6 Hz), 6.97-6.90 (m, 2 H), 6.94 (dd, 1 H, *J* = 8.0, 1.2 Hz), 6.72 (td, 1 H, *J* = 8.4, 1.6 Hz), 6.69-6.63 (m, 1 H), 4.51 (s, 2 H), 2.35 (s, 3 H). | 400.18 |
| **3** | 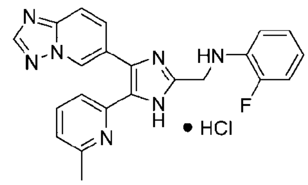 • HCl | (400 MHz, DMSO-$d_6$) δ 9.44 (d, 1 H, *J* = 0.8 Hz), 8.62 (s, 1 H), 7.96 (dd, 1 H, *J* = 9.2, 0.8 Hz), 7.83 (t, 1 H, *J* = 8.0 Hz), 7.80 (dd, 1 H, *J* = 9.2, 1.6 Hz), 7.53 (d, 1 H, *J* = 8.0 Hz), 7.38 (d, 1 H, *J* = 7.6 Hz), 7.10 (ddd, 1 H, *J* = 12.0, 8.0, 1.2 Hz), 7.01 (td, 1 H, *J* = 7.6, 1.2 Hz), 6.88 (br t, 1 H, *J* = 8.6 Hz), 6.70-6.64 (m, 1 H), 4.75 (s, 2 H), 2.54 (s, 3 H) | |
| **4** | 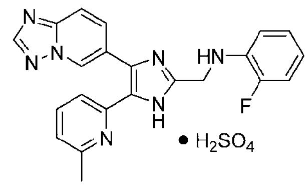 • $H_2SO_4$ | (400 MHz, DMSO-$d_6$) δ 9.40 (dd, 1 H, *J* = 2.0, 0.8 Hz), 8.64 (s, 1 H), 7.99 (dd, 1 H, *J* = 9.2, 0.8 Hz), 7.85 (t, 1 H, *J* = 8.0 Hz), 7.77 (dd, 1 H, *J* = 9.2, 2.0 Hz), 7.42 (pseudo t, 2 H, *J* = 7.4 Hz), 7.11 (ddd, 1 H, *J* = 12.0, 8.0, 1.2 Hz), 7.02 (td, 1 H, *J* = 7.6, 1.2 Hz), 6.82 (td, 1 H, *J* = 8.0, 1.2 Hz), 6.71-6.65 (m, 1 H), 4.73 (s, 2 H), 2.59 (s, 3 H) | |
| **5** | 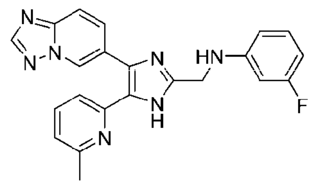 | (400 MHz, $CDCl_3$) δ 8.94 (t, 1 H, *J* = 1.4 Hz), 8.36 (s, 1 H), 7.79 (dd, 1 H, *J* = 9.2, 1.6 Hz), 7.75 (dd, 1 H, *J* = 9.2, 0.8 Hz), 7.46 (t, 1 H, *J* = 7.8 Hz), 7.23 (d, 1 H, *J* = 8.0 Hz), 7.13-= 7.8 Hz), 7.23 7.01 1 H, *J* = 8.0 Hz), 7.13-7.07 (m, 1 H), 7.01 (d, 1 H, *J* = 7.6 Hz), 6.47-6.41 (m, 2 H), 6.37 (dt, 1 H, *J* = 8.8, 2.4 Hz), 4.49 (s, 2 H), 2.49 (s, 3 H) | 400.19 |
| **6** | 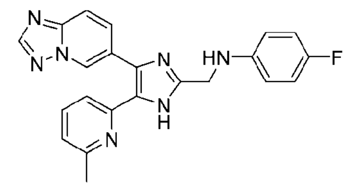 | (400 MHz, $CDCl_3$) δ 8.95 (dd, 1 H, *J* = 1.6, 1.2 Hz), 8.37 (s, 1 H), 7.80 (dd, 1 H, *J* = 9.2, 1.6 Hz), 7.76 (dd, 1 H, *J* = 9.2, 1.2 Hz), 7.46 (t, 1 H, *J* = 7.6 Hz), 7.23 (d, 1 H, *J* = 7.6 Hz), 7.01 (d, 1 H, *J* = 7.6 Hz), 6.92-6.88 (m, 2 H), 6.65-6.62 (m, 2 H), 4.49 (s, 2 H), 2.51 (s, 3 H) | 400.19 |
| **7** | 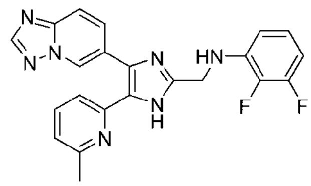 | (400 MHz, $CDCl_3$) δ 8.96 (br s, 1 H), 8.38 (s, 1 H), 7.82 (dd, 1 H, *J* = 9.2, 1.6 Hz), 7.78 (dd, 1 H, *J* = 9.2, 0.8 Hz), 7.47 (t, 1 H, *J* = 7.6 Hz), 7.24 (d, 1 H, *J* = 7.6 Hz), 7.02 (dd, 1 H, *J* = 7.6, 0.4 Hz), 6.94-6.88 (m, 1 H), 6.60-6.51 (m, 2 H), 4.71 (br s, 1 H), 4.58 (d, 2 H, *J* = 3.6 Hz), 2.51 (s, 3 H) | 418.18 |
| **8** | 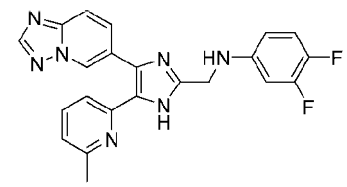 | (400 MHz, $CDCl_3$) δ 8.94 (t, 1 H, *J* = 1.4 Hz), 8.38 (s, 1 H), 7.81 (dd, 1 H, overlapped, *J* = 9.2, 1.6 Hz), 7.78 (dd, 1 H, overlapped, *J* = 9.2, 1.2 Hz), 7.47 (t, 1 H, *J* = 7.8 Hz), 7.24 (d, 1 H, *J* = 8.0 Hz), 7.03 (d, 1 H, *J* = 7.6 Hz), 7.04-6.95 (m, 1 H), 6.52 (ddd, 1 H, *J* = 12.4, 6.8, 2.8 Hz), 6.42-6.37 (m, 1 H), 4.48 (s, 2 H), 2.55 (s, 3 H) | 418.18 |

(continued)

| Example | Structure | $^1$H NMR (ppm) | MS (ESI) m/z (MH$^+$) |
|---|---|---|---|
| **9** | 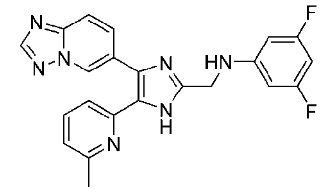 | (400 MHz, $CDCl_3$) δ 8.94 (t, 1 H, *J* = 1.2 Hz), 8.38 (s, 1 H), 7.81 (dd, 1 H, overlapped, *J* = 9.2, 1.6 Hz), 7.79 (dd, 1 H, overlapped, *J* = 9.2, 1.2 Hz), 7.47 (t, 1 H, *J* = 7.8 Hz), 7.24 (d, 1 H, *J* = 8.0 Hz), 7.03 (d, 1 H, *J* = 7.6 Hz), 6.24-6.19 (m, 3 H), 4.71 (br s, 1 H), 4.50 (s, 2 H), 2.55 (s, 3 H) | 418.18 |
| **10** | 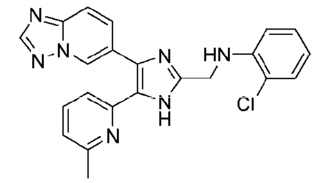 | (400 MHz, $CDCl_3$) δ 8.97 (br s, 1 H), 8.37 (s, 1 H), 7.82 (dd, 1 H, *J* = 9.2, 1.6 Hz), 7.77 (dd, 1 H, *J* = 9.2, 1.0 Hz), 7.46 (t, 1 H, *J* = 7.6 Hz), 7.29 (dd, 1 H, *J* = 7.6, 1.6 Hz), 7.23 (br d, 1 H, *J* = 7.6 Hz), 7.14 (td, 1 H, *J* = 8.4, 1.6 Hz), 7.01 (dd, 1 H, *J* = 7.6, 0.4 Hz), 6.75 (dd, 1 H, *J* = 8.8, 1.4 Hz), 6.72 (td, 1 H, *J* = 8.4, 1.6 Hz), 5.01 (br s, 1 H), 4.60 (br s, 2 H), 2.50 (s, 3 H) | 416.16 |
| **11** | 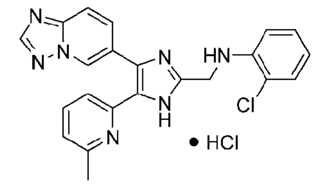 • HCl | (400 MHz, DMSO-$d_6$) δ 9.43 (s, 1 H), 8.61 (s, 1 H), 7.95 (dd, 1 H, *J* = 9.2, 0.8 Hz), 7.82 (t, 1 H, overlapped, *J* = 7.8 Hz), 7.81 (dd, 1 H, overlapped, *J* = 9.2, 2.0 Hz), 7.49 (d, 1 H, *J* = 8.0 Hz), 7.36 (d, 1 H, *J* = 7.6 Hz), 7.32 (dd, 1 H, *J* = 7.8, 1.4 Hz), 7.17 (td, 1 H, *J* = 8.4, 1.2 Hz), 6.87 (dd, 1 H, *J* = 8.4. 1.2 Hz), 6.69 (td, 1 H, *J* = 7.8, 1.4 Hz), 6.15 (br s, 1 H), 4.76 (s, 2 H), 2.55 (s, 3 H) | |
| **12** | 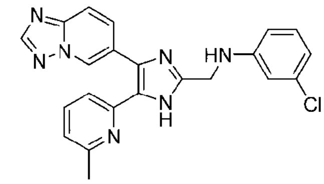 | (400 MHz, $CDCl_3$) δ 8.95 (t, 1 H, *J* = 1.6 Hz), 8.37 (s, 1 H), 7.81 (dd, 1 H, *J* = 9.2, 1.6 Hz), 7.77 (dd, 1 H, *J* = 9.2, 1.2 Hz), 7.47 (t, 1 H, *J* = 7.8 Hz), 7.24 (d, 1 H, *J* = 8.0 Hz), 7.10 (t, 1 H, *J* = 8.0 Hz), 7.02 (d, 1 H, *J* = 7.6 Hz), 6.75 (ddd, 1 H, *J* = 8.0, 2.0, 0.8 Hz), 6.69 (t, 1 H, *J* = 2.0 Hz), 6.57 (ddd, 1 H, *J* = 8.0, 2.4, 0.8 Hz), 4.51 (s, 2 H), 2.52 (s, 3 H) | 416.16 |
| **13** | 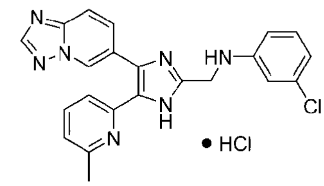 • HCl | (400 MHz, DMSO-$d_6$) δ 9.45 (dd, 1 H, *J* = 1.6, 0.8 Hz), 8.63 (s, 1 H), 7.97 (dd, 1 H, *J* = 9.2, 0.8 Hz), 7.85 (t, 1 H, *J* = 8.0 Hz), 7.81 (dd, 1 H, *J* = 9.2, 1.6 Hz), 7.56 (d, 1 H, *J* = 8.4 Hz), 7.39 (d, 1 H, *J* = 7.6 Hz), 7.15 (t, 1 H, *J* = 8.0 Hz), 6.80 (t, 1 H, *J* = 2.2 Hz), 6.71-6.65 (m, 2 H), 4.71 (s, 2 H), 2.54 (s, 3 H) | |
| **14** | 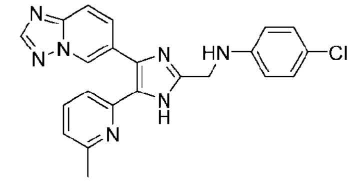 | (400 MHz, $CDCl_3$) δ 8.95 (t, 1 H, *J* = 1.4 Hz), 8.38 (s, 1 H), 7.81 (dd, 1 H, overlapped, *J* = 9.2, 1.4 Hz), 7.78 (dd, 1 H, overlapped, *J* = 9.2, 1.2 Hz), 7.47 (t, 1 H, *J* = 7.6 Hz), 7.23 (br d, 1 H, *J* = 7.6 Hz), 7.16 (m, 2 H), 7.02 (br d, 1 H, *J* = 7.6 Hz), 6.65 (m, 2 H), 4.51 (s, 2 H), 2.54 (s, 3 H) | 416.16 |

(continued)

| Example | Structure | $^1$H NMR (ppm) | MS (ESI) m/z (MH+) |
|---|---|---|---|
| **15** | 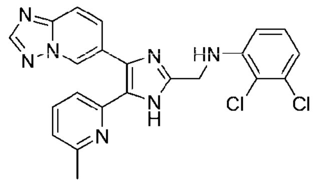 | (400 MHz, $CDCl_3$) δ 11.02 (br s, 1 H), 8.97 (s, 1 H), 8.36 (s, 1 H), 7.81 (dd, 1 H, *J* = 9.2, 1.6 Hz), 7.75 (dd, 1 H, *J* = 9.2, 0.8 = 8.0 7.46 (t, 1 H, *J* = 8.0 Hz), 7.24 (d, 1 H, *J* = 8.0 Hz), 7.03 (t, 1 H, overlapped, *J* = 8.0 Hz), 7.00 (d, 1 H, overlapped, *J* = 8.0 Hz), 6.85 (dd, 1 H, *J* = 8.0, 1.6 Hz), 6.63 (dd, 1 H, *J* = 8.0, 1.6 Hz), 5.15 (t, 1 H, *J* = 5.6 Hz), 4.57 (d, 2 H, *J* = 5.6 Hz), 2.43 (s, 3 H) | 450.12 |
| **16** | 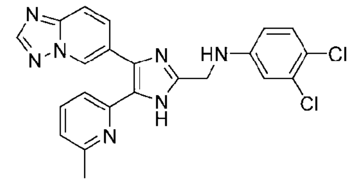 | (400 MHz, $CDCl_3$) δ 8.94 (t, 1 H, *J* = 1.2 Hz), 8.37 (s, 1 H), 7.80 (dd, 1 H, overlapped, *J* = 9.2, 1.6 Hz), 7.77 (dd, 1 H, overlapped, *J* = 9.2, 1.2 Hz), 7.47 (t, 1 H, *J* = 8.0 Hz), 7.24 (br d, 1 H, *J* = 8.0 Hz), 7.20 (d, 1 H, *J* = 8.8 Hz), 7.03 (d, 1 H, *J* = 8.0 Hz), 6.77 (d, 1 H, *J* = 2.8 Hz), 6.53 (dd, 1 H, *J* = 8.8, 2.8 Hz), 4.47 (s, 2 H), 2.51 (s, 3 H) | 450.12 |
| **17** | 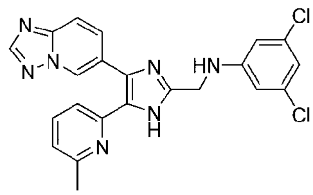 | (400 MHz, $CDCl_3/CD_3OD$) δ 8.90 (s, 1 H), 8.24 (s, 1 H), 7.71 (dd, 1 H, *J* = 9.2, 1.6 Hz), 7.64 (dd, 1 H, *J* = 9.2, 0.8 Hz), 7.45 (t, 1 H, *J* = 7.6 Hz), 7.16 (br d, 1 H, *J* = 7.6 Hz), 6.99 (d, 1 H, *J* = 7.6 Hz), 6.57 (t, 1 H, *J* = 1.6 Hz), 6.51 (d, 2 H, *J* = 1.6 Hz), 4.36 (s, 2 H), 2.45 (s, 3 H) | 450.12 |
| **18** | 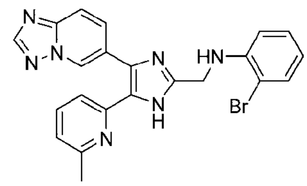 | (400 MHz, $CDCl_3$) δ 10.94 (br s, 1 H), 8.97 (br s, 1 H), 8.36 (s, 1 H), 7.82 (d, 1 H, *J* = 9.2 Hz), 7.75 (d, 1 H, *J* = 9.2 Hz), 7.45 (t, 1 H, overlapped, *J* = 7.8 Hz), 7.43 (dd, 1 H, *J* = 8.0, 1.6 Hz), 7.21 (d, 1 H, *J* = 8.0 Hz), 7.16 (td, 1 H, *J* = 8.4, 1.2 Hz), 6.99 (d, 1 H, *J* = 7.6 Hz), 6.71 (dd, 1 H, *J* = 8.4, 1.2 Hz), 6.63 (td, 1 H, *J* = 8.0, 1.6 Hz), 4.99 (t, 1 H, *J* = 5.6 Hz), 4.57 (d, 2 H, *J* = 5.6 Hz), 2.44 (s, 3 H) | 460.11 |
| **19** | 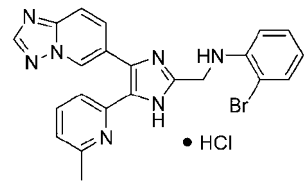 • HCl | (400 MHz, DMSO-$d_6$) δ 9.43 (dd, 1 H, *J* = 1.6, 0.8 Hz), 8.62 (s, 1 H), 7.96 (dd, 1 H, *J* = 9.2, 0.8 Hz), 7.83 (t, 1 H, *J* = 7.6 Hz), 7.80 (dd, 1 H, *J* = 9.2, 1.6 Hz), 7.50 (d, 1 H, *J* = 7.6 Hz), 7.48 (dd, 1 H, *J* = 8.0, 1.6 Hz), 7.37 (d, 1 H, *J* = 7.6 Hz), 7.21 (td, 1 H, *J* = 7.6, 1.6 Hz), 6.87 (dd, 1 H, *J* = 8.0, 1.2 Hz), 6.63 (td, 1 H, *J* = 7.6, 1.2 Hz), 6.04 (br s 1 H), 4.78 (s, 2 H), 2.55 (s, 3 H) | |
| **20** | 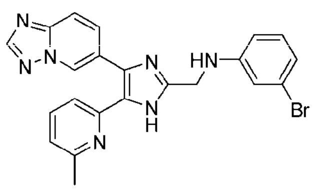 | (400 MHz, $CDCl_3$) δ 8.89 (br s, 1 H), 8.30 (s, 1 H), 7.75 (dd, 1 H, *J* = 9.2, 1.6 Hz), 7.69 (d, 1 H, *J* = 9.2 Hz), 7.44 (br t, 1 H, *J* = 7.6 Hz), 7.12 (br d, 1 H, *J* = 7.6 Hz), 7.00 (d, 1 H, *J* = 8.0 Hz), 6.97 (t, 1 H, overlapped, *J* = 8.0 Hz), 6.82-6.78 (m, 2 H), 6.58 (ddd, 1 H, *J* = 8.2, 2.4, 0.8 Hz), 4.41 (s, 2 H), 2.49 (s, 3 H) | 460.11 |

(continued)

| Example | Structure | $^{1}$H NMR (ppm) | MS (ESI) m/z ($MH^+$) |
|---|---|---|---|
| **21** | 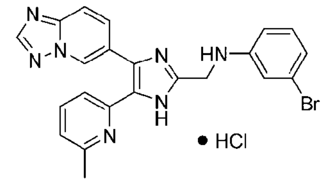 • HCl | (400 MHz, DMSO-$d_6$) δ 9.44 (d, 1 H, *J* = 0.8 Hz), 8.61 (s, 1 H), 7.95 (dd, 1 H, *J* = 9.2, 0.8 Hz), 7.83 (t, 1 H, *J* = 7.8 Hz), 7.82 (dd, 1 H, *J* = 9.2, 1.6 Hz), 7.50 (d, 1 H, *J* = 8.0 Hz), 7.37 (d, 1 H, *J* = 7.6 Hz), 7.08 (t, 1 H, *J* = 8.0 Hz), 6.94 (t, 1 H, *J* = 2.0 Hz), 6.79 (ddd, 1 H, *J* = 7.6, 2.0, 0.8 Hz), 6.71 (ddd, 1 H, *J* = 8.4, 2.0, 0.8 Hz), 4.63 (s, 2 H), 2.55 (s, 3 H) | |
| **22** | 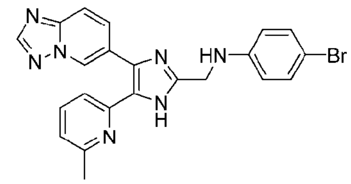 | (400 MHz, $CDCl_3$) δ 8.94 (br s, 1 H), 8.38 (s, 1 H), 7.82-7.77 (m, 2 H), 7.46 (t, 1 H, *J* = 7.6 Hz), 7.29 (m, 2 H), 7.21 (d, 1 H, *J* = 7.6 Hz), 7.02 (d, 1 H, *J* = 7.6 Hz), 6.61 (m, 2 H), 4.51 (s, 2 H), 4.44 (br s, 1 H), 2.54 (s, 3 H) | 460.11 |
| **23** | 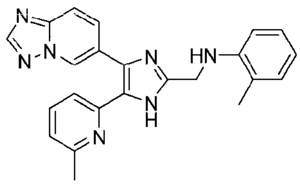 | (400 MHz, $CDCl_3$) δ 8.97 (s, 1 H), 8.37 (s, 1 H), 7.83 (dd, 1 H, *J* = 9.2, 1.6 Hz), 7.78 (dd, 1 H, *J* = 9.2, 0.8 Hz), 7.46 (t, 1 H, *J* = 7.8 Hz), 7.23 (br d, 1 H, *J* = 7.6 Hz), 7.14 (t, 1 H, overlapped, *J* = 8.0 Hz), 7.12 (d, 1 H, overlapped, *J* = 7.6 Hz), 7.01 (d, 1 H, *J* = 8.0 Hz), 6.76 (td, 1 H, *J* =7.6, 0.8 Hz), 6.69 (d, 1 H, *J* = 7.6 Hz), 4.61 (s, 2 H), 2.54 (s, 3 H), 2.26 (s, 3 H) | 396.21 |
| **24** | 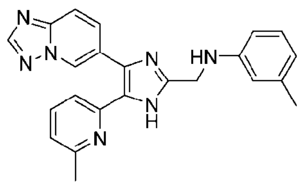 | (400 MHz, $CDCl_3$) δ 8.96 (dd, 1 H, *J* = 1.6, 1.2 Hz), 8.37 (s, 1 H), 7.82 (dd, 1 H, *J* = 9.2, 1.6 Hz), 7.78 (dd, 1 H, *J* = 9.2, 1.2 Hz), 7.46 (t, 1 H, *J* = 7.8 Hz), 7.22 (br d, 1 H, *J* = 8.0 Hz), 7.11 (t, 1 H, *J* = 7.8 Hz), 7.01 (d, 1 H, *J* = 7.6 Hz), 6.63 (dt, 1 H, *J* = 7.6, 0.8 Hz), 6.57-6.53 (m, 2 H), 4.55 (s, 2 H), 2.54 (s, 3 H), 2.29 (s, 3 H) | 396.21 |
| **25** | 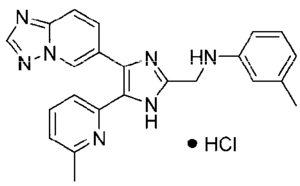 • HCl | (400 MHz, DMSO-$d_6$) δ 9.45 (dd, 1 H, *J* = 1.6, 0.8 Hz), 8.63 (s, 1 H), 7.97 (dd, 1 H, *J* = 9.2, 0.8 Hz), 7.83 (t, 1 H, *J* = 8.0 Hz), 7.81 (dd, 1 H, *J* = 9.2, 1.6 Hz), 7.56 (d, 1 H, *J* = 8.0 Hz), 7.37 (d, 1 H, *J* = 8.0 Hz), 7.02 (t, 1 H, *J* = 7.8 Hz), 6.60 (s, 1 H), 6.54 (dd, 1 H, *J* = 8.0, 2.0 Hz), 6.49 (d, 1 H, *J* = 7.6 Hz), 4.69 (s, 2 H), 2.52 (s, 3 H), 2.21 (s, 3 H) | |
| **26** | 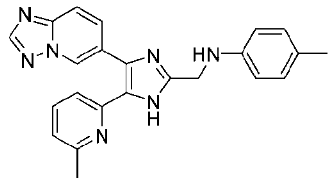 | (400 MHz, $CDCl_3$) δ 8.96 (t, 1 H, *J* = 1.2 Hz), 8.37 (s, 1 H), 7.82 (d d, 1 H, *J* = 9.2, 1.6 Hz), 7.77 (dd, 1 H, *J* = 9.2, 0.8 Hz), 7.45 (t, 1 H, *J* = 7.8 Hz), 7.22 (br d, 1 H, *J* = 8.0 Hz), 7.03 (m, 2 H), 7.02 (d, 1 H, overlapped, *J* = 7.6 Hz), 6.65 (m, 2 H), 4.53 (s, 2 H), 2.53 (s, 3 H), 2.25 (s, 3 H) | 396.21 |
| **27** | 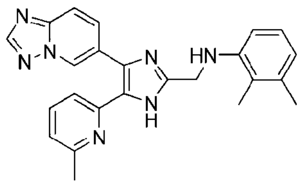 | (400 MHz, $CDCl_3$) δ 8.97 (s, 1 H), 8.37 (s, 1 H), 7.83 (dd, 1 H, *J* = 9.2, 1.6 Hz), 7.78 (dd, 1 H, *J* = 9.2, 0.8 Hz), 7.45 (t, 1 H, *J* = 7.8 Hz), 7.22 (br d, 1 H, *J* = 7.6 Hz), 7.03 (t, 1 H, overlapped, *J* = 7.8 Hz), 7.02 (d, 1 H, overlapped, *J* = 8.0 Hz), 6.69 (d, 1 H, *J* = 7.6 Hz), 6.58 (d, 1 H, *J* = 8.0 Hz), 4.59 (s, 2 H), 2.54 (s, 3 H), 2.31 (s, 3 H), 2.17 (s, 3 H) | 410.23 |

(continued)

| Example | Structure | $^1$H NMR (ppm) | MS (ESI) m/z (MH+) |
|---|---|---|---|
| **28** | 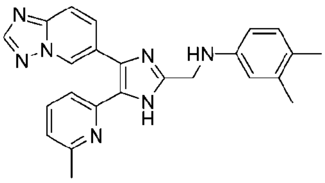 | (400 MHz, $CDCl_3$) δ 8.96 (d, 1 H, *J* = 1.2 Hz), 8.37 (s, 1 H), 7.82 (dd, 1 H, *J* = 9.2, 1.6 Hz), 7.77 (dd, 1 H, *J* = 9.2, 0.8 Hz), 7.45 (t, 1 H, *J* = 7.6 Hz), 7.22 (br d, 1 H, *J* = 8.0 Hz), 7.00 (d, 1 H, *J* = 7.6 Hz), 6.98 (d, 1 H, *J* = 8.0 Hz), 6.57 (d, 1 H, *J* = 2.4 Hz), 6.50 (dd, 1 H, *J* = 8.0, 2.4 Hz), 4.53 (s, 2 H), 2.54 (s, 3 H), 2.20 (s, 3 H), 2.16 (s, 3 H) | 410.23 |
| **29** | 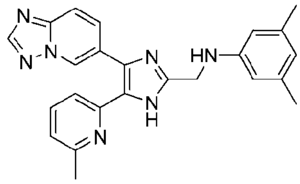 | (400 MHz, $CDCl_3$) δ 8.95 (dd, 1 H, *J* = 1.6, 1.2 Hz), 8.36 (s, 1 H), 7.79 (dd, 1 H, *J* = 9.2, 1.6 Hz), 7.74 (dd, 1 H, *J* = 9.2, 1.2 Hz), 7.45 (t, 1 H, *J* = 7.8 Hz), 7.22 (d, 1 H, *J* = 8.0 Hz), 6.99 (d, 1 H, *J* = 7.6 Hz), 6.44 (br s, 1 H), 6.31 (s, 2 H), 4.49 (s, 2 H), 2.48 (s, 3 H), 2.21 (s, 6 H) | 410.23 |
| **30** | 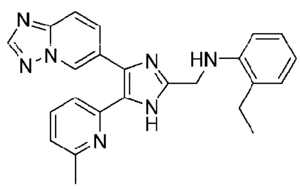 | (400 MHz, $CDCl_3$) δ 8.97 (br s, 1 H), 8.37 (s, 1 H), 7.83 (dd, 1 H, *J* = 9.2, 1.6 Hz), 7.78 (dd, 1 H, *J* = 9.2, 0.8 Hz), 7.46 (t, 1 H, *J* = 7.8 Hz), 7.23 (br d, 1 H, *J* = 7.6 Hz), 7.16-7.11 (m, 2 H), 7.01 (d, 1 H, *J* = 7.6 Hz), 6.80 (td, 1 H, *J* = 7.6, 1.2 Hz), 6.71 (dd, 1 H, *J* = 8.4, 1.2 Hz), 4.60 (s, 2 H), 2.60 (q, 2 H, *J* = 7.6 Hz), 2.52 (s, 3 H), 1.32 (t, 3 H, *J* = 7.6 Hz) | 410.23 |
| **31** | 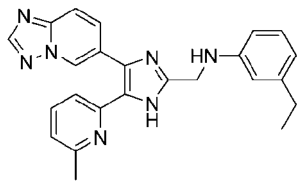 | (400 MHz, $CDCl_3$) δ 8.96 (d, 1 H, *J* = 1.2 Hz), 8.37 (s, 1 H), 7.82 (dd, 1 H, *J* = 9.2, 1.6 Hz), 7.77 (dd, 1 H, *J* = 9.2, 1.2 Hz), 7.45 (t, 1 H, *J* = 7.8 Hz), 7.22 (br d, 1 H, *J* = 8.0 Hz), 7.14 (t, 1 H, *J* = 7.8 Hz), 7.01 (d, 1 H, *J* = 7.6 Hz), 6.66 (dd, 1 H, *J* = 7.6, 0.8 Hz), 6.58 (d, 1 H, *J* = 2.0 Hz), 6.55 (dd, 1 H, *J* = 7.6, 2.0 Hz), 4.55 (s, 2 H), 2.58 (q, 2 H, *J* = 7.6 Hz), 2.53 (s, 3 H), 1.21 (t, 3 H, *J* = 7.6 Hz) | 410.23 |
| **32** | 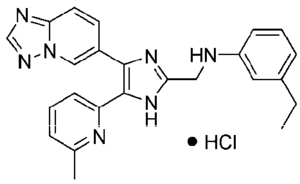 • HCl | (400 MHz, DMSO-$d_6$) δ 9.45 (dd, 1 H, *J* = 1.6, 0.8 Hz), 8.63 (s, 1 H), 7.97 (dd, 1 H, *J* = 9.2, 0.8 Hz), 7.83 (t, 1 H, *J* = 8.0 Hz), 7.81 (dd, 1 H, *J* = 9.2, 1.6 Hz), 7.57 (d, 1 H, *J* = 8.0 Hz), 7.37 (d, 1 H, *J* = 8.0 Hz), 7.05 (t, 1 H, *J* = 7.6 Hz), 6.64 (d, 1 H, *J* = 1.6 Hz), 6.57-6.52 (m, 2 H), 4.71 (s, 2 H), 2.53 (s, 3 H), 2.50 (q, 2 H, *J* = 7.6 Hz), 1.14 (t, 3 H, *J* = 7.6 Hz) | |
| **33** | 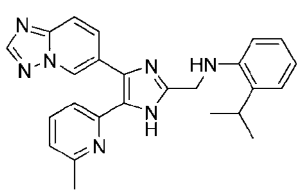 | (400 MHz, $CDCl_3$) δ 8.97 (br s, 1 H), 8.37 (s, 1 H), 7.82 (dd, 1 H, *J* = 9.2, 1.6 Hz), 7.76 (dd, 1 H, *J* = 9.2, 0.8 Hz), 7.45 (t, 1 H, *J* = 7.8 Hz), 7.23 (br d, 1 H, overlapped, *J* = 8.0 Hz), 7.21 (dd, 1 H, overlapped, *J* = 7.6, 1.6 Hz), 7.12 (td, 1 H, *J* = 7.6, 1.6 Hz), 7.00 (d, 1 H, *J* = 7.6 Hz), 6.83 (td, 1 H, *J* = 7.6, 1.2 Hz), 6.71 (dd, 1 H, *J* = 8.0, 1.2 Hz), 4.58 (s, 2 H), 3.00 (heptet, 1 H, *J* = 6.8 Hz), 2.50 (s, 3 H), 1.31 (d, 6 H, *J* = 6.8 Hz) | 424.24 |

(continued)

| Example | Structure | $^1$H NMR (ppm) | MS (ESI) m/z (MH$^+$) |
|---|---|---|---|
| **34** | 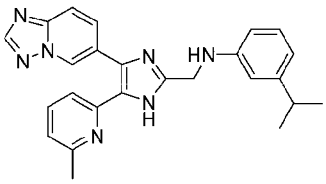 | (400 MHz, $CDCl_3$) δ 8.96 (dd, 1 H, *J* = 1.4, 0.8 Hz), 8.35 (s, 1 H), 7.79 (dd, 1 H, *J* = 9.2, 1.4 Hz), 7.73 (dd, 1 H, *J* = 9.2, 0.8 Hz), 7.44 (t, 1 H, *J* = 7.8 Hz), 7.21 (d, 1 H, *J* = 7.6 Hz), 7.11 (t, 1 H, *J* = 7.8 Hz), 6.99 (dd, 1 H, *J* = 7.8, 0.4 Hz), 6.67 (d, 1 H, *J* = 7.6 Hz), 6.57 (t, 1 H, *J* = 2.4 Hz), 6.50 (ddd, 1 H, *J* = 8.0, 2.4, 0.8 Hz), 4.51 (s, 2 H), 2.80 (heptet, 1 H, *J* = 6.8 Hz), 2.47 (s, 3 H), 1.20 (d, 6 H, *J* = 6.8 Hz) | 424.24 |
| **35** | 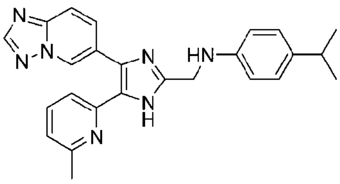 | (400 MHz, $CDCl_3$) δ 8.96 (t, 1 H, *J* = 1.2 Hz), 8.36 (s, 1 H), 7.81 (dd, 1 H, *J* = 9.2, 1.6 Hz), 7.75 (d, 1 H, *J* = 9.2 Hz), 7.44 (t, 1 H, *J* = 7.8 Hz), 7.21 (d, 1 H, *J* = 8.0 Hz), 7.06 (m, 2 H), 6.99 (d, 1 H, *J* = 7.6 Hz), 6.64 (m, 2 H), 4.50 (s, 2 H), 2.80 (heptet, 1 H, *J* = 6.8 Hz), 2.48 (s, 3 H), 1.19 (d, 6 H, *J* = 6.8 Hz) | 424.24 |
| **36** | 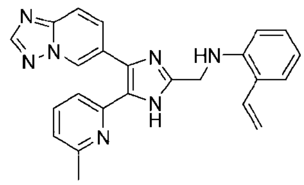 | (400 MHz, $CDCl_3$) δ 8.96 (br s, 1 H), 8.35 (s, 1 H), 7.80 (dd, 1 H, *J* = 9.2, 1.6 Hz), 7.73 (dd, 1 H, *J* = 9.2, 0.8 Hz), 7.44 (t, 1 H, *J* = 7.8 Hz), 7.28 (dd, 1 H, *J* = 7.6, 1.2 Hz), 7.22 (br d, 1 H, *J* = 8.0 Hz), 7.15 (td, 1 H, *J* = 7.8, 1.2 Hz), 6.99 (d, 1 H, *J* = 7.6 Hz), 6.79 (dd, 1 H, overlapped, *J* = 17.2, 11.2 Hz), 6.78 (td, 1 H, overlapped, *J* = 7.6, 0.8 Hz), 6.68 (dd, 1 H, *J* = 8.2, 1.0 Hz), 5.62 (dd, 1 H, *J* = 17.2, 1.4 Hz), 5.34 (dd, 1 H, *J* = 11.2, 1.4 Hz), 4.53 (s, 2 H), 2.45 (s, 3 H) | 408.21 |
| **37** | 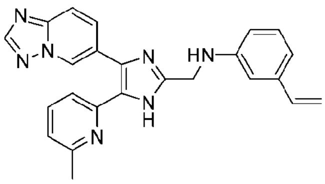 | (400 MHz, $CDCl_3$) δ 10.59 (br s, 1 H), 8.94 (s, 1 H), 8.37 (s, 1 H), 7.81 (d, 1 H, *J* = 9.2 Hz), 7.77 (d, 1 H, *J* = 9.2 Hz), 7.45 (t, 1 H, *J* = 7.8 Hz), 7.20 (br d, 1 H, overlapped, *J* = 7.6 Hz), 7.15 (t, 1 H, overlapped, *J* = 7.8 Hz), 7.00 (d, 1 H, *J* = 8.0 Hz), 6.86 (d, 1 H, *J* = 7.6 Hz), 6.75 (t, 1 H, *J* = 2.0 Hz), 6.63 (dd, 1 H, overlapped, *J* = 17.6, 10.8 Hz), 6.61 (dd, 1 H, overlapped, *J* = 8.0, 2.0 Hz), 5.69 (dd, 1 H, *J* = 17.6, 0.8 Hz), 5.21 (dd, 1 H, *J* = 10.8, 0.8 Hz), 4.55 (s, 2 H), 4.39 (br s, 1 H), 2.51 (s, 3 H) | 408.21 |
| **38** | 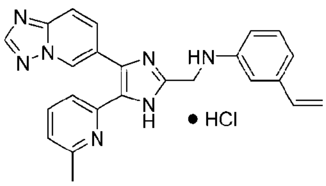 | (400 MHz, DMSO-$d_6$) δ 9.49 (dd, 1 H, *J* = 1.6, 0.8 Hz), 8.65 (s, 1 H), 7.97 (dd, 1 H, *J* = 9.2, 0.8 Hz), 7.86 (dd, 1 H, overlapped, *J* = 9.2, 1.6 Hz), 7.85 (t, 1 H, overlapped, *J* = 7.8 Hz), 7.65 (d, 1 H, *J* = 8.0 Hz), 7.38 (d, 1 H, *J* = 7.6 Hz), 7.12 (t, 1 H, *J* = 7.8 Hz), 6.91 (t, 1 H, *J* = 1.6 Hz), 6.79 (d, 1 H, *J* = 7.6 Hz), 6.71 (dd, 1 H, *J* = 8.0, 1.6 Hz), 6.64 (dd, 1 H, *J* = 17.6, 11.2 Hz), 5.80 (dd, 1 H, *J* = 17.6, 0.8 Hz), 5.20 (dd, 1 H, *J* = 11.2, 0.8 Hz), 4.79 (s, 2 H), 2.51 (s, 3 H) | |

(continued)

| Example | Structure | $^1$H NMR (ppm) | MS (ESI) m/z (MH$^+$) |
|---|---|---|---|
| **39** | 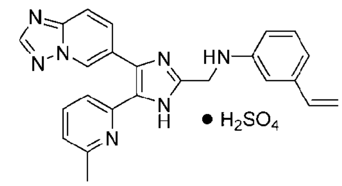 | (400 MHz, DMSO-$d_6$) δ 9.40 (dd, 1 H, *J* = 1.6, 0.8 Hz), 8.63 (s, 1 H), 7.98 (dd, 1 H, *J* = 9.2, 0.8 Hz), 7.84 (t, 1 H, *J* = 8.0 Hz), 7.76 (dd, 1 H, *J* = 9.2, 1.6 Hz), 7.43 (d, 1 H, *J* = 7.6 Hz), 7.40 (d, 1 H, *J* = 8.0 Hz), 7.13 (t, 1 H, *J* = 7.8 Hz), 6.80 (br s, 1 H), 6.79 (d, 1 H, overlapped, *J* = 7.6 Hz), 6.64 (dd, 1 H, overlapped, *J* = 17.6, 11.2 Hz), 6.63 (dd, 1 H, overlapped, *J* = 7.6, 2.0 Hz), 5.74 (dd, 1 H, *J* = 17.6, 0.8 Hz), 5.21 (dd, 1 H, *J* = 11.2, 0.8 Hz), 4.68 (s, 2 H), 2.58 (s, 3 H) | |
| **40** | 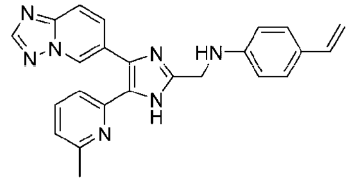 | (400 MHz, $CDCl_3$) δ 10.39 (br s, 1 H), 8.96 (s, 1 H), 8.37 (s, 1 H), 7.82 (dd, 1 H, *J* = 9.2, 1.6 Hz), 7.78 (dd, 1 H, *J* = 9.2, 0.8 Hz), 7.45 (t, 1 H, *J* = 7.6 Hz), 7.28 (m, 2 H), 7.22 (br d, 1 H, *J* = 7.6 Hz), 7.01 (d, 1 H, *J* = 7.6 Hz), 6.69 (m, 2 H), 6.61 (dd, 1 H, *J* = 17.6, 10.8 Hz), 5.55 (dd, 1 H, *J* = 17.6, 0.8 Hz), 5.05 (dd, 1 H, *J* = 10.8, 0.8 Hz), 4.56, (s, 2 H), 2.53 (s, 3 H) | 408.21 |
| **41** | 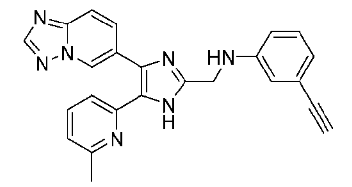 | (400 MHz, $CDCl_3$) δ 8.94 (t, 1 H, *J* = 1.2 Hz), 8.35 (s, 1 H), 7.77 (dd, 1 H, *J* = 9.2, 1.2 Hz), 7.72 (d, 1 H, *J* = 9.2 Hz), 7.46 (t, 1 H, *J* = 7.8 Hz), 7.23 (d, 1 H, *J* = 8.0 Hz), 7.07 (t, 1 H, *J* = 7.8 Hz), 6.99 (d, 1 H, *J* = 7.6 Hz), 6.86 (d, 1 H, *J* = 7.6 Hz), 6.73 (br s, 1 H), 6.61 (br d, 1 H, *J* = 8.0 Hz), 4.45 (s, 2 H), 2.98 (s, 1 H), 2.42 (s, 3 H) | 406.18 |
| **42** | 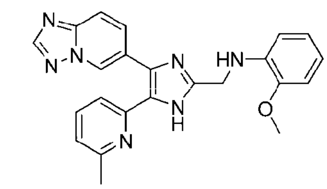 | (400 MHz, $CDCl_3$) δ 8.98 (br s, 1 H), 8.37 (s, 1 H), 7.83 (dd, 1 H, *J* = 9.2, 1.6 Hz), 7.78 (dd, 1 H, *J* = 9.2, 0.8 Hz), 7.45 (t, 1 H, *J* = 7.6 Hz), 7.22 (br d, 1 H, *J* = 7.6 Hz), 7.00 (d, 1 H, *J* = 7.6 Hz), 6.87 (td, 1 H, *J* = 7.6, 1.6 Hz), 6.84 (dd, 1 H, *J* = 8.0, 1.6 Hz), 6.77 (td, 1 H, *J* = 7.8, 1.6 Hz), 6.69 (dd, 1 H, *J* = 7.8, 1.6 Hz), 4.58 (s, 2 H), 3.91 (s, 3 H), 2.53 (s, 3 H) | 412.21 |
| **43** | 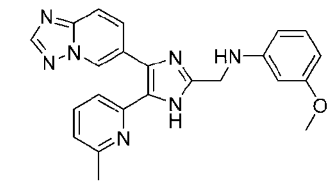 | (400 MHz, $CDCl_3$) δ 8.95 (br s, 1 H), 8.37 (s, 1 H), 7.81 (dd, 1 H, *J* = 9.2, 1.6 Hz), 7.76 (dd, 1 H, *J* = 9.2, 0.8 Hz), 7.45 (t, 1 H, *J* = 7.6 Hz), 7.22 (d, 1 H, *J* = 7.6 Hz), 7.11 (t, 1 H, *J* = 8.0 Hz), 7.01 (d, 1 H, *J* = 7.6 Hz), 6.36-6.31 (m, 2 H), 6.27 (t, 1 H, *J* = 2.4 Hz), 4.52 (s, 2 H), 3.75 (s, 3 H), 2.51 (s, 3 H) | 412.21 |
| **44** | 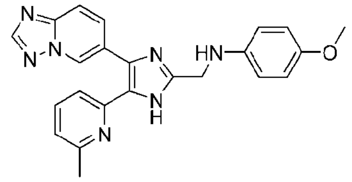 | (400 MHz, $CDCl_3$) δ 8.96 (br s, 1 H), 8.37 (s, 1 H), 7.82 (dd, 1 H, *J* = 9.2, 1.6 Hz), 7.77 (dd, 1 H, *J* = 9.2, 0.8 Hz), 7.45 (t, 1 H, *J* = 7.8 Hz), 7.22 (br d, 1 H, *J* = 7.6 Hz), 7.01 (br d, 1 H, *J* = 8.0 Hz), 6.80 (m, 2 H), 6.70 (m, 2 H), 4.50 (s, 2 H), 3.74 (s, 3 H), 2.53 (s, 3 H) | 412.21 |

(continued)

| Example | Structure | $^1$H NMR (ppm) | MS (ESI) m/z (MH$^+$) |
|---|---|---|---|
| **45** | 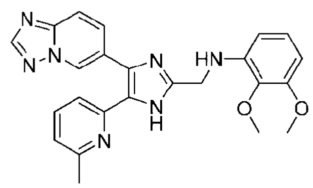 | (400 MHz, $CDCl_3$) δ 8.96 (br s, 1 H), 8.34 (s, 1 H), 7.80 (dd, 1 H, *J* = 9.2, 1.6 Hz), 7.72 (d, 1 H, *J* = 9.2 Hz), 7.42 (t, 1 H, *J* = 7.6 Hz), 7.20 (br d, 1 H, *J* = 7.6 Hz), 6.96 (d, 1 H, *J* = 7.6 Hz), 6.88 (td, 1 H, *J* = 8.2, 0.4 Hz), 6.36 (d, 1 H, overlapped, *J* = 8.0 Hz), 6.34 (d, 1 H, overlapped, *J* = 8.2 Hz), 4.52 (s, 2 H), 3.84 (s, 3 H), 3.81 (s, 3 H), 2.43 (s, 3 H) | 442.22 |
| **46** | 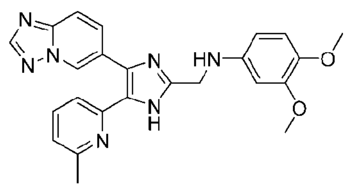 | (400 MHz, $CDCl_3$) δ 8.94 (dd, 1 H, *J* = 1.6, 0.8 Hz), 8.34 (s, 1 H), 7.78 (dd, 1 H, *J* = 9.2, 1.6 Hz), 7.72 (dd, 1 H, *J* = 9.2, 0.8 Hz), 7.44 (t, 1 H, *J* = 8.0 Hz), 7.21 (br d, 1 H, *J* = 8.0 Hz), 6.99 (d, 1 H, *J* = 8.0 Hz), 6.71 (d, 1 H, *J* = 8.4 Hz), 6.30 (d, 1 H, *J* = 2.4 Hz), 6.19 (dd, 1 H, *J* = 8.4, 2.4 Hz), 4.46 (s, 2 H), 3.78 (s, 3 H), 3.76 (s, 3 H), 2.47 (s, 3 H) | 442.22 |
| **47** | 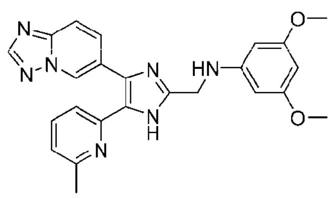 | (400 MHz, $CDCl_3$) δ 10.43 (br s, 1 H), 8.94 (s, 1 H), 8.37 (s, 1 H), 7.81 (d, 1 H, *J* = 9.2 Hz), 7.77 (d, 1 H, *J* = 9.2 Hz), 7.45 (t, 1 H, *J* = 7.6 H), 7.20 (d, 1 H, *J* = 7.6 Hz), 7.01 (d, 1 H, *J* = 7.6 Hz), 5.96 (t, 1 H, *J* = 2.0 Hz), 5.92 (d, 2 H, *J* = 2.0 Hz), 4.52 (d, 2 H, *J* = 2.8 Hz), 4.41 (br s, 1 H), 3.75 (s, 6 H), 2.54 (s, 3 H) | 442.22 |
| **48** | 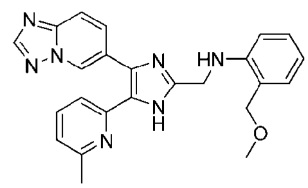 | (400 MHz, $CDCl_3$) δ 8.97 (br s, 1 H), 8.37 (s, 1 H), 7.83 (dd, 1 H, *J* = 9.2, 1.6 Hz), 7.77 (dd, 1 H, *J* = 9.2, 0.8 Hz), 7.45 (t, 1 H, *J* = 7.6 Hz), 7.23 (td, 1 H, overlapped, *J* = 7.6, 1.6 Hz), 7.22 (br d, 1 H, overlapped, *J* = 7.6 Hz) 7.12 (dd, 1 H, *J* = 7.6, 1.6 Hz), 7.01 (d, 1 H, *J* = 8.0 Hz), 6.77-6.73 (m, 2 H), 4.63 (s, 2 H), 4.60 (s, 2 H), 3.42 (s, 3 H), 2.53 (s, 3 H) | 426.22 |
| **49** | 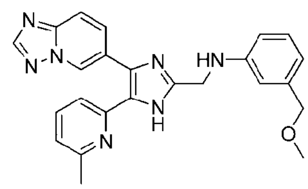 | (400 MHz, $CDCl_3$) δ 8.96 (br s, 1 H), 8.37 (s, 1 H), 7.82 (dd, 1 H, *J* = 9.2, 1.6 Hz), 7.78 (dd, 1 H, *J* = 9.2, 0.8 Hz), 7.45 (t, 1 H, *J* = 7.6 Hz), 7.21 (t, 1 H, overlapped, *J* = 8.0 Hz), 7.20 (br d, 1 H, overlapped, *J* = 7.6 Hz), 7.01 (d, 1 H, *J* = 7.6 Hz), 6.76 (d, 1 H, overlapped, *J* = 8.0 Hz), 6.75 (d, 1 H, overlapped, *J* = 1.6 Hz), 6.67-6.64 (m, 1 H), 4.56 (s. 2 H), 4.40 (s, 2 H), 3.38 (s, 3 H), 2.54 (s, 3 H) | 426.22 |
| **50** | 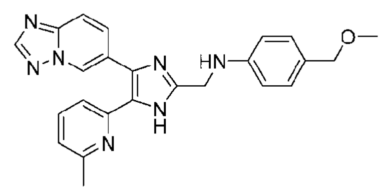 | (400 MHz, $CDCl_3$) δ 10.36 (br s, 1 H), 8.95 (s, 1 H), 8.37 (s, 1 H), 7.82 (br d, 1 H, *J* = 9.2 Hz), 7.78 (br d, 1 H, *J* = 9.2 Hz), 7.45 (t, 1 H, *J* = 7.6 Hz), 7.20 (br d, 1 H, overlapped, *J* = 7.6 Hz), 7.19 (d, 2 H, overlapped, *J* = 8.4 Hz), 7.01 (d, 1 H, *J* = 7.6 Hz), 6.71 (d, 2 H, *J* = 8.4 Hz), 4.55 (s, 2 H), 4.40 (br s, 1 H), 4.34 (s, 2 H), 3.34 (s, 3 H), 2.53 (s, 3 H) | 426.22 |

(continued)

| Example | Structure | $^1$H NMR (ppm) | MS (ESI) m/z (MH$^+$) |
|---|---|---|---|
| **51** | 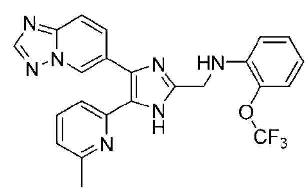 | (400 MHz, $CDCl_3$) δ 10.42 (br s, 1 H), 8.96 (br s, 1 H), 8.37 (s, 1 H), 7.82 (d, 1 H, *J* = 9.2 Hz), 7.78 (d, 1 H, *J* = 9.2 Hz), 7.45 (t, 1 H, *J* = 7.8 Hz), 7.25-7.18 (m, 2 H), 7.15 (td, 1 H, *J* = 7.8, 1.2 Hz), 7.01 (d, 1 H, *J* = 7.6 Hz), 6.81 (dd, 1 H, *J* = 8.0, 1.2 Hz), 6.76 (td, 1 H, *J* = 7.8, 1.6 Hz), 4.85 (t, 1 H, *J* = 5.6 Hz), 4.61 (d, 2 H, *J* = 5.6 Hz), 2.52 (s, 3 H) | 466.18 |
| **52** | 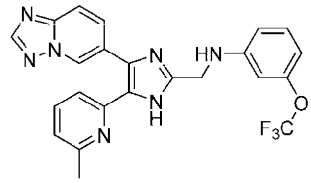 | (400 MHz, $CDCl_3$) δ 8.94 (br s, 1 H), 8.37 (s, 1 H), 7.80 (dd, 1 H, *J* = 9.2, 1.6 Hz), 7.77 (dd, 1 H, *J* = 9.2, 0.8 Hz), 7.46 (t, 1 H, *J* = 7.8 Hz), 7.23 (br d, 1 H, *J* = 8.0 Hz), 7.19 (t, 1 H, *J* = 8.2 Hz), 7.02 (d, 1 H, *J* = 7.6 Hz), 6.64-6.60 (m, 2 H), 6.55 (br s, 1 H), 4.62 (br s, 1 H), 4.52 (s, 2 H), 2.52 (s, 3 H) | 466.18 |
| **53** | 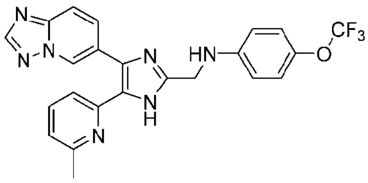 | (400 MHz, $CDCl_3$) δ 8.95 (s, 1 H), 8.38 (s, 1 H), 7.81 (dd 1 H, *J* = 9.2, 1.6 Hz), 7.78 (dd, 1 H, *J* = 9.2, 0.8 Hz), 7.46 (t, 1 H, *J* = 7.8 Hz), 7.23 (d, 1 H, *J* = 8.0 Hz), 7.08 (m, 2 H), 7.02 (d, 1 H, *J* = 7.6 Hz), 6.70 (m, 2 H), 4.53 (s, 2 H), 2.54 (s, 3 H) | 466.18 |
| **54** | 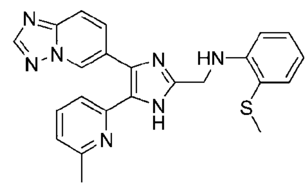 | (400 MHz, $CDCl_3$) δ 11.34 (br s, 1 H), 8.99 (s, 1 H), 8.34 (s, 1 H), 7.83 (br d, 1 H, *J* = 9.2 Hz), 7.72 (br d, 1 H, *J* = 9.2 Hz), 7.43 (t, 1 H, *J* = 7.8 Hz), 7.35 (dd, 1 H, J = 7.6, 1.6 Hz), 7.21 (br d, 1 H, *J* = 7.6 Hz), 7.12 (td, 1 H, *J* = 7.8, 1.6 Hz), 6.95 (d, 1 H, *J* = 8.0 Hz), 6.70 (td, 1 H, *J* = 7.6, 1.2 Hz), 6.65 (dd, 1 H, *J* = 8.0, 1.2 Hz), 5.49 (br t, 1 H, *J* = 4.8 Hz), 4.55 (d, 2 H, *J* = 4.8 Hz), 2.33 (s, 3 H), 2.32 (s, 3 H) | 428.18 |
| **55** | 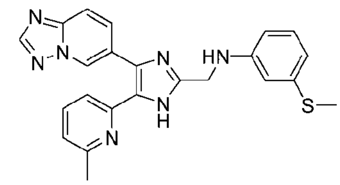 | (400 MHz, $CDCl_3$) δ 11.06 (br s, 1 H), 8.94 (s, 1 H), 8.35 (s, 1 H), 7.78 (dd, 1 H, *J* = 9.2, 1.2 Hz), 7.74 (d, 1 H, *J* = 9.2 Hz), 7.45 (t, 1 H, *J* = 7.8 Hz), 7.21 (br d, 1 H, *J* = 8.0 Hz), 7.07 (t, 1 H, *J* = 7.8 Hz), 6.99 (d, 1 H, *J* = 7.6 Hz), 6.64 (dd, 1 H, *J* = 7.6, 2.0 Hz), 6.54 (t, 1 H, *J* = 1.6 Hz), 6.42 (dd, 1 H, *J* = 8.0, 1.6 Hz), 4.47 (s, 2 H) 4.42 (br s, 1 H), 2.45 (s, 3 H), 2.40 (s, 3 H) | 428.19 |
| **56** | 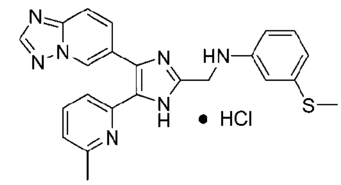 | (400 MHz, DMSO-$d_6$) δ 9.43 (br s, 1 H), 8.62 (s, 1 H), 7.97 (dd, 1 H, *J* = 9.2, 0.8 Hz), 7.84 (t, 1 H, *J* = 7.6 Hz), 7.80 (dd, 1 H, *J* = 9.2, 1.6 Hz), 7.51 (d, 1 H, *J* = 7.6 Hz), 7.38 (d, 1 H, *J* = 7.6 Hz), 7.08 (t, 1 H, *J* = 7.8 Hz), 6.62 (t, 1 H, *J* = 2.0 Hz), 6.55 (d, 1 H, *J* = 7.6 Hz), 6.51 (dd, 1 H, *J* = 8.0, 2.0 Hz), 4.68 (s, 2 H), 2.55 (s, 3 H), 2.41 (s, 3 H) | |

(continued)

| **Example** | **Structure** | **$^1$H NMR** (ppm) | **MS (ESI) m/z ($MH^+$)** |
|---|---|---|---|
| **57** | 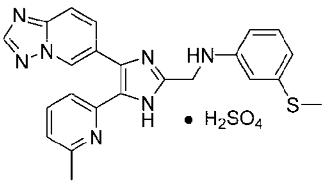 • $H_2SO_4$ | (400 MHz, DMSO-$d_5$) δ 9.40 (dd, 1 H, *J* = 1.6, 0.8 Hz), 8.63 (s, 1 H), 7.98 (d, 1 H, *J* = 9.6 Hz), 7.84 (t, 1 H, *J* = 7.6 Hz), 7.77 (dd, 1 H, *J* = 9.6, 1.6 Hz), 7.43 (d, 1 H, *J* = 7.6 Hz), 7.40 (d, 1 H, *J* = 7.6 Hz), 7.08 (t, 1 H, *J* = 8.0 Hz), 6.60 (t, 1 H, *J* = 2.0 Hz), 6.56 (d, 1 H, *J* = 8.0 Hz), 6.48 (dd, 1 H, *J* = 8.0, 2.0 Hz), 4.65 (s, 2 H), 2.58 (s, 3 H), 2.40 (s, 3 H) | |
| **58** | 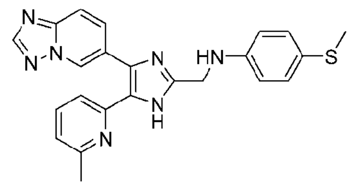 | (400 MHz, $CDCl_3$) δ 11.08 (br s, 1 H), 8.94 (s, 1 H), 8.35 (s, 1 H), 7.78 (dd, 1 H, *J* = 9.2, 1.6 Hz), 7.73 (d, 1 H, *J* = 9.2 Hz), 7.45 (t, 1 H, *J* = 7.8 Hz), 7.21 (br d, 1 H, *J* = 8.0 Hz), 7.17 (m, 2 H), 6.99 (d, 1 H, *J* = 7.6 Hz), 6.60 (m, 2 H), 4.46 (s, 2 H), 2.45 (s, 3 H), 2.37 (s, 3 H) | 428.18 |
| **59** | 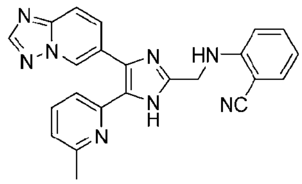 | (400 MHz, $CDCl_3$) δ 8.98 (br s, 1 H), 8.36 (s, 1 H), 7.83 (dd, 1 H, *J* = 9.2, 1.6 Hz), 7.76 (dd, 1 H, *J* = 9.2, 0.8 Hz), 7.47 (t, 1 H, *J* = 7.8 Hz), 7.40 (br d, 1 H, overlapped, *J* = 7.6 Hz), 7.38 (t, 1 H, *J* = 7.8 Hz), 7.25 (br d, 1 H, *J* = 8.0 Hz), 7.00 (d, 1 H, *J* = 7.6), 6.80 (d, 1 H, *J* = 8.4 Hz), 6.75 (td, 1 H, *J* = 8.0, 0.8 Hz), 5.32 (br t, 1 H, *J* = 5.6 Hz), 4.62 (d, 2 H, *J* = 5.6 Hz), 2.41 (s, 3 H) | 407.19 |
| **60** | 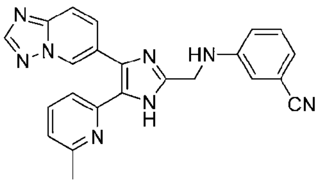 | (400 MHz, $CDCl_3$) δ 8.94 (t, 1 H, *J* = 1.2 Hz), 8.38 (s, 1 H), 7.80 (dd, 1 H, *J* = 9.2, 0.8 Hz), 7.77 (dd, 1 H, *J* = 9.2, 1.6 Hz), 7.55 (t, 1 H, *J* = 8.0 Hz), 7.31 (d, 1 H, *J* = 8.0 Hz), 7.25 (t, 1 H, *J* = 8.0 Hz), 7.08 (d, 1 H, *J* = 8.0 Hz), 7.02 (dt, 1 H, *J* = 7.6, 1.2 Hz), 6.96-6.92 (m, 2 H), 4.56 (s, 2 H), 2.62 (s, 3 H) | 407.19 |
| **61** | 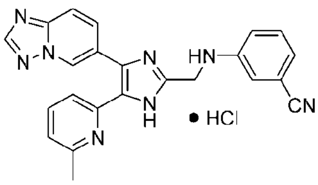 • HCl | (400 MHz, DMSO-$d_6$) δ 9.47 (d, 1 H, *J* = 0.8 Hz), 8.66 (s, 1 H), 7.97 (dd, 1 H, *J* = 9.2, 0.8 Hz), 7.87 (t, 1 H, overlapped, *J* = 7.8 Hz), 7.85 (dd, 1 H, overlapped, *J* = 9.2, 1.6 Hz), 7.63 (d, 1 H, *J* = 7.6 Hz), 7.40 (d, 1 H, *J* = 8.0 Hz), 7.33 (t, 1 H, *J* = 7.8 Hz), 7.16 (t, 1 H, *J* = 1.6 Hz), 7.11 (dd, 1 H, *J* = 8.4, 1.6 Hz), 7.06 (br d, 1 H, *J* = 7.6 Hz), 4.79 (s, 2 H), 2.53 (s, 3 H) | |
| **62** | 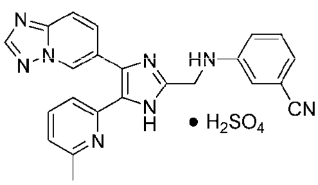 • $H_2SO_4$ | (400 MHz, DMSO-$d_5$) δ 9.39 (br s, 1 H), 8.64 (s, 1 H), 7.99 (d, 1 H, *J* = 9.2 Hz), 7.87 (t, 1 H, *J* = 7.6 Hz), 7.77 (dd, 1 H, *J* = 9.2, 1.6 Hz), 7.45 (d, 1 H, overlapped, *J* = 7.6 H), 7.44 (d, 1 H, overlapped, *J* = 7.6 Hz), 7.34 (t, 1 H, *J* = 8.0 Hz), 7.10 (d, 1 H, *J* = 2.0 Hz), 7.07 (d, 1 H, overlapped, *J* = 7.6 Hz), 7.04 (dd, 1 H, overlapped, *J* = 8.0, 2.0 Hz), 4.70 (s, 2 H), 2.60 (s, 3 H) | |

(continued)

| Example | Structure | $^1$H NMR (ppm) | MS (ESI) m/z (MH$^+$) |
|---|---|---|---|
| **63** | 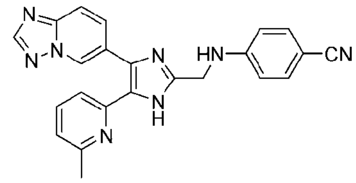 | (400 MHz, $CDCl_3$) δ 8.93 (t, 1 H, *J* = 1.2 Hz), 8.37 (s, 1 H), 7.79 (dd, 1 H, overlapped, *J* = 9.2, 1.6 Hz), 7.76 (dd, 1 H, overlapped, *J* = 9.2, 0.8 Hz), 7.48 (t, 1 H, *J* = 7.8 Hz), 7.43 (m, 2 H), 7.24 (d, 1 H, *J* = 8.0 Hz), 7.03 (d, 1 H, *J* = 7.6 Hz), 6.68 (m, 2 H), 5.12 (br s, 1 H), 4.54 (d, 2 H, *J* = 4.0 Hz), 2.51 (s, 3 H) | 407.19 |
| **64** | 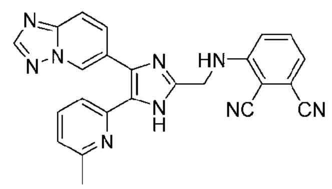 | (400 MHz, $CDCl_3$) δ 8.99 (br s, 1 H), 8.35 (s, 1 H), 7.84 (dd, 1 H, *J* = 9.2, 1.6 Hz), 7.74 (dd, 1 H, *J* = 9.2, 0.8 Hz), 7.50 (t, 1 H, *J* = 7.8 Hz), 7.37 (t, 1 H, *J* = 8.2 Hz), 7.27 (br d, 1 H, *J* = 7.6 Hz), 7.05 (d, 1 H, *J* = 8.8 Hz), 7.01 (d, 1 H, overlapped, *J* = 8.0 Hz), 6.98 (d, 1 H, overlapped, *J* = 7.6 Hz), 5.94 (br t, 1 H, *J* = 5.6 Hz), 4.66 (d, 2 H, *J* = 5.6 Hz), 2.30 (s, 3 H) | 432.19 |
| **65** | 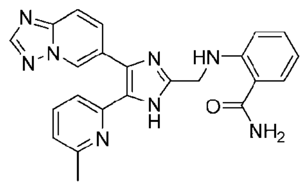 | (400 MHz, $CDCl_3$) δ 9.04 (dd, 1 H, *J* = 1.6, 0.8 Hz), 8.48 (br s, 1 H), 8.33 (s, 1 H), 7.84 (dd, 1 H, *J* = 9.2, 1.6 Hz), 7.72 (d, 1 H, *J* = 9.2 Hz), 7.41 (t, 1 H, *J* = 7.8 Hz), 7.37 (dd, 1 H, *J* = 7.6, 1.2 Hz), 7.29 (td, 1 H, *J* = 8.4, 1.2 Hz), 7.21 (br d, 1 H, *J* = 8.0 Hz), 6.92 (d, 1 H, *J* = 7.6 Hz), 6.83 (d, 1 H, *J* = 8.4 Hz), 6.62 (td, 1 H, *J* = 8.0, 1.0 Hz), 6.25 (br s, 2 H), 4.59 (d, 2 H, *J* = 5.2 Hz), 2.32 (s, 3 H) | 425.20 |
| **66** | 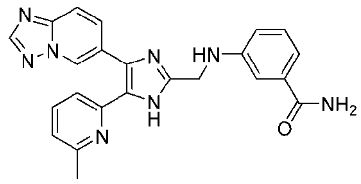 | (400 MHz, $CD_3OD$) δ 9.06 (br s, 1 H), 8.34 (s, 1 H), 7.83 (br d, 1 H, *J* = 9.2 Hz), 7.71 (d, 1 H, *J* = 9.2 Hz), 7.59-7.55 (m, 2 H), 7.22-7.18 (m, 2 H), 7.15 (dt, 1 H, *J* = 7.6, 2.0 Hz), 7.10 (dd, 1 H, *J* = 8.4, 0.8 Hz), 6.86 (ddd, 1 H, *J* = 7.6, 2.0, 0.8 Hz), 4.51 (s, 2 H), 2.50 (s, 3 H) | 425.20 |
| **67** | 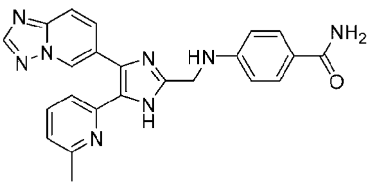 | (400 MHz, $CDCl_3/CD_3OD$) δ 9.03 (br s, 1 H), 8.32 (s, 1 H), 7.81 (dd, 1 H, *J* = 9.2, 1.6 Hz), 7.70 (dd, 1 H, *J* = 9.2, 0.8 Hz), 7.67 (m, 2 H), 7.55 *(t, 1 H, J* = 7.6 Hz), 7.26 (br d, 1 H, *J* = 7.6 Hz), 7.09 (d, 1 H, *J* = 7.6 Hz), 6.71 (m, 2 H), 4.52 (s, 2 H), 2.51 (s, 3 H) | 425.20 |
| **68** | 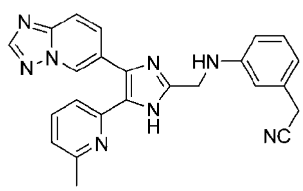 | (400 MHz, $CDCl_3$) δ 10.76 (br s, 1 H), 8.94 (br s, 1 H), 8.37 (s, 1 H), 7.81 (br d, 1 H, *J* = 9.2 Hz), 7.76 (br d, 1 H, *J* = 9.2 Hz), 7.46 (t, 1 H, *J* = 7.6 Hz), 7.21 (br d, 1 H, *J* = 7.6 Hz), 7.20-7.15 (m, 1 H), 7.01 (d, 1 H, *J* = 7.6 Hz), 6.69 (dd, 1 H, *J* = 7.6, 0.8 Hz), 6.62-6.40 (m, 2 H), 4.53 (br s, 1 H, overlapped), 4.52 (br s, 2 H, overlapped), 3.65 (s, 2 H), 2.50 (s, 3 H) | 421.21 |

(continued)

| Example | Structure | $^1$H NMR (ppm) | MS (ESI) m/z (MH$^+$) |
|---|---|---|---|
| **69** | 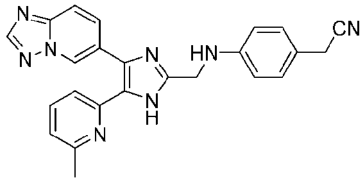 | (400 MHz, $CDCl_3$) δ 10.78 (br s, 1 H), 8.94 (br s, 1 H), 8.36 (s, 1 H), 7.80 (dd, 1 H, *J* = 9.2, 1.2 Hz), 7.76 (d, 1 H, *J* = 9.2 Hz), 7.46 (t, 1 H, *J* = 7.8 Hz), 7.22 (br d, 1 H, *J* = 7.6 Hz), 7.11 (d, 2 H, *J* = 8.4 Hz), 7.01 (d, 1 H, *J* = 8.0 Hz), 6.67 (d, 2 H, *J* = 8.4 Hz), 4.50 (br s, 3 H), 3.61 (s, 2 H), 2.49 (s, 3 H) | 421.21 |
| **70** | 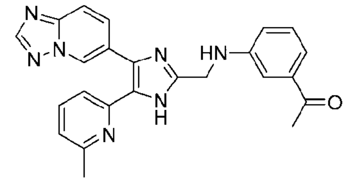 | (400 MHz, $CDCl_3$) δ 11.09 (br s, 1 H), 8.92 (s, 1 H), 8.35 (s, 1 H), 7.79 (d, 1 H, *J* = 9.2 Hz), 7.74 (d, 1 H, *J* = 9.2 Hz), 7.44 (t, 1 H, *J* = 7.8 Hz), 7.30 (d, 1 H, *J* = 7.6 Hz), 7.26-7.24 (m, 1 H), 7.23 (t, 1 H, overlapped, *J* = 7.8 Hz), 7.19 (br d, 1 H, *J* = 8.0 Hz), 7.00 (d, 1 H, *J* = 8.0 Hz), 6.86 (dd, 1 H, *J* = 8.0, 1.8 Hz), 4.68 (br s, 1 H), 4.52 (d, 2 H, *J* = 5.2 Hz), 2.53 (s, 3 H), 2.47 (s, 3 H) | 424.21 |
| **71** | 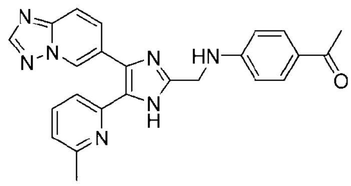 | (400 MHz, $CDCl_3$) δ 8.96 (t, 1 H, *J* = 1.4 Hz), 8.37 (s, 1 H), 7.81 (d, 2 H, *J* = 8.8 Hz), 7.79-7.76 (m, 2 H), 7.54 (t, 1 H, *J* = 7.8 Hz), 7.30 (d, 1 H, *J* = 8.0 Hz), 7.07 (d, 1 H, *J* = 7.6 Hz), 6.70 (d, 2 H, *J* = 8.8 Hz), 4.63 (s, 2 H), 2.59 (s, 3 H), 2.48 (s, 3 H) | 424.21 |
| **72** | 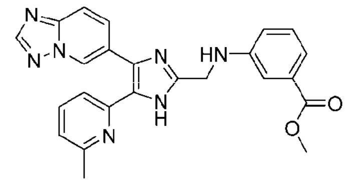 | (400 MHz, $CDCl_3$) δ 8.94 (br s, 1 H), 8.34 (s, 1 H), 7.77 (dd, 1 H, *J* = 9.2, 1.6 Hz), 7.72 (dd, 1 H, *J* = 9.2, 0.8 Hz), 7.44 (t, 1 H, *J* = 7.8 Hz), 7.39 (dt, 1 H, *J* = 7.6, 1.2 Hz), 7.32 (dd, 1 H *J* = 2.4, 1.6 Hz), 7.22 (d, 1 H, overlapped, *J* = 8.0 Hz), 7.19 (t, 1 H, overlapped, *J* = 8.0 Hz), 6.98 (d, 1 H, *J* = 7.6 Hz), 6.81 (ddd, 1 H, *J* = 8.0, 2.4, 0.8 Hz), 4.49 (s, 2 H), 3.84 (s, 3 H), 2.43 (s, 3 H) | 440.20 |
| **73** | 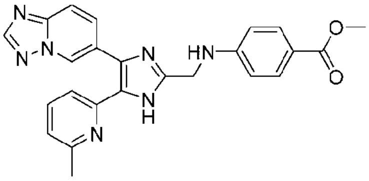 | (400 MHz, $CDCl_3$) δ 10.52 (br s, 1 H), 8.94 (s, 1 H), 8.37 (s, 1 H), 7.89 (m, 2 H), 7.81 (br d, 1 H, *J* = 9.6 Hz), 7.78 (br d, 1 H, *J* = 9.6 Hz), 7.46 (t, 1 H, *J* = 8.0 Hz), 7.21 (d, 1 H, *J* = 8.0 Hz), 7.02 (d, 1 H, *J* = 8.0 Hz), 6.68 (m, 2 H), 4.90 (t, 1 H, *J* = 5.6 Hz), 4.58 (d, 2 H, *J* = 5.6 Hz), 3.85 (s, 3 H), 2.51 (s, 3 H) | 440.20 |
| **74** | 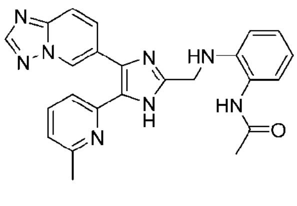 | (400 MHz, DMSO-$d_6$) δ 12.56 (br s, 1 H), 9.58 (s, 1 H), 9.36 (s, 1 H), 8.50 (s, 1 H), 8.00 (br d, 1 H, *J* = 9.2 Hz), 7.82 (d, 1 H, *J* = 9.2 Hz), 7.71 (t, 1 H, *J* = 7.8 Hz), 7.44 (br s, 1 H), 7.16 (d, 1 H, overlapped, *J* = 8.0 Hz), 7.15 (d, 1 H, overlapped, *J* = 7.6 Hz), 7.03 (td, 1 H, *J* = 7.6, 1.2 Hz), 6.81 (d, 1 H, *J* = 7.6 Hz), 6.61 (td, 1 H, *J* = 7.6, 1.2 Hz), 5.65 (t, 1 H, *J* = 6.0 Hz), 4.45 (d, 2 H, *J* = 6.0 Hz), 2.47 (br s, 3 H), 2.09 (s, 3 H) | 439.22 |

(continued)

| Example | Structure | $^1$H NMR (ppm) | MS (ESI) m/z (MH$^+$) |
|---|---|---|---|
| **75** | 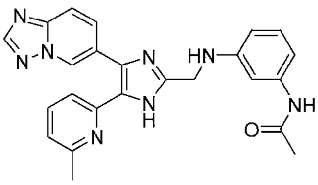 | (400 MHz, DMSO-$d_6$) δ 8.97 (br s, 1 H), 8.35 (s, 1 H), 7.79 (dd, 1 H, *J* = 9.2, 1.6 Hz), 7.73 (d, 1 H, *J* = 9.2 Hz), 7.46 (t, 1 H, *J* = 7.8 Hz), 7.38 (br s, 1 H), 7.24 (d, 1 H, *J* = 8.0 Hz), 7.15 (br s, 1 H), 7.09 (t, 1 H, *J* = 8.0 Hz), 7.01 (d, 1 H, *J* = 7.6 Hz), 6.69 (br d, 1 H, *J* = 8.0 Hz), 6.41 (dd, 1 H, *J* = 8.0, 1.6 Hz), 4.50 (s, 2 H), 2.51 (s, 3 H), 2.13 (s, 3 H) | 439.22 |
| **76** | 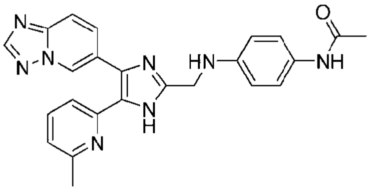 | (400 MHz, DMSO-$d_6$) δ 8.96 (br s, 1 H), 8.37 (s, 1 H), 7.81 (dd, 1 H, *J* = 9.2, 1.6 Hz), 7.76 (dd, 1 H, *J* = 9.2, 0.8 Hz), 7.46 (t, 1 H, *J* = 7.8 Hz), 7.27 (m, 2 H), 7.23 (d, 1 H, *J* = 8.0 Hz), 7.10 (br s, 1 H), 7.01 (d, 1 H, *J* = 7.6 Hz), 6.66 (m, 2 H), 4.50 (s, 2 H), 2.53 (s, 3 H), 2.13 (s, 3 H) | 439.22 |
| **77** | 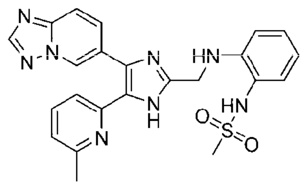 | (400 MHz, $CD_3OD$) δ 9.18 (dd, 1 H, *J* = 1.6, 0.8 Hz), 8.41 (s, 1 H), 7.87 (dd, 1 H, *J* = 9.2, 1.6 Hz), 7.75 (dd, 1 H, *J* = 9.2, 0.8 Hz), 7.66 (t, 1 H, *J* = 7.8 Hz), 7.38 (br d, 1 H, *J* = 7.6 Hz), 7.20 (dd, 1 H, *J* = 7.8, 1.4 Hz), 7.16 (d, 1 H, overlapped, *J* = 7.6 Hz), 7.15 (td, 1 H, overlapped, *J* = 8.0, 1.6 Hz), 6.82 (dd, 1 H, *J* = 8.0, 1.2 Hz), 6.71 (td, 1 H, *J* = 8.0, 1.4 Hz), 4.59 (s, 2 H), 3.05 (s, 3 H), 2.48 (s, 3 H) | 475.19 |
| **78** | 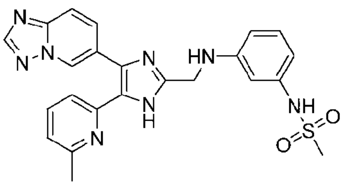 | (400 MHz, $CDCl_3$) δ 8.92 (br s, 1 H), 8.35 (s, 1 H), 7.78 (dd, 1 H, *J* = 9.2, 1.6 Hz), 7.73 (dd, 1 H, *J* = 9.2, 0.8 Hz), 7.46 (t, 1 H, *J* = 7.6 Hz), 7.21 (br d, 1 H, *J* = 7.6 Hz), 7.11 (t, 1 H, overlapped, *J* = 8.2 Hz), 7.10 (br s, 1 H, overlapped), 7.01 (d, 1 H, *J* = 7.6 Hz), 6.58 (t, 1 H, *J* = 2.0 Hz), 6.54 (ddd, 1 H, *J* = 8.2, 2.0, 0.8 Hz), 6.49 (ddd, 1 H, *J* = 8.2, 2.0, 0.8 Hz), 4.50 (s, 2 H), 2.94 (s, 3 H), 2.51 (s, 3 H) | 475.19 |
| **79** | 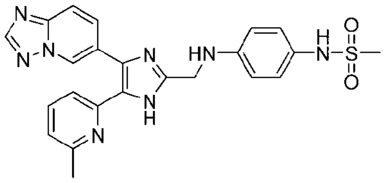 | (400 MHz, $CDCl_3$) δ 8.93 (br s, 1 H), 8.38 (s, 1 H), 7.80 (dd, 1 H, *J* = 9.2, 1.6 Hz), 7.76 (d, 1 H, *J* = 9.2 Hz), 7.46 (t, 1 H, *J* = 7.8 Hz), 7.22 (br d, 1 H, *J* = 8.0 Hz), 7.09 (d, 2 H, *J* = 8.8 Hz), 7.02 (d, 1 H, *J* = 7.6 Hz), 6.67 (d, 2 H, *J* = 8.8 Hz), 6.34 (br s, 1 H), 4.52 (br s, 3 H), 2.93 (s, 3 H), 2.54 (s, 3 H) | 475.19 |
| **80** | 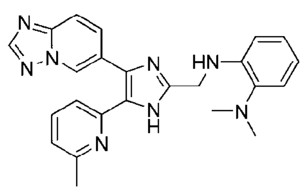 | (400 MHz, $CDCl_3$) δ 8.99 (s, 1 H), 8.36 (s, 1 H), 7.83 (dd, 1 H, *J* = 9.2, 1.6 Hz), 7.77 (dd, 1 H, *J* = 9.2, 0.8 Hz), 7.45 (t, 1 H, *J* = 7.8 Hz), 7.23 (br d, 1 H, *J* = 7.6 Hz), 7.10 (dd, 1 H, *J* = 8.0, 1.2 Hz), 7.02 (td, 1 H, overlapped, *J* = 7.6, 1.2 Hz), 7.00 (d, 1 H, overlapped, *J* = 8.0 Hz), 6.78 (td, 1 H, *J* = 7.6, 1.2 Hz), 6.72 (dd, 1 H, *J* = 8.0, 1.2 Hz), 4.58 (s, 2 H), 2.73 (s, 6 H), 2.50 (s, 3 H) | 425.20 |

(continued)

| Example | Structure | $^1$H NMR (ppm) | MS (ESI) m/z (MH+) |
|---|---|---|---|
| 81 | 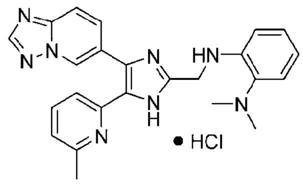 | (400 MHz, DMSO-$d_6$) δ 9.47 (dd, 1 H, *J* = 1.6, 0.8 Hz), 8.63 (s, 1 H), 7.97 (dd, 1 H, *J* = 9.2, 0.8 Hz), 7.87 (t, 1 H, *J* = 7.8 Hz), 7.84 (dd, 1 H, *J* = 9.2, 1.6 Hz), 7.59 (br d, 1 H, overlapped, *J* = 8.0 Hz), 7.58 (d, 1 H, *J* = 7.6 Hz), 7.41 (d, 1 H, *J* = 7.6 Hz), 7.29 (t, 1 H, *J* = 7.4 Hz), 6.99 (dd, 1 H, *J* = 7.6, 0.8 Hz), 6.89 (t, 1 H, *J* = 7.4 Hz), 4.81 (s, 2 H), 3.10 (s, 6 H), 2.56 (s, 3 H) | |
| 82 | 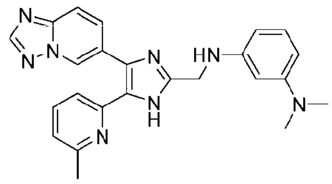 | (400 MHz, $CDCl_3$) δ 8.97 (s, 1 H), 8.36 (s, 1 H), 7.80 (dd, 1 H, *J* = 9.2, 1.6 Hz), 7.75 (d, 1 H, *J* = 9.2 Hz), 7.45 (t, 1 H, *J* = 7.8 Hz), 7.23 (d, 1 H, *J* = 8.0 Hz), 7.07 (t, 1 H, *J* = 8.2 Hz), 7.00 (d, 1 H, *J* = 7.6 Hz), 6.24 (d, 1 H, *J* = 8.0 Hz), 6.14 (br s, 2 H), 4.55 (s, 2 H), 2.90 (s, 6 H), 2.52 (s, 3 H) | 425.21 |
| 83 | 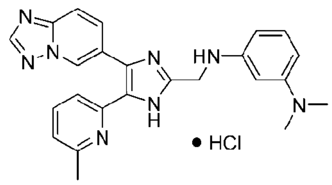 | (400 MHz, DMSO-$d_6$) δ 9.48 (dd, 1 H, *J* = 1.6, 0.8 Hz), 8.64 (s, 1 H), 7.97 (dd, 1 H, *J* = 9.2, 0.8 Hz), 7.86 (t, 1 H, overlapped, *J* = 7.8 Hz), 7.85 (dd, 1 H, overlapped, *J* = 9.2, 1.6 Hz), 7.62 (d, 1 H, *J* = 8.0 Hz), 7.39 (d, 1 H, *J* = 7.6 Hz), 7.28 (t, 1 H, *J* = 8.2 Hz), 7.17 (br s, 1 H), 6.98 (br d, 1 H, *J* = 8.0 Hz), 6.81 (br d, 1 H, *J* = 8.4 Hz), 4.78 (s, 2 H), 3.09 (s, 6 H), 2.52 (s, 3 H) | |
| 84 | 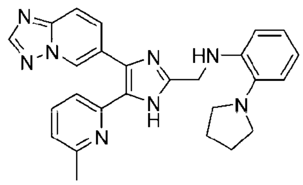 | (400 MHz, $CDCl_3$) δ 10.41 (br s, 1 H), 8.99 (s, 1 H), 8.36 (s, 1 H), 7.84 (dd, 1 H, *J* = 9.2, 1.2 Hz), 7.76 (d, 1 H, *J* = 9.2 Hz), 7.45 (t, 1 H, *J* = 7.8 Hz), 7.23 (br s, 1 H), 7.10 (dd, 1 H, *J* = 7.6, 1.2 Hz), 7.00 (td, 1 H, overlapped, *J* = 7.6, = 1.2 Hz), 6.99 (d, 1 H, overlapped, *J* = 7.6 Hz), 6.77 (td, 1 H, *J* = 7.6, 1.2 Hz), 6.72 (d, 1 H, *J* = 8.0 Hz), 4.58 (s, 2 H), 3.12 (br s, 4 H), 2.51 (s, 3 H), 1.98 (br s, 4 H) | 451.22 |
| 85 | 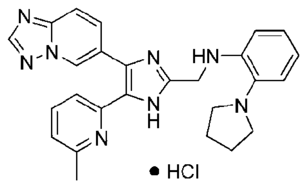 | (400 MHz, DMSO-$d_5$) δ 9.47 (dd, 1 H, *J* = 2.0, 0.8 Hz), 8.63 (s, 1 H), 7.97 (dd, 1 H, *J* = 9.2, 0.8 Hz), 7.87 (t, 1 H, *J* = 7.8 Hz), 7.84 (dd, 1 H, *J* = 9.2, 2.0 Hz), 7.60 (d, 1 H, *J* = 8.0 Hz), 7.57 (d, 1 H, *J* = 8.0 Hz), 7.41 (d, 1 H, *J* = 7.6 Hz), 7.30 (td, 1 H, *J* = 7.8, 0.8 Hz), 7.02 (dd, 1 H, *J* = 7.8, 1.2 Hz), 6.88 (td, 1 H, *J* = 8.0, 1.2 Hz), 4.82 (s, 2 H), 3.72 (br s, 4 H), 2.55 (s, 3 H), 2.17 (m, 4 H) | |
| 86 | 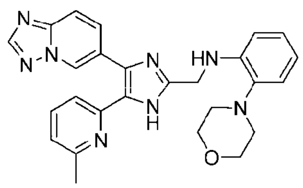 | (400 MHz, $CDCl_3$) δ 8.96 (s, 1 H), 8.37 (s, 1 H), 7.82 (dd, 1 H, *J* = 9.2, 1.6 Hz), 7.78 (dd, 1 H, *J* = 9.2, 1.2 Hz), 7.48 (t, 1 H, *J* = 7.6 Hz), 7.25 (br d, 1 H, *J* = 7.6 Hz), 7.09 (dd, 1 H, *J* = 8.0, 1.6 Hz), 7.05 (td, 1 H, overlapped, *J* = 7.6. 1.6 Hz), 7.02 (d, 1 H, overlapped, *J* = 7.6 Hz), 6.80 (td, 1 H, *J* = 7.6, 1.2 Hz), 6.71 (dd, 1 H, *J* = 8.0, 1.2 Hz), 4.57 (s, 2 H), 3.90 (br t, 4 H, *J* = 4.6 Hz), 2.96 (br t, 4 H, *J* = 4.6 Hz), 2.54 (s, 3 H) | 467.22 |

(continued)

| Example | Structure | $^1$H NMR (ppm) | MS (ESI) m/z (MH$^+$) |
|---|---|---|---|
| **87** | 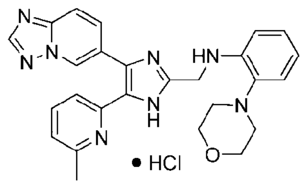 | (400 MHz, DMSO-$d_5$) δ 9.46 (dd, 1 H, *J* = 1.6, 0.8 Hz), 8.64 (s, 1 H), 7.98 (dd, 1 H, *J* = 9.2, 0.8 Hz), 7.84 (t, 1 H, *J* = 7.8 Hz), 7.82 (dd, 1 H, *J* = 9.2, 1.6 Hz), 7.57 (d, 1 H, *J* = 8.0 Hz), 7.38 (d, 1 H, *J* = 7.6 Hz), 7.06 (dd, 1 H, *J* = 7.6, 1.2 Hz), 6.98 (td, 1 H, *J* = 7.8, 1.2 Hz), 6.82 (dd, 1 H, *J* = 7.8, 1.2 Hz), 6.70 (td, 1 H, *J* = 7.6, 1.2 Hz), 4.80 (s, 2 H), 3.84 (br t, 4 H, *J* = 4.4 Hz), 2.89 (br t, 4 H, *J* = 4.4 Hz), 2.53 (s, 3 H) | |
| **88** | 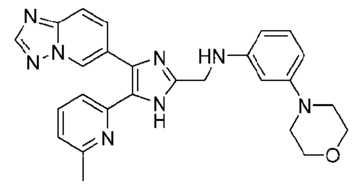 | (400 MHz, $CDCl_3$) δ 8.96 (t, 1 H, *J* = 1.2 Hz), 8.37 (s, 1 H), 7.82-7.76 (m, 2 H), 7.48 (t, 1 H, *J* = 7.6 Hz), 7.24 (br d, 1 H, *J* = 7.6 Hz), 7.12 (t, 1 H, *J* = 8.0 Hz), 7.03 (d, 1 H, *J* = 7.6 Hz), 6.39 (dd, 1 H, *J* = 8.0, 1.6 Hz), 6.34 (t, 1 H, *J* = 2.0 Hz), 6.29 (dd, 1 H, *J* = 8.0, 2.0 Hz), 4.56 (s, 2 H), 3.85-3.82 (m, 4 H), 3.15-3.12(m,4H),2.56(s,3H) | 467.23 |
| **89** | 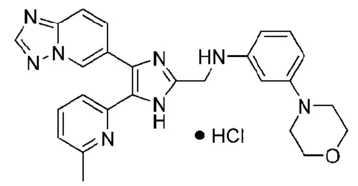 | (400 MHz, DMSO-$d_6$) δ 9.47 (dd, 1 H, *J* = 1.6, 0.8 Hz), 8.64 (s, 1 H), 7.97 (dd, 1 H, *J* = 9.2, 0.8 Hz), 7.85 (t, 1 H, *J* = 8.0 Hz), 7.84 (dd, 1 H, *J* = 9.2, 1.6 Hz), 7.60 (d, 1 H, *J* = 8.0 Hz), 7.39 (d, 1 H, *J* = 8.0 Hz), 7.17 (t, 1 H, *J* = 8.0 Hz), 6.86 (br s, 1 H), 6.76 (br s, 1 H), 6.59 (br d, 1 H, *J* = 7.6 Hz), 4.76 (s, 2 H), 3.92 (br s, 4 H), 3.34 (br s, 4 H), 2.52 (s, 3 H) | |
| **90** | 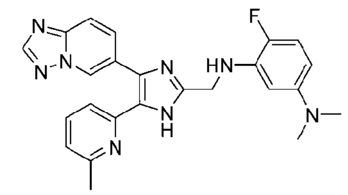 | (400 MHz, $CDCl_3$) δ 8.95 (t, 1 H, *J* = 1.2 Hz), 8.35 (s, 1 H), 7.78 (dd, 1 H, *J* = 9.2, 1.6 Hz), 7.70 (dd, 1 H, *J* = 9.2, 1.2 Hz), 7.43 (t, 1 H, *J* = 7.8 Hz), 7.21 (d, 1 H, *J* = 8.0 Hz), 6.94 (d, 1 H, *J* = 7.6 Hz), 6.80-6.73 (m, 1 H), 6.14 (br d, 1 H, *J* = 7.6 Hz), 6.00-5.95 (m, 1 H), 4.49 (s, 2 H), 2.79 (s, 6 H), 2.31 (s, 3 H) | 443.21 |
| **91** | 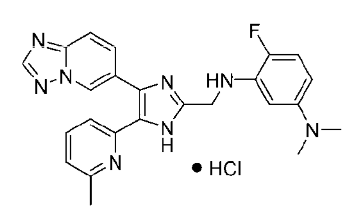 | (400 MHz, DMSO-$d_6$) δ 9.49 (dd, 1 H, *J* = 1.6, 0.8 Hz), 8.63 (s, 1 H), 7.97 (dd, 1 H, *J* = 9.2, 0.8 Hz), 7.85 (dd, 1 H, overlapped, *J* = 9.2, 1.6 Hz), 7.84 (t, 1 H, overlapped, *J* = 8.0 Hz), 7.60 (d, 1 H, *J* = 8.0 Hz), 7.39 (br s, 1 H, overlapped), 7.38 (d, 1 H, overlapped, *J* = 8.0 Hz), 7.24 (pseudo t, 1 H, *J* = 9.8 Hz), 6.97 (br s, 1 H), 4.86 (s, 2 H), 3.10 (s, 6 H), 2.52 (s, 3 H) | |
| **92** | 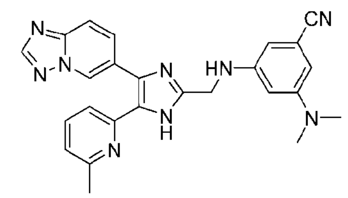 | (400 MHz, $CDCl_3$) δ 8.94 (t, 1 H, *J* = 1.2 Hz), 8.38 (s, 1 H), 7.80 (dd, 1 H, overlapped, *J* = 9.2, 1.6 Hz), 7.78 (dd, 1 H, overlapped, *J* = 9.2, 1.2 Hz), 7.49 (t, 1 H, *J* = 7.6 Hz), 7.25 (d, 1 H, *J* = 7.6 Hz), 7.04 (d, 1 H, *J* = 7.6 Hz), 6.38 (dd, 1 H, *J* = 2.4, 1.2 Hz), 6.30 (t, 1 H, *J* = 1.6 Hz), 6.18 (t, 1 H, *J* = 2.2 Hz), 4.53 (s, 2 H), 2.92 (s, 6 H), 2.56 (s, 3 H) | 450.21 |

(continued)

| Example | Structure | $^1$H NMR (ppm) | MS (ESI) m/z (MH$^+$) |
|---|---|---|---|
| 93 | 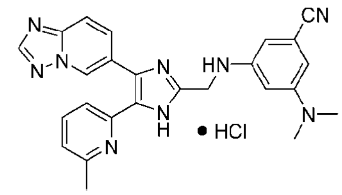 | (400 MHz, DMSO-$d_5$) δ 9.44 (dd, 1 H, *J* = 2.0, 0.8 Hz), 8.63 (s, 1 H), 7.97 (dd, 1 H, *J* = 9.2, 0.8 Hz), 7.85 (t, 1 H, *J* = 7.8 Hz), 7.81 (dd, 1 H, *J* = 9.2, 2.0 Hz), 7.55 (d, 1 H, *J* = 8.0 Hz), 7.40 (d, 1 H, *J* = 7.6 Hz), 6.47 (s, 2 H), 6.37 (br s, 1 H), 4.73 (s, 2 H), 2.90 (s, 6 H), 2.55 (s, 3 H) | |
| 94 | 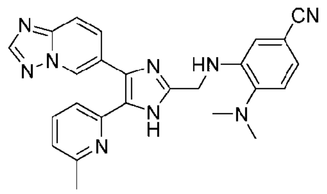 | (400 MHz, $CDCl_3$) δ 8.96 (t, 1 H, *J* = 1.6 Hz), 8.38 (s, 1 H), 7.82 (dd, 1 H, *J* = 9.2, 1.6 Hz), 7.79 (dd, 1 H, *J* = 9.2, 0.8 Hz), 7.53 (t, 1 H, *J* = 7.8 Hz), 7.31 (d, 1 H, *J* = 8.0 Hz), 7.08-7.03 (m, 3 H), 6.87 (d, 1 H, *J* = 1.6 Hz), 5.44 (br s, 1 H), 4.55 (s, 2 H), 2.74 (s, 6 H), 2.58 (s, 3 H) | 450.21 |
| 95 | 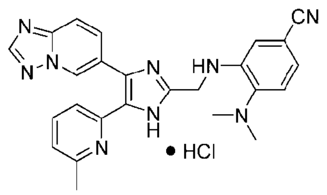 | (400 MHz, DMSO-$d_6$) δ 9.44 (dd, 1 H, *J* = 1.6, 0.8 Hz), 8.64 (s, 1 H), 7.99 (dd, 1 H, *J* = 9.2, 0.8 Hz), 7.84 (t, 1 H, overlapped, *J* = 7.8 Hz), 7.82 (dd, 1 H, *J* = 9.2, 0.8 Hz), 7.51 (d, 1 H, *J* = 8.0 Hz), 7.39 (d, 1 H, *J* = 7.6 Hz), 7.15 (dd, 2 H, overlapped, *J* = 7.6, 0.8 Hz), 7.14 (d, 1 H, overlapped, *J* = 1.2 Hz), 4.79 (s, 2 H), 2.71 (s, 6 H), 2.56 (s, 3 H) | |
| 96 | 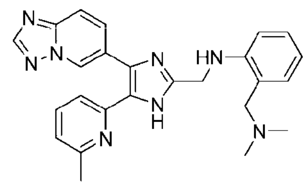 | (400 MHz, $CDCl_3$) δ 10.57 (br s, 1 H), 8.97 9.2, (s, 1 H), 8.34 (s, 1H), 7.82 (dd, Hz), *J* = 9.2, 1.2 Hz), 7.74 (d, 1 H, *J* = 9.2 Hz), 7.43 (t, 1 H, *J* = 7.8 Hz), 7.21 (br s, 1 H, overlapped), 7.17 (td, 1 H, *J* = 8.0, 1.6 Hz), 7.01 (dd, 1 H, *J* = 7.6, 1.6 Hz), 6.98 (d, 1 H, *J* = 7.6 Hz), 6.72-6.66 (m, 2 H), 4.61 (s, 2 H), 3.54 (s, 2 H), 2.51 (s, 3 H), 2.28 (s, 6 H) | 439.23 |
| 97 | 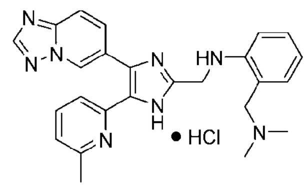 | (400 MHz, DMSO-$d_6$) δ 10.03 (br s, 1 H), 9.48 (dd, 1 H, *J* = 1.6, 0.8 Hz), 8.62 (s, 1 H), 7.95 (dd, 1 H, *J* = 9.2, 0.8 Hz), 7.87-7.82 (m, 2 H), 7.64 (d, 1 H, *J* = 7.6 Hz), 7.38 (d, 1 H, *J* = 7.6 Hz), 7.33-7.27 (m, 2 H), 6.95 (br s, 1 H), 6.78-6.74 (m, 2 H), 4.81 (s, 2 H), 4.43 (s, 2H),2.77(s,6H),2.52(s,3H) | |
| 98 | 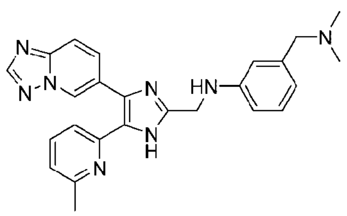 | (400 MHz, $CDCl_3$) δ 9.02 (br s, 1 H), 8.35 (s, 1 H), 7.82 (dd, 1 H, *J* = 9.2, 1.6 Hz), 7.74 (dd, 1 H, *J* = 9.2, 0.8 Hz), 7.46 (t, 1 H, *J* = 7.6 Hz), 7.27 (br d, 1 H, *J* = 7.6 Hz), 7.13 (t, 1 H, *J* = 7.8 Hz), 7.00 (d, 1 H, *J* = 7.6 Hz), 6.90 (br s, 1 H), 6.67 (d, 1 H, *J* = 7.6 Hz), 6.61 (dd, 1 H, *J* = 8.0, 2.0 Hz), 4.54 (s, 2 H), 3.48 (s, 2 H), 2.51 (s, 3 H), 2.30 (s, 6 H) | 439.23 |

(continued)

| Example | Structure | $^1$H NMR (ppm) | MS (ESI) m/z (MH$^+$) |
|---|---|---|---|
| 99 | 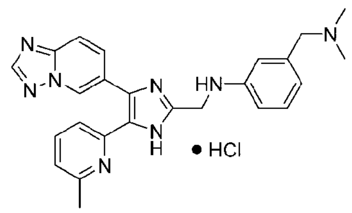 • HCl | (400 MHz, DMSO-d$_6$) δ 10.69 (br s, 1 H), 9.48 (dd, 1 H, *J* = 1.6, 0.8 Hz), 8.63 (s, 1 H), 7.96 (dd, 1 H, *J* = 9.2, 0.8 Hz), 7.84 (t, 1 H, *J* = 8.0 Hz), 7.83 (dd, 1 H, *J* = 9.2, 1.6 Hz), 7.61 (d, 1 H, *J* = 8.0 Hz), 7.37 (d, 1 H, *J* = 8.0 Hz), 7.21 (t, 1 H, *J* = 8.2 Hz), 7.09 (br s, 1 H), 6.85 (dd, 1 H, overlapped, *J* = 8.4, 2.2 Hz), 6.82 (d, 1 H, *J* = 8.0 Hz), 4.76 (s, 2 H), 4.16 (d, 1 H, *J* = 4.8 Hz), 2.66 (d, 6 H, *J* = 4.4 Hz), 2.51 (s, 3 H) | |
| 100 | 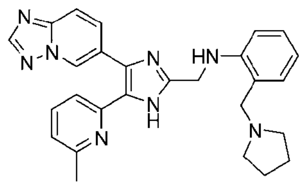 | (400 MHz, CDCl$_3$) δ 10.40 (br s, 1 H), 8.98 (s, 1 H), 8.35 (s, 1 H), 7.83 (d, 1 H, *J* = 9.2 Hz), 7.75 (d, 1 H, *J* = 9.2 Hz), 7.43 (t, 1 H, *J* = 7.6 Hz), 7.20 (br s, 1 H, overlapped), 7.15 (td, 1 H, *J* = 8.0, 1.6 Hz), 7.04 (d, 1 H, *J* = 8.0 Hz), 6.98 (d, 1 H, *J* = 7.6 Hz), 6.69 (pseudo t, 2 H, *J* = 7.2 Hz), 4.58 (s, 2 H), 3.72 (s, 2 H), 2.56 (br s, 4 H), 2.50 (s, 3 H), 1.80 (br s, 4 H) | 465.25 |
| 101 | 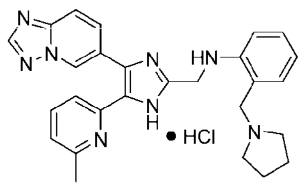 • HCl | (400 MHz, DMSO-*d*$_6$) δ 10.29 (br s, 1 H), 9.47 (t, 1 H, *J* = 0.8 Hz), 8.62 (s, 1 H), 7.95 (d, 1 H, *J* = 9.2 Hz), 7.87-7.83 (m, 2 H), 7.65 (d, 1 H, *J* = 8.0 Hz), 7.38 (br d, 2 H, *J* = 7.6 Hz), 7.27 (td, 1 H, *J* = 8.0, 1.2 Hz), 6.75 (t, 1 H, overlapped, *J* = 7.6 Hz), 6.72 (d, 1 H, overlapped, *J* = 7.6 Hz), 4.82 (s, 2 H), 4.47 (s, 2 H), 3.44 (br s, 2 H), 3.17 (br s, 2 H), 2.52 (s, 3 H), 2.04 (br s, 2 H), 1.94 (br s, 2 H) | |
| 102 | 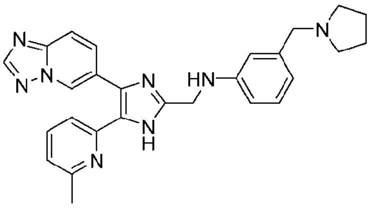 | (400 MHz, CDCl$_3$) δ 9.04 (s, 1 H), 8.35 (s, 1 H), 7.83 (dd, 1 H, *J* = 9.2, 1.6 Hz), 7.73 (d, 1 H, J = 9.2 Hz), 7.47 (t, 1 H, *J* = 7.8 Hz), 7.28 (br d, 1 H, *J* = 7.6 Hz), 7.11 (t, 1 H, *J* = 7.8 Hz), 7.00 (d, 1 H, *J* = 8.0 Hz), 6.95 (br s, 1 H), 6.68 (d, 1 H, *J* = 7.6 Hz), 6.60 (dd, 1 H, *J* = 8.0, 2.0 Hz), 4.54 (s, 2 H), 3.65 (s, 2 H), 2.61 (br s, 4 H), 2.51 (s, 3 H), 1.78-1.75 (m, 4 H) | 465.25 |
| 103 | 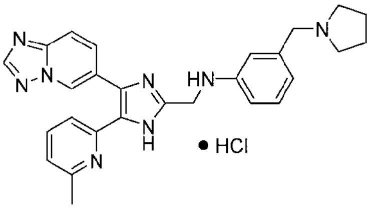 • HCl | (400 MHz, DMSO-d$_6$) δ 10.93 (br s, 1 H), 9.48 (dd, 1 H, *J* = 1.2, 1.6 Hz), 8.63 (s, 1 H), 7.96 (dd, 1 H, *J* = 9.2, 0.8 Hz), 7.86-7.81 (m, 2 H), 7.61 (d, 1 H, *J* = 8.0 Hz), 7.37 (d, 1 H, *J* = 7.6 Hz), 7.19 (t, 1 H, *J* = 7.8 Hz), 7.15 (br s, 1 H), 6.86-6.81 (m, 2 H), 4.76 (s, 2 H), 4.22 (d, 2 H, *J* = 5.6 Hz), 3.29 (m, 2 H), 3.00 (m, 2 H), 2.51 (s, 3 H), 1.92 (m, 2 H), 1.82 (m, 2 H) | |
| 104 | 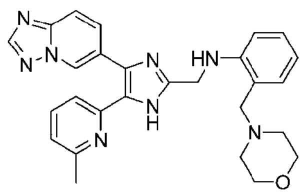 | (400 MHz, CDCl$_3$) δ 10.35 (br s, 1 H), 8.97 (s, 1 H), 8.36 (s, 1 H), 7.83 (d, 1 H, *J* = 9.2 Hz), 7.76 (d, 1 H, *J* = 9.2 Hz), 7.44 (t, 1 H, *J* = 7.6 Hz), 7.21 (br s, 1 H, overlapped), 7.19 (td, 1 H, *J* = 7.6, 1.6 Hz), 7.04 (dd, 1 H, *J* = 7.6, 1.6 Hz), 6.99 (d, 1 H, *J* = 7.6 Hz), 6.73-6.68 (m, 2 H), 4.57 (s, 2 H), 3.71 (br s, 4 H), 3.63 (s, 2 H), 2.50 (br s, 7 H) | 481.25 |

(continued)

| Example | Structure | $^1$H NMR (ppm) | MS (ESI) m/z (MH$^+$) |
|---|---|---|---|
| 105 | 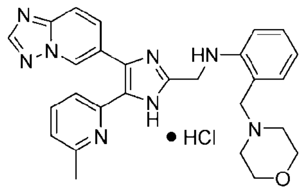 | (400 MHz, DMSO-$d_6$) δ 9.46 (s, 1 H), 8.62 (s, 1 H), 7.96 (dd, 1 H, *J* = 9.2, 0.8 Hz), 7.85 (t, 1 H, overlapped, *J* = 8.0 Hz), 7.83 (dd, 1 H, overlapped, *J* = 9.2, 1.6 Hz), 7.60 (d, 1 H, *J* = 8.0 Hz), 7.38 (br d, 2 H, *J* = 8.0 Hz), 7.29 (td, 1 H, *J* = 8.0, 1.2 Hz), 6.75 (t, 1 H, overlapped, *J* = 8.0 Hz), 6.74 (d, 1 H, overlapped, *J* = 8.0 Hz), 4.82 (s, 2 H), 4.45 (s, 2 H), 3.93 (br s, 4 H), 3.34 (br s, 4 H), 2.53 (s, 3 H) | |
| 106 | 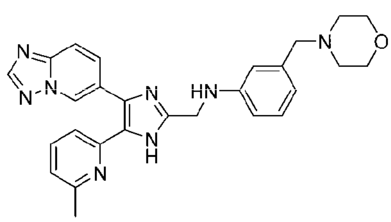 | (400 MHz, $CDCl_3$) δ 10.78 (br s, 1 H), 8.97 (s, 1 H), 8.35 (s, 1 H), 7.81 (dd, 1 H, *J* = 9.2, 1.6 Hz), 7.74 (d, 1 H, *J* = 9.2 Hz), 7.45 (t, 1 H, *J* = 7. 8 Hz),7.23 (br s, 1H), 7.13 (t, 1H, *J* = 7.8 Hz), 7.00 (d, 1H, *J* = 7.6 Hz), 1 H, *J* = 7.8 Hz), 7.00 (d, 1 H, *J* = 7.6 Hz), 6.79 (br s, 1H), 6.71 (d, 1H, *J* = 7.6 Hz), 6.59 (dd, 1 H, *J* = 8.0, 1.6 Hz), 4.53 (s, 2 H), 4.44 (br s, 1 H), 3.66 (m, 4 H), 3.45 (s, 2 H), 2.49 (s, 3 H), 2.44 (br s, 4 H) | 481.25 |
| 107 | 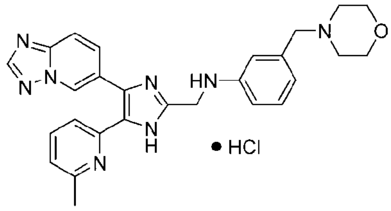 | (400 MHz, DMSO-$d_6$/$D_2O$) δ 9.36 (dd, 1 H, *J* = 1.8, 1.0 Hz), 8.62 (s, 1 H), 7.95 (dd, 1 H, *J* = 9.4, 1.0 Hz), 7.87 (t, 1 H, *J* = 7.8 Hz), 7.82 (dd, 1 H, *J* = 9.4, 1.8 Hz), 7.48 (d, 1 H, *J* = 8.0 Hz), 7.42 (d, 1 H, *J* = 7.6 Hz), 7.28 (t, 1 H, *J* = 7.8 Hz), 6.90 (br s, 1 H), 6.86-6.82 (m, 2 H), 4.68 (s, 2 H Hz), 4.23 (s, 2 H), 3.70 (br s, 4 H), 3.22 (br s, 2 H), 3.14 (br s, 2 H), 2.58 (s, 3 H) | |
| 108 | 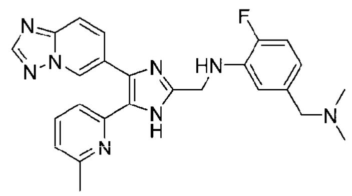 | (400 MHz, $CDCl_3$) δ 9.06 (s, 1 H), 8.34 (s, 1 H), 7.84 (dd, 1 H, *J* = 9.2, 1.6 Hz), 7.72 (d, 1 H, *J* = 9.2 Hz), 7.48 (t, 1 H, *J* = 7.6 Hz), 7.32 (br s, 1 H, ), 7.18 (br s, 1 H), 7.00 (d, 1 H, *J* = 7.6 Hz), 6.90 (dd, 1 H, *J* = 11.2, 8.0 Hz), 6.55-6.51 (m, 1 H), 4.78 (br s, 1 H), 4.59 (d, 2 H, *J* = 6.4 Hz), 3.54 (s, 2 H), 2.50 (s, 3 H), 2.34 (s, 6 H) | 457.22 |
| 109 | 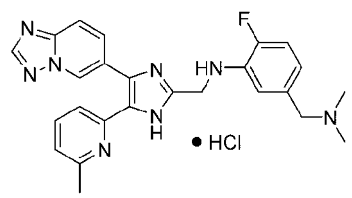 | (400 MHz, DMSO-$d_6$) δ 10.73 (br s, 1 H), 9.46 (t, 1 H, *J* = 1.2 Hz), 8.62 (s, 1 H), 7.96 (dd, 1 H, *J* = 9.2, 0.8 Hz), 7.84 (dd, 1 H, overlapped, *J* = 9.2, 1.6 Hz), 7.82 (t, 1 H, overlapped, *J* = 7.8 Hz), 7.55 (d, 1 H, *J* = 8.0 Hz), 7.37 (dd, 1 H, overlapped, *J* = 8.2, 2.0 Hz), 7.36 (d, 1 H, *J* = 7.6 Hz), 7.18 (dd, 1 H, *J* = 11.8, 8.2 Hz), 6.82-6.78 (m, 1 H), 4.79 (s, 2 H), 4.17 (br s, 2 H), 2.64 (d, 6 H, *J* = 1.6 Hz), 2.52 (s, 3 H) | |
| 110 | 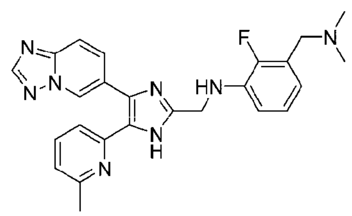 | (400 MHz, $CDCl_3$) δ 8.99 (br s, 1 H), 8.36 (s, 1 H), 7.83 (dd, 1 H, *J* = 9.2, 1.6 Hz), 7.76 (d, 1 H, *J* = 9.2 Hz), 7.46 (t, 1 H, *J* = 7.8 Hz), 7.24 (br s, 1 H), 6.99 (d, 1 H, overlapped, *J* = 8.0 Hz), 6.95 (t, 1 H, overlapped, *J* = 8.0 Hz), 6.76-6.70 (m, 2 H), 4.73 (br s, 1 H), 4.56 (d, 2 H, *J* = 5.6 Hz), 3.60 (s, 2 H), 2.50 (s, 3 H), 2.36 (s, 6 H) | 457.23 |

(continued)

| Example | Structure | $^1$H NMR (ppm) | MS (ESI) m/z (MH$^+$) |
|---|---|---|---|
| 111 | 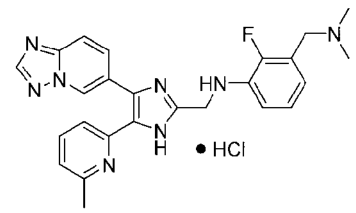 | (400 MHz, DMSO-$d_6$) δ 10.62 (br s, 1 H), 9.46 (s, 1 H), 8.62 (s, 1 H), 7.96 (d, 1 H, *J* = 9.2 Hz), 7.84 (t, 1 H, overlapped, *J* = 8.0 Hz), 7.83 (d, 1 H, overlapped, *J* = 9.2 Hz), 7.57 (d, 1 H, *J* = 8.0 Hz), 7.37 (d, 1 H, *J* = 8.0 Hz), 7.10 (t, 1 H, *J* = 7.6 Hz), 7.02 (t, 1 H, *J* = 7.6 Hz), 6.93 (pseudo t, 1 H, *J* = 6.6 Hz), 4.78 (s, 2 H), 4.30 (d, 2 H, *J* = 4.8 Hz), 2.72 (d, 6 H, *J* = 4.4 Hz), 2.53 (s, 3 H) | |
| 112 | 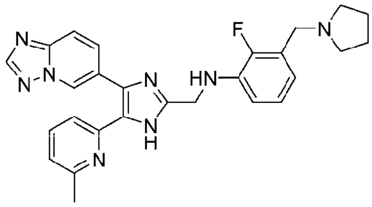 | (400 MHz, $CDCl_3$) δ 11.11 (br s, 1 H), 8.98 (s, 1 H), 8.35 (s, 1 H), 7.81 (dd, 1 H, *J* = 9.2, 1.6 Hz), 7.73 (d, 1 H, *J* = 9.2 Hz), 7.44 (t, 1 H, *J* = 7.8 Hz), 7.23 (br s, 1 H), 6.97 (d, 1 H, *J* = 8.0 Hz), 6.91 (t, 1 H, *J* = 8.0 Hz), 6.75 (td, 1 H, *J* = 8.0, 1.2 Hz), 6.65 (td, 1 H, *J* = 8.0, 1.2 Hz), 4.66 (br s, 1 H), 4.51 (d, 2 H, *J* = 5.6 Hz), 3.66 (s, 2 H), 2.59 (br s, 4 H), 2.42 (s, 3 H), 1.81-1.75 (m, 4 H) | 483.24 |
| 113 | 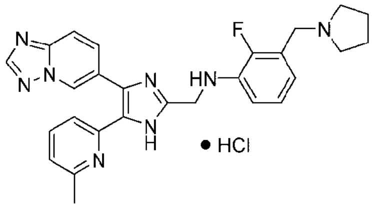 | (400 MHz, DMSO-$d_6$) δ 10.84 (br s, 1 H), 9.46 (s, 1 H), 8.62 (s, 1 H), 7.95 (d, 1 H, *J* = 9.2 Hz), 7.84 (t, 1 H, *J* = 7.8 Hz), 7.83 (dd, 1 H, *J* = 9.2, 1.6 Hz), 7.57 (d, 1 H, *J* = 8.0 Hz), 7.36 (d, 1 H, *J* = 7.6 Hz), 7.08 (t, 1 H, *J* = 7.8 Hz), 7.03-6.96 (m, 2 H), 4.77 (s, 2 H), 4.36 (d, 2 H, *J* = 5.2 Hz), 3.39 (m, 2 H), 3.07 (m, 2 H), 2.53 (s, 3 H), 2.03-1.87 (m, 4 H) | |
| 114 | 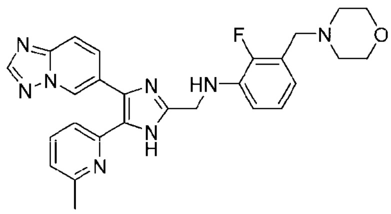 | (400 MHz, $CDCl_3$) δ 8.97 (s, 1 H), 8.36 (s, 1 H), 7.82 (dd, 1 H, *J* = 9.2, 2.0 Hz), 7.76 (dd, 1 H, *J* = 9.2, 0.8 Hz), 7.46 (t, 1 H, *J* = 7.6 Hz), 7.23 (br d, 1 H, *J* = 7.6 Hz), 7.00 (d, 1 H, *J* = 7.6 Hz), 6.95 (t, 1 H, *J* = 8.0 Hz), 6.76 (t, 1 H, *J* = 7.6 Hz), 6.70 (td, 1 H, *J* = 8.0, 1.6 Hz), 4.67 (br s, 1 H), 4.54 (d, 2 H, *J* = 4.8 Hz), 3.73 (m, 4 H), 3.59 (s, 2 H), 2.53 (br s, 4 H), 2.47 (s, 3 H) | 499.24 |
| 115 | 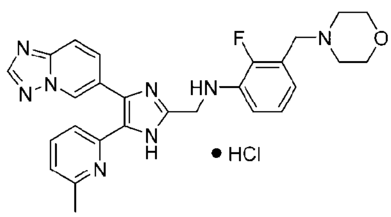 | (400 MHz, DMSO-$d_6$) δ 11.18 (br s, 1 H), 9.46 (dd, 1 H, *J* = 1.6, 1.2 Hz), 8.62 (s, 1 H), 7.96 (dd, 1 H, *J* = 9.2, 0.8 Hz), 7.84 (t, 1 H, overlapped, *J* = 7.8 Hz), 7.83 (dd, 1 H, overlapped, *J* = 9.2, 1.6 Hz), 7.57 (d, 1 H, *J* = 7.6 Hz), 7.37 (d, 1 H, *J* = 8.0 Hz), 7.10 (t, 1 H, *J* = 7.8 Hz), 7.05-6.99 (m, 2 H), 4.78 (s, 2 H), 4.34 (s, 2 H), 3.93 (br s, 2 H), 3.81 (br t, 2 H, *J* = 11.8 Hz) 3.29 (br s, 2 H), 3.13 (br s, 2H),2.53(s,3H) | |
| 116 | 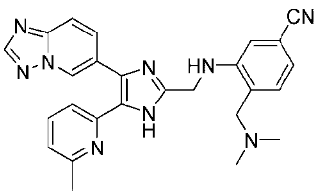 | (400 MHz, $CDCl_3$) δ 8.99 (s, 1 H), 8.36 (s, 1 H), 7.86 (dd, 1 H, *J* = 9.2, 1.6 Hz), 7.78 (dd, 1 H, *J* = 9.2, 0.8 Hz), 7.47 (t, 1 H, *J* = 7.6 Hz), 7.23 (br d, 1 H, *J* = 7.6 Hz), 7.09 (d, 1 H, *J* = 7.6 Hz), 7.01 (d, 1 H, *J* = 7.6 Hz), 6.98 (dd, 1 H, *J* = 7.6, 1.6 Hz), 6.93 (br s, 1 H), 4.59 (s, 2 H), 3.63 (s, 2 H), 2.53 (s, 3 H), 2.33 (s, 6 H) | 464.23 |

(continued)

| Example | Structure | $^1$H NMR (ppm) | MS (ESI) m/z (MH$^+$) |
|---|---|---|---|
| **117** | 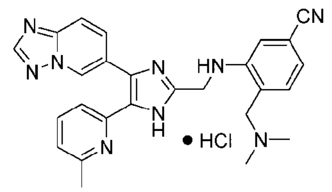 • HCl | (400 MHz, DMSO-$d_6$) δ 9.45 (dd, 1 H, *J* = 1.6, 0.8 Hz), 8.60 (s, 1 H), 7.94 (dd, 1 H, *J* = 9.2, 0.8 Hz), 7.85 (t, 1 H, overlapped, *J* = 7.6 Hz), 7.84 (dd, 1 H, overlapped, J = 9.2, 1.6 Hz), 7.57 (d, 1 H, *J* = 7.6 Hz), 7.52 (d, 1 H, *J* = 8.0 Hz), 7.39 (d, 1 H, *J* = 7.6 Hz), 7.30 (br s, 1 H), 7.19-7.17 (m, 2 H), 4.79 (s, 2 H), 4.48 (s, 2 H), 2.78 (s, 6 H), 2.55 (s, 3 H) | |
| **118** | 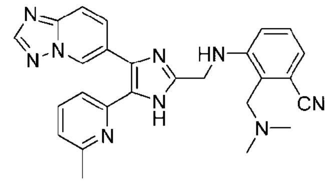 | (400 MHz, DMSO-$d_6$) δ 12.70 (br s, 1 H), 9.54 (s, 1 H), 8.50 (s, 1 H), 7.99 (dd, 1 H, *J* = 9.2, 2.0 Hz), 7.83 (dd, 1 H, *J* = 9.2, 0.8 Hz), 7.72 (t, 1 H, *J* = 7.8 Hz), 7.52 (br s, 1 H), 7.32 (t, 1 H, *J* = 7.6 Hz), 7.17-7.13 (m, 2 H), 7.07 (d, 1 H, *J* = 8.0 Hz), 7.02 (dd, 1 H, *J* = 7.6, 0.8 Hz), 4.48 (d, 2 H, *J* = 5.6 Hz), 3.68 (s, 2 H), 2.47 (s, 3 H), 2.23 (s, 6 H) | 464.23 |
| **119** | 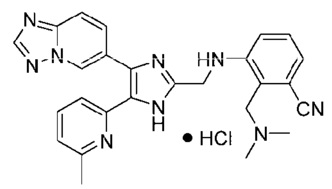 • HCl | (400 MHz, DMSO-$d_6$) δ 9.48 (t, 1 H, J = 1.2 Hz), 8.60 (s, 1 H), 7.92 (dd, 1 H, *J* = 9.2, 1.2 Hz), 7.86 (dd, 1 H, overlapped, *J* = 9.2, 1.6 Hz), 7.85(t, 1 H, overlapped, *J* = 8.0 Hz), 7.64 (d, 1 H, *J* = 8.0 Hz) 7.49 (t, 1 H, *J* = 7.8 Hz), 7.37 (d, 1 H, *J* = 8.0 Hz), 7.24 (dd, 1 H, *J* = 7.6, 0.8 Hz), 7.10 (dd, 1 H, *J* = 8.0, 0.8 Hz), 4.81 (s, 2 H), 4.61 (s, 2 H), 2.89 (s, 6H),2.52(s,3H) | |
| **120** | 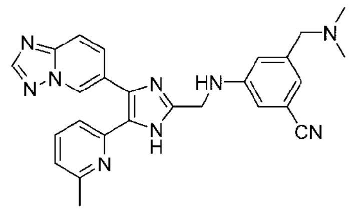 | (400 MHz, $CDCl_3$) δ 9.01 (s, 1 H), 8.36 (s, 1 H), 7.83 (dd, 1 H, *J* = 9.2, 1.6 Hz), 7.76 (dd, 1 H, *J* = 9.2, 0.8 Hz), 7.49 (t, 1 H, *J* = 7.6 Hz), 7.28 (br d, 1 H, *J* = 7.6 Hz), 7.15 (br s, 1 H), 7.03 (d, 1 H, *J* = 7.6 Hz), 6.94 (s, 1 H), 6.82 (dd, 1 H, *J* = 2.0, 1.2 Hz), 4.86 (br s, 1 H), 4.55 (d, 2 H, *J* = 5.6 Hz), 3.48 (s, 2 H), 2.54(s,3H),2.32(s,6H) | 464.23 |
| **121** | 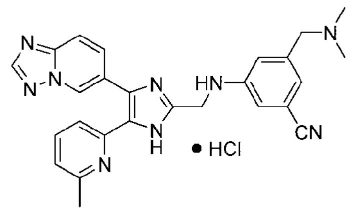 • HCl | (400 MHz, DMSO-$d_6$) δ 10.77 (br s, 1 H), 9.46 (dd, 1 H, *J* = 1.6, 0.8 Hz), 8.60 (s, 1 H), 7.94 (dd, 1 H, *J* = 9.2, 0.8 Hz), 7.85 (dd, 1 H, overlapped, *J* = 9.2, 1.6 Hz), 7.84 (t, 1 H, overlapped, *J* = 7.8 Hz), 7.55 (d, 1 H, *J* = 7.6 Hz), 7.36 (d, 1 H, overlapped, *J* = 8.0 Hz), 7.35 (s, 1 H, overlapped), 7.23 (br s, 2 H), 4.71 (s, 2 H), 4.22 (s, 2 H), 2.67 (s, 6 H), 2.53 (s, 3 H) | |
| **122** | 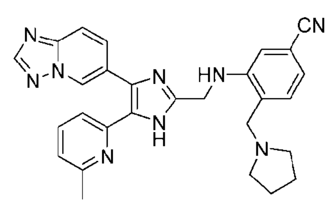 | (400 MHz, $CDCl_3$) δ 10.40 (br s, 1 H), 8.96 (s, 1 H), 8.36 (s, 1 H), 7.84 (dd, 1 H, *J* = 9.2, 1.6 Hz), 7.77 (d, 1 H, *J* = 9.2 Hz), 7.46 (t, 1 H, *J* = 7.6 Hz), 7.24 (br d, 1 H, *J* = 7.6 Hz), 7.09 (d, 1 H, *J* = 7.6 Hz), 7.00 (d, 1 H, *J* = 7.6 Hz), 6.96 (dd, 1 H, *J* = 7.6, 1.6 Hz), 6.86 (br s, 1 H), 4.55 (s, 2 H), 3.74 (s, 2 H), 2.54 (br s, 4 H), 2.51 (s, 3 H), 1.81 (br s, 4 H) | 490.25 |

(continued)

| Example | Structure | $^1$H NMR (ppm) | MS (ESI) m/z (MH$^+$) |
|---|---|---|---|
| 123 | 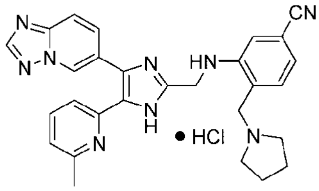 | (400 MHz, DMSO-$d_6$) δ 10.47 (br s, 2 H), 9.46 (s, 1 H), 8.61 (s, 1 H), 7.95 (dd, 1 H, *J* = 9.2, 0.4 Hz), 7.86 (t, 1 H, overlapped, *J* = 7.6 Hz), 7.85 (dd, 1 H, overlapped, *J* = 9.2, 1.2 Hz), 7.60 (d, 1 H, *J* = 7.6 Hz), 7.59 (d, 1 H, *J* = 8.0 Hz), 7.39 (d, 1 H, *J* = 7.6 Hz), 7.32 (br s, 1 H), 7.18 (dd, 1 H, *J* = 7.6, 1.6 Hz), 7.14 (d, 1 H, *J* = 1.6 Hz), 4.82 (s, 2 H), 4.53 (s, 2 H), 3.23 (br s, 4 H), 2.55 (s, 3 H), 1.99 (br s, 4 H) | |
| 124 | 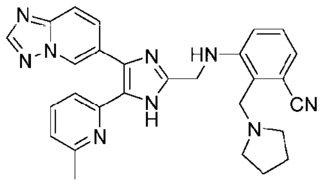 | (400 MHz, DMSO-$d_6$) δ 12.71 (br s, 1 H), 9.52 (s, Hz), 8.50 (s, 1 H), 7.98 9.2 Hz), *J* = 9.2, 1.6 Hz), 7.83 (d, 1 H, *J* = 9.2 Hz), 7.71 (t, 1 H, *J* = 7.8 Hz), 7.51 (br s, 1 H), 7.37 (t, 1 H, *J* = 5.2 Hz), 7.31 (t, 1 H, *J* = 8.0 Hz), 7.16 (d, 1 H, J = 7.6 Hz), 7.05 (d, 1 H, *J* = 8.0 Hz), 7.00 (dd, 1 H, *J* = 8.0, 1.2 Hz), 4.47 (d, 2 H, *J* = 5.2 Hz), 3.87 (s, 2 H), 2.51 (br s, 4 H), 2.47 (s, 3 H), 1.74 (br s, 4 H) | 490.25 |
| 125 | 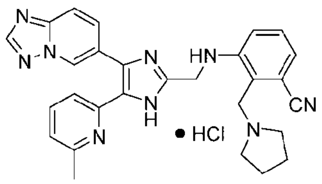 | (400 MHz, DMSO-$d_6$/$D_2O$) δ 9.41 (dd, 1 H, *J* = 1.6, 0.8 Hz), 8.61 (s, 1 H), 7.94 (dd, 1 H, *J* = 9.2, 0.8 Hz), 7.89 (t, 1 H, *J* = 7.8 Hz), 7.84 (dd, 1 H, *J* = 9.2, 1.6 Hz), 7.55 (d, 1 H, *J* = 8.0 Hz), 7.50 (t, 1 H, *J* = 7.6 Hz), 7.42 (d, 1 H, *J* = 7.6 Hz), 7.25 (dd, 1 H, *J* = 7.6, 0.8 Hz), 7.09 (d, 1 H, *J* = 7.6 Hz), 4.78 (s, 2 H), 4.64 (s, 2 H), 3.45 (br s, 4 H), 2.58 (s, 3 H), 2.06 (br s, 4 H ) | |
| 126 | 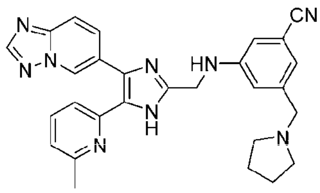 | (400 MHz, $CDCl_3$) δ 8.99 (s, 1 H), 8.36 (s, 1 H), 7.82 (dd, 1 H, *J* = 9.2, 1.6 Hz), 7.77 (d, 1 H, *J* = 9.2 Hz), 7.48 (t, 1 H, *J* = 7.6 Hz), 7.27 (br d, 1 H, *J* = 7.6 Hz), 7.13 (br s, 1 H), 7.03 (d, 1 H, *J* = 7.6 Hz), 6.97 (br s, 1 H), 6.81 (dd, 1 H, *J* = 2.4, 1.2 Hz), 4.81 (br s, 1 H), 4.54 (d, 2 H, *J* = 5.6 Hz), 3.62 (s, 2 H), 2.57 (br s, 4 H), 2.54 (s, 3 H), 1.79 (br s, 4 H) | 490.25 |
| 127 | 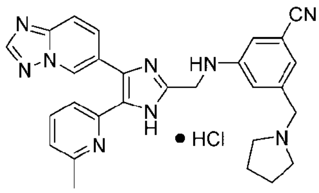 | (400 MHz, DMSO-$d_6$) δ 10.96 (br s 1 H), 9.45 (dd, 1 H, *J* = 1.6, 0.8 Hz), 8.60 (s, 1 H), 7.94 (dd, 1 H, *J* = 9.2, 0.8 Hz), 7.84 (dd, 1 H, overlapped, *J* = 9.2, 1.6 Hz), 7.83 (t, 1 H, overlapped, *J* = 7.8 Hz), 7.54 (d, 1 H, *J* = 8.0 Hz), 7.39 (br s, 1 H), 7.36 (d, 1 H, *J* = 7.6 Hz), 7.26 (s, 1 H), 7.21 (dd, 1 H, *J* = 2.0, 1.6 Hz), 4.72 (s, 2 H), 4.29 (s, 2 H), 3.02 (br s, 4 H), 2.53 (s, 3 H), 1.95 (br s, 2 H), 1.84 (br s, 2 H) | |
| 128 | 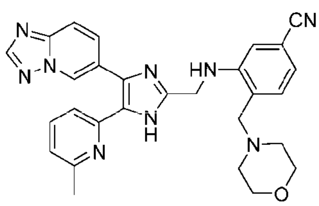 | (400 MHz, $CDCl_3$) δ 8.97 (s, 1 H), 8.37 (s, 1 H), 7.84 (dd 1 H, *J* = 9.2, 1.6 Hz), 7.79 (dd, 1 H, *J* = 9.2, 1.2 Hz), 7.49 (t, 1 H, *J* = 7.8 Hz), 7.29 (br d, 1 H, *J* = 8.0 Hz), 7.12 (d, 1 H, *J* = 7.6 Hz), 7.03 (d, 1 H, *J* = 7.6 Hz), 6.99 (dd, 1 H, *J* = 7.6, 1.6 Hz), 6.93 (br s, 1 H), 4.57 (s, 2 H), 3.75 (br s, 4 H, overlapped), 3.72 (s, 2 H, overlapped), 2.55 (br s, 7 H) | 506.25 |

(continued)

| Example | Structure | $^1$H NMR (ppm) | MS (ESI) m/z (MH$^+$) |
|---|---|---|---|
| 129 | 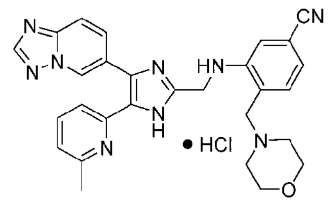 | (400 MHz, DMSO-$d_6$) δ 9.45 (d, 1 H, *J* = 0.4 Hz), 8.61 (s, 1 H), 7.96 (d, 1 H, *J* = 9.2 Hz), 7.86 (t, 1 H, overlapped, *J* = 8.0 Hz), 7.85 (dd, 1 H, overlapped, *J* = 9.2, 1.6 Hz), 7.60 (d, 1 H, *J* = 8.0 Hz), 7.57 (d, 1 H, *J* = 8.0 Hz), 7.39 (d, 1 H, *J* = 8.0 Hz), 7.19 (s, 1 H), 7.17 (dd, 1 H, overlapped, *J* = 8.0, 1.2 Hz), 4.82 (s, 2 H), 4.52 (s, 2 H), 3.91 (br s, 4 H), 3.32 (br s, 4 H), 2.55 (s, 3 H) | |
| 130 | 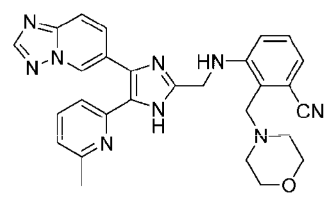 | (400 MHz, DMSO-$d_6$) δ 12.70 (br s, 1 H), 9.48 (br s, 1 H), 8.50 (s, 1 H), 7.95 (dd, 1 H, *J* = 9.2, 1.6 Hz), 7.84 (d, 1 H, *J* = 9.2 Hz), 7.71 (t, 1 H, *J* = 7.6 Hz), 7.44 (br s, 1 H), 7.34 (t, 1 H, *J* = 8.0 Hz), 7.28 (t, 1 H, *J* = 5.6 Hz), 7.6 (d, 1 H, *J* = 7.6 Hz), 7.07 (d, 1 H, *J* = 8.4 Hz), 7.03 (dd, 1 H, *J* = 7.6, 1.2 Hz), 4.49 (d, 2 H, *J* = 5.6 Hz), 3.76 (s, 2 H), 3.58 (m,4H),2.48 (s, 3 H), 2.41 (br s, 4 H) | 506.24 |
| 131 | 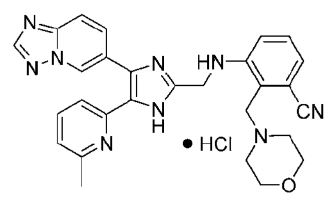 | (400 MHz, DMSO-$d_6$) δ 9.46 (d, 1 H, *J* = 0.8 Hz), 8.60 (s, 1 H), 7.94 (dd, 1 H, *J* = 9.2, 0.8 Hz), 7.85 (t, 1 H, overlapped, *J* = 7.6 Hz), 7.84 (dd, 1 H, overlapped, *J* = 9.2, 1.6 Hz), 7.60 (d, 1 H, *J* = 7.6 Hz), 7.47 (t, 1 H, *J* = 8.0 Hz), 7.37 (d, 1 H, *J* = 8.0 Hz), 7.22 (dd, 1 H, *J* = 7.6, 0.8 Hz), 7.08 (d, 1 H, *J* = 8.0 Hz), 4.82 (s, 2 H), 4.57 (s, 2 H), 3.95 (br t, 4 H, *J* = 4.4 Hz), 3.40 (br s, 4 H), 2.53 (s, 3 H) | |
| 132 | 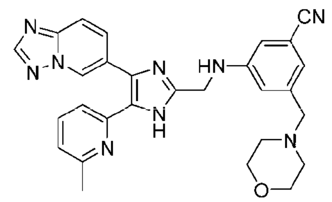 | (400 MHz, $CDCl_3$) δ 8.97 (s, 1 H), 8.38 (s, 1 H), 7.82 (dd, 1 H, *J* = 9.2, 1.6 Hz), 7.78 (dd, 1 H, *J* = 9.2, 0.8 Hz), 7.48 (t, 1 H, *J* = 7.6 Hz), 7.28 (br d, 1 H, *J* = 7.6 Hz), 7.06 (br s, 1 H, overlapped), 7.04 (d, 1 H, *J* = 7.6 Hz), 7.01 (s, 1 H), 6.84 (dd, 1 H, *J* = 2.0, 1.2 Hz), 4.79 (br s, 1 H), 4.54 (d, 2 H, *J* = 7.6 Hz), 3.70 (m, 4 H), 3.48 (s, 2 H), 2.55 (s, 3 H), 2.47 (brs, 4 H) | 506.24 |
| 133 | 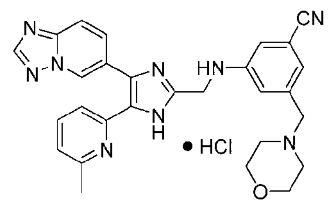 | (400 MHz, DMSO-$d_6$) δ 9.45 (dd, 1 H, *J* = 1.6, 0.8 Hz), 8.61 (s, 1 H), 7.95 (dd, 1 H, *J* = 9.2, 0.8 Hz), 7.85 (t, 1 H, overlapped, *J* = 7.6 Hz), 7.84 (dd, 1 H, overlapped, *J* = 9.2, 1.6 Hz), 7.54 (d, 1 H, *J* = 7.6 Hz), 7.43 (br s, 1 H), 7.37 (d, 1 H, *J* = 7.6 Hz), 7.28 (s, 1 H), 7.23 (dd, 1 H, *J* = 2.2, 1.4 Hz), 4.74 (s, 2 H), 4.28 (s, 2 H), 3.83 (br s, 4 H), 3.81 (br s, 2 H), 3.08 (br s, 2 H), 2.54 (s, 3 H) | |
| 134 | 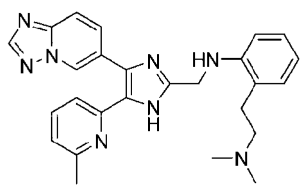 | (400 MHz, $CDCl_3$) δ 8.98 (s, 1 H), 8.36 (s, 1 H), 7.83 (dd 1 H, *J* = 9.2, 1.6 Hz), 7.76 (dd, 1 H, J = 9.2, 0.8 Hz), 7.45 (t, 1 H, *J* = 7.8 Hz), 7.23 (br d, 1 H, *J* = 7.6 Hz), 7.13 (td, 1 H, *J* = 8.0, 1.2 Hz), 7.04 (dd, 1 H, *J* = 8.0, 1.2 Hz), 6.99 (d, 1 H, *J* = 8.0 Hz), 6.72 (d, 1 H, overlapped, *J* = 7.6 Hz), 6.71 (td, 1 H, overlapped, *J* = 8.0, 1.2 Hz), 4.56 (s, 2 H), 2.81 (t, 2 H, *J* = 6.4 Hz), 2.63 (t, 2 H, *J* = 6.4 Hz), 2.52 (s, 3 H), 2.36 (s, 6 H) | 453.25 |

(continued)

| Example | Structure | $^1$H NMR (ppm) | MS (ESI) m/z (MH+) |
|---|---|---|---|
| 135 | 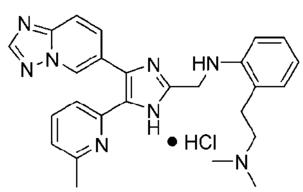 | (400 MHz, DMSO-d$_6$/D$_2$O) δ 9.40 (dd, 1 H, *J* = 1.8, 0.8 Hz), 8.63 (s, 1 H), 7.96 (dd, 1 H, *J* = 9.2, 0.8 Hz), 7.86 (t, 1 H, *J* = 7.8 Hz), 7.82 (dd, 1 H, *J* = 9.2, 1.8 Hz), 7.48 (d, 1 H, *J* = 8.0 Hz), 7.41 (d, 1 H, *J* = 7.6 Hz), 7.17-7.12 (m, 2 H), 6.75-671 (m, 2 H), 4.73 (s, 2 H), 3.32 (br t, 2 H, *J* = 8.4 Hz), 3.02 (br t, 2 H, *J* = 8.4 Hz), 2.90 (s, 6 H), 2.58 (s, 3 H) | |
| 136 | 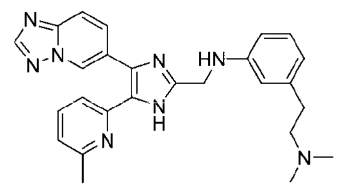 | (400 MHz, CDCl$_3$) δ 9.08 (s, 1 H), 8.32 (s, 1 H), 7.83 (dd, 1 H, J = 9.2, 1.6 Hz), 7.68 (d, 1 H, *J* = 9.2 Hz), 7.47 (t, 1 H, *J* = 7.6 Hz), 7.34 (br d, 1 H, J = 7.6 Hz), 7.04 (t, 1 H, *J* = 7.6 Hz), 6.99 (dd, 1 H, *J* = 7.6, 0.4 Hz), 6.66 (br s, 1 H), 6.53-6.49 (m, 2 H), 4.48 (s, 2 H), 2.89-2.83 (m 4 H), 2.56 (s, 6 H), 2.48 (s, 3 H) | 453.25 |
| 137 | 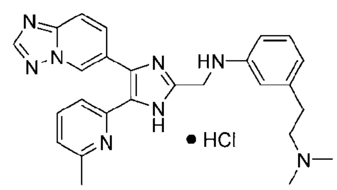 | (400 MHz, DMSO-*d*$_6$) δ 10.47 (br s, 1 H), 9.49 (t, 1 H, *J* = 0.8 Hz), 8.63 (s, 1 H), 7.97 (dd, 1 H, *J* = 9.2, 0.8 Hz), 7.85 (dd, 1 H, overlapped, *J* = 9.2, 1.2 Hz), 7.84 (t, 1 H, overlapped, *J* = 7.8 Hz), 7.64 (d, 1 H, *J* = 8.0 Hz), 7.37 (d, 1 H, *J* = 7.6 Hz), 7.10 (t, 1 H, *J* = 8.0 Hz), 6.81 (br s, 1 H), 6.66 (dd, 1 H, *J* = 8.0, 1.6 Hz), 6.58 (d, 1 H, *J* = 8.0 Hz), 4.75 (s, 2 H), 3.30-3.25 (m, 2 H), 2.94-2.89 (m, 2 H), 2.77 (d, 6 H, *J* = 4.8 Hz), 2.51 (s, 3 H) | |
| 138 | 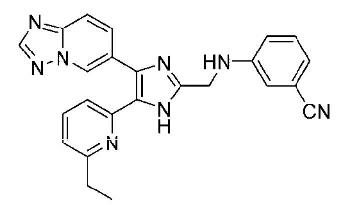 | (400 MHz, CDCl$_3$) δ 10.40 (br s, 1 H), 8.94 (s, 1 H), 8.38 (s, 1 H), 7.83 (dd, 1 H, *J* = 9.2, 1.2 Hz), 7.80 (dd, 1 H, J = 9.2, 0.8 Hz), 7.49 (t, 1 H, *J* = 7.8 Hz), 7.30-7.27 (m, 1 H), 7.24 (d, 1 H, *J* = 8.0 Hz), 7.06 (dt, 1 H, overlapped, *J* = 7.6, 1.2 Hz), 7.05 (d, 1 H, overlapped, *J* = 7.6 Hz), 6.94-6.93 (m, 2 H), 4.72 (t, 1 H, *J* = 5.2 Hz), 4.54 (d, 2 H, *J* = 5.2 Hz), 2.82 (q, 2 H, *J* = 7.6 Hz), 1.31 (t, 3 H, *J* = 7.6 Hz) | 421.21 |
| 139 | 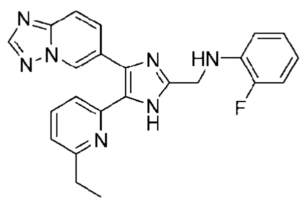 | (400 MHz, CDCl$_3$) δ 10.37 (br s, 1 H), 8.96 (s, 1 H), 8.38 (s, 1 H), 7.84 (dd, 1 H, *J* = 9.2, 1.2 Hz), 7.79 (dd, 1 H, *J* = 9.2, 0.8 Hz), 7.48 (t, 1 H, *J* = 7.8 Hz), 7.22 (d, 1 H, *J* = 8.0 Hz), 7.06-7.00 (m, 3 H), 6.80 (td, 1 H, *J* = 8.0, 1.2 Hz), 6.76-6.70 (m, 1 H), 4.62 (br s, 1 H, overlapped), 4.60 (s, 2 H), 2.80 (q, 2 H, *J* = 7.6 Hz), 1.29 (t, 3 H, *J* = 7.6 Hz) | 414.20 |

**Practice Example 5**

Preparation of *N*-((4-([1,2,4]triazolo[1,5-*a*]pyridin-6-yl)-5-(6-methylpyridin-2-yl)-1*H*-imidazol-2-yl)methyl)-2-fluoro-*N*-methylaniline (Example 140)

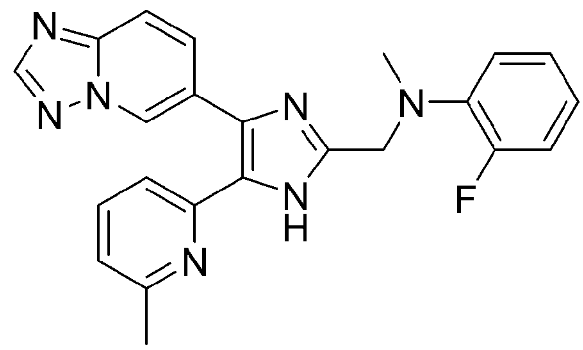

To a stirred solution of 1-((1,2,4)triazolo(1,5-*a*)pyridin-6-yl)-2-(6-methylpyridin-2-yl)ethane-1,2-dione (0.20 g, 0.75 mmol) in a mixture of *tert*-butyl methyl ether (10 mL) and MeOH (8 mL) were added 2-((2-fluorophenyl)(methyl)amino)acetaldehyde (190 mg, 1.13 mmol) and $NH_4OAc$ (0.15 g, 1.88 mmol), and the mixture was stirred at room temperature for 2 h. The pH of the mixture was adjusted to 8 with saturated aqueous $NaHCO_3$ solution. After removal of the solvent, the reaction mixture was extracted with $CHCl_3$ (2 × 100 mL), and the $CHCl_3$ solution was washed with water (20 mL) and brine (20 mL), dried over anhydrous $Na_2SO_4$, filtered, and evaporated to dryness under reduced pressure. The residue was purified by MPLC on silica gel using a mixture of MeOH and $CH_2Cl_2$ (1:19 (v/v)) as eluent to give the titled compound (90 mg, 32%) as a pale yellow solid. $^1H$ NMR (400 MHz, $CDCl_3$) δ 8.97 (br s, 1 H), 8.37 (s, 1 H), 7.82 (dd, 1 H, *J* = 9.2, 1.6 Hz), 7.78 (dd, 1 H, *J* = 9.2, 1.2 Hz), 7.49 (t, 1 H, *J* = 7.8 Hz), 7.25 (br d, 1 H, *J* = 7.6 Hz), 7.14-7.06 (m, 3 H), 7.04 (d, 1 H, *J* = 7.6 Hz), 7.00-6.94 (m, 1 H), 4.44 (s, 2 H), 2.91 (s, 3 H), 2.58 (s, 3 H); MS (ESI) *m/z* 414.20 ($MH^+$).

**Practice Example 6**

Preparation of 3-((4-([1,2,4]triazolo[1,5-a]pyridin-6-yl)-5-(6-methylpyridin-2-yl)-1*H*-imidazol-2-yl)methyl)benzonitrile (Example 145)

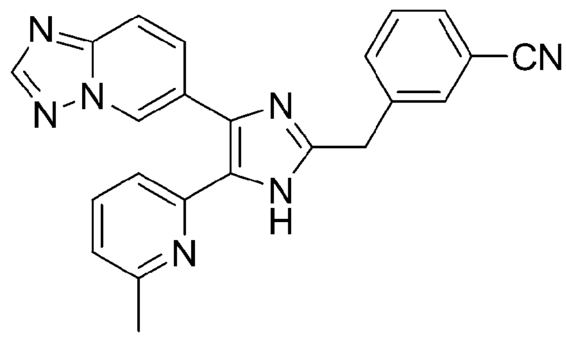

To a stirred solution of 1-((1,2,4)triazolo(1,5-*a*)pyridin-6-yl)-2-(6-methylpyridin-2-yl)ethane-1,2-dione (4.00 g, 15.02 mmol) in a mixture of *tert*-butyl methyl ether (30 mL) and MeOH (30 mL) were added 3-(fomylmethyl)benzonitrile (prepared according to the method described in WO 02/096875 A1) (6.54 g, 45.07 mmol) and $NH_4OAc$ (11.58 g, 150.24 mmol), and the mixture was stirred at room temperature for 90 min. The pH of the mixture was adjusted to 8 with saturated aqueous $NaHCO_3$ solution. After removal of solvent, the mixture was extracted with EtOAc (2 × 150 mL), and the EtOAc solution was washed with water (50 mL) and brine (50 mL), dried over anhydrous $Na_2SO_4$, filtered, and evaporated to dryness under reduced pressure. The residue was purified by MPLC on silica gel using a mixture of MeOH and $CH_2Cl_2$ (1:19 (v/v)) as eluent to give the titled compound (1.92 g, 33%) as a yellow solid. $^1H$ NMR (400 MHz, DMSO-$d_6$) δ 12.70 (br s, 1 H), 9.53 (br s, 1 H), 8.49 (s, 1 H), 7.96 (dd, 1 H, *J* = 9.2, 1.8 Hz), 7.84 (d, 1 H, *J* = 2.0 Hz), 7.82 (d, 1 H, *J* = 9.2 Hz), 7.74-7.71 (m, 2 H), 7.69 (t, 1 H, overlapped, *J* = 7.6 Hz), 7.56 (t, 1 H, *J* = 7.8 Hz), 7.47 (br s, 1 H), 7.15 (d, 1 H, *J* = 7.6 Hz), 4.18 (s, 2 H), 2.47 (s, 3 H); MS (ESI) m/z 392.18 ($MH^+$).

**Practice Example 7**

Preparation of 3-((4-([1,2,4]triazolo[1,5-*a*]pyridin-6-yl)-5-(6-methylpyridin-2-yl)-1*H*-imidazol-2-yl)methyl)benzamide (Example 147)

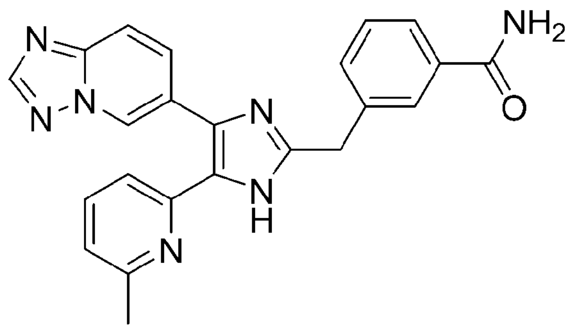

To a stirred solution of 3-((4-([1,2,4]triazolo[1,5-*a*]pyridin-6-yl)-5-(6-methylpyridin-2-yl)-1*H*-imidazol-2-yl)methyl)benzonitrile (41 mg, 0.10 mmol) in EtOH (2 mL) were added 28% $H_2O_2$ (13.9 mL, 0.11 mmol) and 1N NaOH (0.39 mL, 0.39 mmol) at room temperature. The mixture was heated to at 60°C for 1 h and then, to it, was added 1 N HCl at 0°C to adjust pH 7-8. After removal of the solvent, the residue was extracted with $CH_2Cl_2$ (2 × 15 mL). The $CH_2Cl_2$ solution was washed with water (5 mL) and brine (5 mL), dried over anhydrous $Na_2SO_4$, filtered, and evaporated to dryness under reduced pressure. The residue was purified by MPLC on silica gel using a mixture of MeOH and $CH_2Cl_2$ (1:9 (v/v)) as eluent to give the titled compound (15 mg, 35%) as a yellow solid. $^1H$ NMR (400 MHz, $CDCl_3$) δ 8.93 (br s, 1 H), 8.32 (s, 1 H), 7.77 (s, 1 H), 7.76 (dd, 1 H, overlapped, *J* = 9.2, 1.6 Hz), 7.69 (d, 1 H, *J* = 9.2 Hz), 7.56 (d, 1 H, *J* = 8.0 Hz), 7.44 (t, 1 H, *J* = 7.6 Hz), 7.39 (d, 1 H, *J* = 7.6 Hz), 7.26 (t, 1 H, *J* = 8.0 Hz), 7.21 (d, 1 H, *J* = 7.6 Hz), 6.98 (d, 1 H, *J* = 7.6 Hz), 6.60 (br s, 1 H), 6.27 (br s, 1 H), 4.15 (s, 2 H), 2.44 (s, 3 H); MS (ESI) *m/z* 410.19 ($MH^+$).

The compounds listed in the following Table 2 were prepared in an analogous manner to those described in the Practice Examples 5-7 above. The mass spectroscopy data of these compounds are included in the Table 2.

**TABLE 2**

| Example | Structure | $^1H$ NMR (ppm) | MS (ESI) m/z ($MH^+$) |
|---|---|---|---|
| **140** | 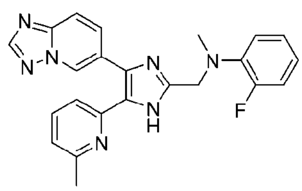 | (400 MHz, $CDCl_3$) δ 8.97 (br s, 1 H), 8.37 (s, 1 H), 7.82 (dd, 1 H, *J* = 9.2, 1.6 Hz), 7.78 (dd, 1 H, *J* = 9.2, 1.2 Hz), 7.49 (t, 1 H, *J* = 7.8 Hz), 7.25 (br d, 1 H, *J* = 7.6 Hz), 7.14-7.06 (m, 3 H), 7.04 (d, 1 H, *J* = 7.6 Hz), 7.00-6.94 (m, 1 H), 4.44 (s, 2 H), 2.91 (s, 3 H), 2.58 (s, 3 H) | 414.20 |
| **141** | 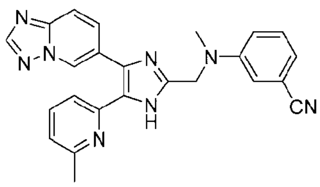 | (400 MHz, $CDCl_3$) δ 8.93 (s, 1 H), 8.37 (s, 1 H), 7.80-7.78 (m, 2 H), 7.48 (t, 1 H, *J* = 7 6 Hz), 7.35-7.30 (m, 1 H), 7.24 (br d, 1 H, *J* = 8.0 Hz), 7.08-7.05 (m, 3 H), 7.02 (d, 1 H, *J* = 7.6 Hz), 4.67 (s, 2 H), 3.14 (s, 3 H), 2.50 (s, 3 H) | 421.20 |

(continued)

| Example | Structure | $^1$H NMR (ppm) | MS (ESI) m/z (MH$^+$) |
|---|---|---|---|
| **142** | 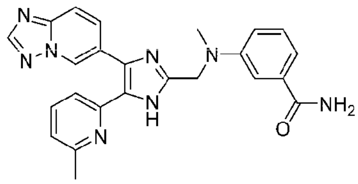 | (400 MHz, $CDCl_3/CD_3OD$) δ 8.86 (dd, 1 H, *J* = 1.6, 1.2 Hz), 8.18 (s, 1 H), 7.62 (dd, 1 H, *J* = 9.2, 1.6 Hz), 7.57 (dd, 1 H, *J* = 9.2, 1.2 Hz), 7.43 (t, 1 H, *J* = 8.0 Hz), 7.24 (dd, 1 H, *J* = 2.4, 1.6 Hz), 7.14 (t, 1 H, overlapped, *J* = 8.0 Hz), 7.13 (d, 1 H, overlapped, *J* = 8.0 Hz), 7.04 (ddd, 1 H, *J* = 8.0, 1.2, 0.8 Hz), 6.97 (d, 1 H, *J* = 7.6 Hz), 6.83 (ddd, 1 H, *J* = 8.0, 2.4, 0.8 Hz), 4.54 (s, 2 H), 2.99 (s, 3 H), 2.38 (s, 3 H) | 439.22 |
| **143** | 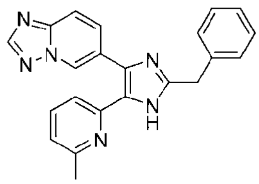 | (400 MHz, $CDCl_3$) δ 8.94 (br s, 1 H), 8.36 (s, 1 H), 7.81 (dd, 1 H, *J* = 9.2, 1.6 Hz), 7.77 (dd, 1 H, *J* = 9.2, 0.8 Hz), 7.44 (t, 1 H, *J* = 7.8 Hz), 7.40-7.28 (m, 5 H), 7.20 (br d, 1 H, *J* = 8.0 Hz), 6.99 (d, 1 H, *J* = 7.6 Hz), 4.21 (s, 2H), 2.51 (s, 3 H) | 367.18 |
| **144** | 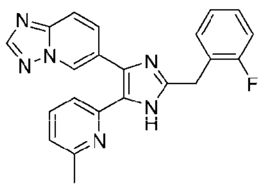 | (400 MHz, $CDCl_3$) δ 8.93 (t, 1 H, *J* = 1.2 Hz), 8.37 (s, 1 H), 7.80 (dd, 1 H, overlapped, *J* = 9.2, 1.6 Hz), 7.77 (dd, 1 H, overlapped, *J* = 9.2, 0.8 Hz), 7.46 (t, 1 H, *J* = 7.8 Hz), 7.39 (td, 1 H, *J* = 7.6, 1.6 Hz), 7.31-7.25 (m, 1H), 7.21 (br d, 1H, *J* = 8.0 Hz), 7.14 (td, 1 H, overlapped, *J* = 7.6. 1.2 Hz), 7.10 (dd, 1 H, *J* = 8.4, 1.2 Hz), 7.02 (br d, 1 H, *J* = 7.6 Hz), 4.24 (s, 2 H), 2.55 (s, 3 H) | 385.17 |
| **145** | 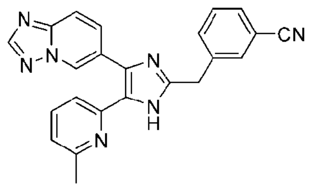 | (400 MHz, DMSO-$d_6$) δ 12.70 (br s, 1H), 9.53 (br s, 1 H), 8.49 (s, 1 H), 7.96 (dd, 1 H, *J* = 9.2, 1.8 Hz), 7.84 (d, 1 H, *J* = 2.0 Hz), 7.82 (d, 1 H, *J* = 9.2 Hz), 7.74-7.71 (m, 2 H), 7.69 (t, 1 H, overlapped, *J* = 7.6 Hz), 7.56 (t, 1 H, *J* = 7.8 Hz), 7.47 (br s, 1 H), 7.15 (d, 1 H *J* = 7.6 Hz), 4.18 (s, 2 H), 2.47 (s, 3H) | 392.18 |
| **146** | 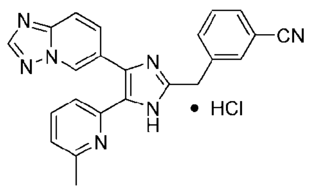 • HCl | (400 MHz, DMSO-$d_6$) δ 9.51 (dd, 1 H, *J* = 1.6, 0.8 Hz), 8.65 (s, 1 H), 8.08 (br s, 1 H), 7.97 (dd, 1 H, overlapped, *J* = 9.2, 0.8 Hz), 7.95 (br d, overlapped, 1 H, *J* = 8.0 Hz), 7.87 (dd, 1 H, *J* = 9.2, 1.6 Hz), 7.85-7.79 (m, 2 H), 7.63 (dd, 1 H, overlapped, *J* = 7.6, 1.2 Hz), 7.61 (t, 1 H, overlapped, *J* = 7.6 Hz), 7.36 (d, 1 H, *J* = 7.6 Hz), 4.55 (s, 2 H), 2.50 (s, 3 H) | |
| **147** | 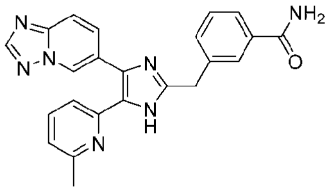 | (400 MHz, $CDCl_3$) δ 8.93 (br s, 1 H), 8.32 (s, 1 H), 7.77 (s, 1 H), 7.76 (dd, 1 H, overlapped, *J* = 9.2, 1.6 Hz), 7.69 (d, 1 H, *J* = 9.2 Hz), 7.56 (d, 1 H, *J* = 8.0 Hz), 7.44 (t, 1 H, *J* = 7.6 Hz), 7.39 (d, 1 H, *J* = 7.6 Hz), 7.26 (t, 1 H, *J* = 8.0 Hz), 7.21 (d. 1 H, *J* = 7.6 Hz), 6.98 (d, 1 H, *J* = 7.6 Hz), 6.60 (br s, 1 H), 6.27 (br s, 1 H), 4.15 (s, 2 H), 2.44 (s, 3 H) | 410.19 |
| **148** | 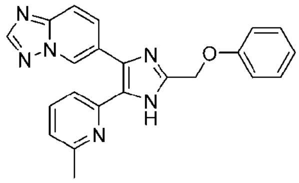 | (400 MHz, $CDCl_3$) δ 8.94 (s, 1 H), 8.37 (s, 1 H), 7.77-7.72 (m, 2 H), 7.51 (t, 1 H, *J* = 7.6 Hz), 7.32-7.25 (m, 3H), 7.06 (d, 1 H, *J* = 7.6 Hz), 7.00 (t, 1 H, overlapped, *J* = 7.6 Hz), 6.97 (d, 2 H, overlapped, *J* = 8.0 Hz), 5.22 (s, 2H), 2.54 (s, 3 H) | 383.17 |

(continued)

| Example | Structure | **$^1$H NMR** (ppm) | MS (ESI) m/z (MH$^+$) |
|---|---|---|---|
| **149** | 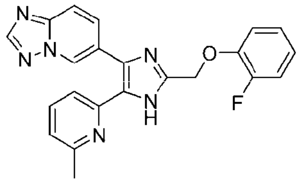 | (400 MHz, $CDCl_3$) δ 8.95 (t, 1 H, *J* = 1.2 Hz), 8.38 (s, 1 H), 7.80 (dd, 1 H, *J* = 9.2, 0.8 Hz), 7.77 (dd, 1 H, *J* = 9.2, 1.2 Hz), 7.56 (t, 1H, *J* = 8.0 Hz), 7.31 (d, 1 H, *J* = 7.6 Hz), 7.19 (td, 1 H, *J* = 8.0, 1.6 Hz), 7.14-7.06 (m, 3 H), 7.01-6.95 (m, 1 H), 5.34 (s, 2 H), 2.64 (s, 3 H) | 401.17 |
| **150** | 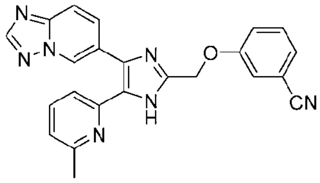 | (400 MHz, $CDCl_3/CD_3OD$) δ 8.94 (dd, 1 H, *J* = 1.6, 1.2 Hz), 8.32 (s, 1 H), 7.78 (dd, 1 H, *J* = 9.2, 1.6 Hz), 7.73 (dd, 1 H, *J* = 9.2, 1.2 Hz), 7.52 (t, 1 H, *J* = 7.8 Hz), 7.42-7.37 (m, 1 H), 7.31-7.26 (m, 3 H), 7.25 (br d, 1 H, overlapped, *J* = 8.0 Hz), 7.07 (d, 1 H, *J* = 7.6 Hz), 5.23 (s, 2 H), 2.55 (s, 3H) | 408.17 |
| **151** | 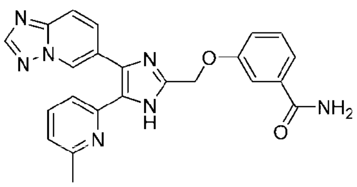 | (400 MHz, $CDCl_3CD_3OD$) δ 8.94 (t, 1 H, *J* = 1.6 Hz), 8.31 (s, 1 H), 7.76 (dd, 1 H, *J* = 9.2, 1.6 Hz), 7.70 (dd, 1 H, *J* = 9.2, 0.8 Hz), 7.51-7.47 (m, 2 H), 7.42 (ddd, 1 H, *J* = 8.0, 2.4, 1.2 Hz), 7.31 (t, 1 H, *J* = 8.0 Hz), 7.23 (d, 1H, *J* = 7.6 Hz), 7.12 (ddd, 1 H, *J* = 8.0, 2.4, 0.8 Hz), 7.04 (d, 1 H, *J* = 7.6 Hz), 5.22 (s, 2 H), 2.51 (s, 3 H) | 426.18 |
| **152** | 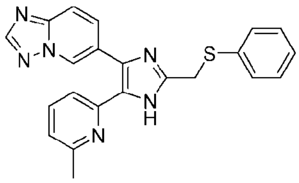 | (400 MHz, $COCl_3$) δ 8.89 (t, 1 H, *J* = 1.4 Hz), 8.35 (s, 1 H), 7.76-7.19 (m, 2 H), 7.45 (t, 1H, *J* = 7.8 Hz), 7.39-7.36 (m, 2 H), 7.30-7.26 (m, 2 H), 7.23-7.19 (m, 2 H), 7.01 (d, 1 H, *J* = 7.6 Hz), 4.26 (s, 2 H), 2.49 (s, 3 H) | 399.15 |
| **153** | 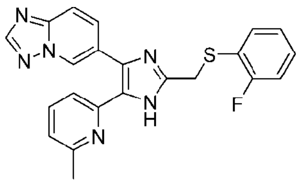 | (400 MHz, $CDCl_3$) δ 8.86 (br s, 1 H), 8.37 (s, 1 H), 7.78 (dd, 1H, *J* = 9.2, 0.8 Hz), 7.72 (dd, 1 H, *J* = 9.2, 1.6 Hz), 7.54-7.48 (m, 2 H), 7.31-7.27 (m, 1 H), 7.25 (br d, 1 H, *J* = 8.0 Hz), 7.13-7.08 (m, 2 H), 7.06 (d, 1 H, *J* = 8.0 Hz), 4.30 (s, 2 H), 2.64 (s, 3 H) | 417.15 |

**Biological Data**

The biological activity of the compounds of the invention may be assessed using the following assays:

Cell-Free Assay for Evaluating Inhibition of ALK5 Kinase Phosphorylation

ALK5 protein was expressed in Sf9 insect cells as human recombinant GST-fusion protein using the baculovirus expression system. Expressed protein was purified by affinity chromatography using GSH-agarose (Sigma-Aldrich). Kinase assay was performed in 96-well FlashPlates™ from Perkin Elmer (Boston, MA, USA) in a 50 μL reaction volume. The reaction cocktail was pipetted in four steps in the following order: 20 μL of assay buffer (standard buffer), 5 μL of ATP solution in $H_2O$, 5 μL of each test compounds of formula (I) in 10% DMSO, 10 μL of GSK3 (14-27) (200 ng)/10 μL of ALK5 solution (1 ng) (premixed). The reaction cocktail contained 60 mM HEPES-NaOH, pH 7.5, 3 mM $MgCl_2$, 3 mM $MaCl_2$, 3 μM $Na_3VO_4$, 1.2 mM DTT, 50 μg/mL $PEG_{20000}$, 1 μM [γ-$^{33}$P]-ATP (approximately 2.5 $10^5$ **Error! Digit expected.**cpm per well), 200 ng/10 μL GSK3 (14-27), and 1 ng/10 μL ALK5. The reaction cocktail was incubated at 30°C for 60 min. The reaction was stopped with 50 μL of 2% (v/v) $H_3PO_4$, and plates were aspirated and washed two times with 200 μL 0.9% (w/v) NaCl. Assay was performed with a BeckmanCoulter Biomek 2000 robotic system. Incorporation of $^{33}P_i$ (counting of "cpm") was determined with a microplate scintillation counter

(Microbeta, Wallac).

Compounds of formula (I) typically exhibited $IC_{50}$ values of less than 1 μM; some exhibited $IC_{50}$ values of less than 0.1 μM; and some even exhibited $IC_{50}$ values less than 10 nM, which is shown in the table 3.

**Table 3**

| Example | $IC_{50}$ (nM) | Example | $IC_{50}$ (nM) |
|---|---|---|---|
| **1** | 13.10 | **51** | 12.30 |
| **2** | 6.68 | **52** | 23.40 |
| **5** | 9.41 | **53** | 60.10 |
| **6** | 17.40 | **54** | 9.93 |
| **7** | 8.96 | **55** | 6.70 |
| **8** | 9.84 | **58** | 39.60 |
| **9** | 6.39 | **59** | 19.50 |
| **10** | 9.41 | **60** | 4.83 |
| **12** | 6.16 | **63** | 32.10 |
| **14** | 10.70 | **64** | 20.60 |
| **15** | 12.30 | **65** | 29.70 |
| **16** | 7.54 | **66** | 9.14 |
| **17** | 14.60 | **67** | 54.60 |
| **18** | 6.54 | **68** | 11.00 |
| **20** | 4.53 | **69** | 24.50 |
| **22** | 17.10 | **70** | 12.80 |
| **23** | 13.90 | **71** | 59.10 |
| **24** | 9.07 | **72** | 16.60 |
| **26** | 15.60 | **73** | 78.00 |
| **27** | 18.00 | **74** | 39.50 |
| **28** | 17.40 | **75** | 15.40 |
| **29** | 14.30 | **76** | 39.00 |
| **30** | 10.60 | **77** | 15.30 |
| **31** | 8.01 | **78** | 15.10 |
| **33** | 14.20 | **79** | 46.60 |
| **34** | 16.20 | **138** | 9.40 |
| **35** | 46.00 | **139** | 23.20 |
| **36** | 14.20 | **140** | 49.80 |
| **37** | 11.90 | **141** | 118.00 |
| **40** | 29.60 | **142** | 153.00 |
| **42** | 14.40 | **143** | 19.80 |
| **43** | 14.30 | **144** | 25.10 |
| **44** | 48.10 | **145** | 12.40 |
| **45** | 57.50 | **147** | 40.00 |
| **46** | 44.20 | **148** | 13.10 |
| **47** | 14.40 | **149** | 25.40 |
| **48** | 24.10 | **150** | 23.90 |
| **49** | 15.30 | **151** | 22.50 |
| **50** | 50.70 | **152** | 20.70 |

**Cell-Free Assay for Evaluating Inhibition of ALK4 Kinase Phosphorylation**

Inhibition of the ALK4 kinase phosphorylation by test compounds of formula (I) can be determined in a similar manner to that described above for ALK5 inhibition except that GST-tagged ALK4 (Invitrogen Corporation) and RBER-CHKtide are used in place of the GST-tagged ALK5 and GSK3 (14-27).

Compounds of formula (I) typically exhibited $IC_{50}$ values of less than 1 μM; some exhibited $IC_{50}$ values of less

than 0.1 μM; and some even exhibited $IC_{50}$ values less than 10 nM.

**Kinase Selectivity Profiling**

Kinase assays were performed in 96-well FlashPlates™ from Perkin Elmer in a 50 μL reaction volume. The reaction cocktail was pipetted in four steps in the following order: 15 μL of ATP solution in $H_2O$, 20 μL of assay buffer (standard buffer), 5 μL of Example 2 in 10% DMSO, 10 μL of enzyme/substrate mixture in $H_2O$. The reaction cocktail contained 70 mM HEPES-NaOH, pH 7.5, 3 mM $MnCl_2$, 3 μM $Na_3VO_4$, 1.2 mM DTT, 1 μM [γ-$^{33}$P]-ATP (approximately 6 $10^5$ **Error! Digit expected.**cpm per well), protein kinase (variable amounts), and substrate (variable amounts). The reaction cocktails were incubated at 30°C for 60 min. The reaction was stopped with 50 μL of 2% (v/v) $H_3PO_4$, and plates were aspirated and washed two times with 200 μL 0.9% (w/v) NaCl. ALL assays were performed with a Beckman-Coulter Biomek 2000/SL robotic system. Incorporation of $33P_i$ (counting of "cpm") was determined with a microplate scintillation counter.

**Table 4**

| protein kinase | $IC_{50}$(μM) | % inhibition at 10 μM | % inhibition at 1 μM |
|---|---|---|---|
| ALK5 | 0.00668 | | |
| ALK4 | 0.0173 | | |
| P38α | 1.720 | | |
| VEGF-R1 | 0.391 | | |
| VEGF-R2 | 0.097 | | |
| VEGF-R3 | 0.257 | | |
| ALK1 | | 66 | 20 |
| ALK2 | | 71 | 17 |
| ALK3 | | 27 | -6 |
| AKT1 | | 3 | 0 |
| CDK1/CycA | | 2 | 5 |
| CHK1 | | 5 | -2 |
| DAPK1 | | 11 | 5 |
| EGF-R wt | | 43 | -8 |
| ERK1 | | 66 | 17 |
| GSK3α | | 15 | 10 |
| MEK1 wt | | 47 | 14 |
| MET wt | | 12 | -5 |
| MST1 | | 3 | -7 |
| PAK1 | | -3 | -7 |
| PDGFRα wt | | 91 | 53 |
| PDGFRβ | | 87 | 47 |
| PKA | | 18 | -7 |
| PKCα | | 4 | -9 |
| ROCK1 | | 9 | 3 |
| RPS6KA1 | | 14 | 6 |
| STK23 | | -1 | -4 |

**Assay for Evaluating Cellular Inhibition of TGF-β Signaling**

HaCaT-3TP-Luc stable cells or 4T1-3TP-Luc stable cells that have p3TP-Luc (neo) expression plasmid were seeded at 2.5× 104 cells/well or 5×105 cells/well in 96-well plate, respectively. Cells were concomitantly treated with TGF-β1 (2 ng/mL) in 0.2% FBS in the presence or absence of each test compounds of formula (I) at approximately 60-70% confluence for 24 h at 37°C in 5% $CO_2$. Cell lysates were prepared using Luciferase Assay System (Promega) according to the manufacturer's instruction, and luminescence was measured by a luminometer, Micro Lumat Plus (Berthold, Germany).

Compounds of formula (I) typically exhibited $IC_{50}$ values of less than 1 μM; some exhibited $IC_{50}$ values of less than 0.1 μM; and some even exhibited $IC_{50}$ values of less than 10 nM.

**Immunofluorescence Assay**

MCF10A cells were plated on the cover glass in 6-well plate at 2 × $10^5$ cells/well. After 12 h, when cells were attached, 10% FBS medium was changed to 0.5% FBS medium. Twenty-four hours later, cells were treated with TGF-β1 (2 ng/mL) with or without Example 2 (1 μM) for 2 h. Then, cells were fixed with 4% formaldehyde solution for 30 min at room temperature and quenched with quenching solution (50 mM $NH_4Cl$ in PBS) for 15 min. After being washed three times with PBS, cells were incubated with blocking/permeabilization solution (1% BSA and 0.1% Triton X-100 in PBS) for 1 h at room temperature and incubated with anti-Smad2/3 antibody (BD Biosciences, Franklin Lakes, NJ, USA) overnight at 4°C. Fluorescence was visualized by Cy3-conjugated goat anti-mouse IgG (Jackson ImmunoResearch Laboratories, Bar Harbor, ME, USA). Nuclei of the same cells were stained with DAPI solution. Cells were analyzed using the LSM 510 META laser confocal microscopy system (Carl Zeiss, Germany).

**Example 2 suppressed TGF-β1-induced Smad2/3 nuclear translocation in MCF10A cells.**

**Wound Healing Assay**

MCF10A cells were seeded at 2 × $10^5$ cells/well in 6-well plate. When each well was occupied by cells over 80% of the area, 10% FBS was changed to 0.2% FBS. After 24 h, wound was made by a plastic pipette tip, and then cells were treated with TGF-β1 (2 ng/mL) with or without Example 2 (1 μM) for 16 h. Wound area change from 0 to 16 h was calculated based on Image J program (National Institutes of Health, MD, USA) based on phase-contrast images of cells taken by microscope.

**Example 2 suppressed TGF-β1-induced cell migration in MCF10A cells.**

**Matrigel Invasion Assay**

The upper surface of Transwells (6.5 mm diameter, 8 m pore size; Corning, Lowell, MA, USA) were coated with 20 μL diluted Matrigel (BD Biosciences). 4T1 cells were seeded at 4 × $10^4$ cells/well on the upper chamber of transwell in serum free medium with or without TGF-β1 (2 ng/mL) in the presence or absence of Example 2. The lower chamber was filled with 10% FBS with TGF-β1 (2 ng/mL) in the presence or absence of Example 2. After incubation for 20 h at 37°C in 5% $CO_2$, the cells remaining on the upper surface of the membrane were removed with a cotton swab, and DAPI-stained cells remaining on the bottom surface were observed using fluorescence microscopy. Average cell number per view field was obtained from 5 random fields.

**Example 2 suppressed TGF-β1-induced cell invasion in matrigel invasion assay.**

**Cell Growth Study**

Either 4T1 cells or MCF1 0A cells were seeded in 96-well plate at 5×$10^3$ cells per well. After cells were attached, cells were treated with Example 2 dissolved in DMSO in 0.2% serum medium. After incubation for four days, cell viability was determined by SRB assay.

Example 2 showed no effect on 4T1 cell growth and slightly increased MCF10A cell growth without significance, thus, suggesting that the anti-metastatic effect of Example 2 were not due to the primary tumor growth inhibition

**Anti-metastatic Effect on BALB/c 4T1 xenografted mice model**

Female BALB/c mice were purchased from Orient Bio Inc. (Seoul, Korea). Animals were maintained in a temperature-controlled room (22°C) and supplied with food and water ad libitum. 4T1 cells (1.2×$10^4$ cells) were suspended in PBS and implanted into the left #4 mammary fat pad of five to six-week-old female BALB/c mice (day 0). In Experiment 1, treatment was started after tumor implantation (day 0). Example 3 (13.6 or 27.3 mg/kg) dissolved in water was given to mice orally BID five consecutive days per week for four weeks. In Experiment 2, treatment was started on day 4. Example 2 (5, 10, 20, or 40 mg/kg) dissolved in artificial gastric fluid formulation was given to mice orally five consecutive days per week for three weeks. In Experiment 3, treatment was started on day 4. Example 2 (5, 10, 20, or 40 mg/kg) dissolved in artificial gastric fluid formulation was given to mice orally every other day (three times per week) for 24 days. In Experiment 4, 4T1 cells (**Error! Digit expected**.1×104 cells) were suspended in PBS and implanted into the left #4 mammary fat pad of ten-week-old female BALB/c mice (day 0). Treatment was started on day 10. Example 61 (43.6 mg/kg) dissolved in saline was given to mice intraperitoneally every other day for 2.5 weeks. In all Experiments, mice were sacrificed at 24 to 72 h after the last dosing, and 15% India ink solution (Hardy Diagnostics) in PBS was

immediately injected into the trachea. The India ink-stained lungs were isolated and destained with Feket's solution (60% ethanol, 3% formaldehyde, and 4% acetic acid in PBS) for at least 20 min. Number of metastatic nodule was counted on the surface of left lobe of lung, and picture of lung was taken with digital camera. The tumor size was measured using calipers, and the tumor volume was calculated by using the following equation:

$$\text{Tumor volume} = (0.5236) \times (\text{width})^2 \times (\text{length})$$

Examples 2, 3, and 61 significantly reduced the number of metastastic nodules on the lung.

In Experiment 3, Western blot analysis was performed to examine the effect of Example 2 on the Smad2 phosphorylation in tumor tissues. Either vehicle buffer (4 mM HCl, 1 mg/mL BSA) or TGF-β1 (50 ng/mouse) in vehicle buffer was given to mice intravenously at 2 h before mice were sacrificed. Tumor tissues from mice were lysed in RIPA buffer [50 mM Tris, pH 7.5, 150 mM NaCl, 0.1% sodium dodecyl sulfate, 0.5% sodium deoxycholate, 1% NP-40, 1 mM NaF, 1 mM $Na_3VO_4$, 1 mM PMSF, a protease inhibitor cocktail (1 tablet of Roche Diagnostics GmbH protease inhibitor cocktail/10 mL) (Roche)] for 20 min on ice. Lysates were cleared by centrifugation at 13000 rpm at 4°C for 20 min. Protein content of supernatants was determined using Micro-BCA (bicinchoninic acid) protein assay kit (Thermo Scientific). Lysates containing 20-50 μg total protein were separated by electrophoresis on polyacrylamide gel and then electrophoretically transferred to polyvinylidene difluoride transfer membranes (Millipore, Billerica, MA, USA). Membranes were blocked with 5% BSA (Sigma-Aldrich) in PBS containing 0.5% Tween-20 (PBST) for 1 h and incubated overnight at 4º̲C with one of following antibodies: anti-phospho-Smad2 (Millipore), anti-Smad2/3 (BD Transduction Laboratories, NJ, USA), or anti-β-actin (Sigma-Aldrich) in PBST containing 1% BSA. Membranes were washed three times with PBST and incubated with either horseradish peroxidase (HRP)-conjugated goat anti-mouse antibody or HRP-conjugated goat anti-rabbit antibody (SantaCruz Biotechnology, Santa Cruz, CA, USA) at room temperature for 1 h. Bound antibodies were detected using Western Blotting Luminol Reagent (SantaCruz Biotechnology). Band intensities were analyzed using a densitometer LAS-3000 imager (FUJIFILM, Tokyo, Japan).

**Example 2 suppressed TGF-β1-induced Smad2 phosphorylation in tumor tissues.**

**Anti-metastatic Effect on MMTV/c-Neu mice breast cancer model**

MMTV/c-Neu female transgenic mice were purchased from Jackson Laboratory (Bar Harbor, ME, USA). Animals were maintained in a temperature-controlled SPF room (22°C) and supplied with food and water ad libitum. In Experiment 1, Example 61(43.6 mg/kg) dissolved in saline was given to thirty two-week-old MMTV/c-Neu mice intraperitoneally every other day for three weeks. In Experiment 2, Example 3 (43.6 mg/kg) dissolved in saline was given to thirty two-week-old MMTV/c-Neu mice intraperitoneally every other day for ten weeks. Mice were sacrificed at 24 h after the last dosing, and tissues of mammary tumor and lung were analyzed by hematoxylin and eosin (H&E) staining. To analyse β-casein mRNA level in tissues of mammary tumor and lung, total RNAs were isolated from these tissues using TRIzol reagent (Invitrogen Corporation) and RNeasy Mini kit (Qiagen) according to the manufacturer's instruction. The cDNAs were synthesized from 2 μg of total RNAs using random primer (Invitrogen Corporation) by MMLV RTase (Invitrogen Corporation) for 1 h at 37°C and subjected to PCR amplification using Taq polymerase (Promega) and following gene-specific primers: mouse GAPDH (forward) 5'-ATG TGT CCG TCG TGG ATC TGA-3' and (reverse) 5'-TTG AAG TCG CAG GAG ACA ACC-3', mouse β-casein (forward) 5'-TCC CAC AAA ACA TCC AGC C-3' and (reverse) 5'-ACG GAA TGT TGT GGA GTG G-3'. Amplified DNA was analyzed by agarose gel electrophoresis.

Example 61 significantly reduced the number of metastastic lesions in the lung. Significant level of β-casein (a mammary differentiation marker) mRNA was detected in the lung of MMTV/c-Neu mice. Examples 3 and 61 significantly inhibited β-casein mRNA expression level in the lung, demonstrating their anti-metastatic effect.

Activity of MMP-9 and MMP-2 in the primary mammary tumor was measured by gelatin zymography. Tumor tissues from mice (30 mg) was lysed in 500 μL RIPA buffer (50 mM Tris, 150 mM NaCl, 0.1% sodium dodecyl sulfate, 0.5% sodium deoxycholate, 1% NP-40, protease inhibitor without EDTA) for 10-20 min on ice. Lysates were cleared by centrifugation at 13000 rpm at 4°C for 10 min. Protein content of supernatants was determined using Micro-BCA protein assay kit (Thermo Scientific). Loading samples were prepared by adding loading buffer (0.5 M Tris, pH 6.8, 50% glycerol, 10% SDS, and 1% bromophenol blue solution) into lysates containing 15 μg of total protein. Loading samples were heated at 60°C for 5 min and separated by electrophoresis on 10% polyacrylamide gel containing 0.2% gelatin. Gel was washed twice with washing buffer [2.5% Triton-X100, 0.05 M Tris-HCl, pH 7.5, and 0.1 M NaCl] for 30 min at room temperature. Then, the gel was incubated in incubation buffer [0.05 M Tris-HCl, pH 7.5, 0.15 M NaCl, 0.01 M $CaCl_2$, 0.02% $NaN_3$, and 1 μM $ZnCl_2$] at 37°C for 16-18 h with shaking. Gel was stained by 0.5% Coomassie blue R250 solution containing 5% methanol and 10% acetic acid for 2-4 h at room temperature and destained twice by destaining solution

(5% methanol and 10% acetic acid) for 30 min at room temperature. Gel image was obtained using a densitometer LAS-3000 imager (FUJIFILM) in cybergreen mode.

**Example 3 significantly inhibited activity of MMP-9 and MMP-2 in the primary mammary tumor.**

**Anti-fibrotic Effect on Bile Duct-ligated Liver Fibrosis Model**

Six-week-old male Sprague-Dawley (SD) rats were purchased from Orient Bio Inc.. In Experiment 1, SD rats weighing 180-200 g were randomly divided into five experimental groups: sham-operated control rats (n = 5), sham-operated rats treated with Example 3 (43.6 mg/kg, n = 5), bile duct-ligated (BDL) rats (n = 10), BDL rats treated with either 21.8 or 43.6 mg/kg of Example 3 (n = 10). In Experiment 2, SD rats weighing 180-200 g were randomly divided into five experimental groups: sham-operated control rats (n = 5), BDL rats (n = 10), BDL rats treated with either 5, 10, or 20 mg/kg of Example 2 (n = 10). For BDL, the animals were anesthetized with zoletil (20 mg/kg) and xylazine (10 mg/kg), and the common bile duct was exposed and double-ligated using 3-0 silk. The first ligature was placed below the junction of the hepatic duct, and the second was placed above the entrance of the pancreatic duct. The common bile duct was then cut between the double ligatures. In sham-operated rats, an incision was made in the abdomen and then closed without any treatment. Treatment was started within 2 h after surgical procedure. Either Example 3 dissolved in saline (Experiment 1) or Example 2 dissolved in artificial gastric fluid formulation (Experiment 2) was given to rats orally three times per week for four weeks starting from BDL surgery. Animals were maintained in a temperature controlled room (at 21°C) and supplied with autoclaved food and water. At 48 h after the last dosing, animals were killed, and the serum, spleens and livers were removed. The livers were sagittally sliced into several parts, snap frozen in liquid nitrogen, and kept at -70°C. A part of livers was immersed into 10% neutral buffered-formalin for histopathological examinations. All experimental procedures were conducted in accordance with our institutional guidelines.The activity of serum alanine aminotransferase (ALT) and aspartate aminotransferase (AST) was determined using a spectrophotometric enzyme assay kit (Asan Pharm. Co., Ltd., Hwaseong-si, Korea) according to the manufacturer's instruction. Automated instrument was also used to assay general serum biochemistry. Liver specimens were fixed in 10% neutral buffered-formalin prior to routine processing in paraffin-embedded blocks. Sections (5 μm thick) were cut and stained using hematoxylin and eosin (H&E), and examined by light microscopy. Liver tissues were lysed in RIPA buffer [50 mM Tris, pH 7.5, 150 mM NaCl, 1 mM EDTA, 0.1% sodium dodecyl sulfate, 0.5% sodium deoxycholate, 1% NP-40, 50 mM NaF, 1 mM $Na_3VO_4$, 1 mM PMSF, a protease inhibitor cocktail (1 tablet of Roche Diagnostics GmbH protease inhibitor cocktail/10 mL) (Roche)] for 20 min on ice. Lysates were cleared by centrifugation at 13000 rpm at 4°C for 20 min. Protein content of supernatants was determined using Micro-BCA protein assay kit (Thermo Scientific). Lysates containing 20-60 μg total protein were separated by electrophoresis on 6-10% sodium dodecyl sulfate-polyacrylamide gel and then transferred to nitrocellulose (Whatman®, Germany) or polyvinylidene difluoride membranes (Millipore). Membranes were blocked with 5% BSA (Sigma-Aldrich) or 5% nonfat dry milk solution for 1 h and incubated overnight at 4ºC with one of following antibodies: rabbit antiphospho-Smad3 (Cell Signaling Technology, Beverly, MA, USA), rabbit anti-α-SMA (Millipore), mouse anti-fibronectin, mouse anti-vimentin (BD Biosciences), or mouse anti-β-actin (Sigma-Aldrich). Membranes were washed three times with Tris-buffered saline and incubated with either HRP-conjugated goat anti-rabbit antibody or HRP-conjugated goat anti-mouse antibody (SantaCruz Biotechnology) at room temperature for 1 h. Bound antibodies were detected using an ECL kit (GE Healthcare, Princeton, NJ, USA). Band intensities were analyzed using a densitometer LAS-3000 imager (FUJIFILM).

BDL rats showed body weight loss and organ (liver and spleen) weight increase compared with sham-operated control rats. Examples 2 and 3 recovered body weight loss and decreased organ (liver and spleen) weight in BDL rats. A significant increase in serum ALT and AST was observed in BDL rats as compared with sham-operated animals. Examples 2 and 3 improved serum ALT and AST in BDL rats. Examples 3 inhibited Smad signaling and suppressed α-SMA, fibronectin, and vimentin in BDL rat liver. Examples 2 suppressed α-SMA and fibronectin in BDL rat liver. BDL rat livers showed typical histological changes characterized by central ± central architecture disruption and bridge fibrosis formation compared with that of normal rat livers. Examples 2 and 3 greatly abolished BDL-induced histological change.

**Table 5**

| Groups (mg/kg) | | Body weight(g) | Organ weight | | | |
|---|---|---|---|---|---|---|
| | | | Liver (g) | Spleen (g) | Liver/Body (%) | Spleen/Body(%) |
| Sham | vehicle | 357 ± 5.2 | 11.7 ± 0.36 | 0.78 ± 0.06 | 3.28 ± 0.07 | 0.22 ± 0.02 |
| | Example 3 (43.6) | 354 ± 12.2== | 11.8 ± 0.71 | 0.81 ± 0.04 | 3.34 ± 0.12 | 0.23 ± 0.01 |

(continued)

| Groups (mg/kg) | | Body weight(g) | Organ weight | | | |
|---|---|---|---|---|---|---|
| | | | Liver (g) | Spleen (g) | Liver/Body (%) | Spleen/Body(%) |
| BDL | vehicle | 308 ± 6.1** | 22.8 ± 1.30** | 2.01 ± 0.14** | 7.43 ± 0.44** | 0.65 ± 0.04** |
| | Example 3 (21.8) | 340 ± 9.6$^{=}$ | 16.4 ± 10.72$^{**,==}$ | 1.30 ± 0.07** | 4.85 ± 0.26$^{**,==}$ | 0.39 ± 0.03$^{**,==}$ |
| | Example 3 (43.6) | 335 ± 8.4 | 16.1 ± 0.57$^{**,==}$ | 1.19 ± 0.09** | 4.84 ± 0.26$^{**,==}$ | 0.36 ± 0.03$^{**,==}$ |

Data represents the mean ± S.E. (n = 5-8).
**: $p$ < 0.01 vs. sham. #: $p$ < 0.05 vs. BDL. ##: $p$ < 0.01 vs. BDL.

**Table 6**

| Groups (mg/kg) | | Body weight(g) | Organ weight | | | |
|---|---|---|---|---|---|---|
| | | | Liver(g) | Spleen (g) | Liver/Body(%) | Spleen/Body(%) |
| Sham | vehicle | 345 ± 6.3 | 12.1 ± 0.77 | 0.83 ± 0.05 | 3.51 ± 0.21 | 0.24 ± 0.02 |
| BDL | vehicle | 315 ± 16.1* | 20.3 ± 2.45** | 2.54 ± 0.29** | 6.39 ± 0.51** | 0.80 ± 0.05** |
| | Example 2 (5) | 324 ± 7.3 | 17.5 ± 1.82 | 2.00 ± 0.28** | 5.46 ± 0.62 | 0.63 ± 0.09** |
| | Example 2 (10) | 312 ± 10.4 | 15.8 ± 1.88 | 1.57 ± 0.12$^{=}$ | 5.08 ± 0.60 | 0.51 ± 0.05$^{=}$ |
| | Example 2 (20) | 312 ± 8.4* | 15.2 ± 1.80 | 1.50 ± 0.16$^{=}$ | 4.88 ± 0.63 | 0.48 ± 0.05$^{=}$ |

Data represents the mean ± S.E. (n = 5-8).
*: $p$ < 0.05 vs. sham. **: $p$ < 0.01 vs. sham. #: $p$ < 0.05 vs. BDL.

**Anti-fibrotic Effect on Bleomycin-induced Lung Fibrosis Model**

Six-week-old male ICR mice were purchased from Orient Bio Inc.. Mice weighing 31-35 g were randomly divided into five experimental groups: sham-operated control mice (saline, n = 6), bleomycin (BLM)-treated mice (n = 10), BLM-treated mice treated with either 5, 10, or 20 mg/kg of Example 2 (n = 10). For the induction of lung fibrosis, mice were anesthetized with zoletil (10 mg/kg) and xylazine (5 mg/kg) and were given BLM (given as BLM sulfate, 1 mg/kg) (MBcell, Los Angeles, CA, USA) dissolved in 60 μL of saline once on day 0 through intratracheal instillation. Example 2 dissolved in artificial gastric fluid formulation was given to mice orally five times per week for two weeks starting from day 7. Animals were maintained in a temperature controlled room (at 21°C) and supplied with autoclaved food and water. At three weeks post-surgery, animals were killed, and the lungs were removed. The lungs were sagittally sliced into several parts, snap frozen in liquid nitrogen, and kept at -70°C. A part of lungs was immersed into 10% neutral buffered-formalin for histopathological examinations. All experimental procedures were conducted in accordance with our institutional guidelines. Lung specimens were fixed in 10% neutral buffered-formalin prior to routine processing in paraffin-embedded blocks. Sections (5 μm thick) were cut, stained using hematoxylin and eosin (H&E), and examined by light microscopy. Lung tissues were lysed in RIPA buffer [50 mM Tris, pH 7.5, 150 mM NaCl, 1 mM EDTA, 0.1% sodium dodecyl sulfate, 0.5% sodium deoxycholate, 1% NP-40, 50 mM NaF, 1 mM $Na_3VO_4$, 1 mM PMSF, a protease inhibitor cocktail (1 tablet of Roche Diagnostics GmbH protease inhibitor cocktail/10 mL) (Roche)] for 20 min on ice. Lysates were cleared by centrifugation at 13000 rpm at 4°C for 20 min. Protein content of supernatants was determined using Micro-BCA protein assay kit (Thermo Scientific). Lysates containing 20-50 μg total protein were separated by electrophoresis on 6-10% sodium dodecyl sulfate-polyacrylamide gel and then transferred to nitrocellulose (Whatman®). Membranes were blocked with 5% nonfat dry milk solution for 1 h and incubated overnight at 4ºC with either rabbit anti-α-SMA (Millipore) or mouse anti-fibronectin (BD Biosciences). Membranes were washed three times with Tris-buffered saline and incubated with either HRP-conjugated goat anti-rabbit antibody or HRP-conjugated goat anti-mouse antibody (SantaCruz Biotechnology) at room temperature for 1 h. Bound antibodies were detected using an ECL kit (GE Healthcare). Band intensities were analyzed using a densitometer LAS-3000 imager (FUJIFILM).

BLM-induced fibrotic lungs showed the elevated levels of α-SMA and fibronectin compared with those of sham-operated animals. Example 2 suppressed α-SMA and fibronectin in BLM-induced fibrotic lung. Lung tissues from the BLM-treated mice showed typical histology that pulmonary interalveolar septa became thickened and infiltrated by inflammatory cells with collagen depositions in the interstitium disclosed. Example 2 greatly reduced BLM-induced histological changes at all dose levels tested.

## Claims

**1.** A pharmaceutical composition, comprising one or more of the compounds

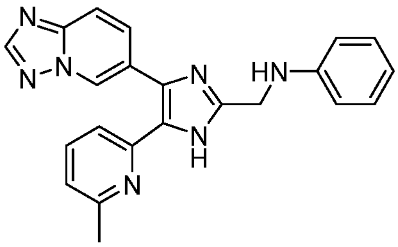,
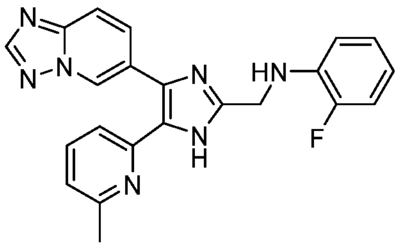,
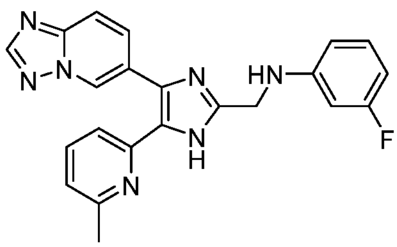,
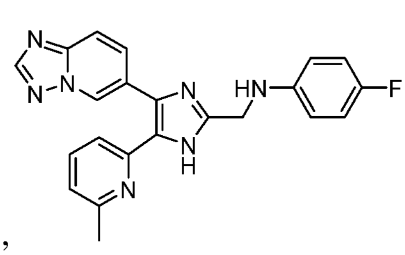,
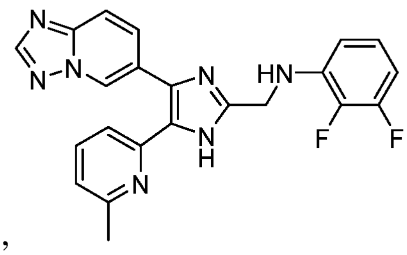,
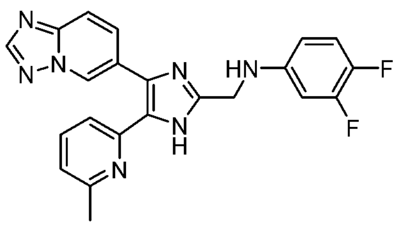,
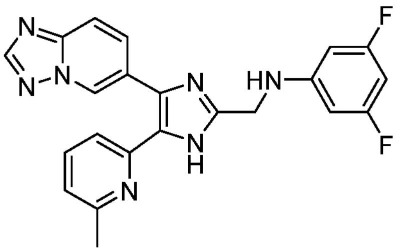,
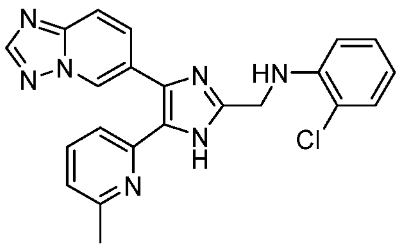,
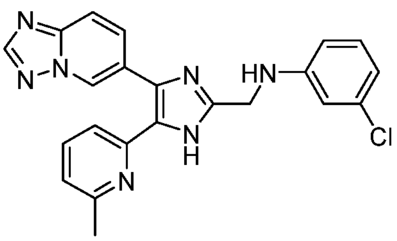,
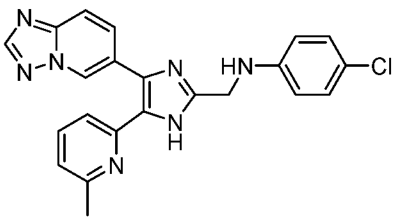,
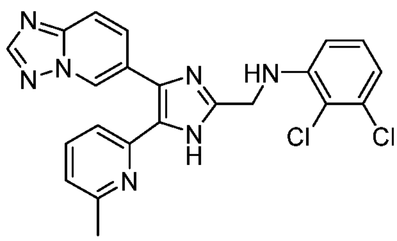,
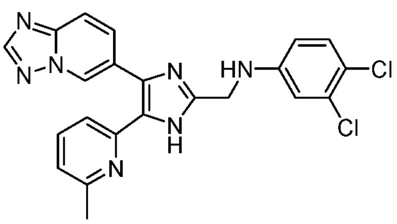,
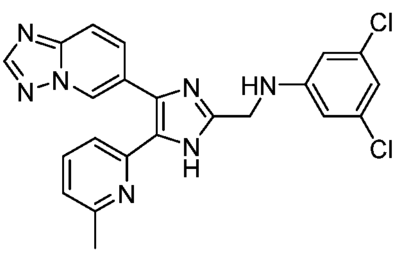,
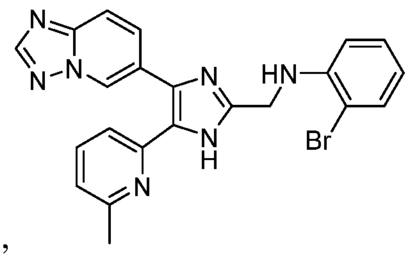,

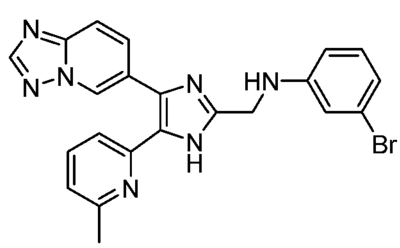,
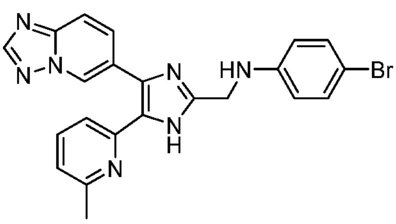,
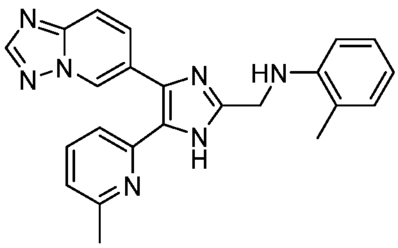,
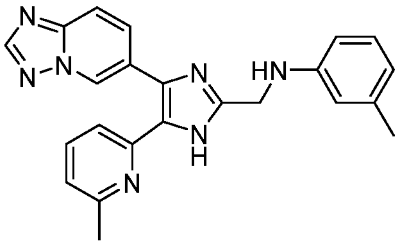,
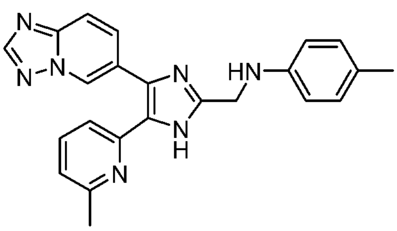,
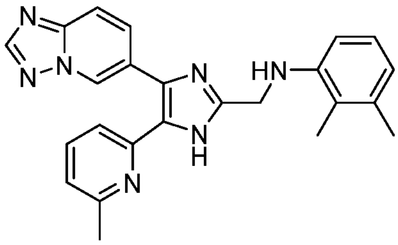,
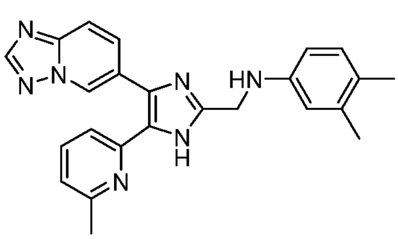,
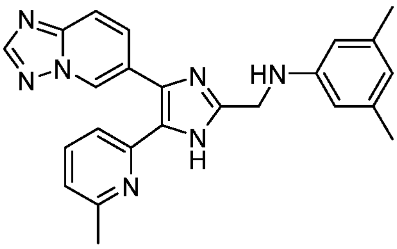,
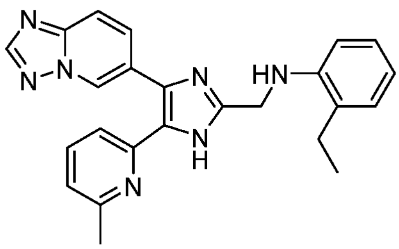,
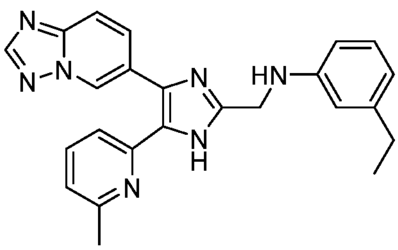,
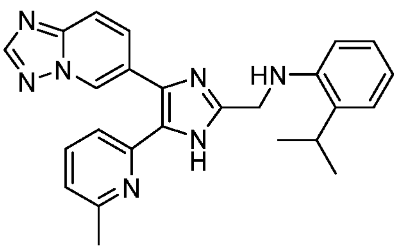,
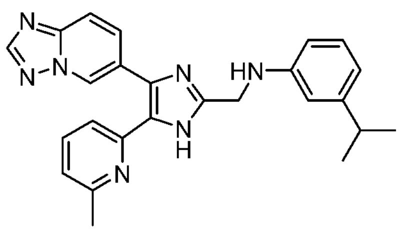,
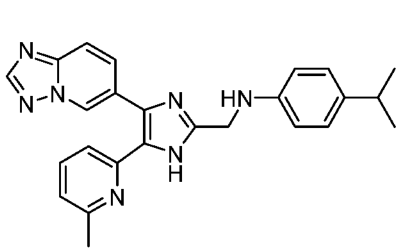,
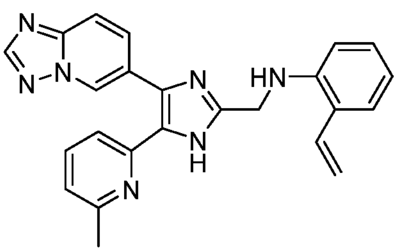,
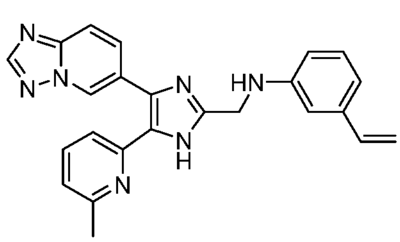,

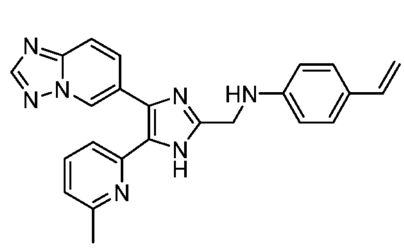,
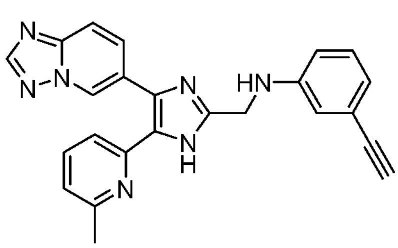,
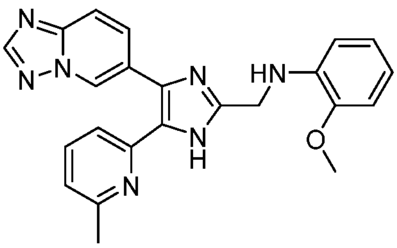,

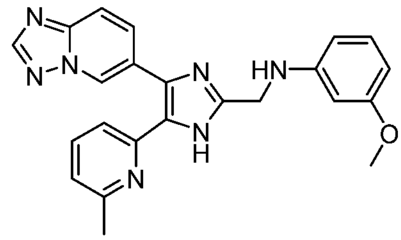,
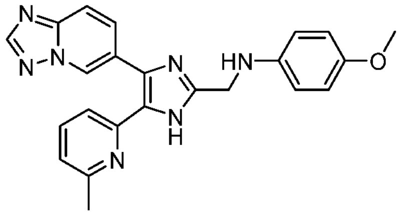,
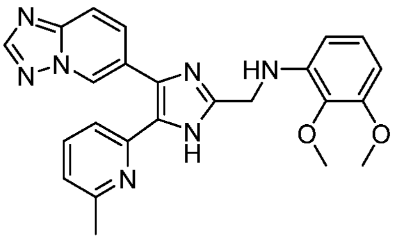,

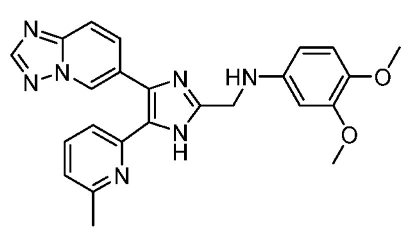,
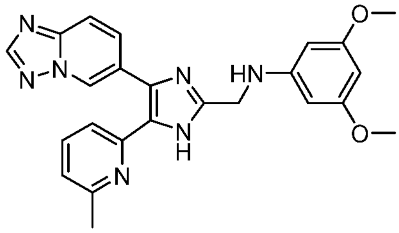,
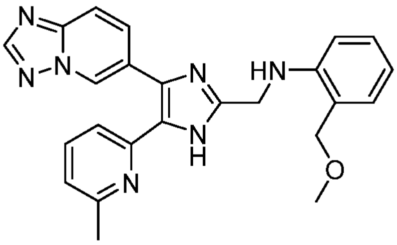,

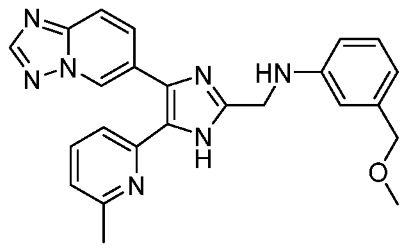,
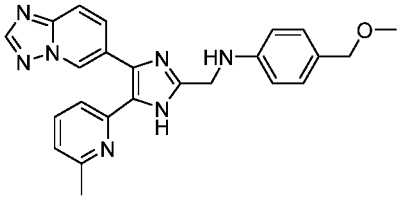,
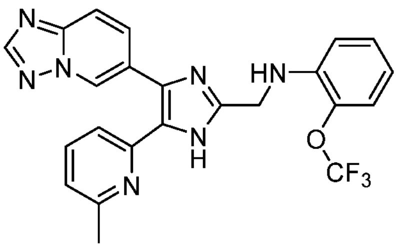,

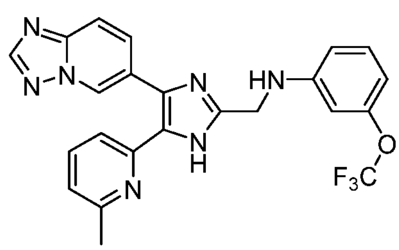,
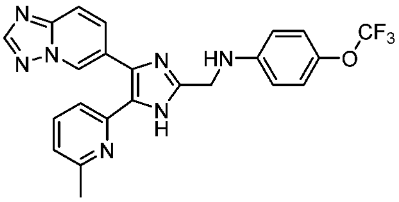,
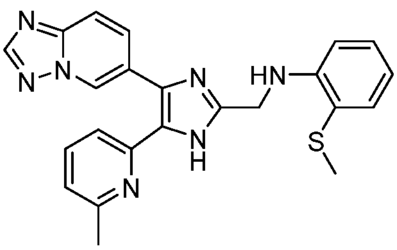,

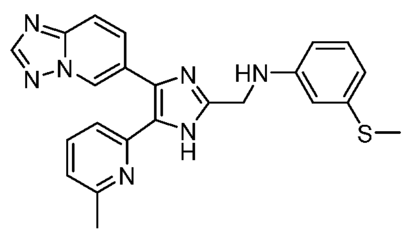,
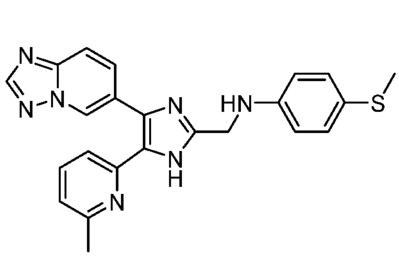,
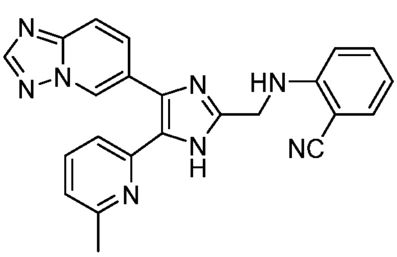,

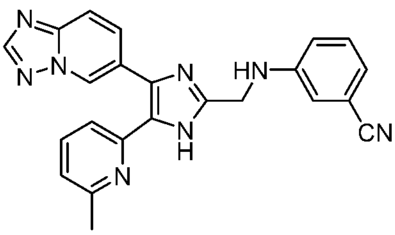 , 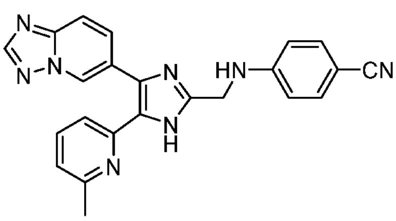 , 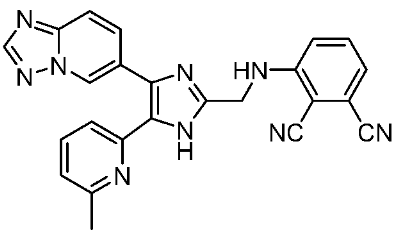

, 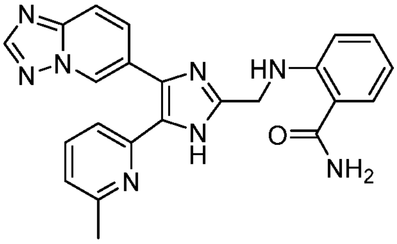 , 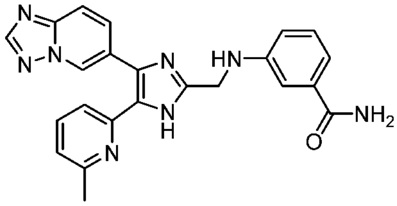 , 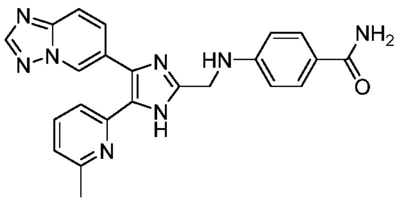

, 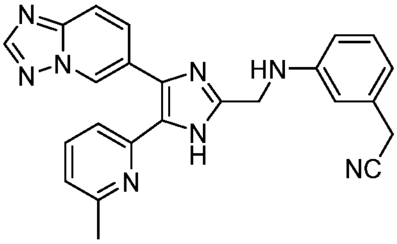 , 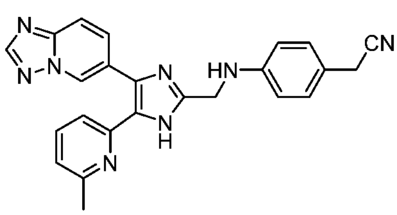 , 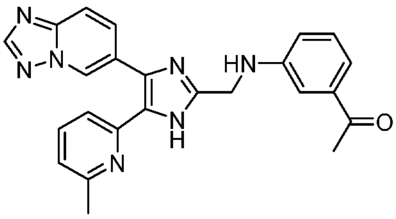

, 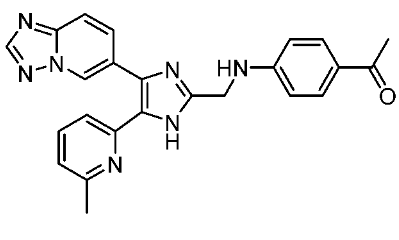 , 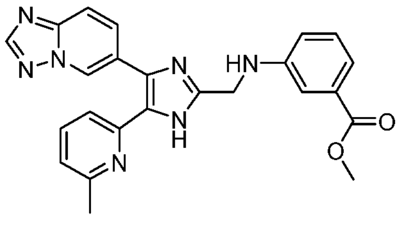 ,

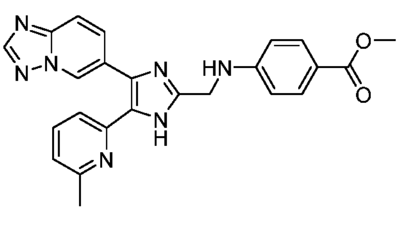 , 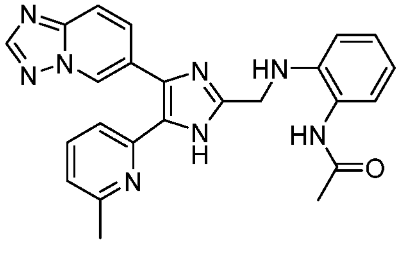 , 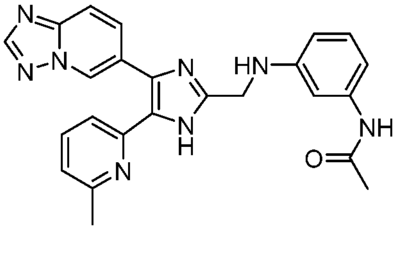

, 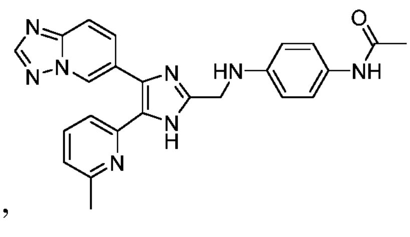 , 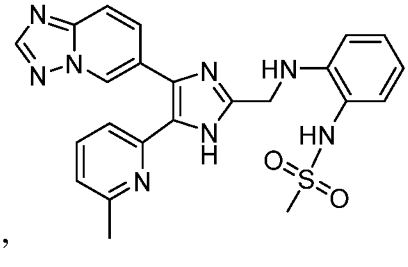 , 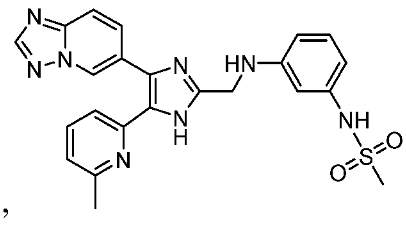

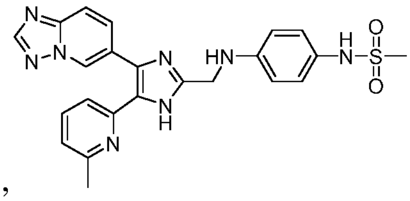,
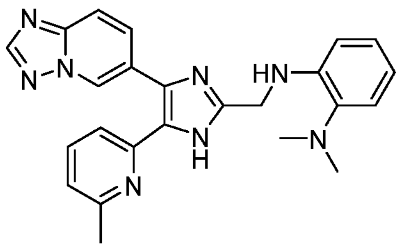,
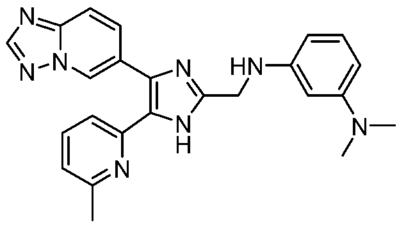,

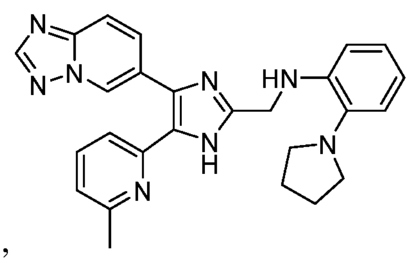,
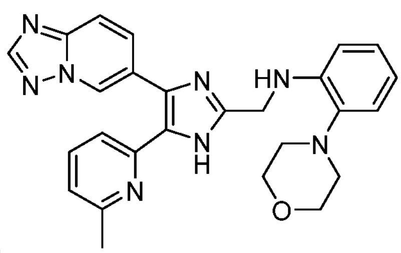,

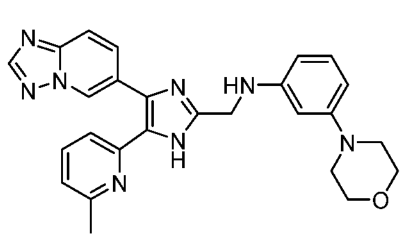,
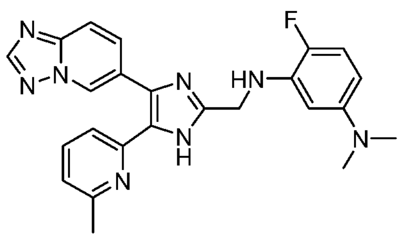,
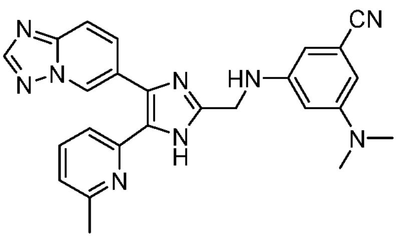,

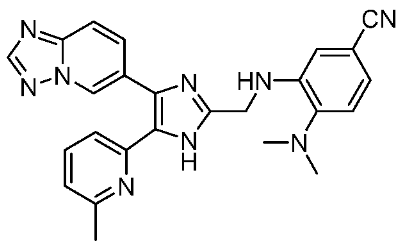,
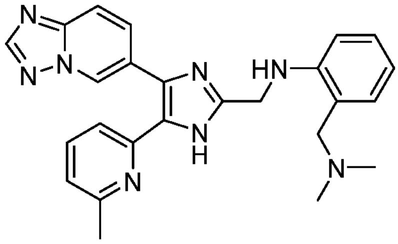,
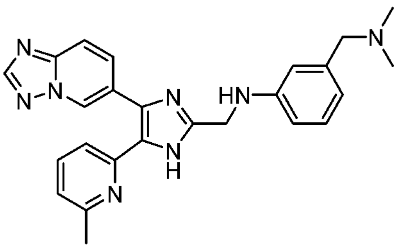,

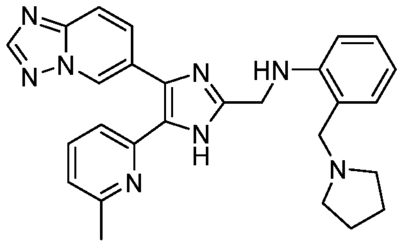,
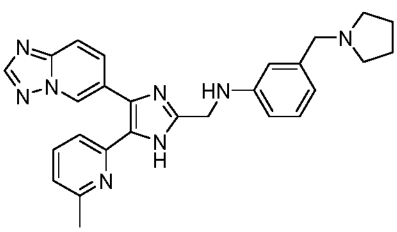,
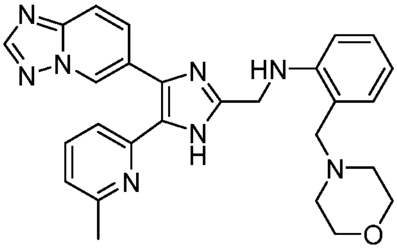,

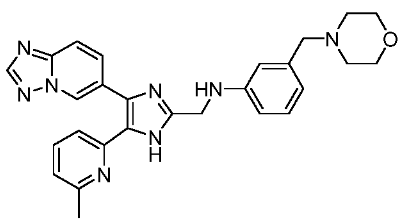,
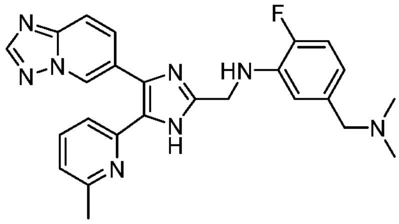,
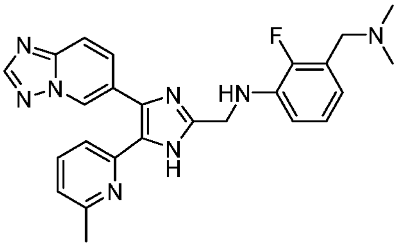,

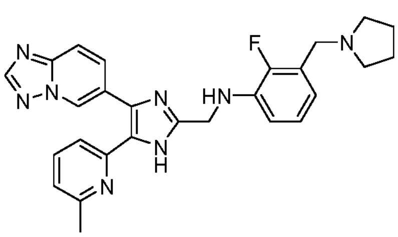,
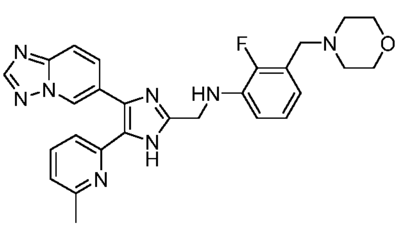,
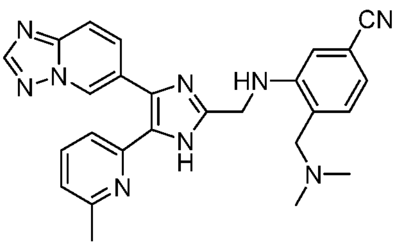,

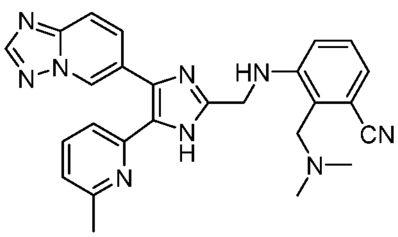,
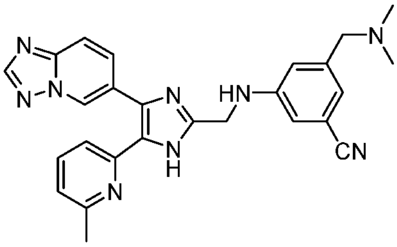,
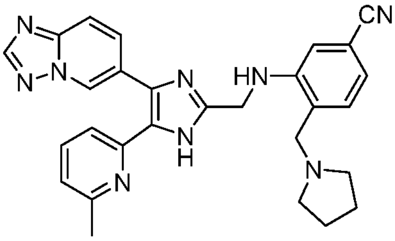,

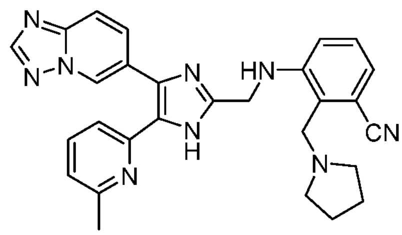,
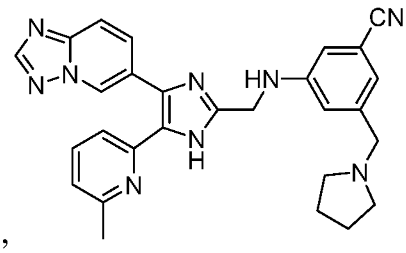,
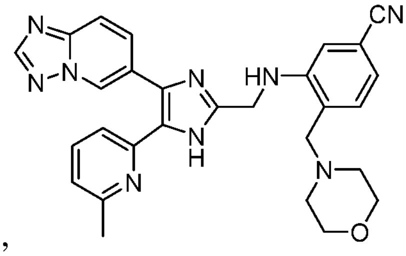

,

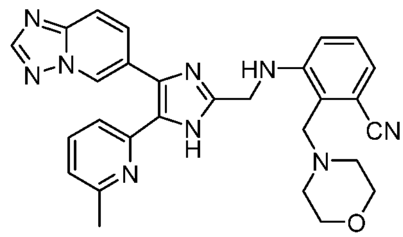,
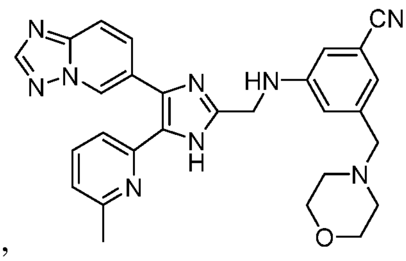,
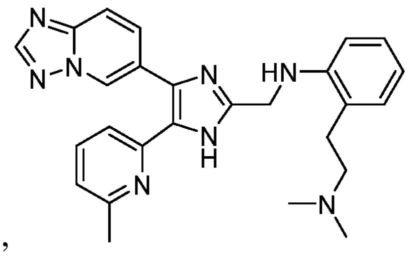

,
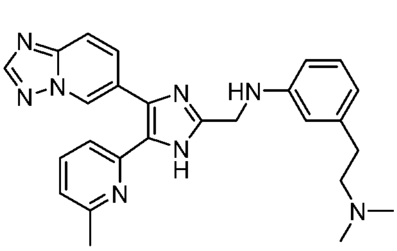,
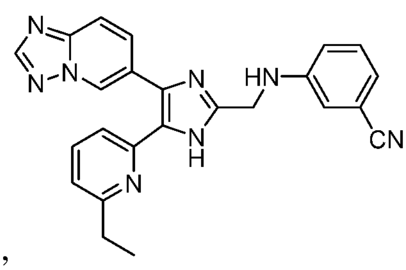,
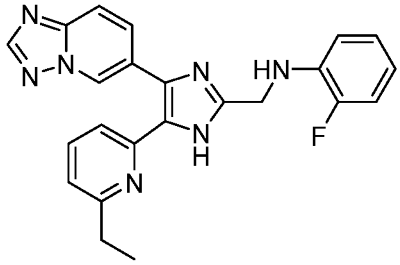

,

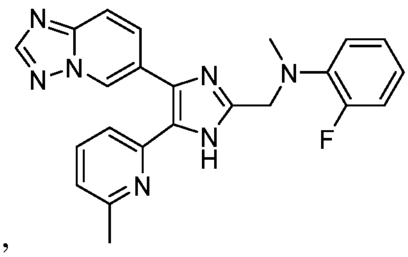

,

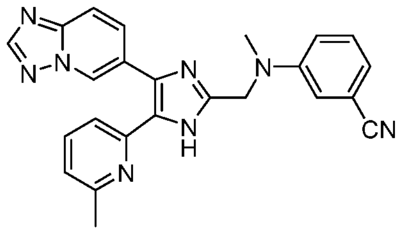

,

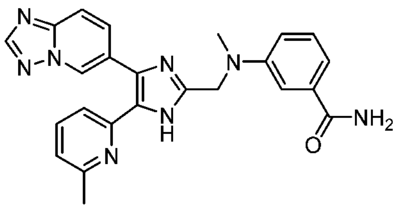

,

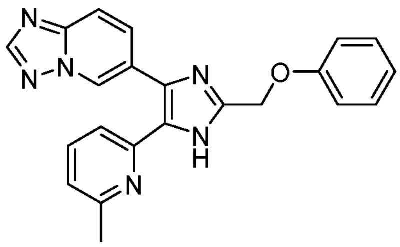

,

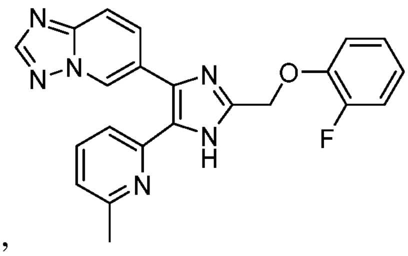

,

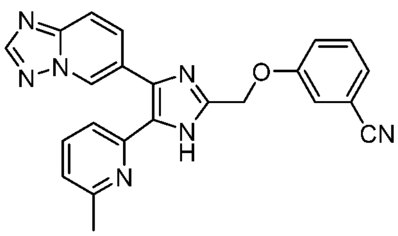

,

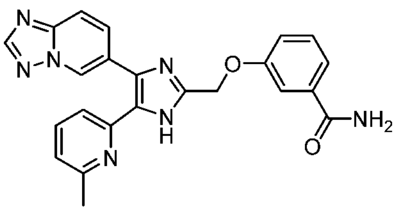

,

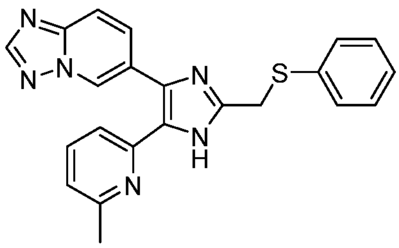

,

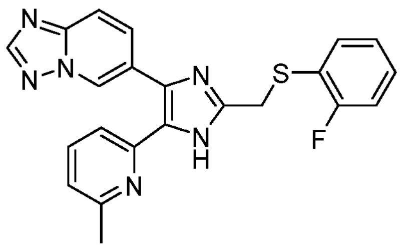

,

or a pharmaceutically acceptable salt or hydrate thereof, wherein

- the pharmaceutical composition is an oral, buccal or sub-lingual dosage form delivering 50-5000 mg of the one or more compounds daily; or
- the pharmaceutical composition is a topical formulation further comprising 1 - 98 % of a carrier; or
- the pharmaceutical composition is a parenteral dosage form comprising 25-250 mg of the one or more compounds per single dose.

**2.** A pharmaceutical composition according to claim 1, wherein

- the oral, buccal or sub-lingual dosage form is a tablet or capsule comprising 25 - 500 mg of the one or more compounds; or
- the topical formulation comprises a carrier selected from a cream base, an ointment base, ethanol and/or oleyl alcohol; or
- the parenteral formulation is a sterile aqueous solution further comprising salts or monosaccharides in a quantity to make the formulation isotonic with blood.

**3.** A pharmaceutical composition according to claim 1, wherein the oral, buccal or sublingual dosage form is a tablet

further comprising excipients such as starch or lactose, or a capsule or ovule either alone or in admixture with excipients, or an elixir or suspension containing flavoring or coloring agents.

4. A pharmaceutical composition according to claim 1 or 2, wherein the parenteral dosage form is formulated for intravenous, intramuscular, subcutaneous or intracoronary injection.

5. A pharmaceutical composition according to any of the previous claims, wherein the composition comprises the compound

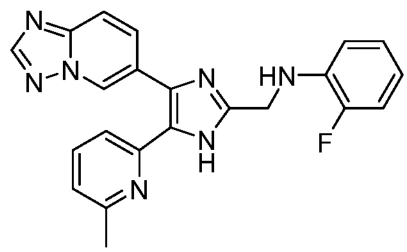

as a pharmaceutically acceptable salt selected from the compounds

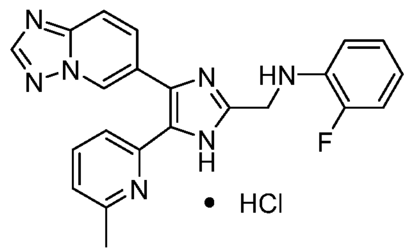

and

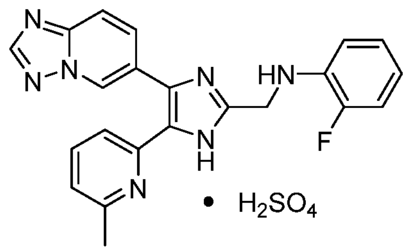

.

6. A pharmaceutical composition according to any of the previous claims, for use in treating, preventing, or reducing a disease in human selected from the group consisting of glomerulonephritis, diabetic nephropathy, lupus nephritis, hypertension-induced nephropathy, renal interstitial fibrosis, renal fibrosis resulting from complications of drug exposure, HIV-associated nephropathy, transplant nephropathy, liver fibrosis due to all etiologies, hepatic dysfunction attributable to infections, alcohol-induced hepatitis, disorders of the biliary tree, cystic fibrosis, pulmonary fibrosis, interstitial lung disease, acute lung injury, adult respiratory distress syndrome, idiopathic pulmonary fibrosis, chronic obstructive pulmonary disease, pulmonary disease due to infectious or toxic agents, post-infarction cardiac fibrosis, congestive heart failure, dilated cardiomyopathy, myocarditis, intimal thickening, vascular stenosis, hypertensior induced vascular remodeling, pulmonary arterial hypertension, coronary restenosis, peripheral restenosis, carotid restenosis, stent-induced restenosis, atherosclerosis, ocular scarring, corneal scarring, proliferative vitreoretinopathy, glaucoma, intraocular pressure, excessive or hypertrophic scar or keloid formation in the dermis occurring during wound healing resulting from trauma or surgical wounds, peritoneal and sub-dermal adhesion, dermatomyositis, polymyositis, scleroderma, fibrosclerosis, progressive systemic sclerosis, arthritis, osteoporosis, ulcers, impaired neurological function, male erectile dysfunction, Peyronie's disease, Dupuytren's contracture, Alzheimer's disease, Raynaud's syndrome, radiation-induced fibrosis, thrombosis, tumor metastasis growth, multiple myeloma, melanoma, glioma, glioblastomas, leukemia, sarcomas, leiomyomas, mesothelioma, and carcinornas of lung, breast, colon, kidney, ovary, cervix, liver, biliary tract, gastrointestinal tract, pancreas, prostate, head, and neck.

**7.** A pharmaceutical composition according to any of the previous claims, for use in treating, preventing, or reducing a disease in human selected from the group consisting of diabetic nephropathy, renal interstitial fibrosis, liver fibrosis due to all etiologies, hepatic dysfunction attributable to infections, alcohol-induce hepatitis, disorders of the biliary tree, pulmonary fibrosis, interstitial lung disease, idiopathic pulmonary fibrosis, chronic obstructive pulmonary disease, pulmonary disease due to infectious or toxic agents, pulmonary arterial hypertension, coronary restenosis, peripheral restenosis, carotid restenosis, stent induced restenosis, ocular scarring, corneal scarring, glaucoma, intraocular pressure, excessive or hypertrophic scar or keloid formation in the dermis occurring during wound healing resulting from trauma or surgical wounds, peritoneal and sub-dermal adhesion, dermatomyositis, polymyositis, scleroderma fibrosclerosis, progressive systemic sclerosis, Peyronie's disease, radiation-induce fibrosis, tumor metastasis growth, multiple myeloma, melanoma, glioma, glioblastomas, leukemia, sarcomas, leiomyomas, mesothelioma, and carcinornas of lung, breast, colon, kidney, ovary, cervix, liver, biliary tract, gastrointestinal tract, pancreas, prostate, head, and neck.

**8.** A pharmaceutical composition according to any of the previous claims, for use in treating, preventing, or reducing a disease in human selected from the group consisting of excessive or hypertrophic scar or keloid formation in the dermis occurring during wound healing resulting from trauma or surgical wounds peritoneal and sub-dermal adhesion, dermatomyositis, polymyositis, scleroderma, fibrosclerosis, progressive systemic sclerosis.

**9.** A pharmaceutical composition according to claim 6 or 7 for use in treating, preventing, or reducing a disease in human selected from the group consisting of melanoma, glioma, glioblastomas, leukemia, and carcinornas of lung, breast, colon, kidney, liver, gastrointestinal tract, pancreas, prostate, head, and neck.

**10.** A pharmaceutical composition, comprising one or more of the compounds

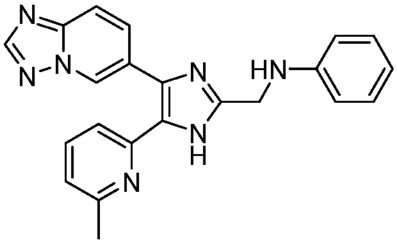
,
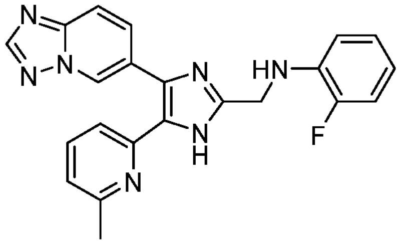
,
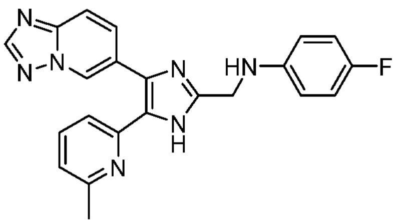
,
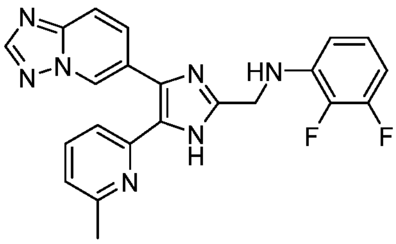
,
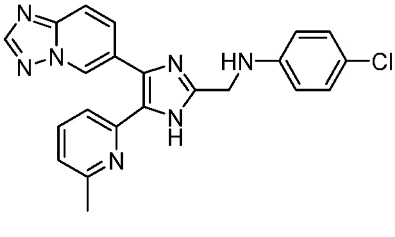
,
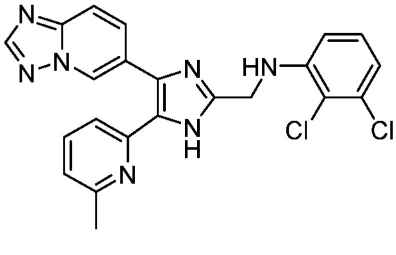
,

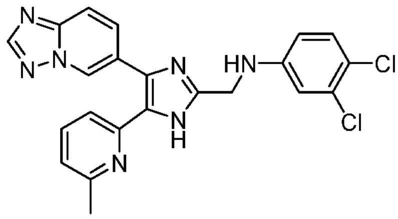,
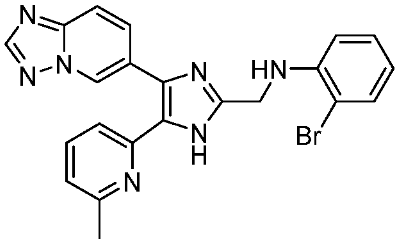,

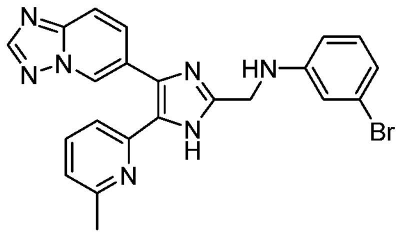,
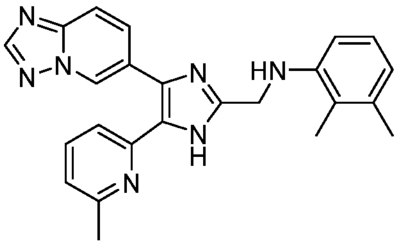,

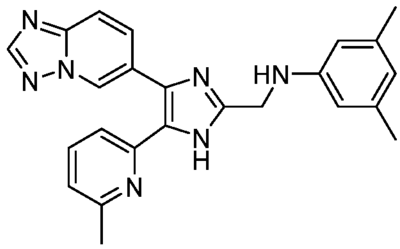,
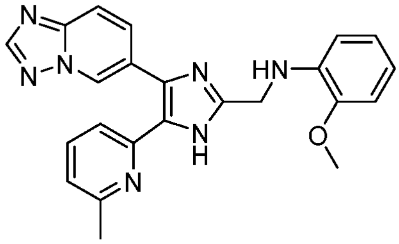,

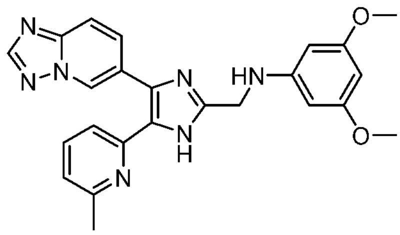,
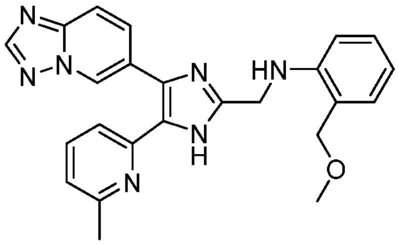,

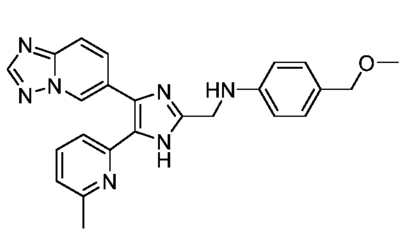,
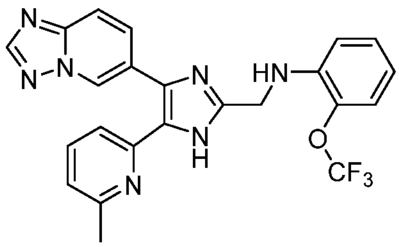,

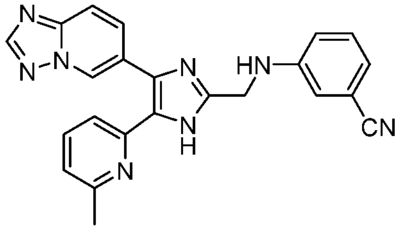,
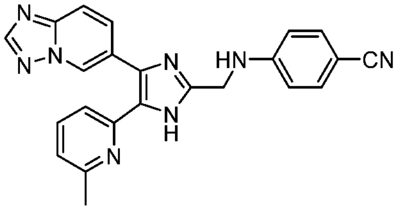,

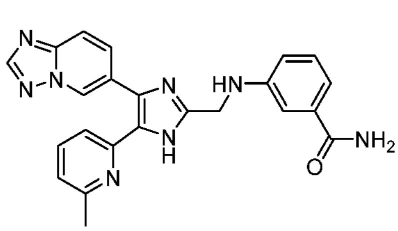,
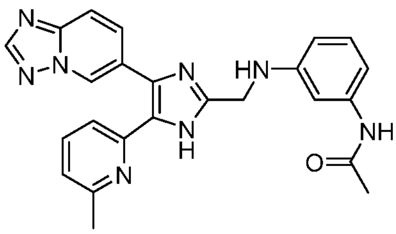,

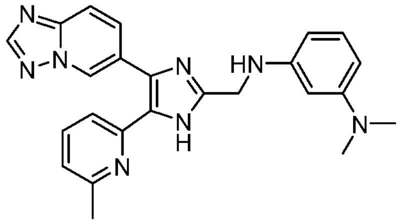,
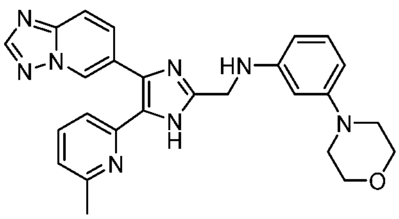,

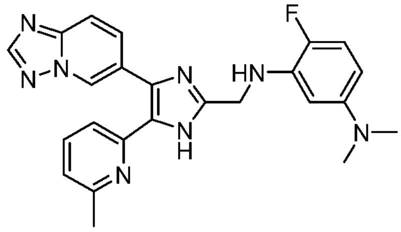,
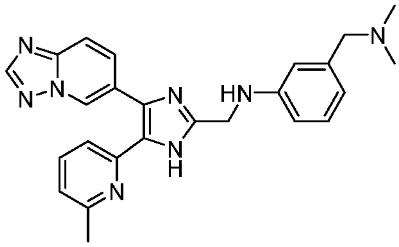,

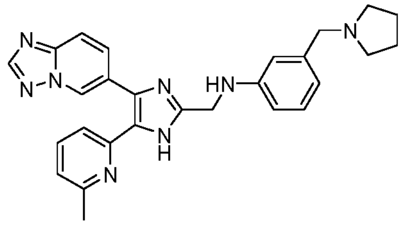,
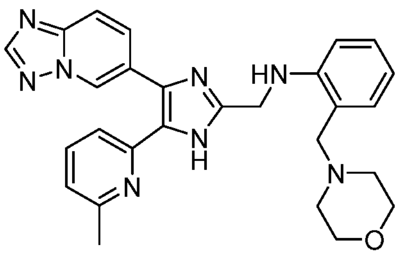,

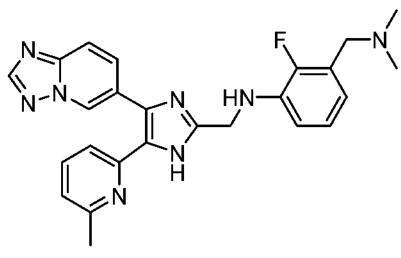,
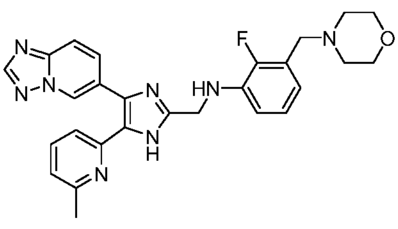,

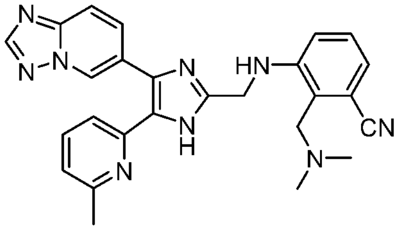,
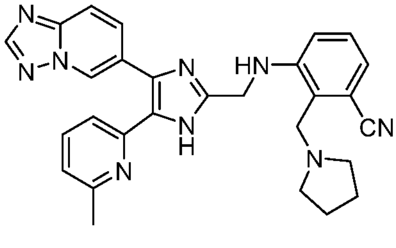,

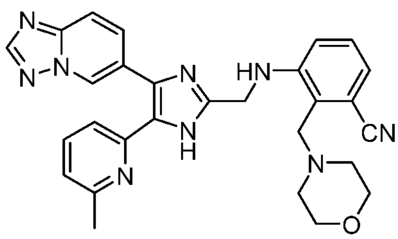,
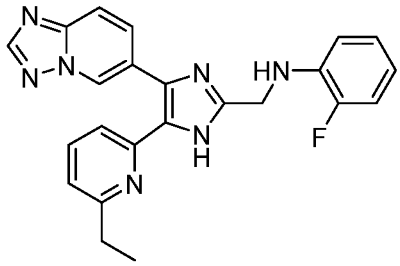,

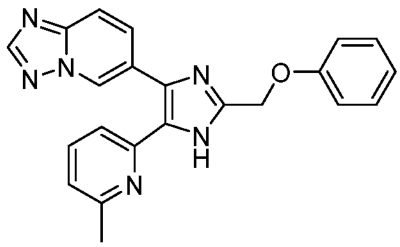,
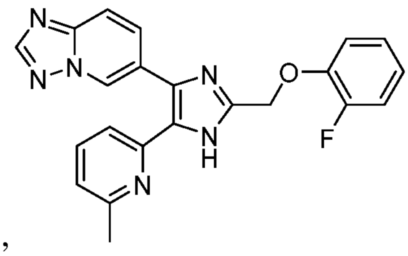,

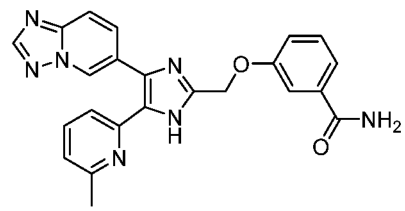,
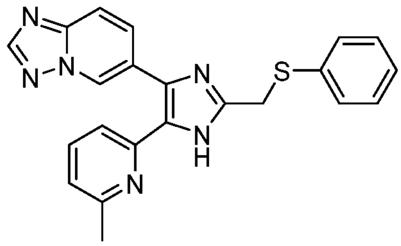

or a pharmaceutically acceptable salt or hydrate thereof, for use in treating, preventing, or reducing a disease in human selected from the group consisting of excessive or hypertrophic scar or keloid formation in the dermis occurring during wound healing resulting from trauma or surgical wounds, peritoneal and sub-dermal adhesion, dermatomyositis, polymyositis, scleroderma, fibrosclerosis, progressive systemic sclerosis, melanoma, glioma, glioblastomas, leukemia, and carcinornas of lung, breast, colon, kidney, liver, gastrointestinal tract, pancreas, prostate, head, and neck.

**Patentansprüche**

1. Pharmazeutische Zusammensetzung, umfassend eine oder mehrere der Verbindungen

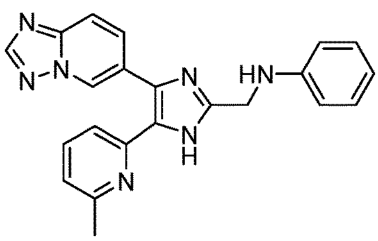,
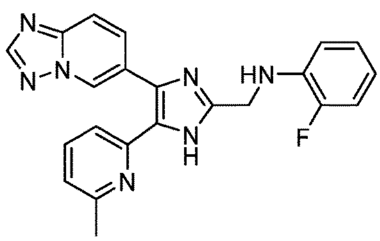,
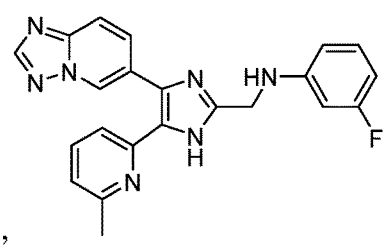

, 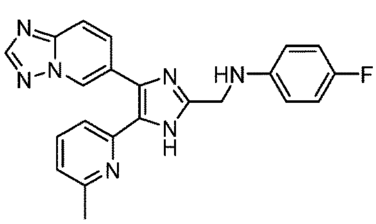 , 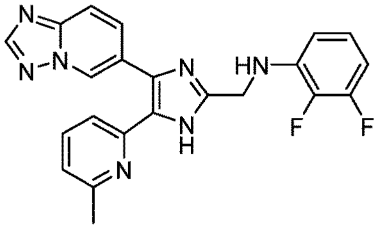 , 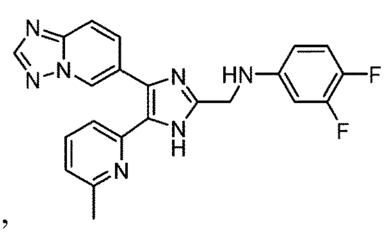

, 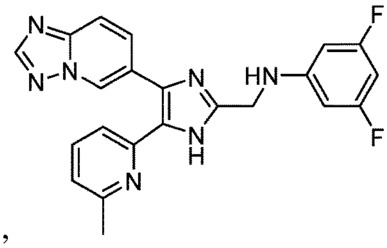 , 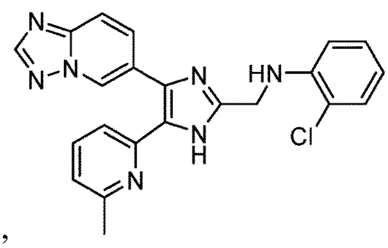 ,

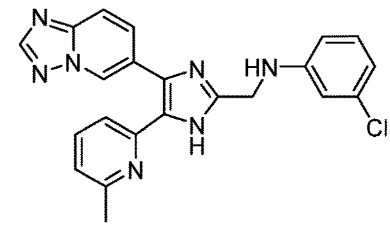 , 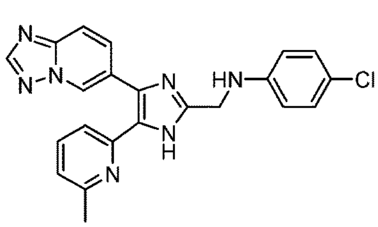 , 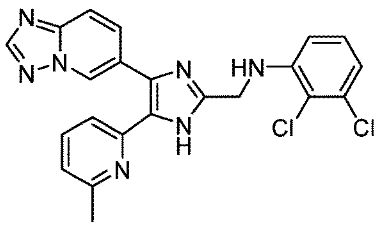 ,

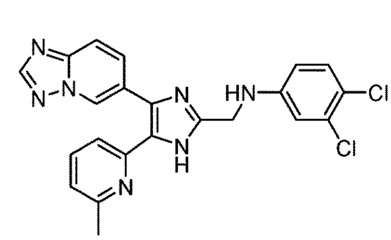 , 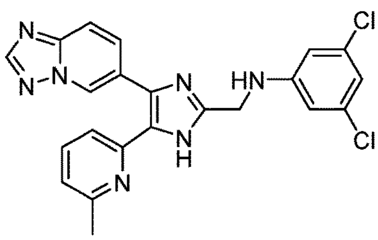 , 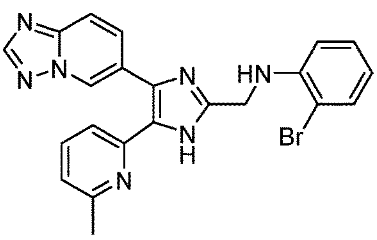 ,

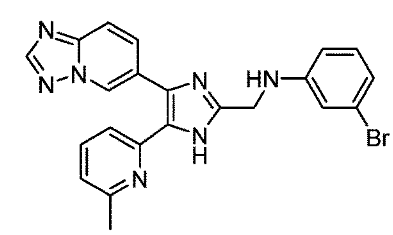 , 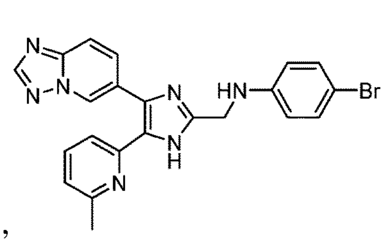 , 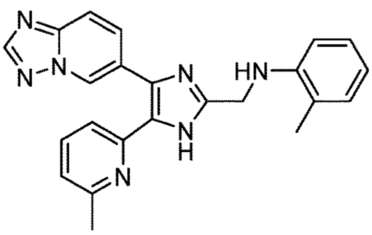 ,

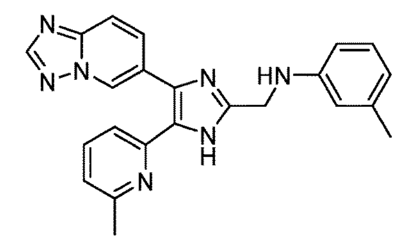 , 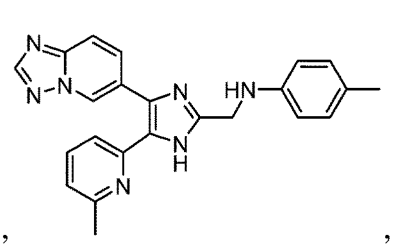 , 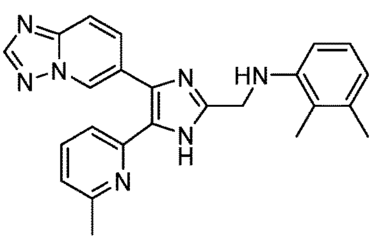 ,

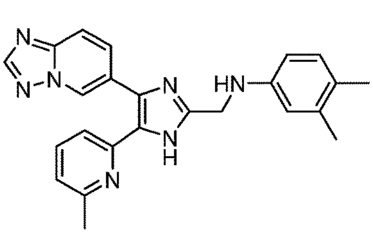 , 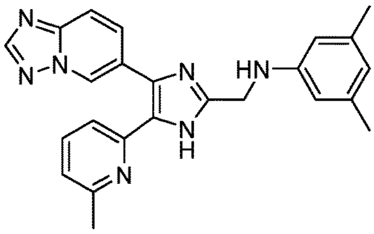 , 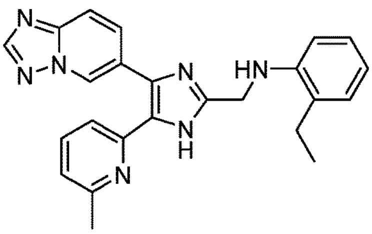 ,

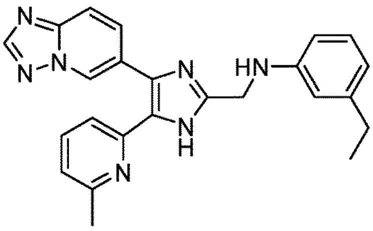 , 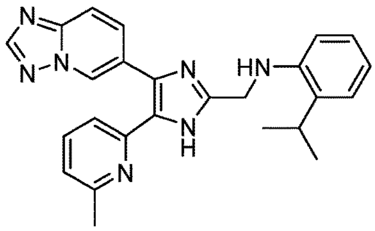 , 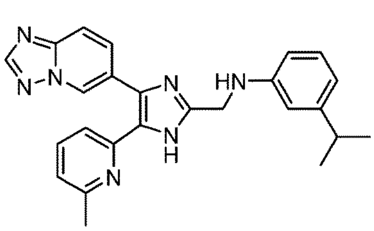 ,

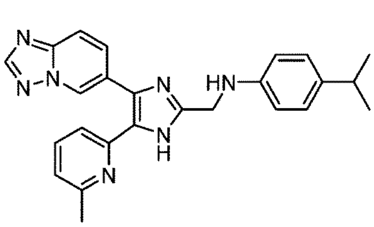 , 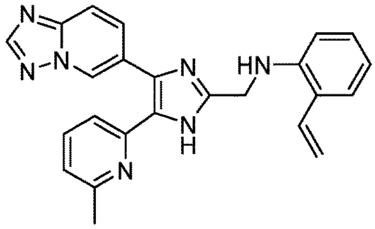 , 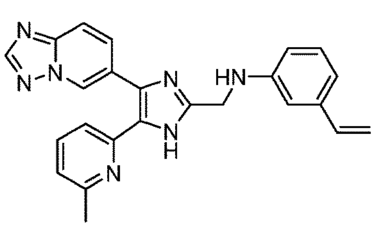 ,

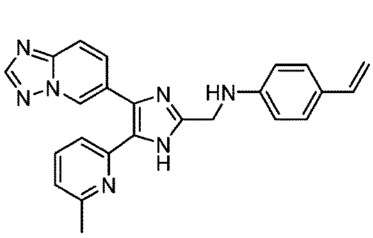 , 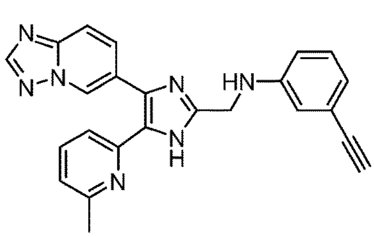 , 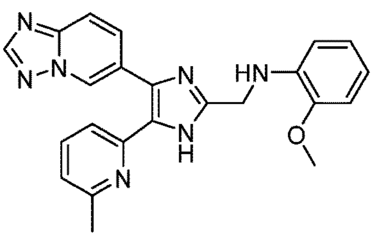 ,

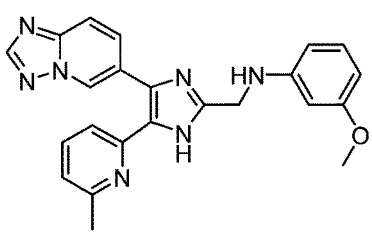 , 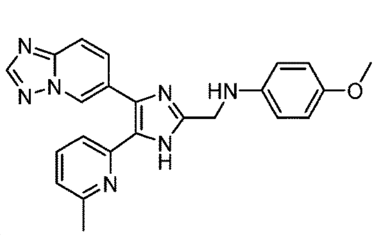 , 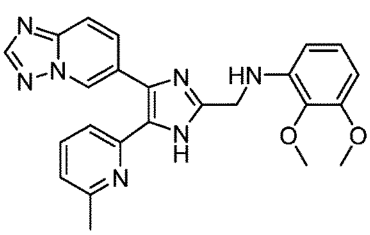 ,

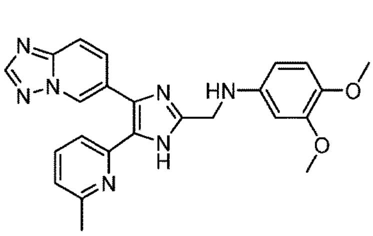 , 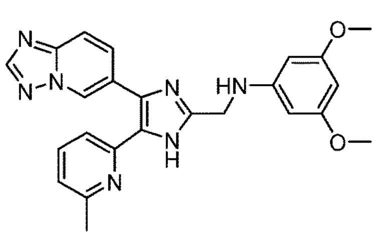 , 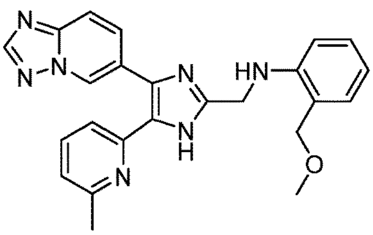 ,

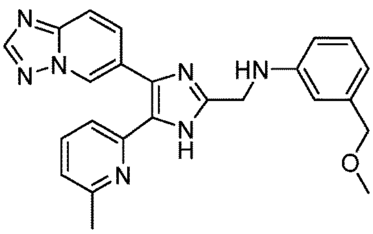 , 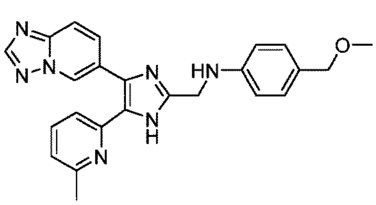 , 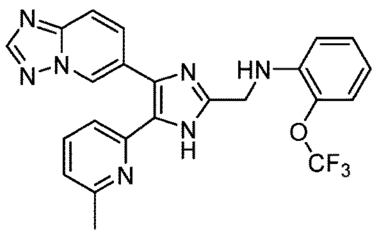 ,

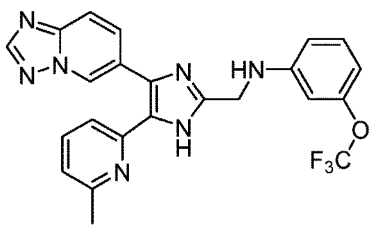 , 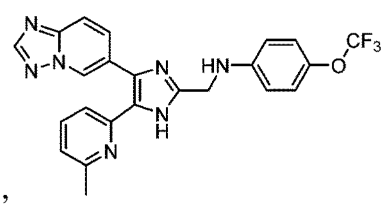 , 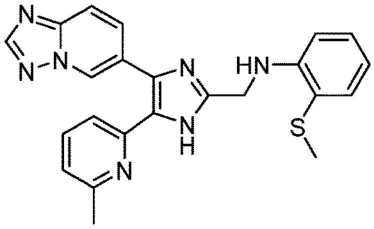 ,

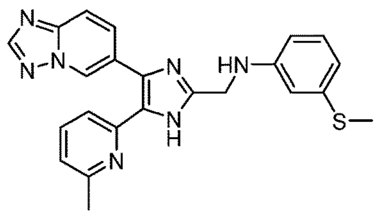 , 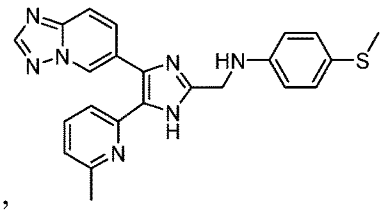 , 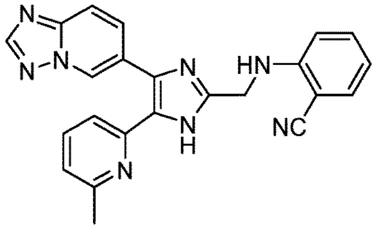 ,

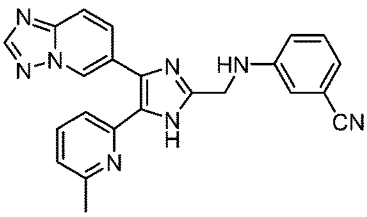 , 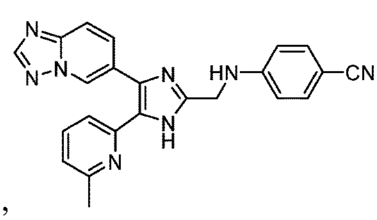 , 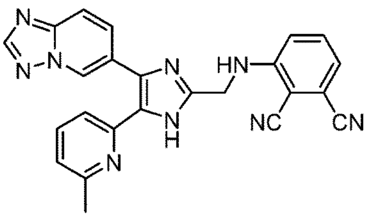

, 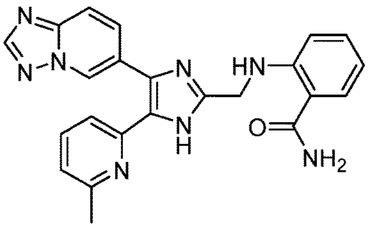 , 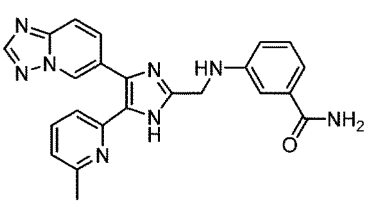 , 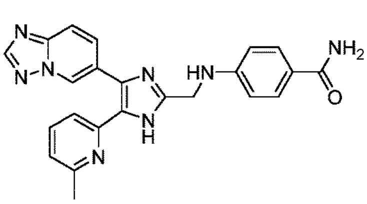

, 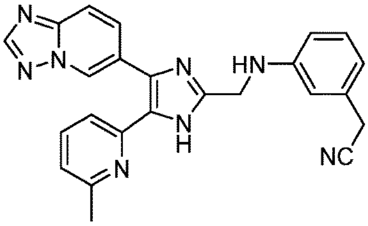 , 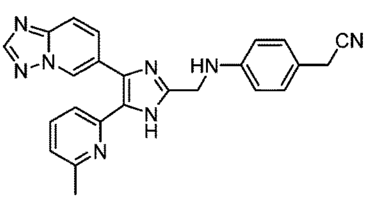 , 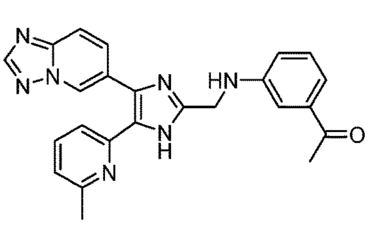

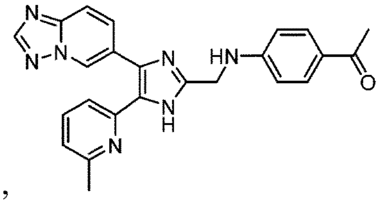

,

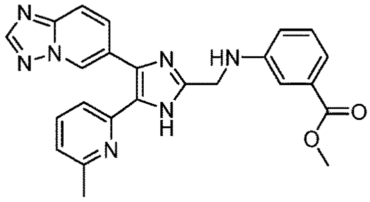

,

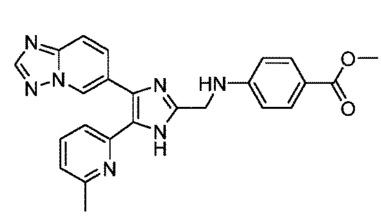

,

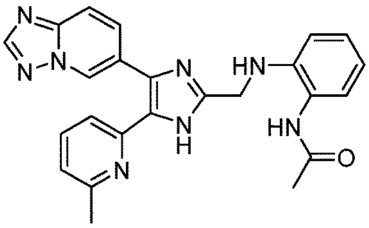

,

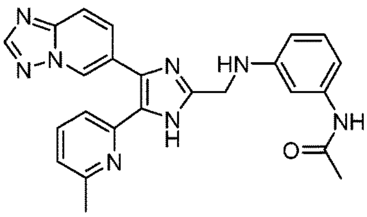

,

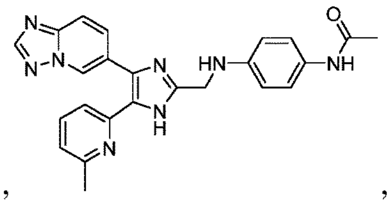

,

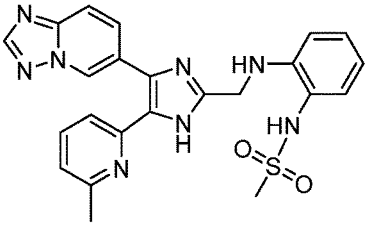

,

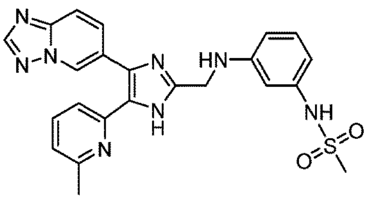

,

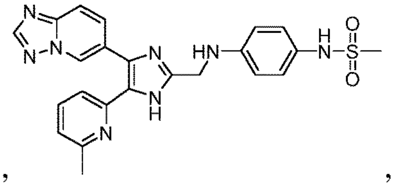

,

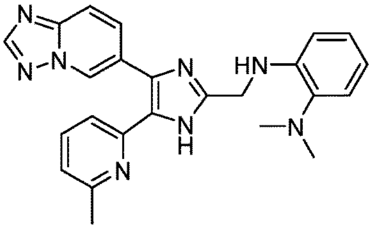

,

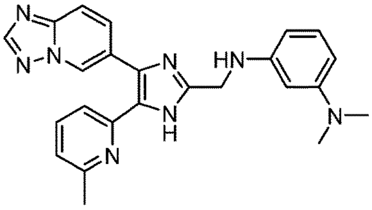

,

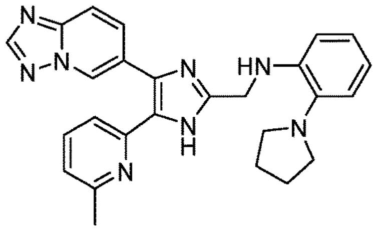

,

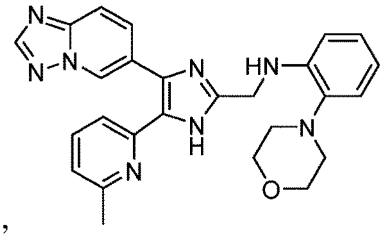

,

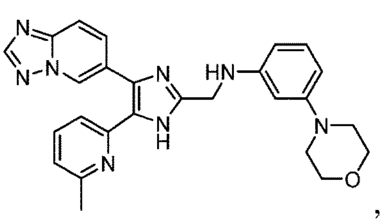

,

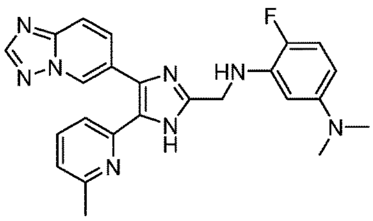

,

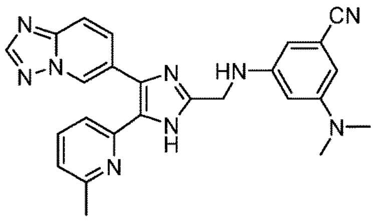

,

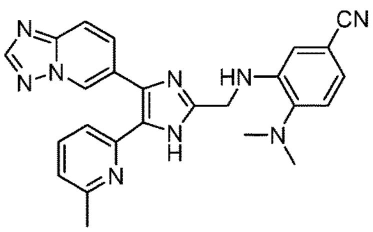 , 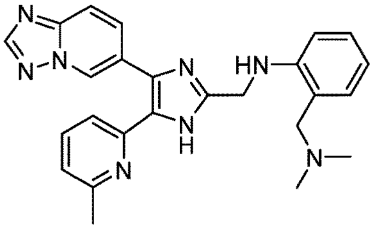 , 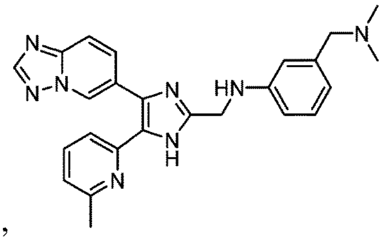 ,

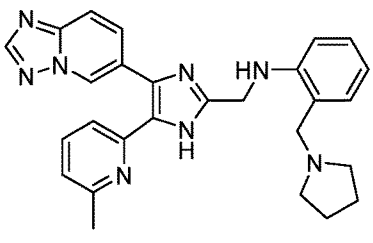 , 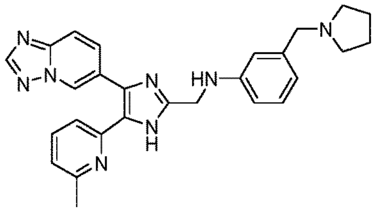 , 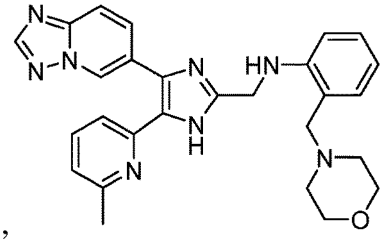 ,

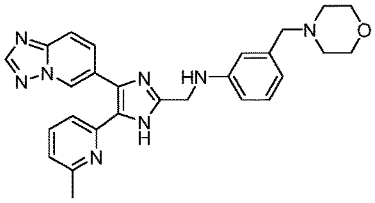 , 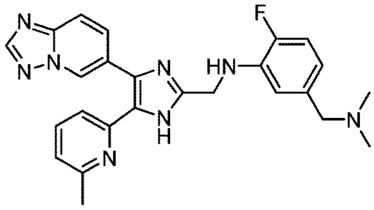 , 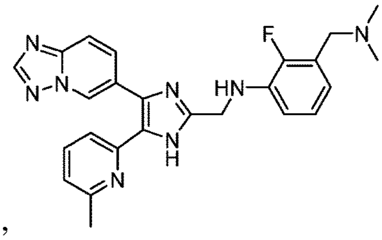 ,

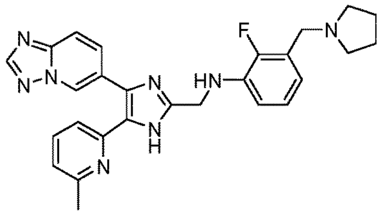 , 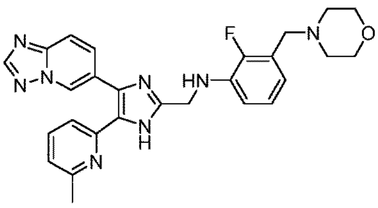 , 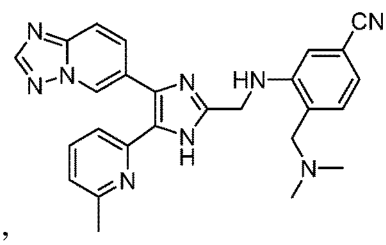 ,

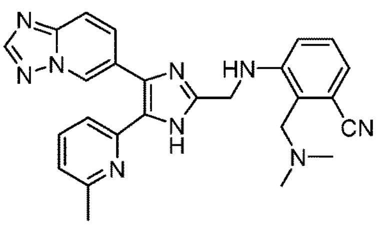 , 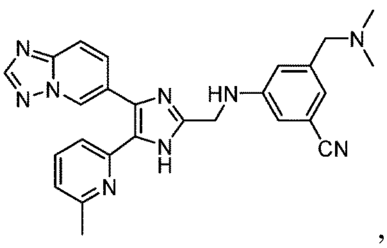 , 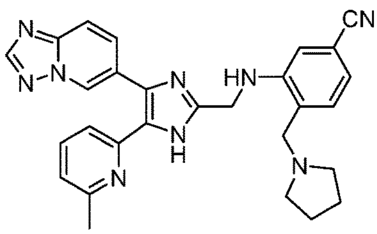 ,

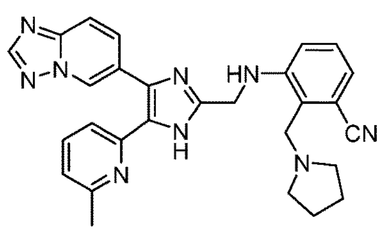 , 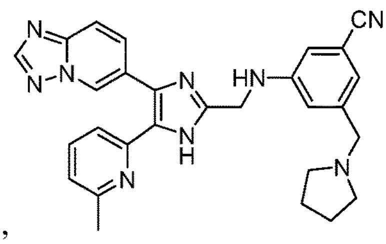 , 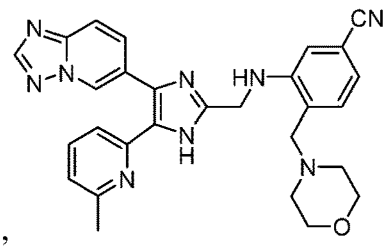

,

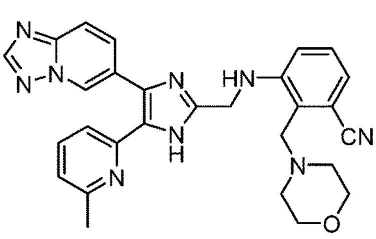, 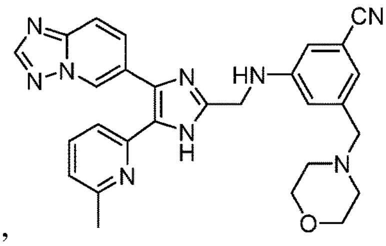, 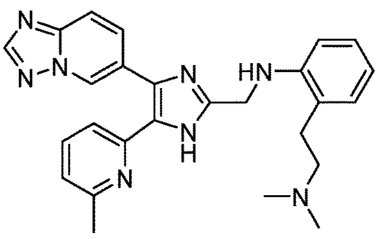

, 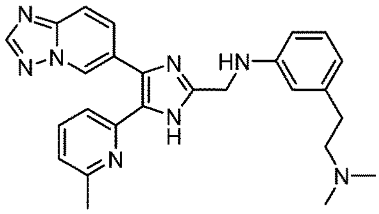, 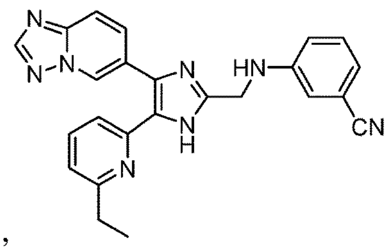, 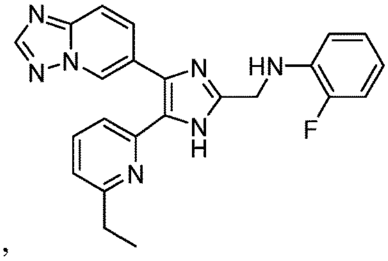

, 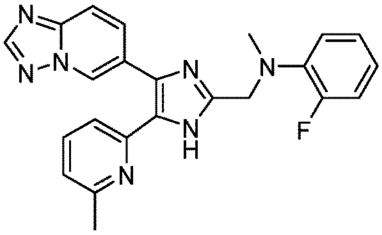, 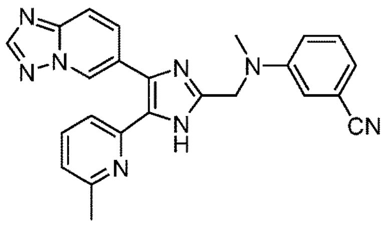, 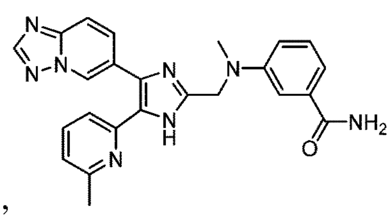

, 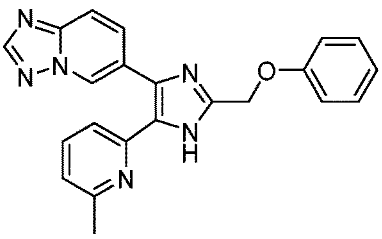, 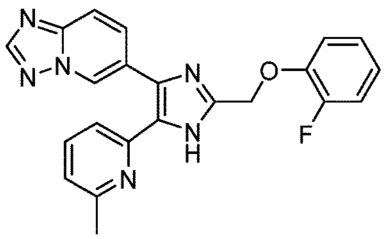,

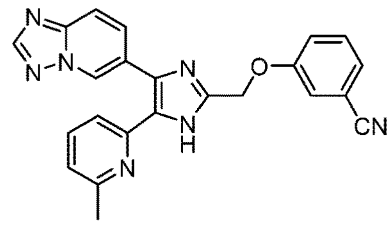, 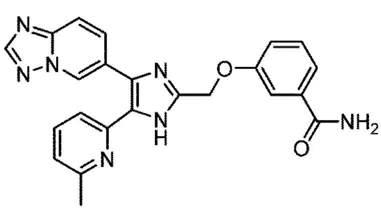, 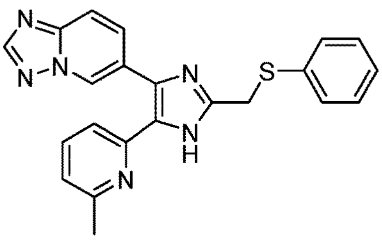;

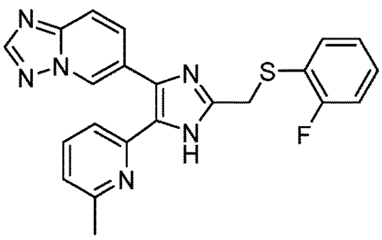,

oder ein pharmazeutisch akzeptables Salz oder Hydrat davon, wobei

- die pharmazeutische Zusammensetzung eine orale, bukkale oder sublinguale Dosierungsform ist, die 50-5000

mg von der einen oder den mehreren Verbindung(en) täglich abgibt; oder

- die pharmazeutische Zusammensetzung eine topische Formulierung ist, ferner umfassend 1-98 % eines Trägers; oder
- die pharmazeutische Zusammensetzung eine parenterale Dosierungsform ist, die 25-250 mg von der einen oder den mehreren Verbindung(en) pro Einzeldosis umfasst.

**2.** Pharmazeutische Zusammensetzung nach Anspruch 1, wobei

- die orale, bukkale oder sublinguale Dosierungsform eine Tablette oder Kapsel ist, die 25-500 mg von der einen oder den mehreren der Verbindungen umfasst; oder
- die topische Formulierung einen Träger umfasst, ausgewählt aus einer Cremebasis, einer Salbenbasis, Ethanol und/oder Oleylalkohol; oder
- die parenterale Formulierung eine sterile wässrige Lösung ist, ferner umfassend Salze oder Monosaccharide in einer Menge, um die Formulierung isotonisch mit Blut zu machen.

**3.** Pharmazeutische Zusammensetzung nach Anspruch 1, wobei die orale, bukkale oder sublinguale Dosierungsform eine Tablette ist, die ferner Exzipienten wie etwa Stärke oder Laktose umfasst, oder eine Kapsel oder Ovule, entweder allein oder in Beimischung mit Exzipienten, oder ein Elixir oder eine Suspension ist, enthaltend Aroma- oder Farbstoffe.

**4.** Pharmazeutische Zusammensetzung nach Anspruch 1 oder 2, wobei die parenterale Dosierungsform für intravenöse, intramuskuläre, subkutane oder intrakoronare Injektion formuliert ist.

**5.** Pharmazeutische Zusammensetzung nach einem der vorhergehenden Ansprüche, wobei die Zusammensetzung die Verbindung

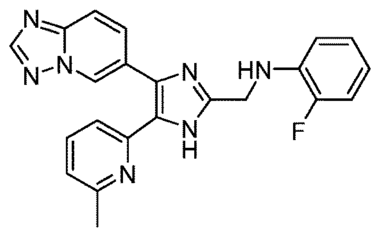

als ein pharmazeutisch akzeptables Salz umfasst, ausgewählt aus den Verbindungen

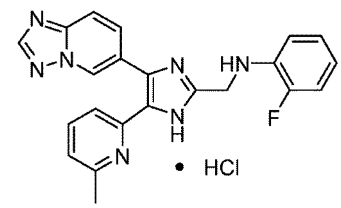

und

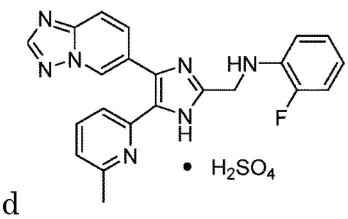

.

**6.** Pharmazeutische Zusammensetzung nach einem der vorhergehenden Ansprüche zur Verwendung bei Behandlung, Prävention oder Verminderung einer Krankheit beim Menschen, ausgewählt aus der Gruppe bestehend aus Glomerulonephritis, diabetischer Nephropathie, Lupus Nephritis, durch Hypertonie induzierter Nephropathie, interstitieller Nierenfibrose, Nierenfibrose infolge von Komplikationen durch Drogenmissbrauch, HIV-assoziierter Nephropathie, Transplantationsnephropathie, Leberfibrose aufgrund von allen Ätiologien, hepatischer Dysfunktion aufgrund von Infektionen, durch Alkohol induzierter Hepatitis, Störungen der Gallenwege, zystischer Fibrose, Lungenfibrose, interstitieller Lungenerkrankung, akuter Lungenverletzung, posttraumatischer Lungeninsuffizienz, idiopathischer Lungenfibrose, chronisch obstruktiver Lungenerkrankung, Lungenerkrankung aufgrund von infektiösen oder toxischen Stoffen, postinfarktischer Herzfibrose, kongestiver Herzinsuffizienz, dilatativer Kardiomyopathie, Myokarditis, Intimaverdickung, Gefäßstenose, Hypertension-induzierter Gefäßremodellierung, pulmonal-arterieller Hypertonie, Koronarrestenose, peripherer Restenose, karotider Restenose, durch Stent induzierter Restenose, Atherosklerose, Augenvernarbung, Hornhautvernarbung, proliferativer Vitreoretinopathie, Glaukom, Augeninnendruck, exzessiver oder hypertrophischer Narben- oder Keloidbildung in der Dermis auftretend während der Wundheilung resultierend

von einem Trauma oder chirurgischen Wunden peritonealer und subdermaler Adhäsion, Dermatomyositis, Polymyositis, Skleroderma, Fibrosklerose, progressiver systemischer Sklerose, Arthritis, Osteoporose, Geschwüren, beeinträchtigter neurologischer Funktion, männlicher erektiler Dysfunktion, Peyronie-Krankheit, Dupuytren-Kontraktur, Alzheimer Krankheit, Raynaud-Syndrom, strahlungsinduzierter Fibrose, Thrombose, Tumormetastasenwachstum, multiplem Myeloma, Melanoma, Glioma, Glioblastoma, Leukämie, Sarkoma, Leiomyoma, Mesothelioma und Karzinomen von Lunge, Brust, Kolon, Nieren, Eierstöcken, Gebärmutterhals, Leber, Gallentrakt, Magen-Darm-Trakt, Bauchspeicheldrüse, Prostata, Kopf und Hals.

**7.** Pharmazeutische Zusammensetzung nach einem der vorhergehenden Ansprüche zur Verwendung beiBehandlung, Prävention oder Verminderung einer Krankheit beim Menschen, ausgewählt aus der Gruppe bestehend aus diabetischer Nephropathie, interstitieller Nierenfibrose, Leberfibrose aufgrund von allen Ätiologien, hepatischer Dysfunktion aufgrund von Infektionen, durch Alkohol induzierter Hepatitis, Störungen der Gallenwege, Lungenfibrose, interstitieller Lungenerkrankung, idiopathischer Lungenfibrose, chronisch obstruktiver Lungenerkrangung, Lungenerkrankung aufgrund von infektiösen oder toxischen Stoffen, pulmonal-arterieller Hypertonie, Koronarrestenose, peripherer Restenose, karotider Restenose, durch Stent induzierter Restenose, Augenvernarbung, Hornhautvernarbung, Glaukom, Augeninnendruck, exzessiver oder hypertrophischer Narben- oder Keloidbildung in der Dermis auftretend während der Wundheilung resultierend von einem Trauma oder chirurgischen Wunden, peritonealer und subdermaler Adhäsion, Dermatomyositis, Polymyositis, Skleroderma, Fibrosklerose, progressiver systemischer Sklerose, Peyronie-Krankheit, strahlungsinduzierter Fibrose, Tumormetastasenwachstum, multiplem Myeloma, Melanoma, Glioma, Glioblastoma, Leukämie, Sarkoma, Leiomyoma, Mesothelioma und Karzinomen von Lunge, Brust, Kolon, Nieren, Eierstöcken, Gebärmutterhals, Leber, Gallentrakt, Magen-Darm-Trakt, Bauchspeicheldrüse, Prostata, Kopf und Hals.

**8.** Pharmazeutische Zusammensetzung nach einem der vorhergehenden Ansprüche zur Verwendung bei Behandlung, Prävention oder Verminderung einer Krankheit beim Menschen, ausgewählt aus der Gruppe bestehend aus exzessiver oder hypertrophischer Narben- oder Keloidbildung in der Dermis auftretend während der Wundheilung resultierend von einem Trauma oder chirurgischen Wunden, peritonealer und subdermaler Adhäsion, Dermatomyositis, Polymyositis, Skleroderma, Fibrosklerose, progressiver systemischer Sklerose.

**9.** Pharmazeutische Zusammensetzung nach Anspruch 6 oder 7 zur Verwendung bei Behandlung, Prävention oder Verminderung einer Krankheit beim Menschen, ausgewählt aus der Gruppe bestehend aus Melanoma, Glioma, Glioblastoma, Leukämie und Karzinomen von Lunge, Brust, Kolon, Nieren, Leber, Magen-Darm-Trakt, Bauchspeicheldrüse, Prostata, Kopf und Hals.

**10.** Pharmazeutische Zusammensetzung, umfassend eine oder mehrere der Verbindungen

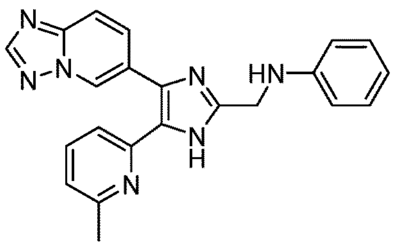 ,

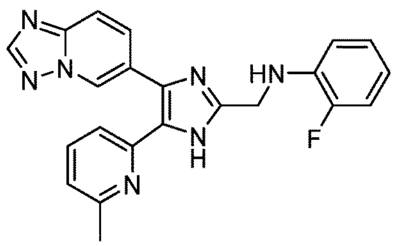 ,

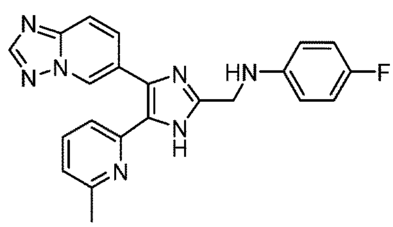 ,

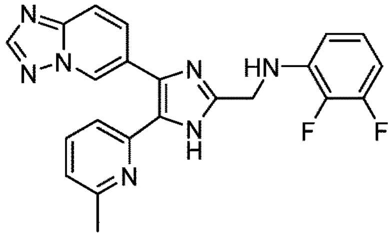 ,

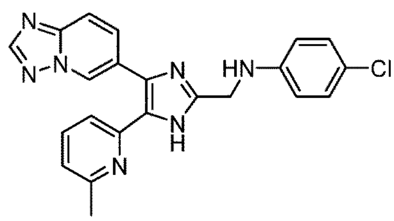,
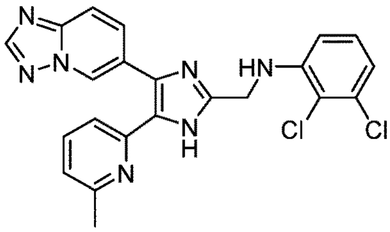,

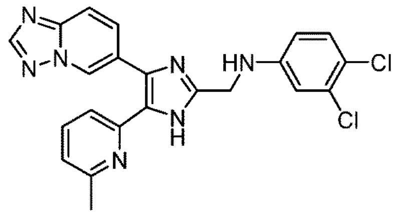,
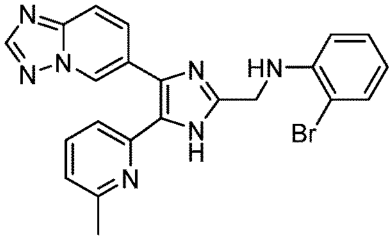,

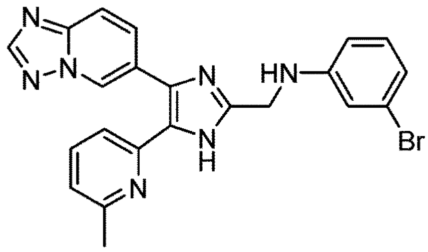,
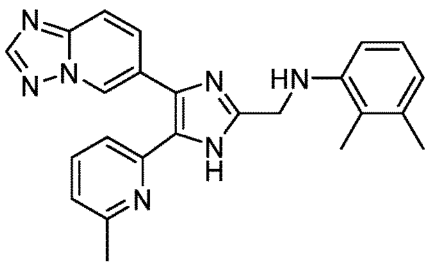,

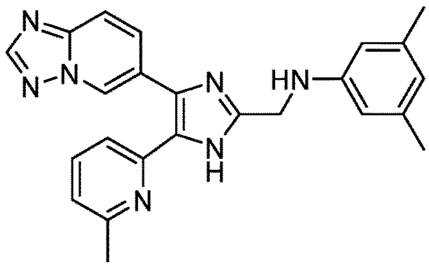,
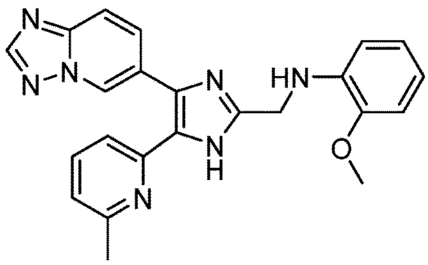,

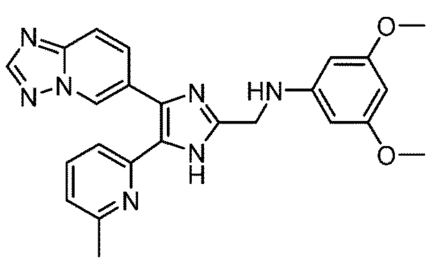,
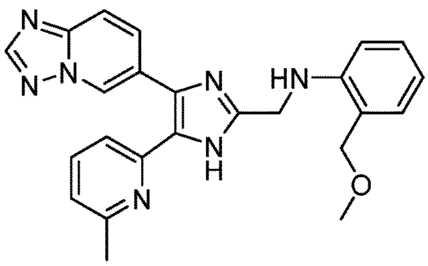,

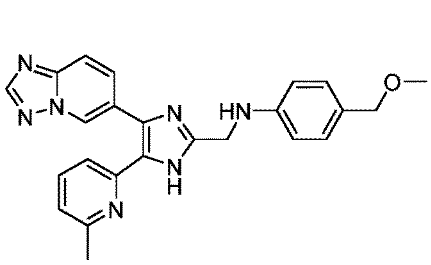,
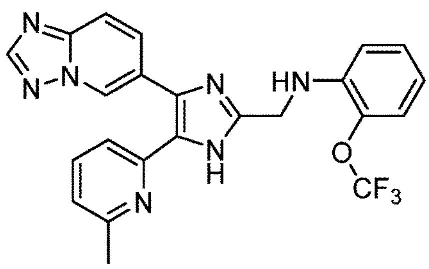,

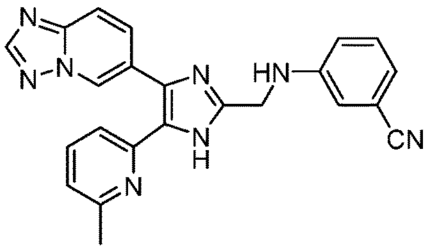,

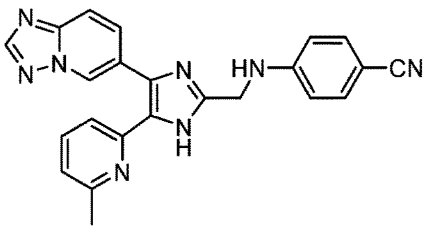,

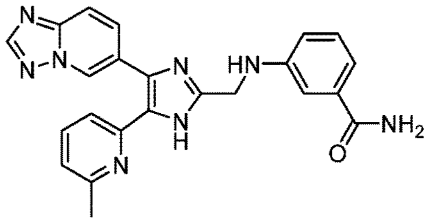,

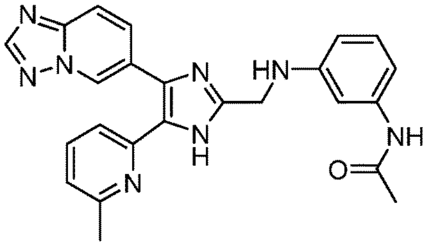,

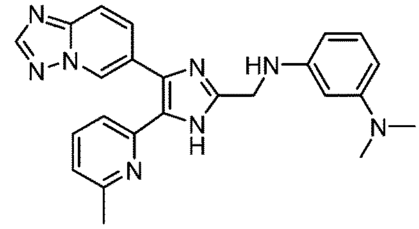,

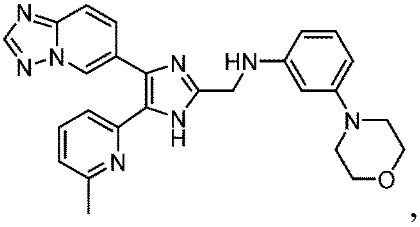,

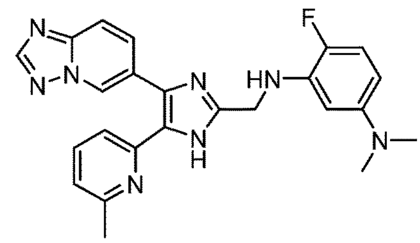,

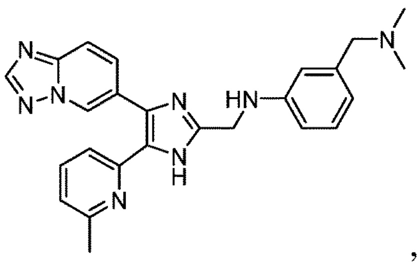,

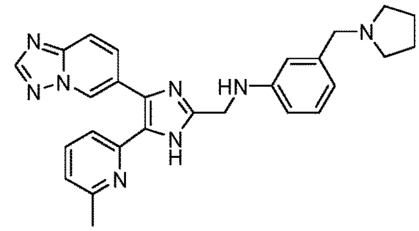,

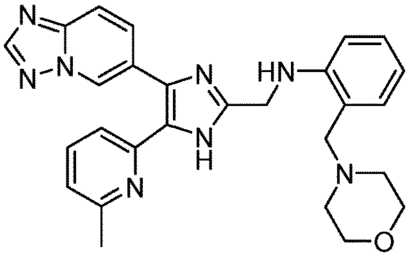,

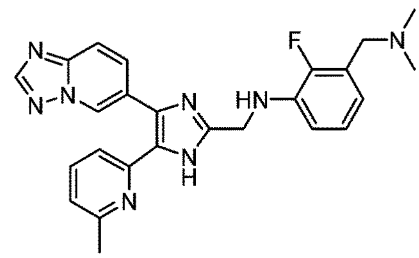,

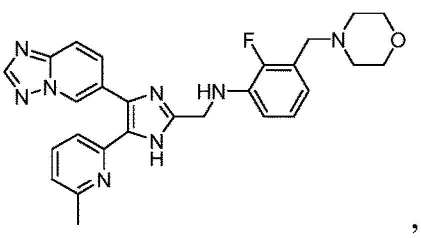,

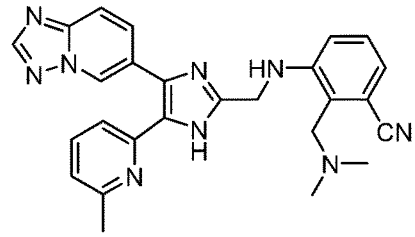, 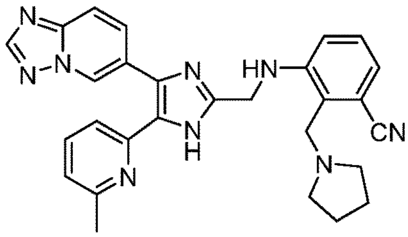,

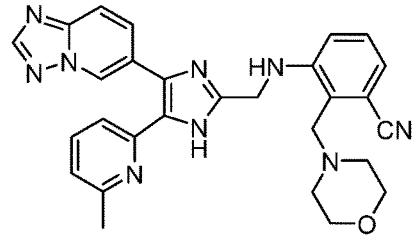, 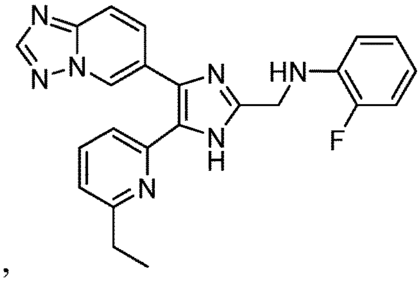,

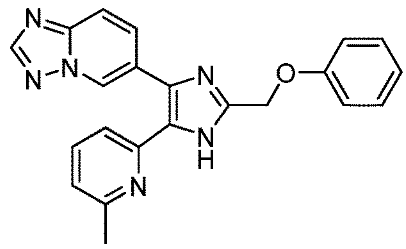, 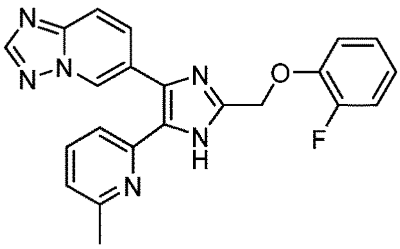,

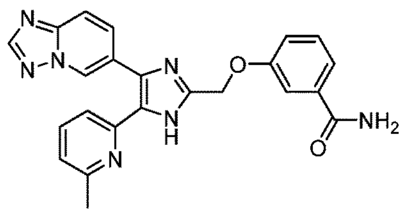, 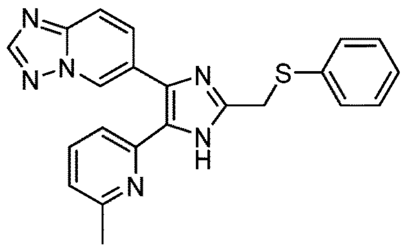

oder ein pharmazeutisch akzeptables Salz oder Hydrat davon zur Verwendung bei Behandlung, Prävention oder Verminderung einer Krankheit beim Menschen, ausgewählt aus der Gruppe bestehend aus exzessiver oder hypertrophischer Narben- oder Keloidbildung in der Dermis auftretend während der Wundheilung resultierend von einem Trauma oder chirurgischen Wunden, peritonealer und subdermaler Adhäsion, Dermatomyositis, Polymyositis, Skleroderma, Fibrosklerose, progressiver systemischer Sklerose, Melanoma, Glioma, Glioblastoma, Leukämie und Karzinomen von Lunge, Brust, Kolon, Nieren, Leber, Magen-Darm-Trakt, Bauchspeicheldrüse, Prostata, Kopf und Hals.

## Revendications

**1.** Composition pharmaceutique comprenant un ou plusieurs composés

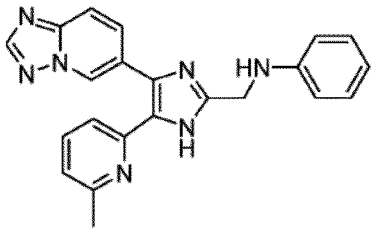, 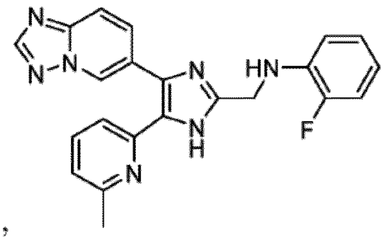, 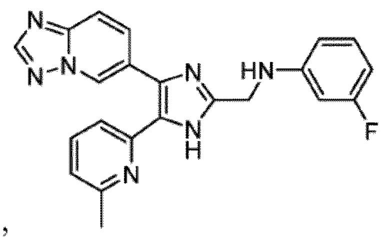

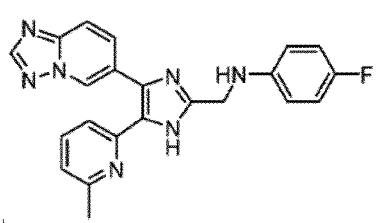, 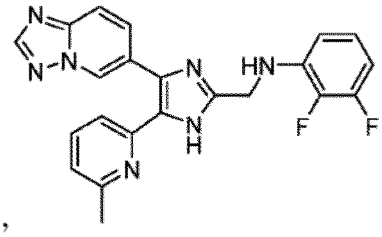, 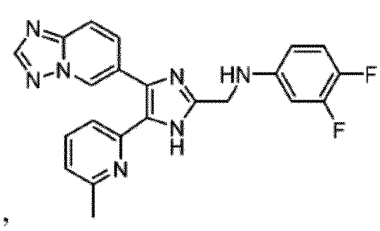

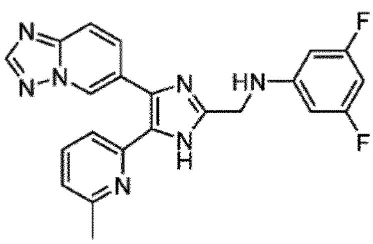, 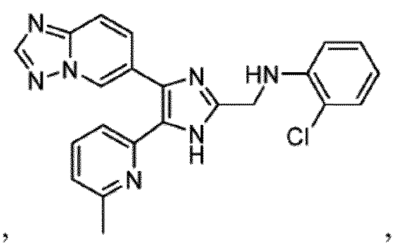,

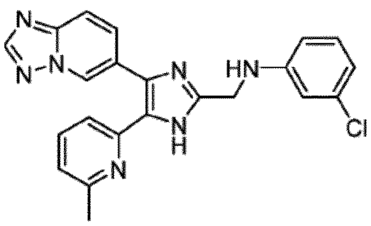, 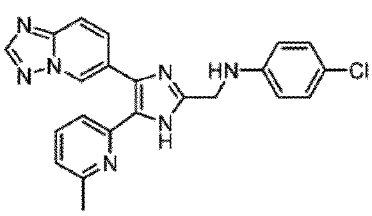, 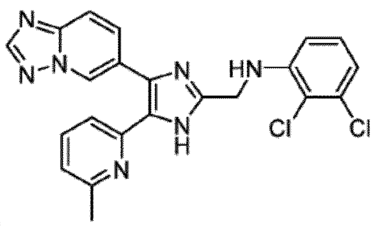,

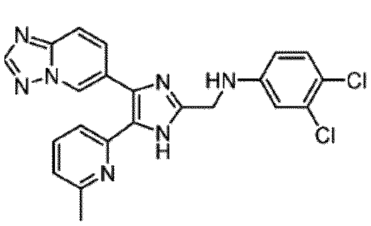, 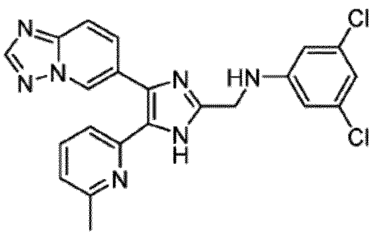, 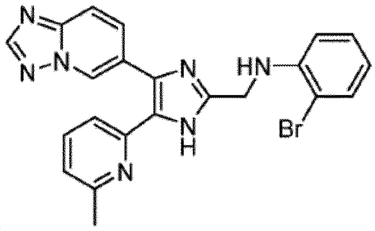,

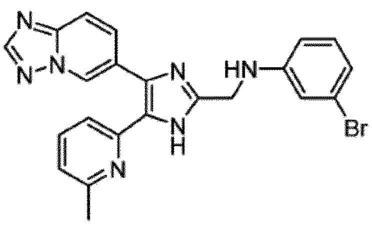, 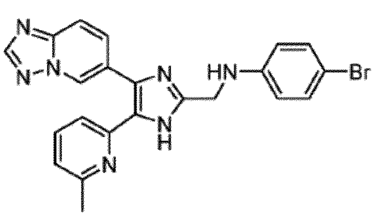, 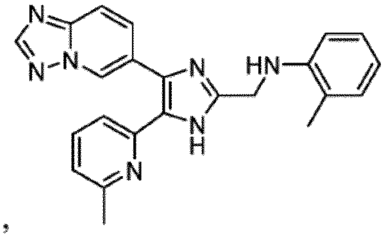,

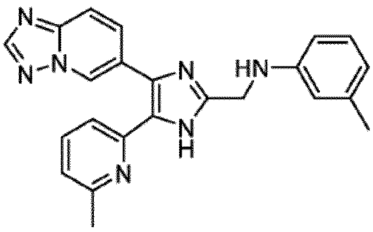 , 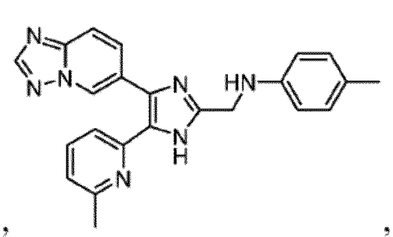 , 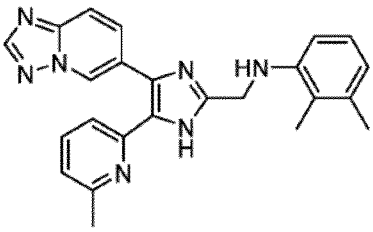 ,

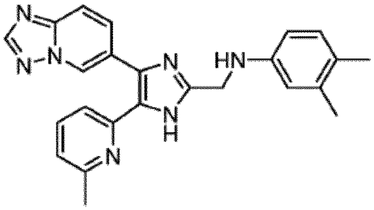 , 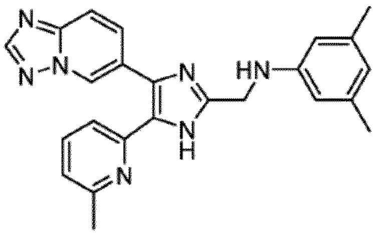 , 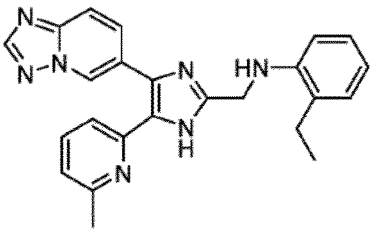 ,

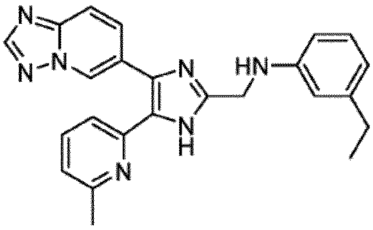 , 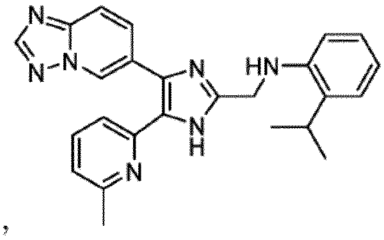 , 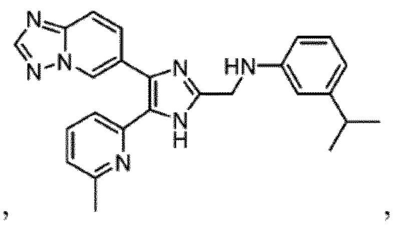 ,

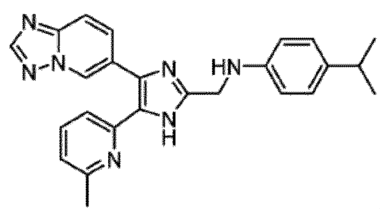 , 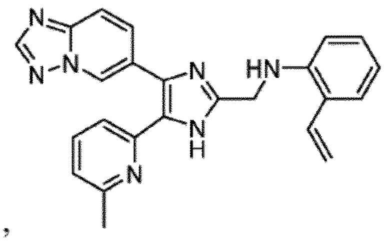 , 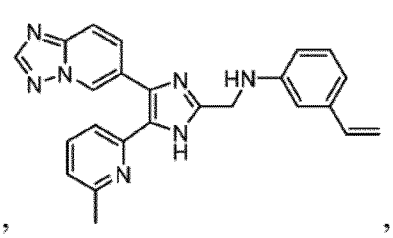 ,

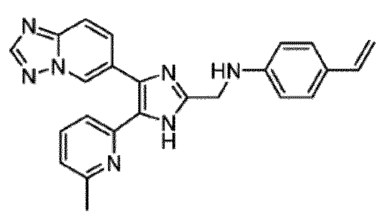 , 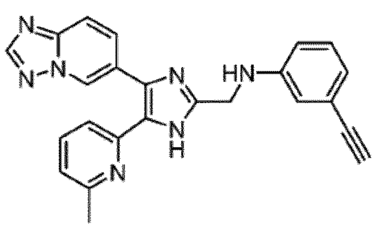 , 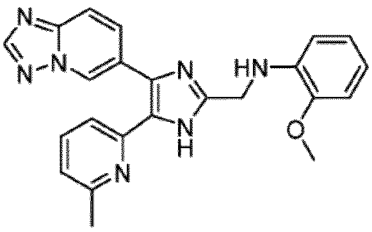 ,

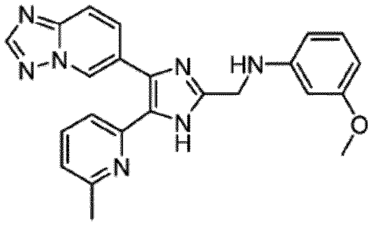 , 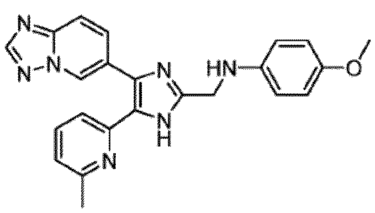 , 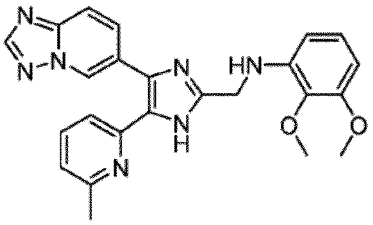 ,

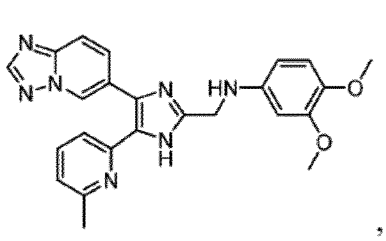,
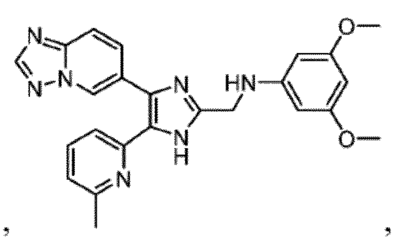,
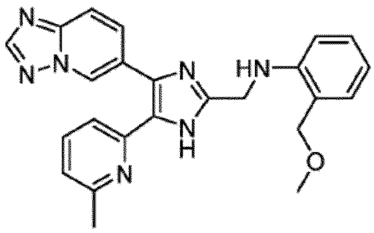,

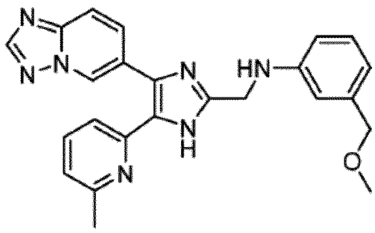,
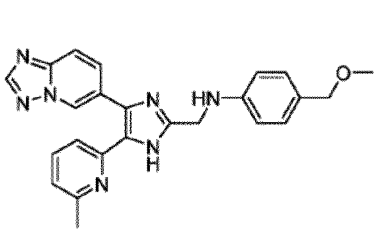,
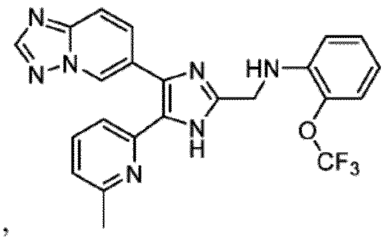,

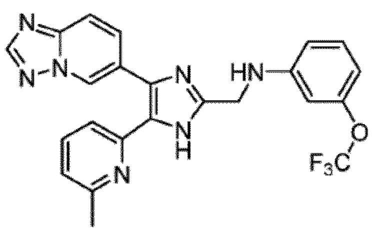,
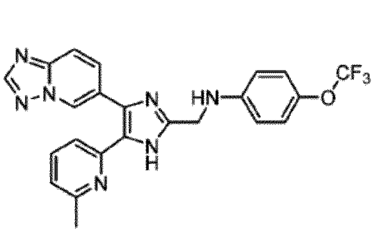,
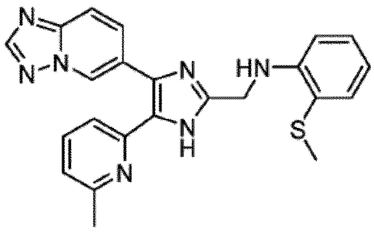,

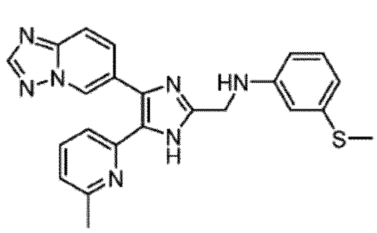,
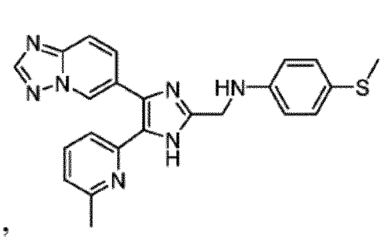,
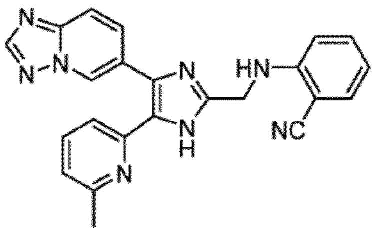,

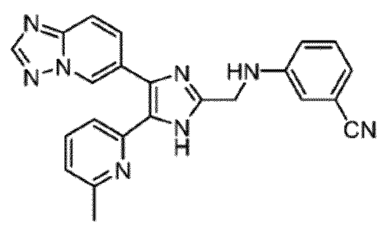,
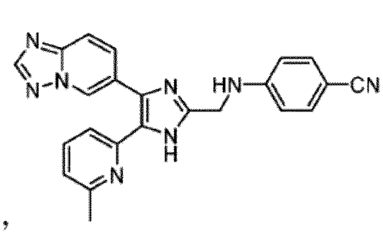,
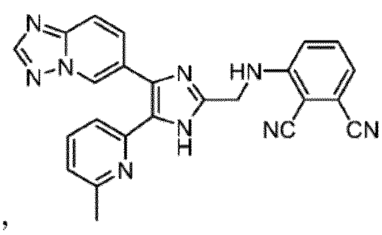

,
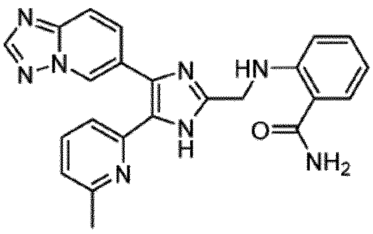,
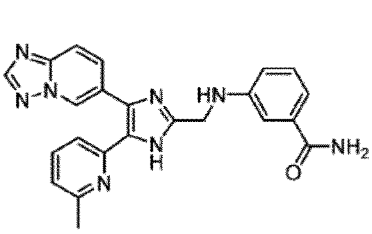,
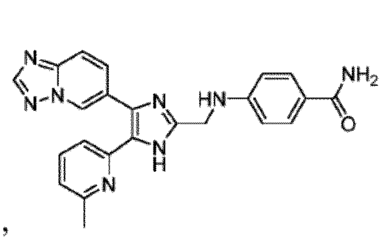

,
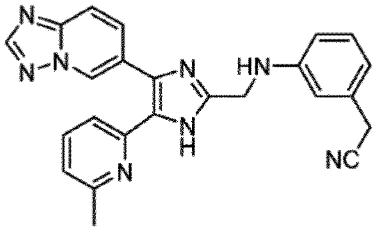
,
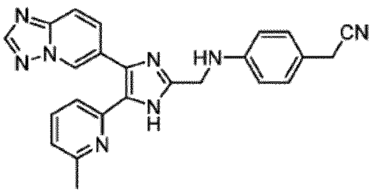
,
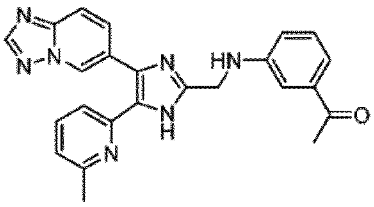

,
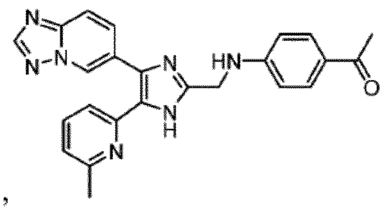
,
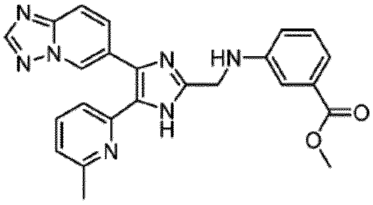
,

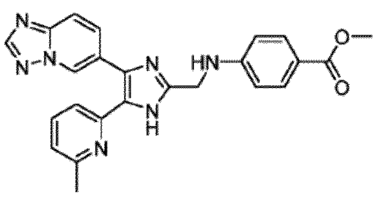
,
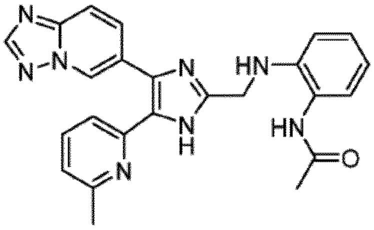
,
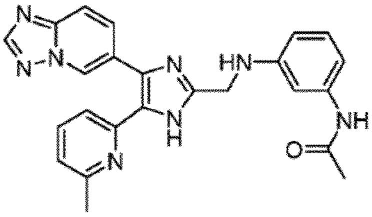

,
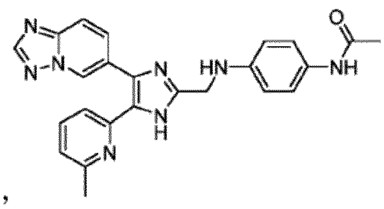
,
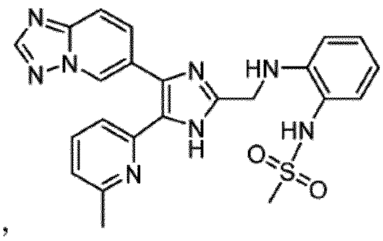
,
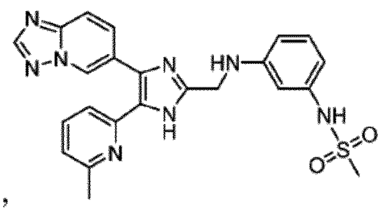

,
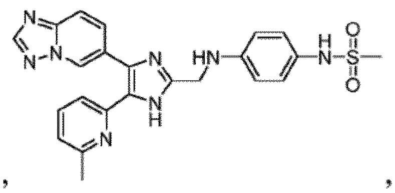
,
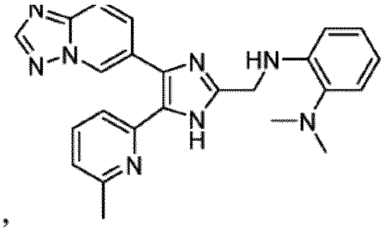
,
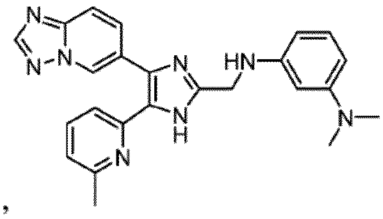

,
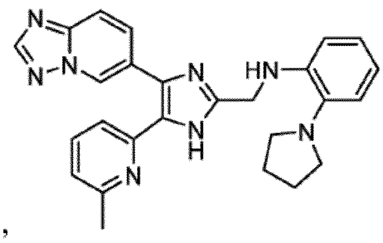
,
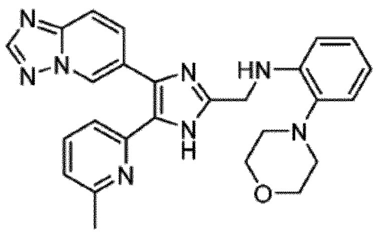
,

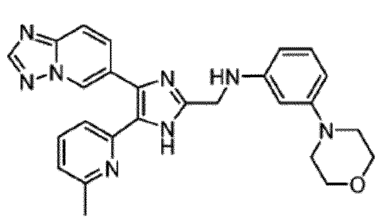 , 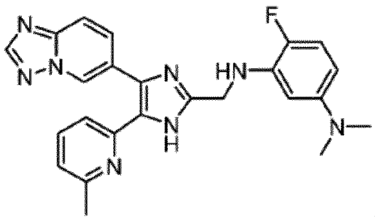 , 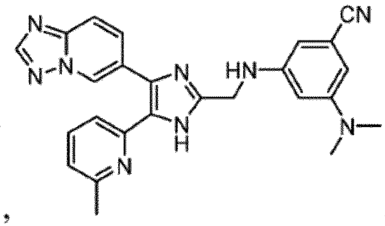 ,

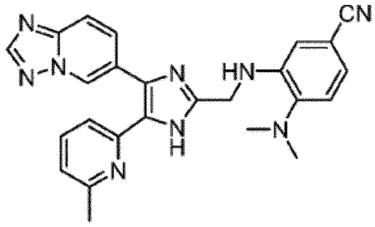 , 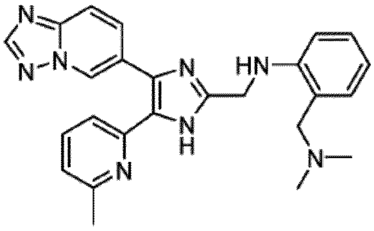 , 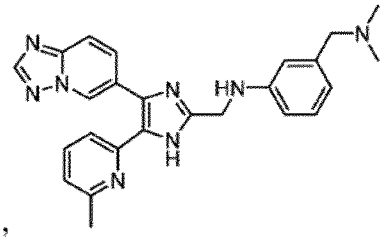 ,

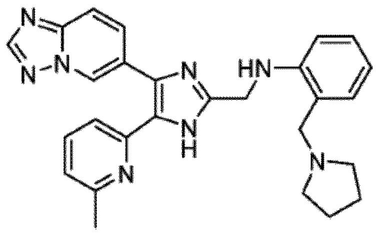 , 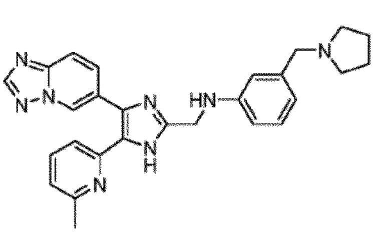 , 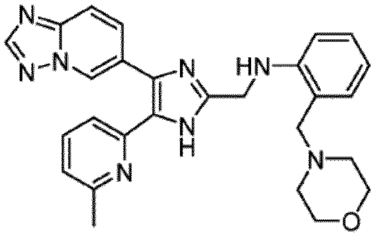 ,

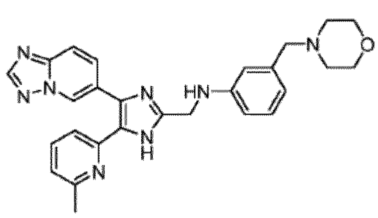 , 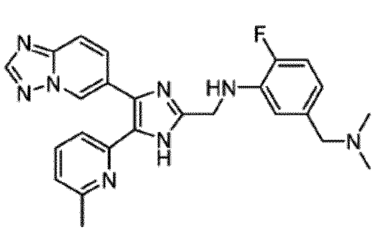 , 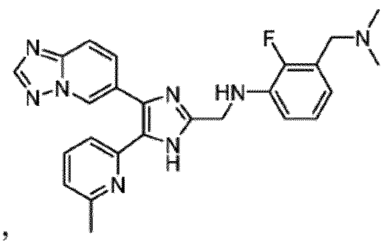 ,

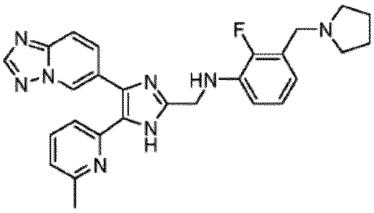 , 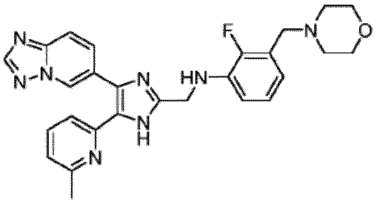 , 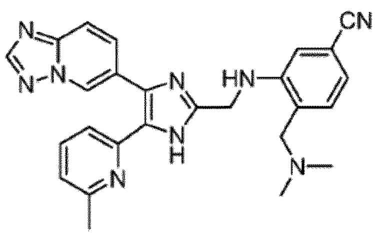 ,

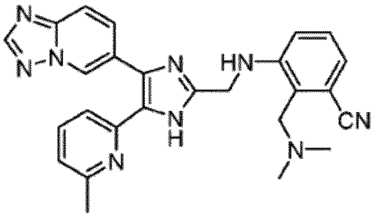 , 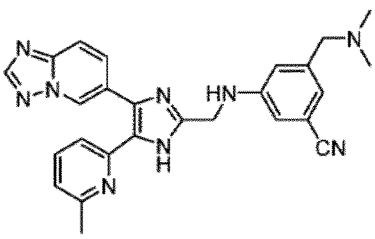 , 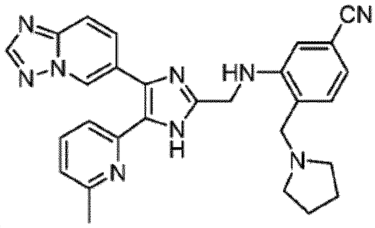 ,

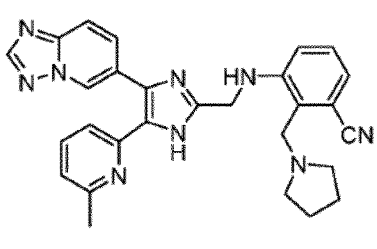

,

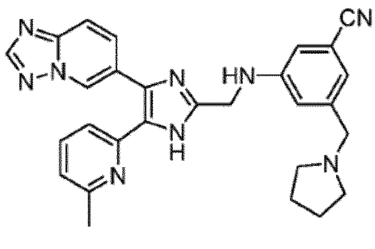

,

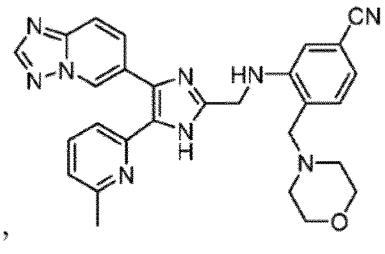

,

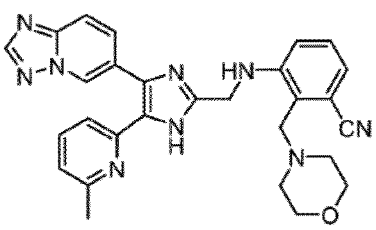

,

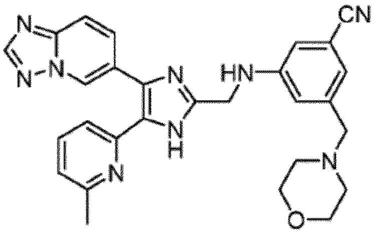

,

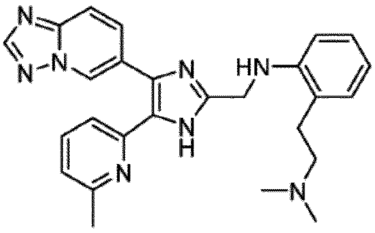

,

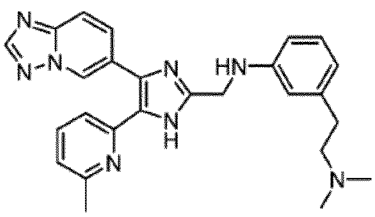

,

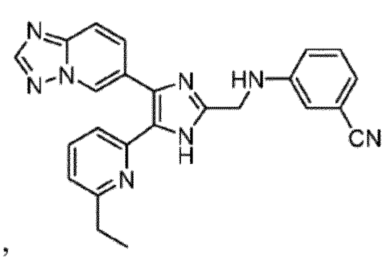

,

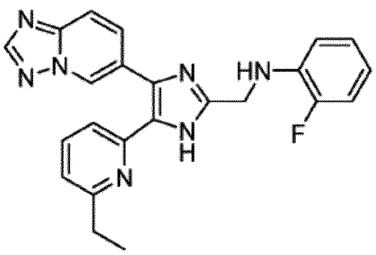

,

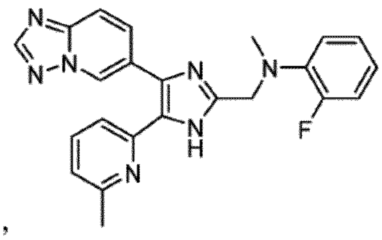

,

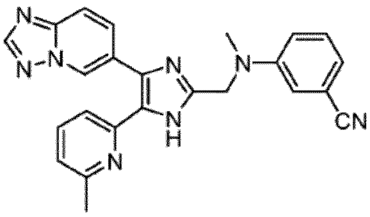

,

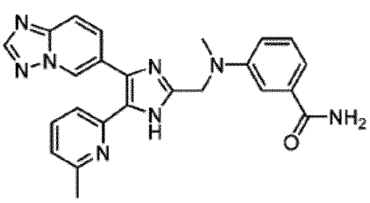

,

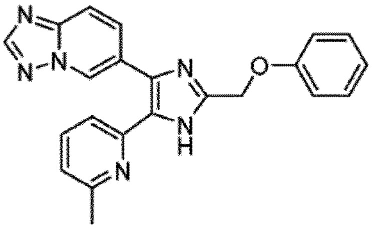

,

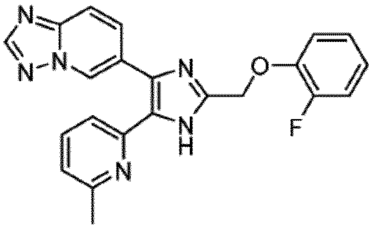

,

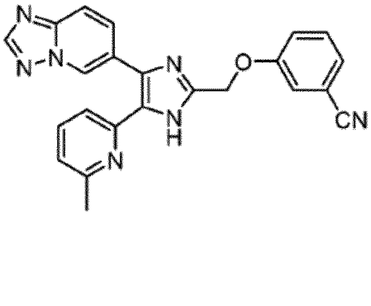

,

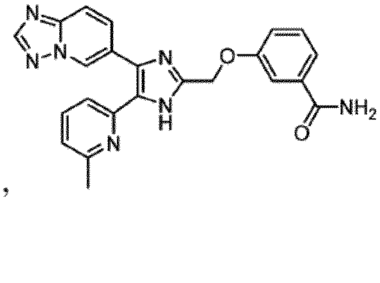

,

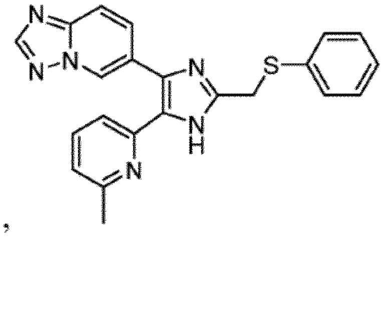

,

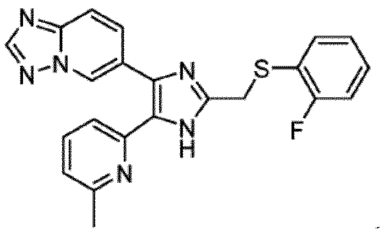

ou un sel ou un hydrate pharmaceutiquement acceptable de ceux-ci,

- la composition pharmaceutique étant une forme posologique orale, buccale ou sublinguale libérant de 50 à 5 000 mg du ou des composés par jour ; ou
- la composition pharmaceutique étant une formulation topique comprenant en outre de 1 à 98 % d'un véhicule ; ou
- la composition pharmaceutique étant une forme posologique parentérale comprenant de 25 à 250 mg du ou des composés par dose unique.

**2.** Composition pharmaceutique selon la revendication 1, dans laquelle

- la forme posologique orale, buccale ou sublinguale est un comprimé ou une gélule comprenant de 25 à 500 mg du ou des composés ; ou
- la formulation topique comprend un véhicule choisi parmi une base pour crème, une base pour pommade, de l'éthanol et/ou un alcool oléylique ; ou
- la formulation parentérale est une solution aqueuse stérile comprenant en outre des sels ou des monosaccharides en une quantité rendant la formulation isotonique avec le sang.

**3.** Composition pharmaceutique selon la revendication 1, dans laquelle la forme posologique orale, buccale ou sublinguale est un comprimé comprenant en outre des excipients tels que de l'amidon ou du lactose, ou une gélule ou un ovule seul ou en mélange avec des excipients ou un élixir ou une suspension contenant des agents aromatisants ou colorants.

**4.** Composition pharmaceutique selon la revendication 1 ou 2, dans laquelle la forme posologique parentérale est formulée pour une injection intraveineuse, intramusculaire, sous-cutanée ou intracoronarienne.

**5.** Composition pharmaceutique selon l'une quelconque des revendications précédentes, la composition comprenant le composé

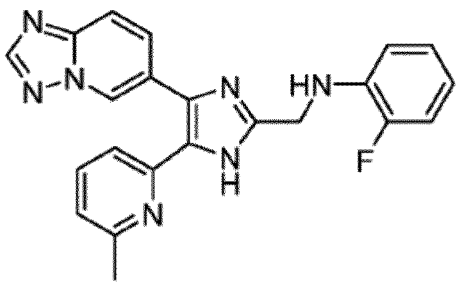

sous forme de sel pharmaceutiquement acceptable choisi parmi les composés

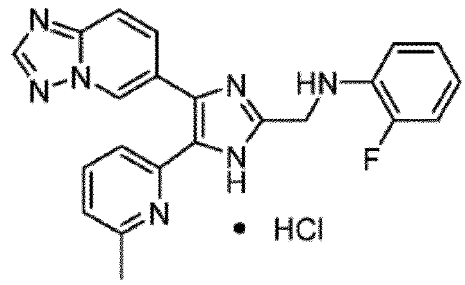

et

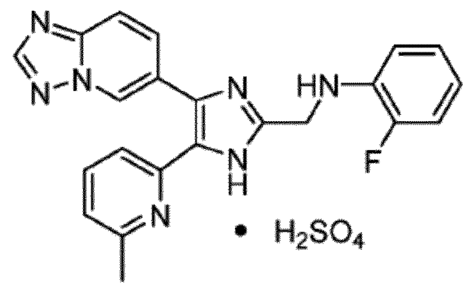

.

6. Composition pharmaceutique selon l'une quelconque des revendications précédentes, destinée à être utilisée dans le traitement, la prévention ou la réduction d'une maladie chez l'homme choisie dans le groupe constitué par une glomérulonéphrite, une néphropathie diabétique, une néphropathie lupique, une néphropathie induite par une hypertension, une fibrose interstitielle rénale, une fibrose rénale résultant de complications due à une exposition à des médicaments, une néphropathie associée au VIH, une néphropathie due à une greffe, une fibrose hépatique due à toutes les étiologies, un dysfonctionnement hépatique attribuable à des infections, une hépatite induite par l'alcool, les troubles de l'arbre biliaire, une fibrose kystique, une fibrose pulmonaire, une maladie pulmonaire interstitielle, une lésion pulmonaire aiguë, le syndrome de détresse respiratoire de l'adulte, une fibrose pulmonaire idiopathique, une maladie pulmonaire obstructive chronique, une maladie pulmonaire due à des agents infectieux ou toxiques, une fibrose cardiaque post-infarctus, une insuffisance cardiaque congestive, une cardiomyopathie dilatée, une myocardite, un épaississement intime, une sténose vasculaire, un remodelage vasculaire induit par une hypertension, une hypertension artérielle pulmonaire, une resténose coronaire, une resténose périphérique, une resténose carotidienne, une resténose induite par un stent, une athérosclérose, une cicatrisation oculaire, une cicatrisation cornéenne, une vitréorétinopathie proliférative, un glaucome, une pression intraoculaire, une cicatrice excessive ou hypertrophique ou la formation de chéloïdes dans le derme lors de la cicatrisation résultant de traumatismes ou de plaies chirurgicales, une adhérence péritonéale et sous-cutanée, une dermatomyosite, une polymyosite, une sclérodermie, une fibrosclérose, une sclérose systémique progressive, une arthrite, une ostéoporose, des ulcères, un dysfonctionnement neurologique, une dysfonction érectile masculine, la maladie de Peyronie, la contracture de Dupuytren, la maladie d'Alzheimer, le syndrome de Raynaud, une fibrose induite par rayonnement, une thrombose, une croissance de métastases tumorales, un myélome multiple, un mélanome, un gliome, les glioblastomes, une leucémie, les sarcomes, les léiomyomes, un mésothéliome et les carcinomes du poumon, du sein, du côlon, des reins, des ovaires, du col de l'utérus, du foie, des voies biliaires, des voies gastro-intestinales, du pancréas, de la prostate, de la tête et du cou.

7. Composition pharmaceutique selon l'une quelconque des revendications précédentes, destinée à être utilisée dans le traitement, la prévention ou la réduction d'une maladie chez l'homme choisie dans le groupe constitué par une néphropathie diabétique, une fibrose interstitielle rénale, une fibrose hépatique due à toutes les étiologies, un dysfonctionnement hépatique attribuable à des infections, une hépatite induite par l'alcool, les troubles de l'arbre biliaire, une fibrose pulmonaire, une maladie pulmonaire interstitielle, une fibrose pulmonaire idiopathique, une maladie pulmonaire obstructive chronique, une maladie pulmonaire due à des agents infectieux ou toxiques, une hypertension artérielle pulmonaire, une resténose coronaire, une resténose périphérique, une resténose carotidienne, une resténose induite par un stent, une cicatrisation oculaire, une cicatrisation cornéenne, un glaucome, une pression intraoculaire, une cicatrice excessive ou hypertrophique ou la formation de chéloïdes dans le derme lors de la cicatrisation résultant de traumatismes ou de plaies chirurgicales, une adhérence péritonéale et sous-cutanée, une dermatomyosite, une polymyosite, une sclérodermie, une fibrosclérose, une sclérose systémique progressive,

la maladie de Peyronie, une fibrose induite par rayonnement, une croissance de métastases tumorales, un myélome multiple, un mélanome, un gliome, les glioblastomes, une leucémie, les sarcomes, les léiomyomes, un mésothéliome et les carcinomes du poumon, du sein, du côlon, des reins, des ovaires, du col de l'utérus, du foie, des voies biliaires, des voies gastro-intestinales, du pancréas, de la prostate, de la tête et du cou.

**8.** Composition pharmaceutique selon l'une quelconque des revendications précédentes, destinée à être utilisée dans le traitement, la prévention ou la réduction d'une maladie chez l'homme choisie dans le groupe constitué par une cicatrice excessive ou hypertrophique ou la formation de chéloïdes dans le derme lors de la cicatrisation résultant de traumatismes ou de plaies chirurgicales, une adhérence péritonéale et sous-cutanée, une dermatomyosite, une polymyosite, une sclérodermie, une fibrosclérose, une sclérose systémique progressive.

**9.** Composition pharmaceutique selon la revendication 6 ou 7, destinée à être utilisée dans le traitement, la prévention ou la réduction d'une maladie chez l'homme choisie dans le groupe constitué par un mélanome, un gliome, les glioblastomes, une leucémie et les carcinomes du poumon, du sein, du côlon, des reins, du foie, des voies gastro-intestinales, du pancréas, de la prostate, de la tête et du cou.

**10.** Composition pharmaceutique comprenant un ou plusieurs des composés

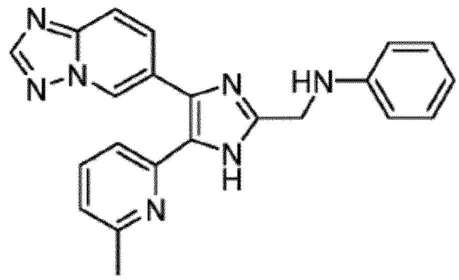 ,

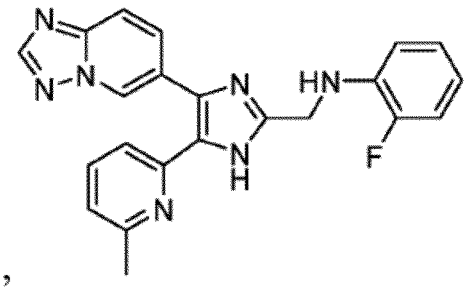 ,

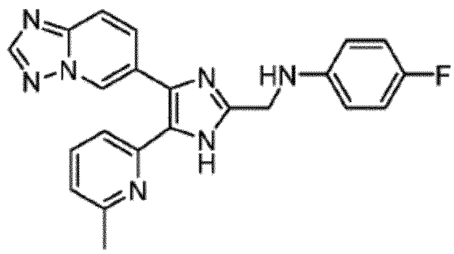 ,

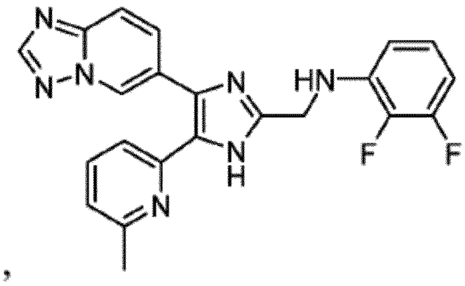 ,

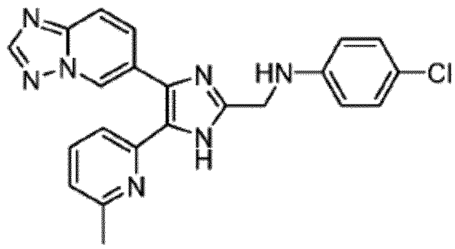 ,

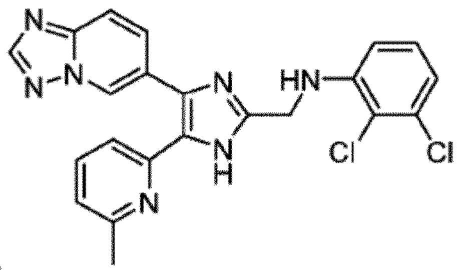 ,

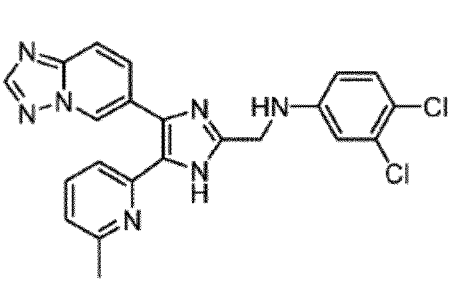

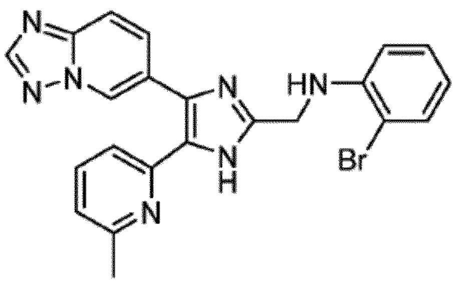

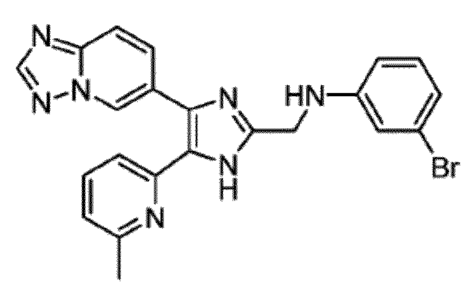

,

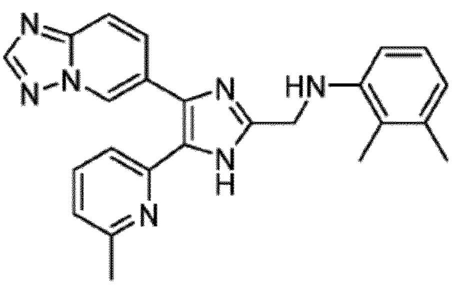

,

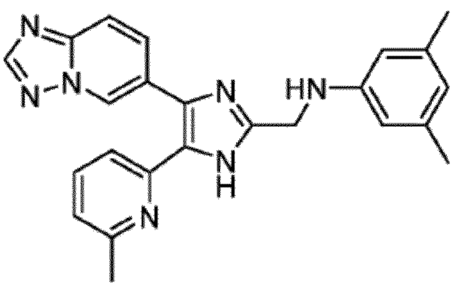

,

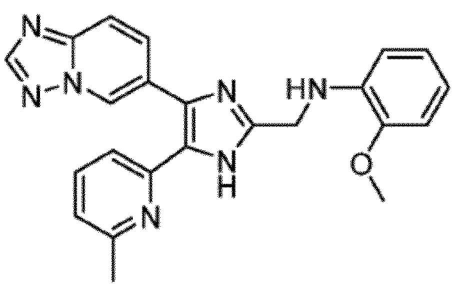

,

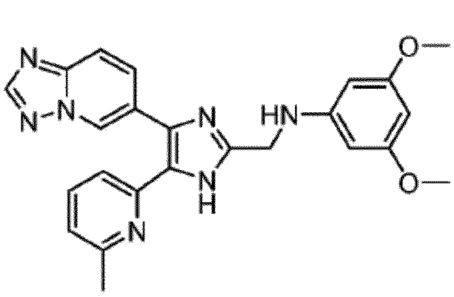

,

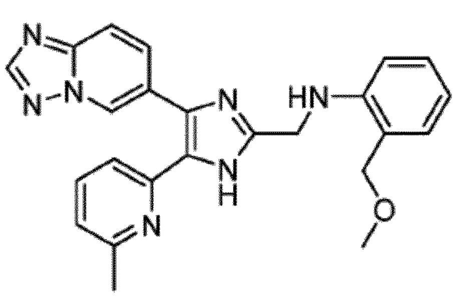

,

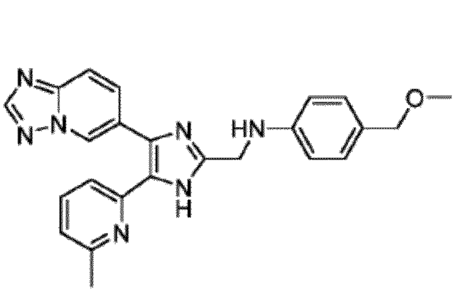

,

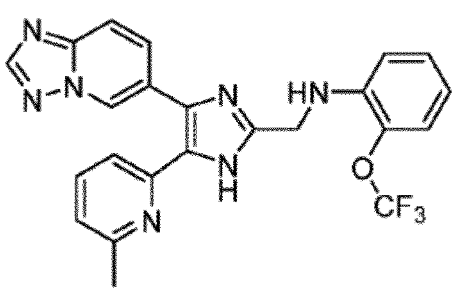

,

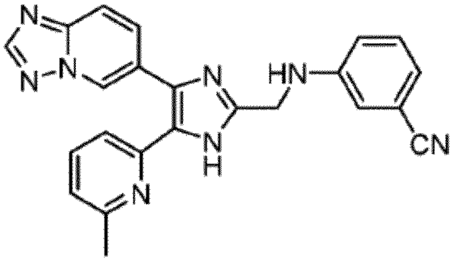,

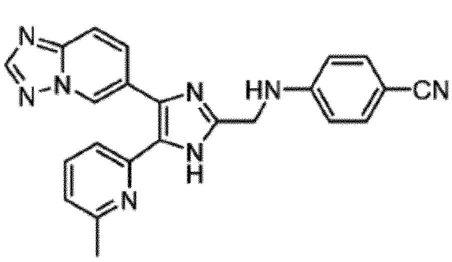,

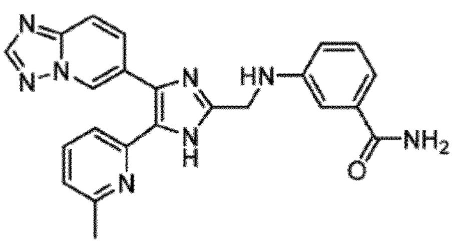,

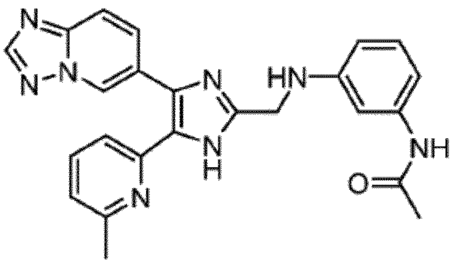,

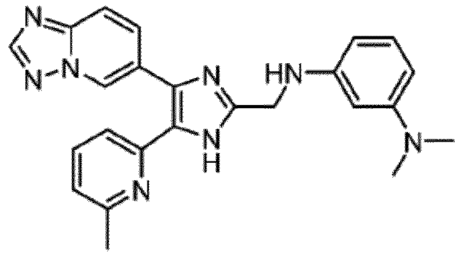,

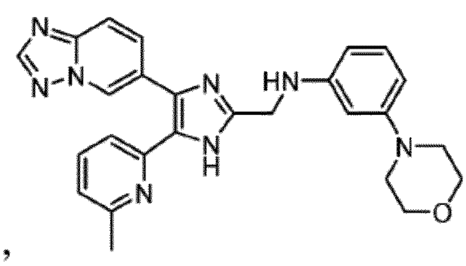,

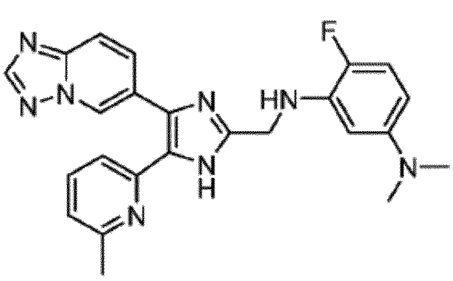,

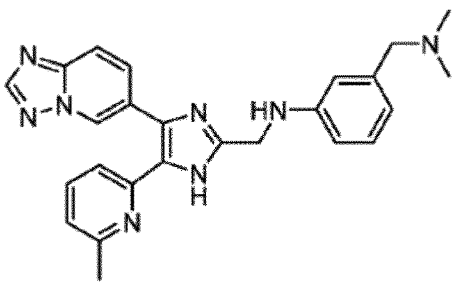,

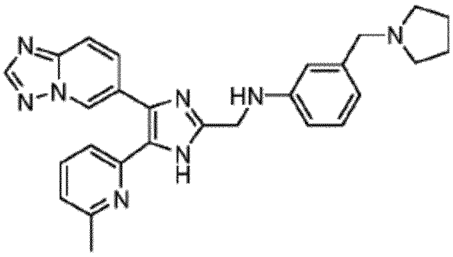,

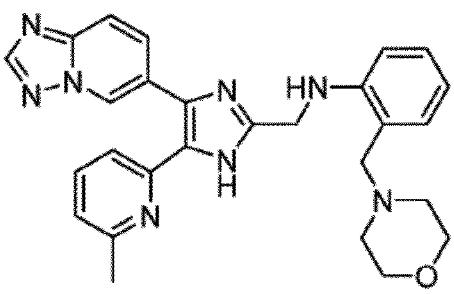,

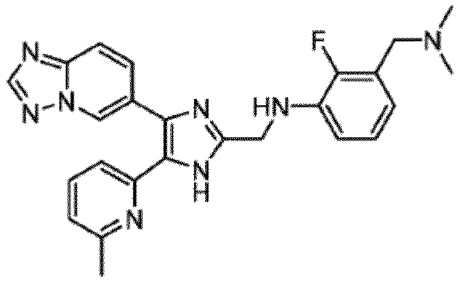,
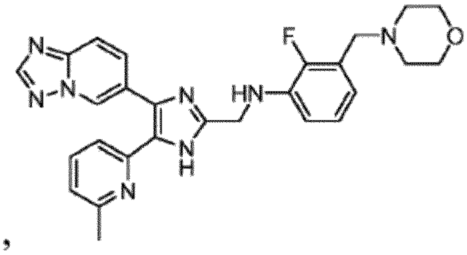,

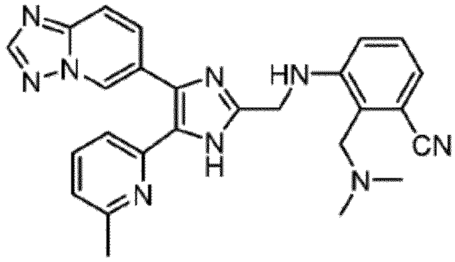,
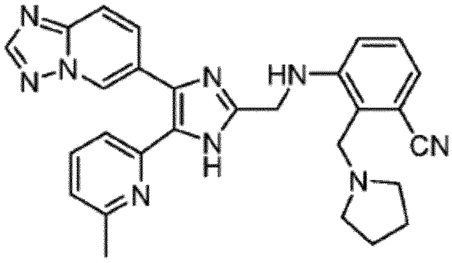,

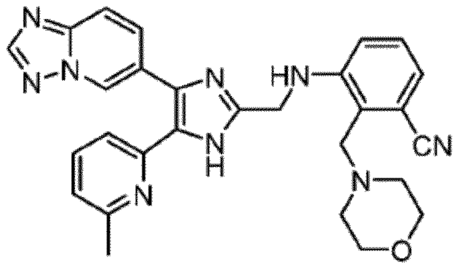,
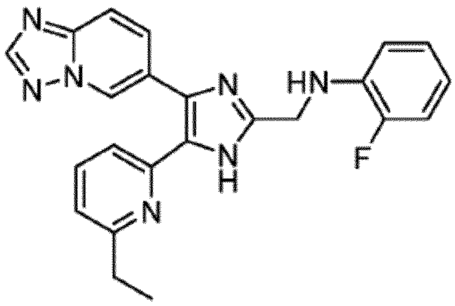,

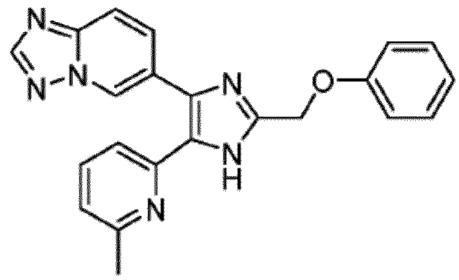,
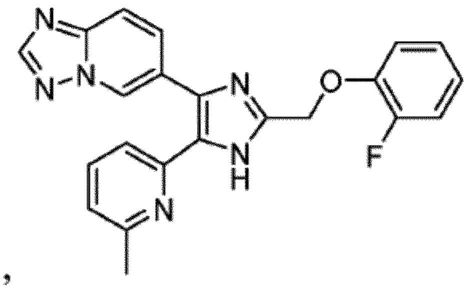,

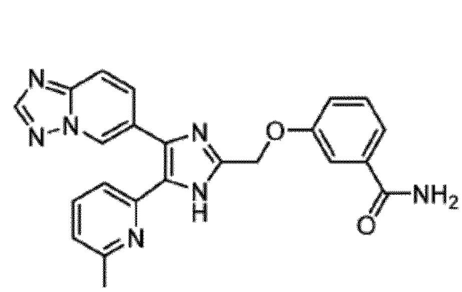
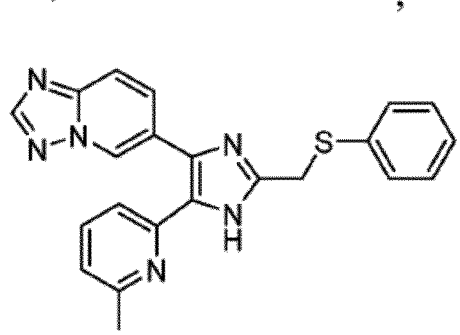

ou un sel ou hydrate pharmaceutiquement acceptable de ceux-ci, destinée à être utilisée dans le traitement, la prévention ou la réduction d'une maladie chez l'homme choisie dans le groupe constitué par une cicatrice excessive ou hypertrophique ou la formation de chéloïdes dans le derme lors de la cicatrisation résultant de traumatismes ou

de plaies chirurgicales, une adhérence péritonéale et sous-cutanée, une dermatomyosite, une polymyosite, une sclérodermie, une fibrosclérose, une sclérose systémique progressive, un mélanome, un gliome, les glioblastomes, une leucémie et les carcinomes du poumon, du sein, du côlon, des reins, du foie, des voies gastro-intestinales, du pancréas, de la prostate, de la tête et du cou.

【Figure 1】

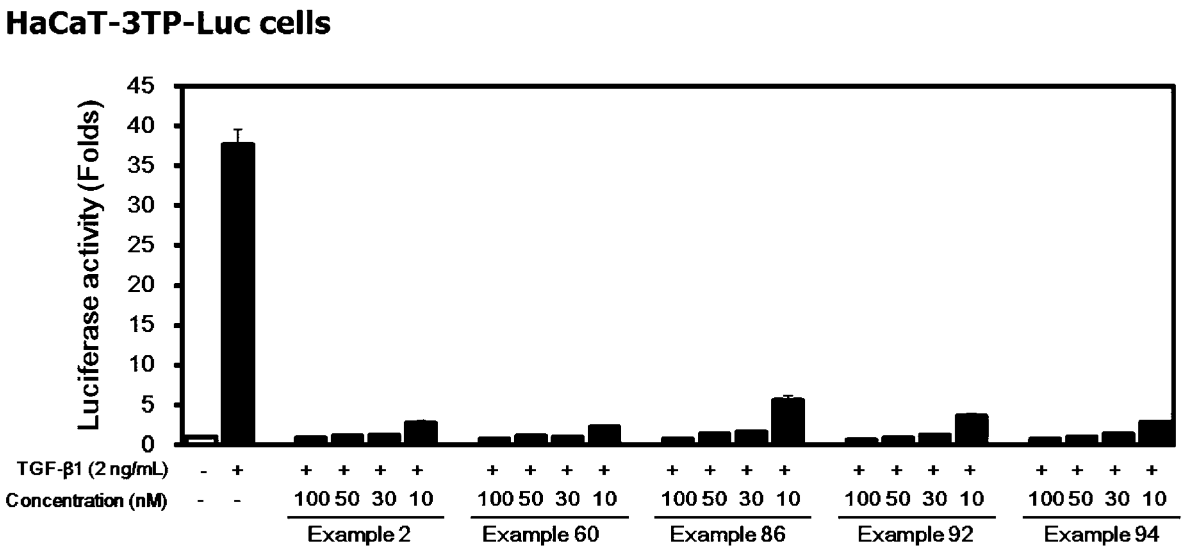
HaCaT-3TP-Luc cells
Luciferase activity (Folds)
45
40
35
30
25
20
15
10
5
0
TGF-β1 (2 ng/mL) - + + + + + + + + + + + + + + + + + + + + + +
Concentration (nM) - - 100 50 30 10 100 50 30 10 100 50 30 10 100 50 30 10 100 50 30 10
Example 2
Example 60
Example 86
Example 92
Example 94

【Figure 2】

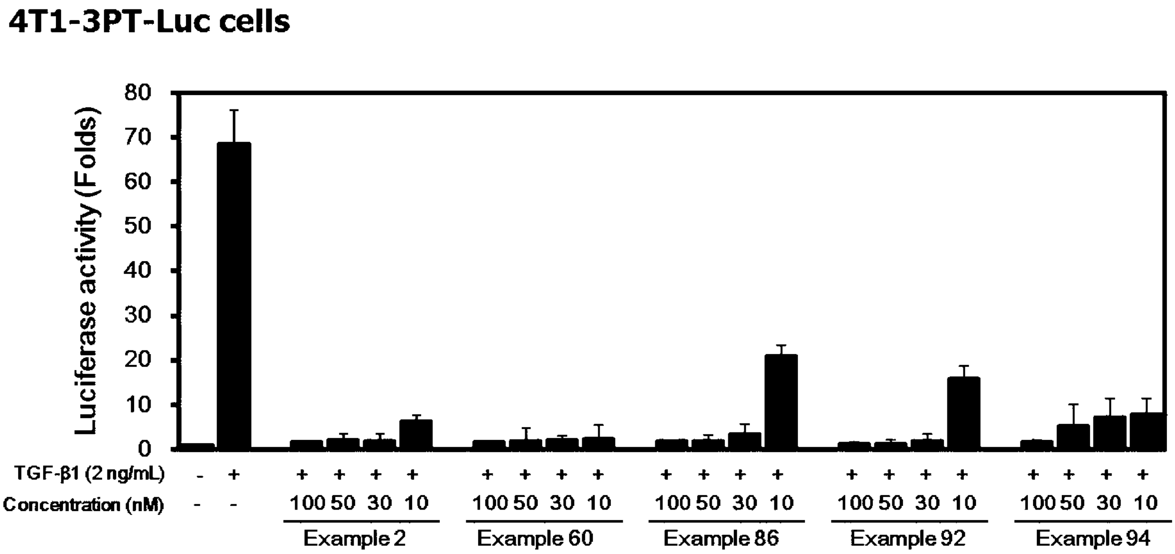
4T1-3PT-Luc cells
Luciferase activity (Folds)
80
70
60
50
40
30
20
10
0
TGF-β1 (2 ng/mL) - + + + + + + + + + + + + + + + + + + + + +
Concentration (nM) - - 100 50 30 10 100 50 30 10 100 50 30 10 100 50 30 10 100 50 30 10
Example 2
Example 60
Example 86
Example 92
Example 94

【Figure 3】

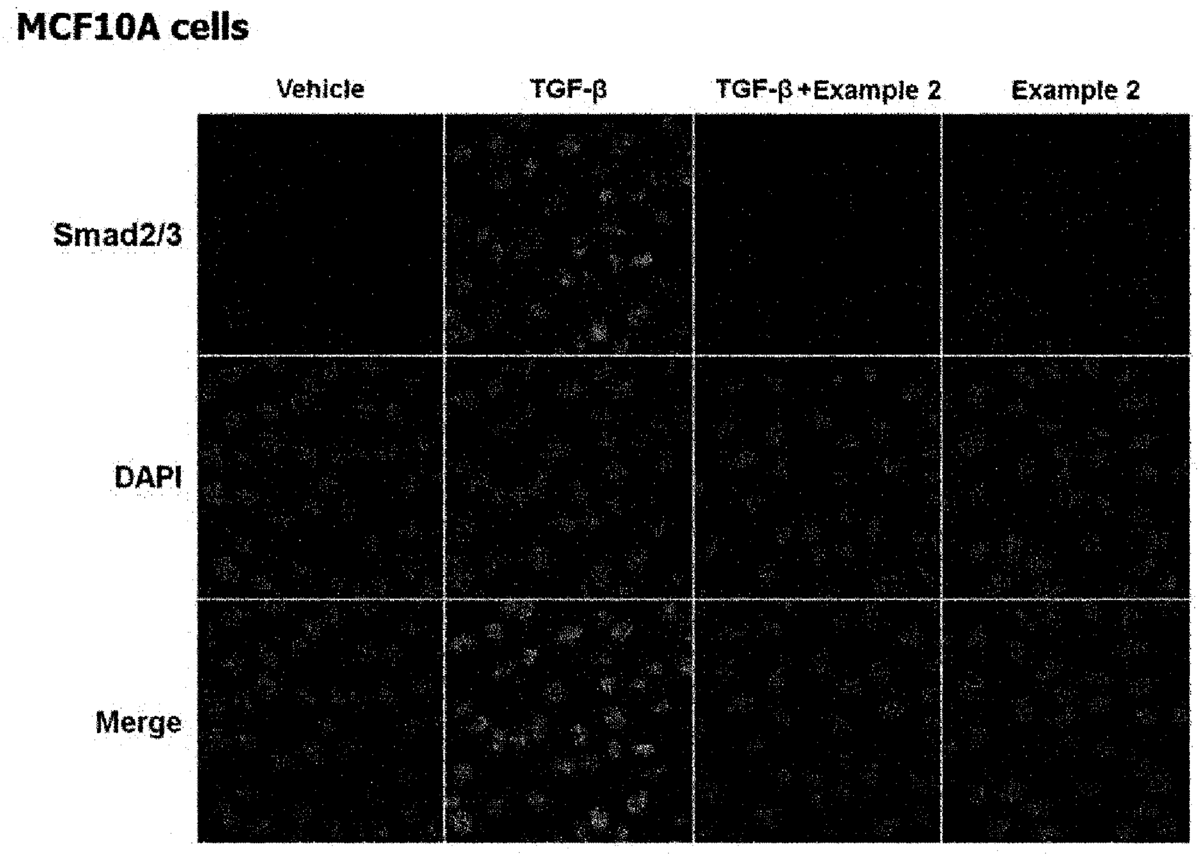
MCF10A cells
Vehicle
TGF-β
TGF-β+Example 2
Example 2
Smad2/3
DAPI
Merge

【Figure 4】

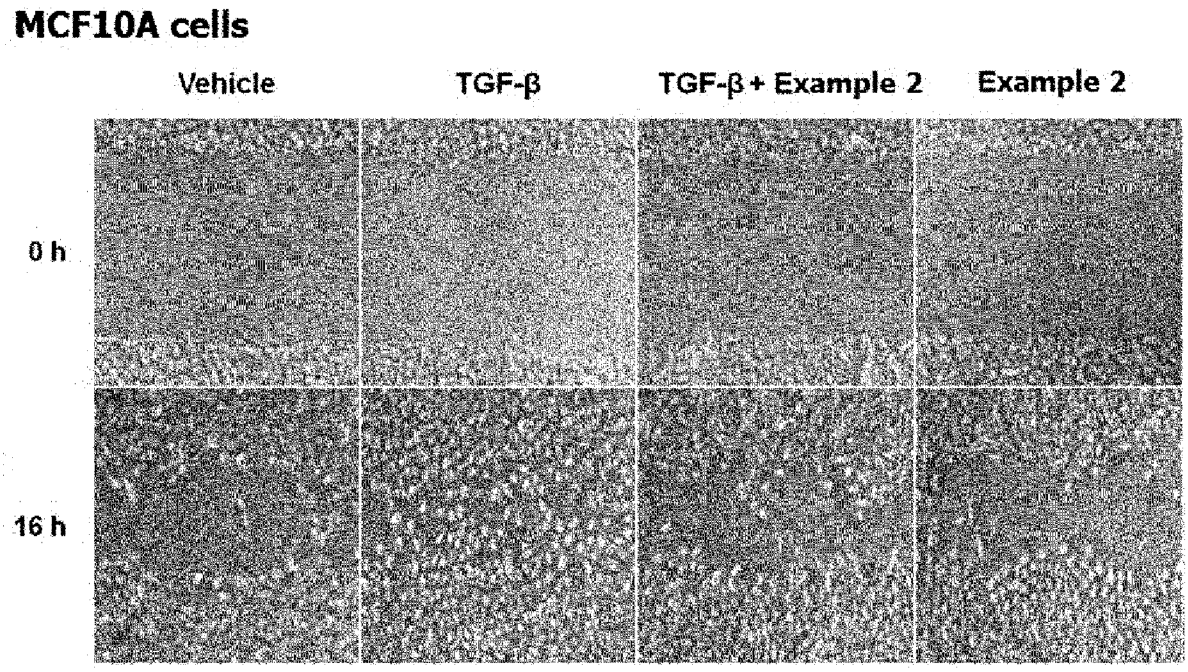

【Figure 5a】

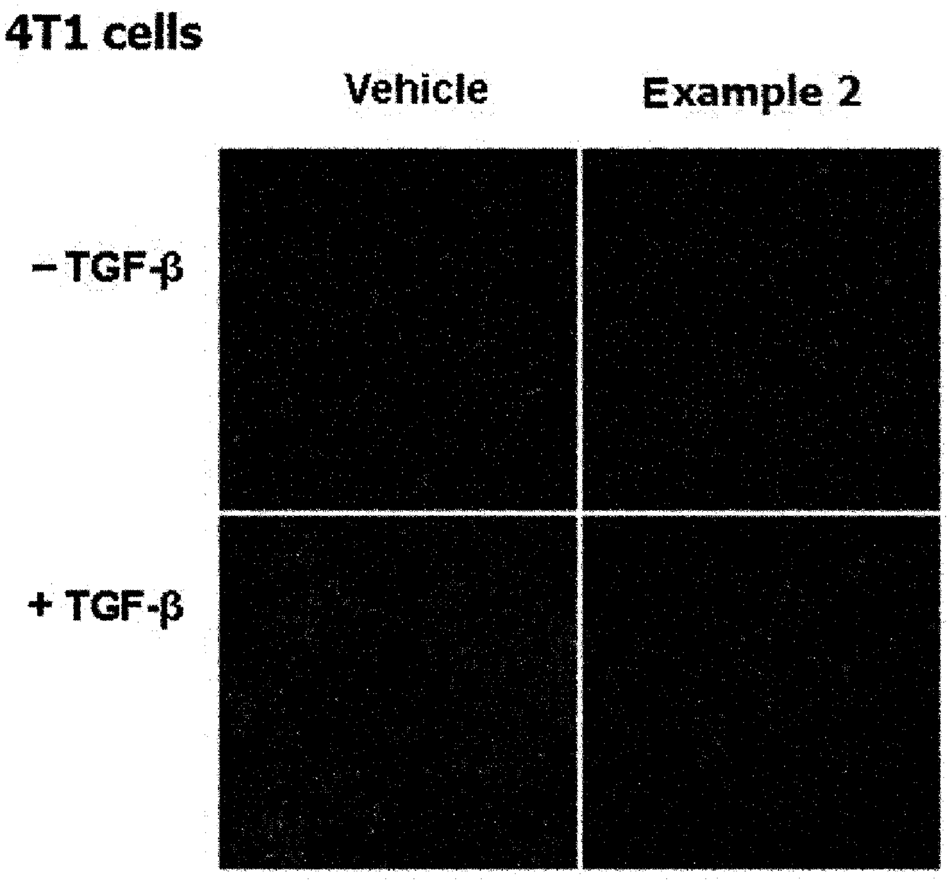

【Figure 5b】

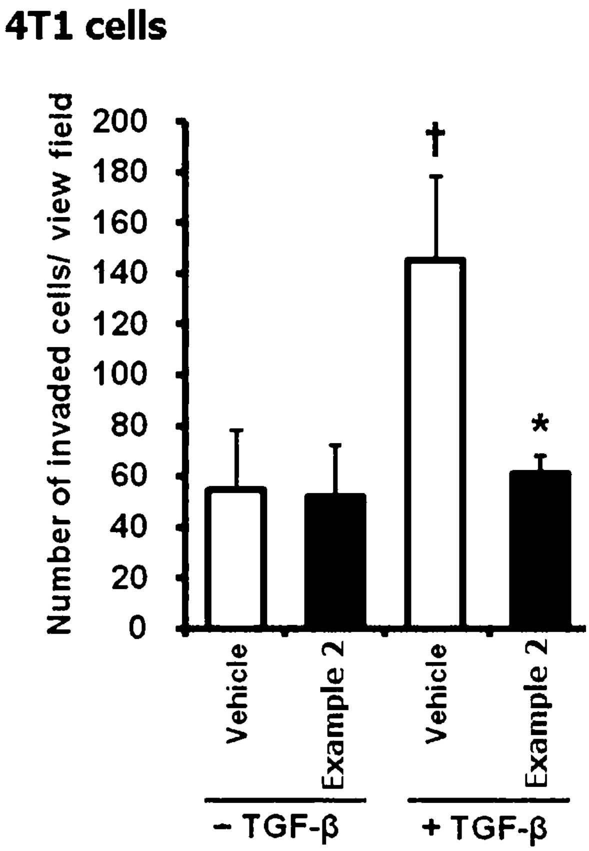


Data represents the mean number of invaded cells per view field ± S.D. (n = 3).
†: significantly different from vehicle group ($P < 0.05$).
*: significantly different from TGF-β- treated group ($P < 0.05$).

【Figure 6】

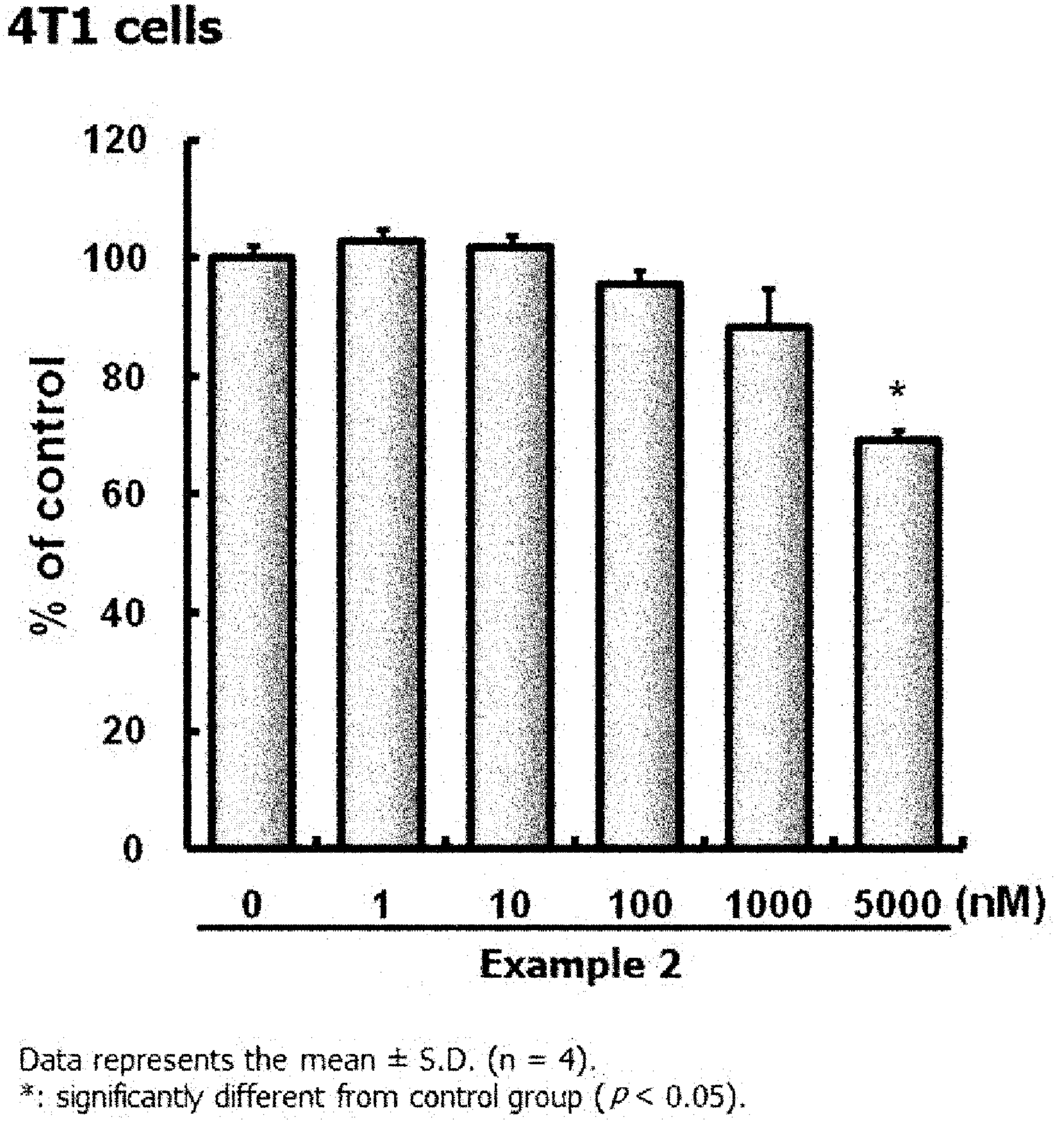
4T1 cells
% of control
120
100
80
60
40
20
0
*
0
1
10
100
1000
5000 (nM)
Example 2
Data represents the mean ± S.D. (n = 4).
*: significantly different from control group (p < 0.05).

【Figure 7】

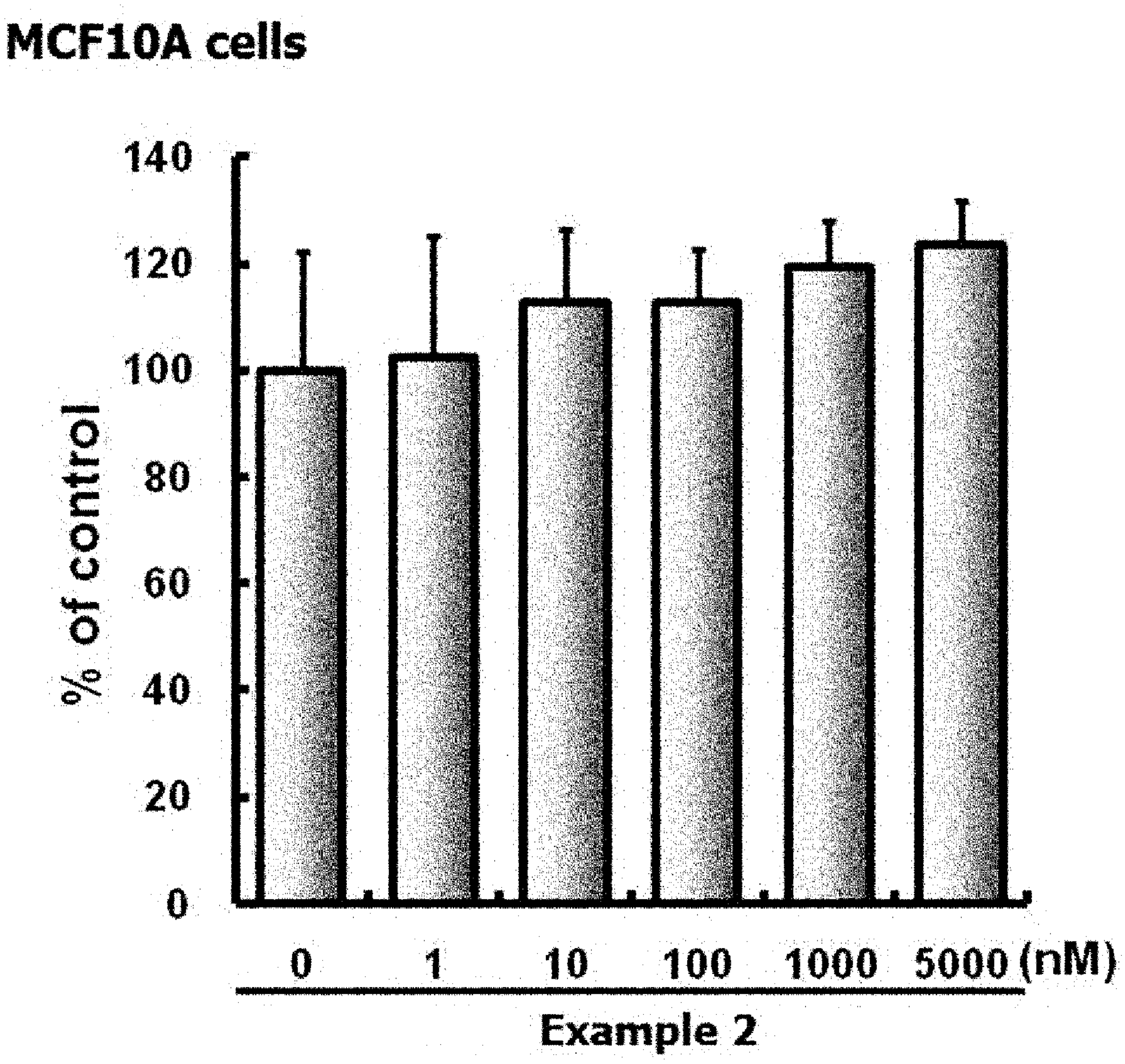


Data represents the mean ± S.D. (n = 4).

【Figure 8a】

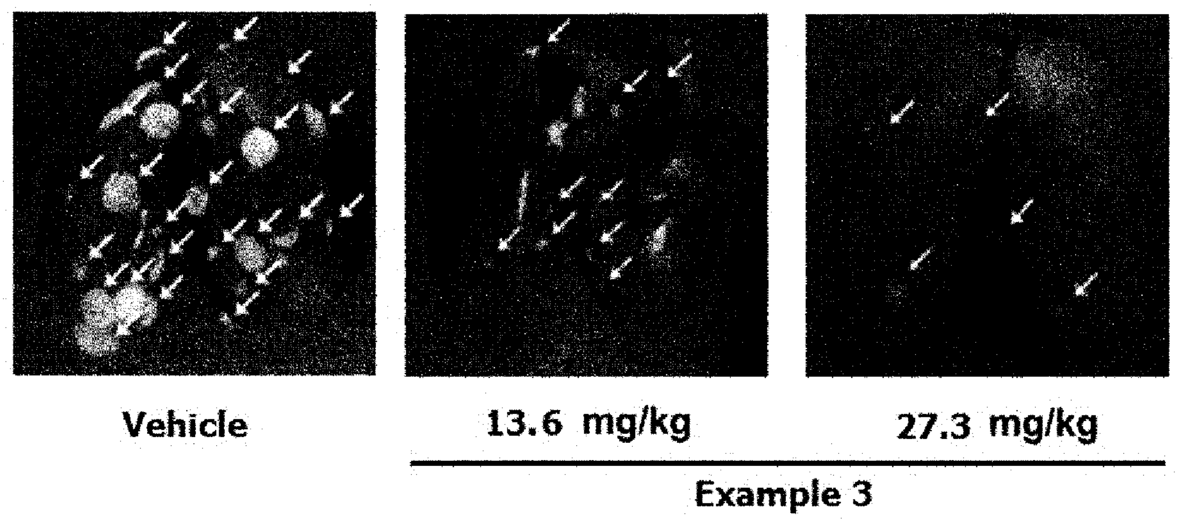

【Figure 8b】

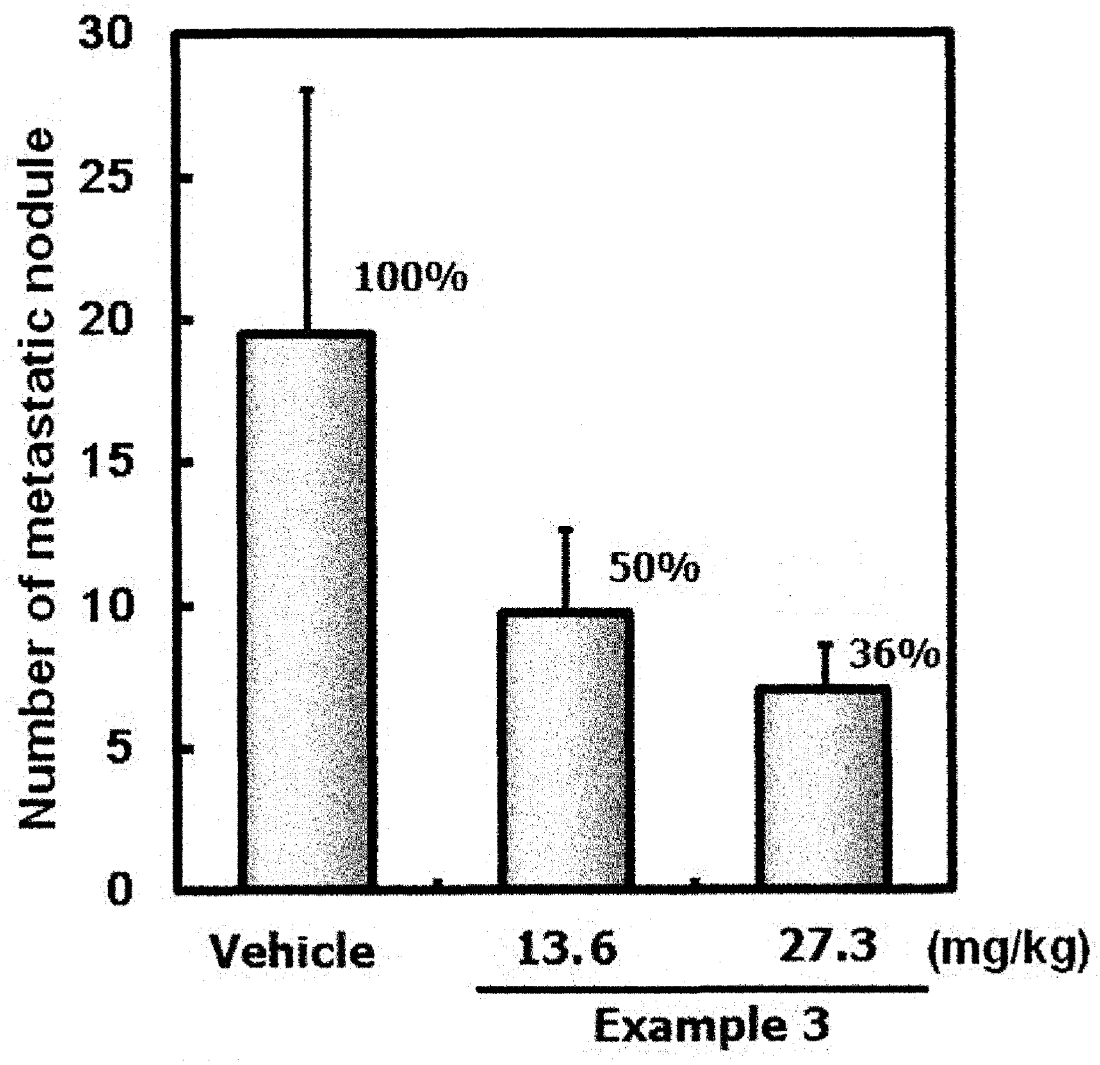


Data represents the mean ± S.E. (n = 8).

【Figure 9a】

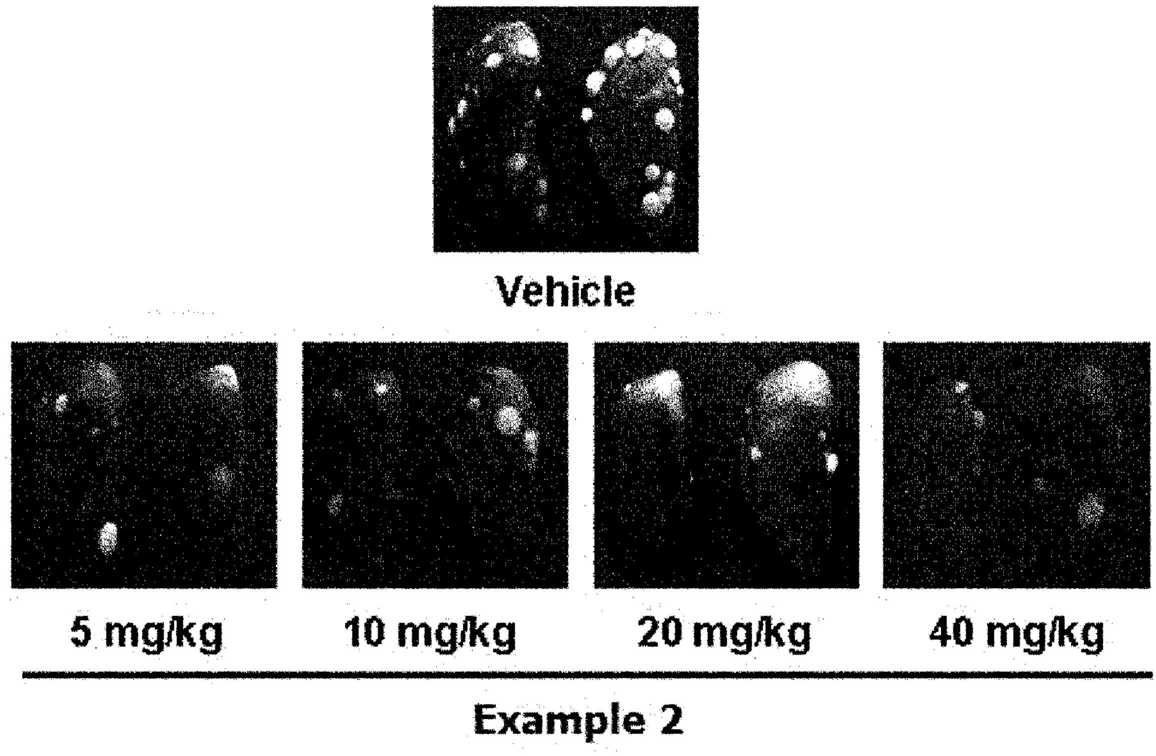

【Figure 9b】

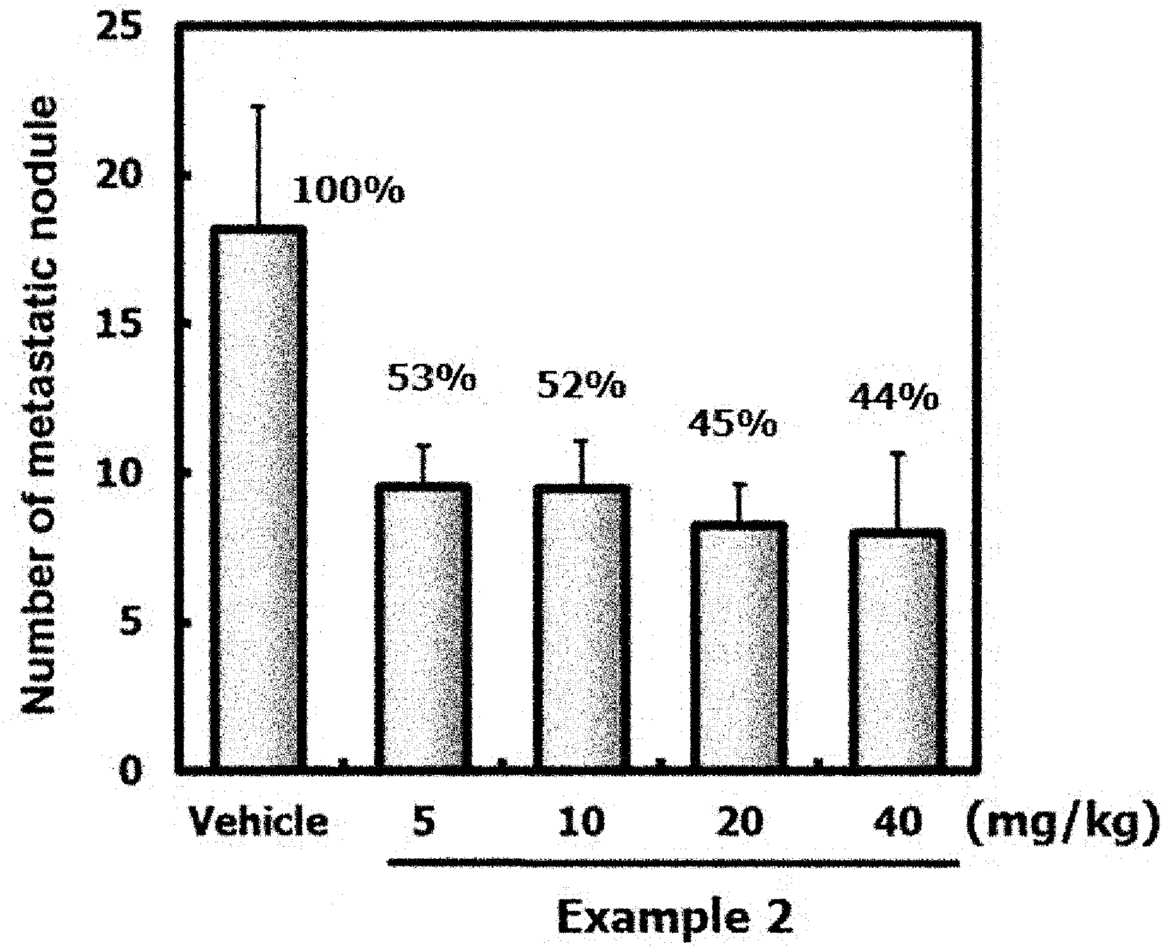


Data represents the mean ± S.E. (n = 7-10).

【Figure 10a】

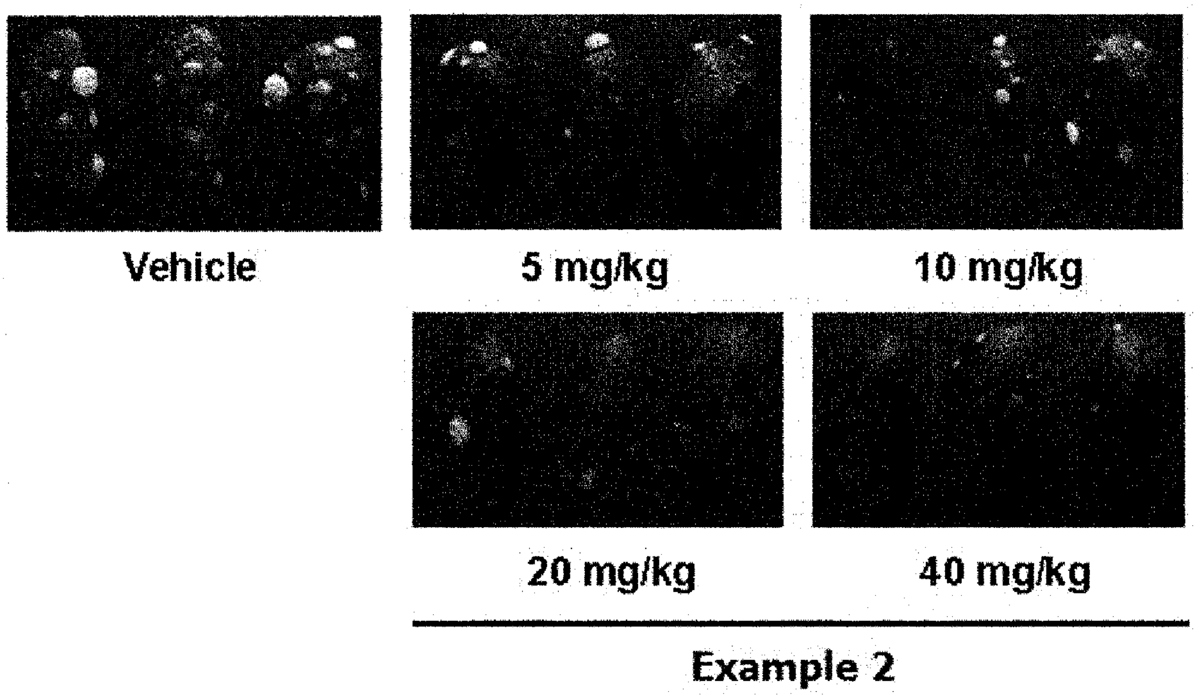

【Figure 10b】

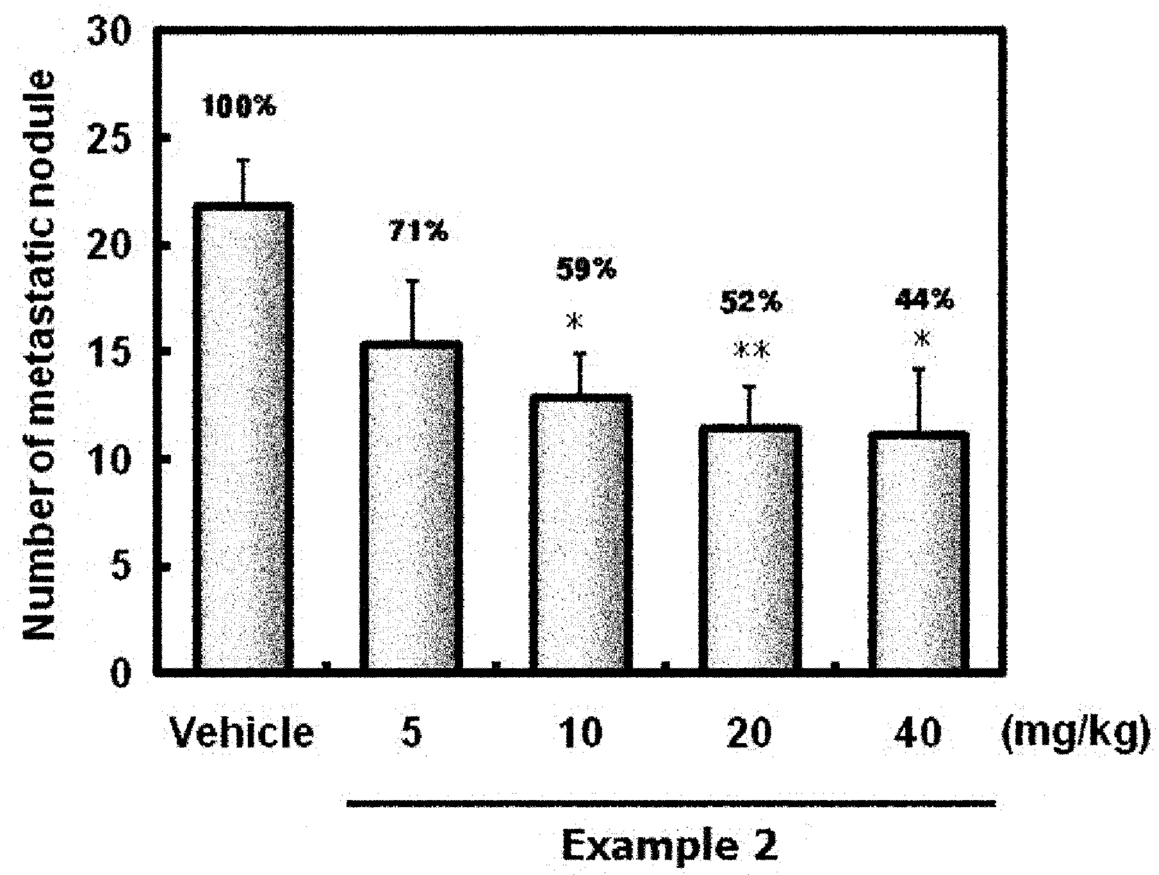


Data represents the mean ± S.E. (n = 9−10).
*: significantly different from vehicle group ($p < 0.05$).
**: significantly different from vehicle group ($p < 0.01$).

【Figure 10c】

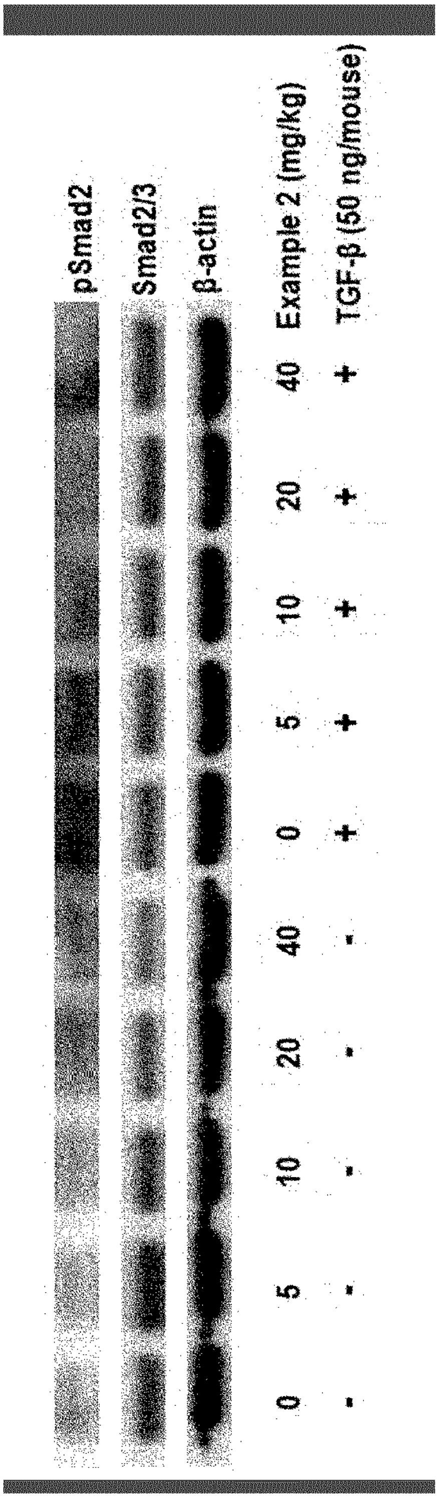
pSmad2
Smad2/3
β-actin
Example 2 (mg/kg) 0 5 10 20 40 0 5 10 20 40
TGF-β (50 ng/mouse) - - - - - + + + + +

【Figure 11a】

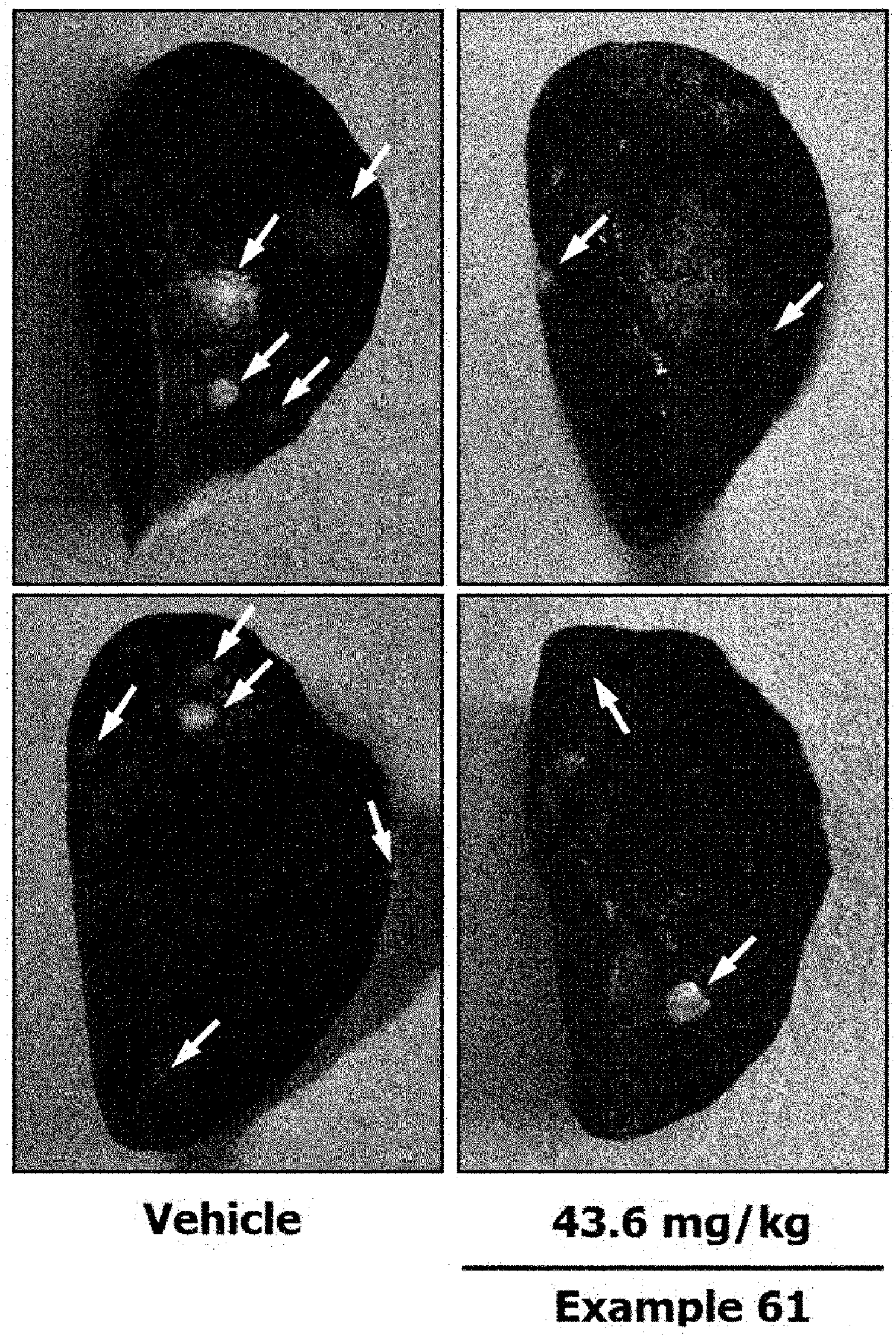

【Figure 11b】

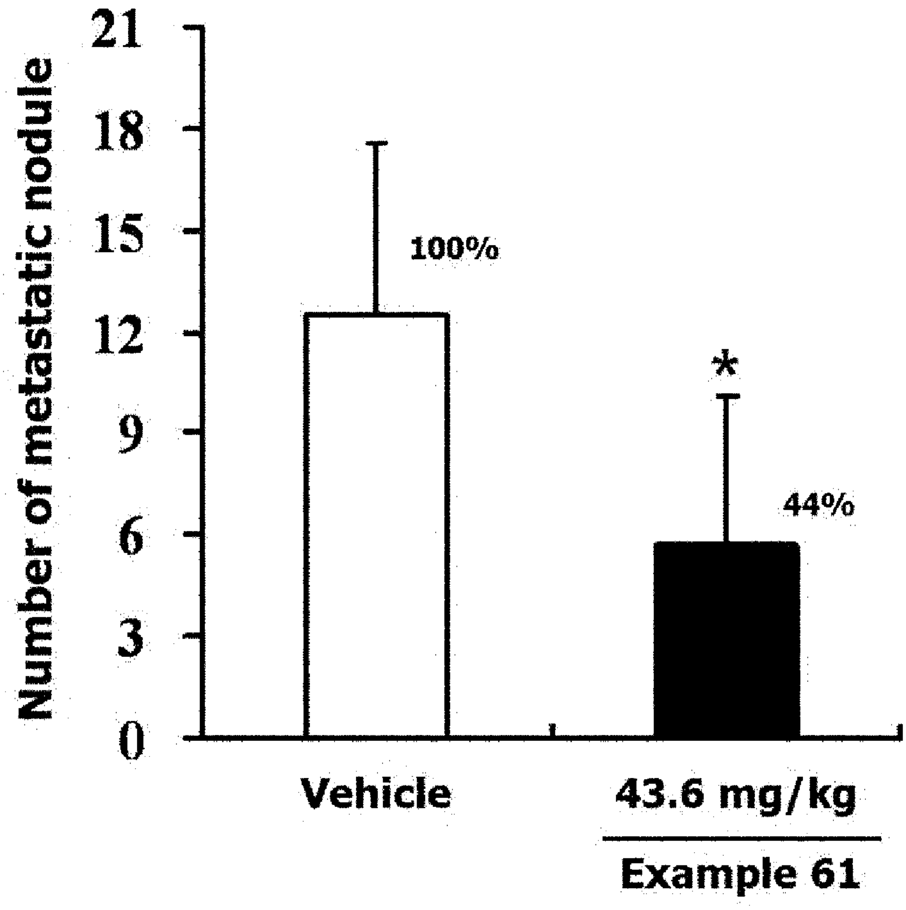


Data represents the mean ± S.D. (n = 8).
*: significantly different from vehicle group ($p < 0.05$).

【Figure 11c】

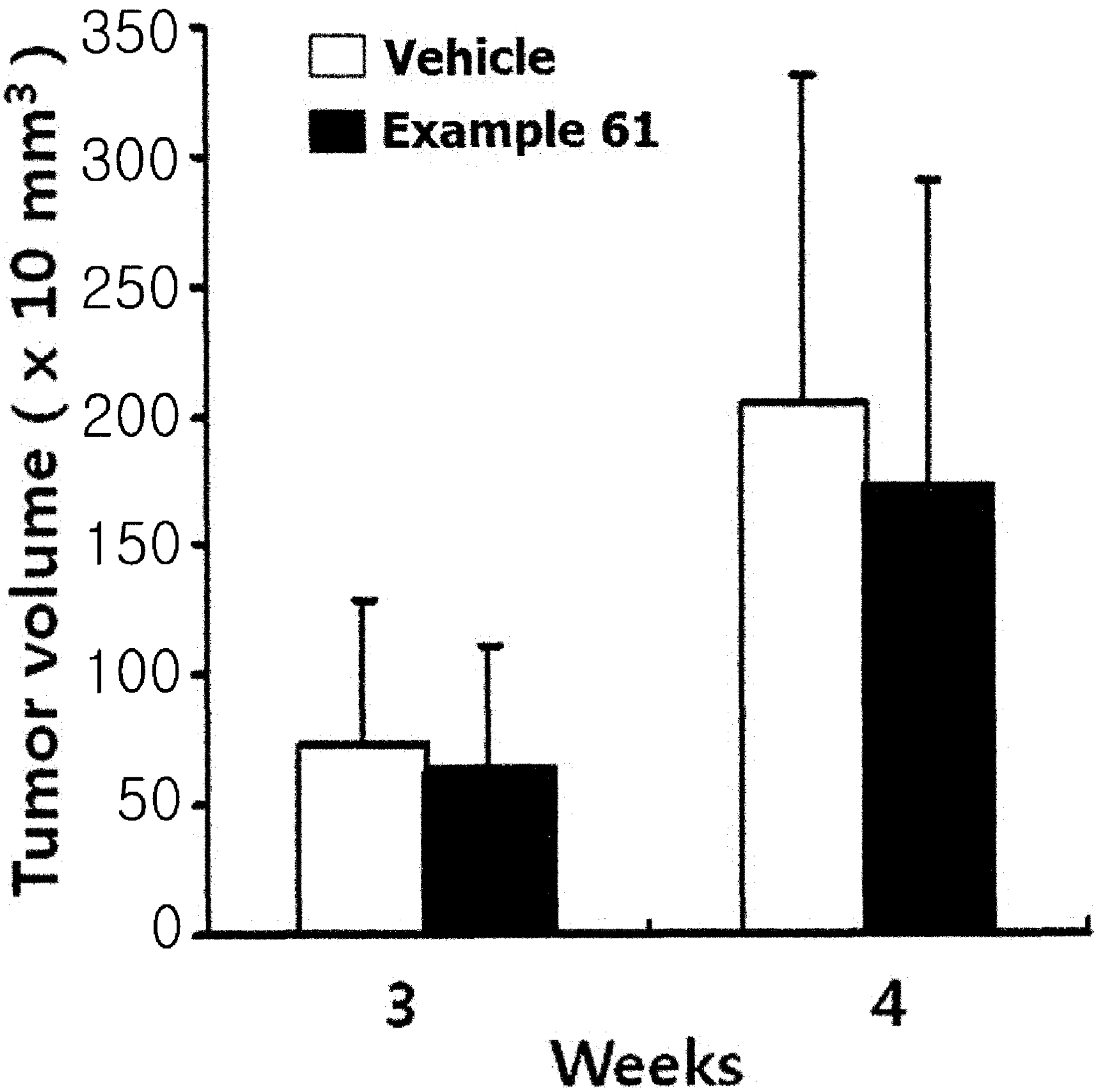


Data represents the mean ± S.D. (n = 6).

【Figure 12a】

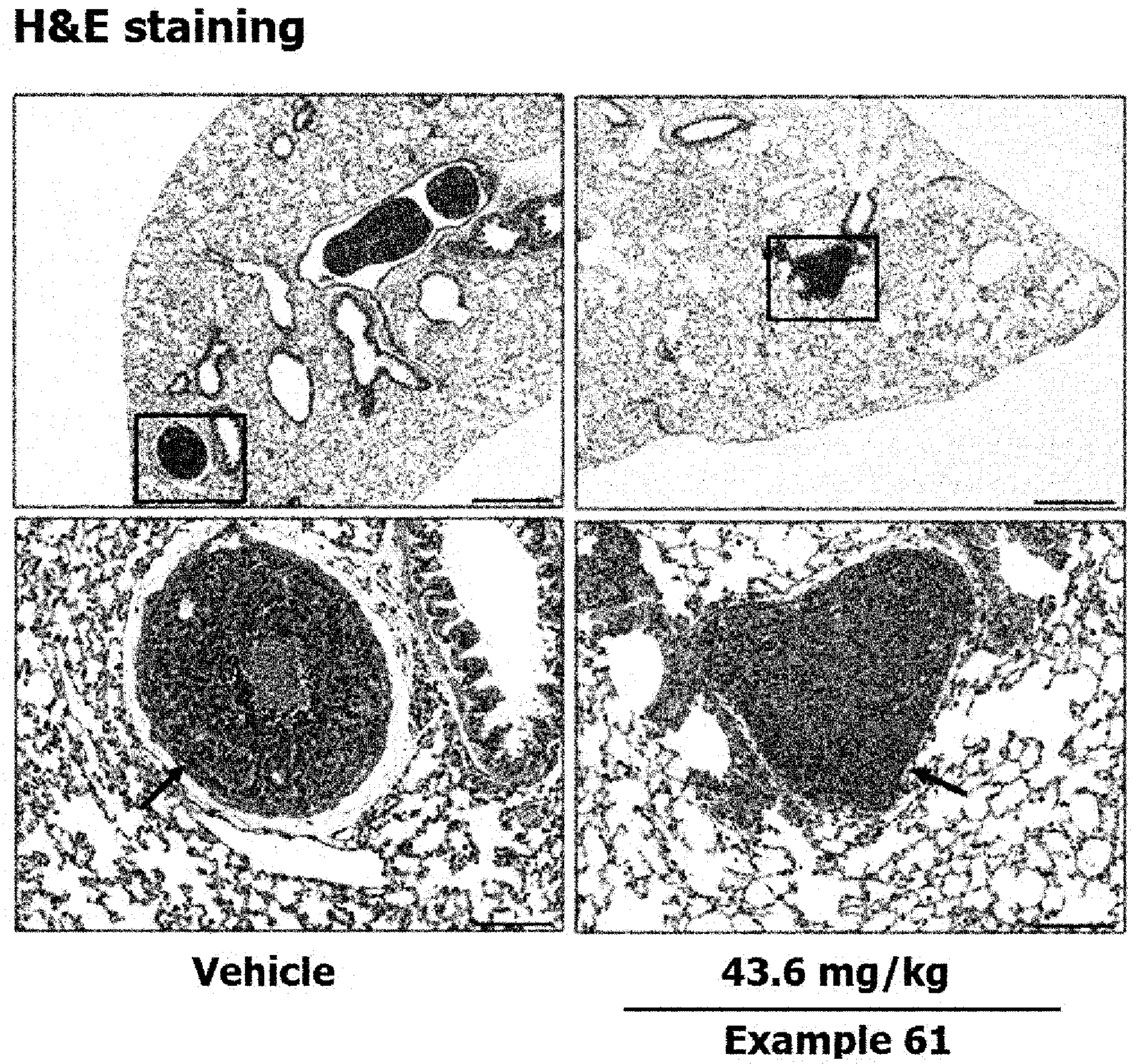


Arrows indicate metastatic lesions in the lung.

【Figure 12b】

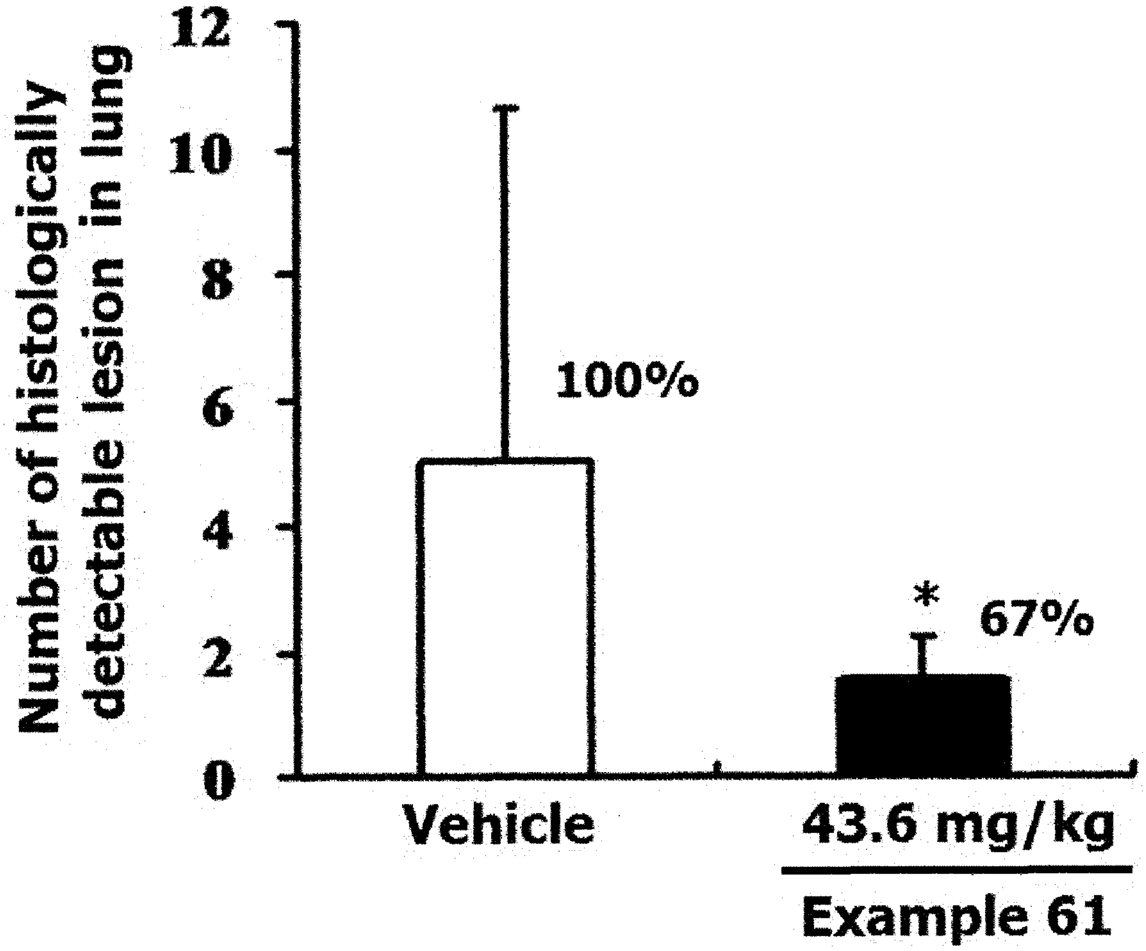


Data represents the mean ± S.D. (n = 3).
*: significantly different from vehicle group ($p < 0.05$).

【Figure 12c】

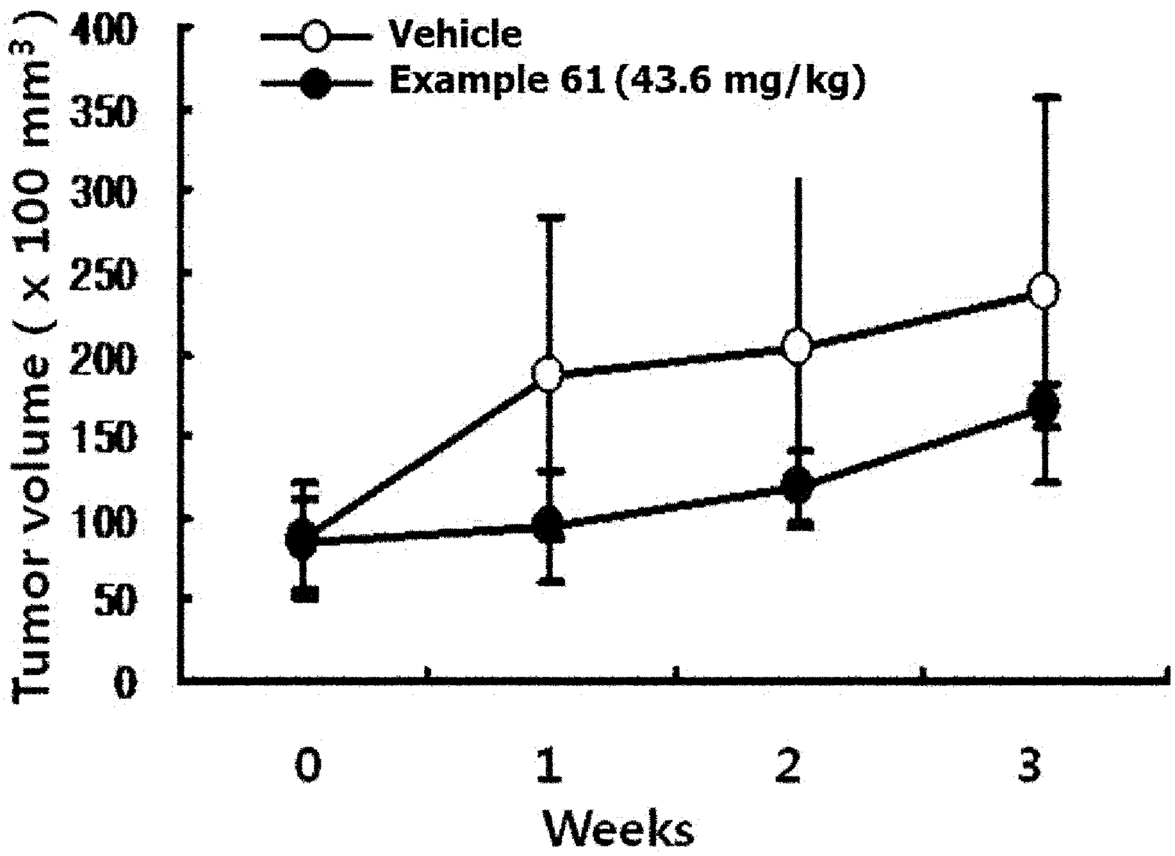


Data represents the mean ± S.D. (n = 3).

【Figure 12d】

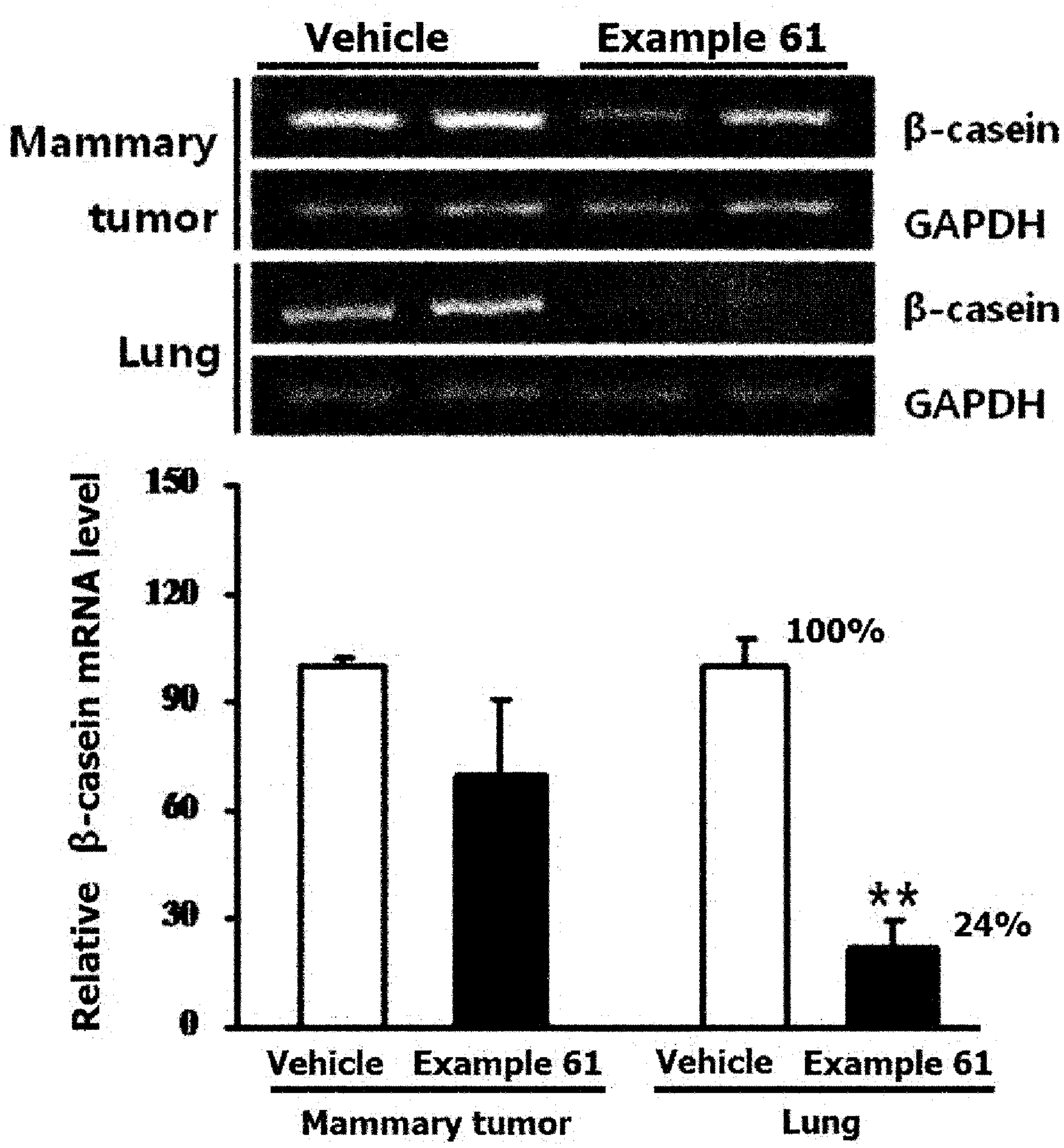


Data represents the mean ± S.D. (n = 6).
**: significantly different from vehicle group in lung ($p < 0.01$).

【Figure 13a】

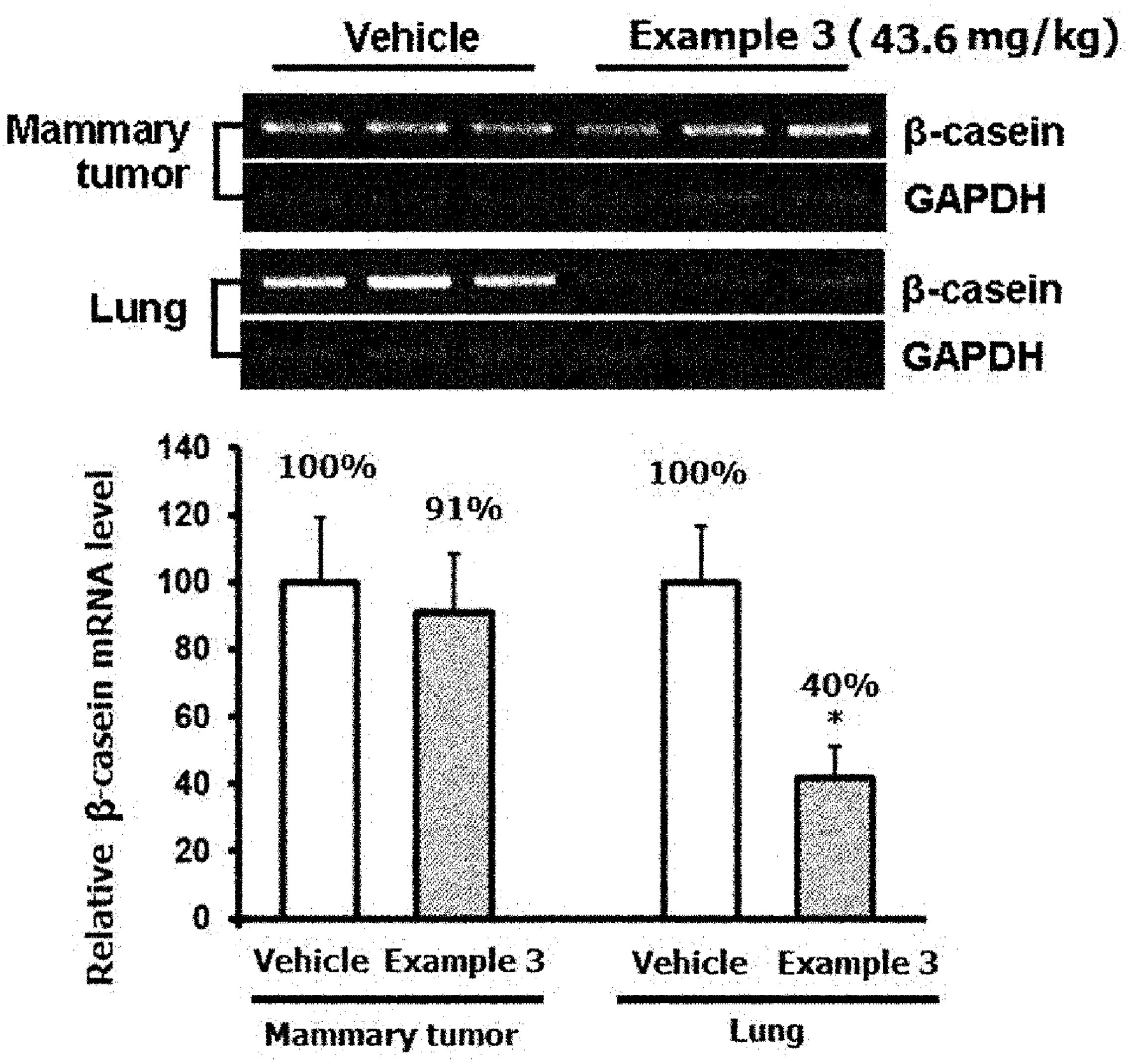


Data represents the mean ± S.E. (n = 7–10).
* : significantly different from vehicle group in lung ($p < 0.05$).

【Figure 13b】

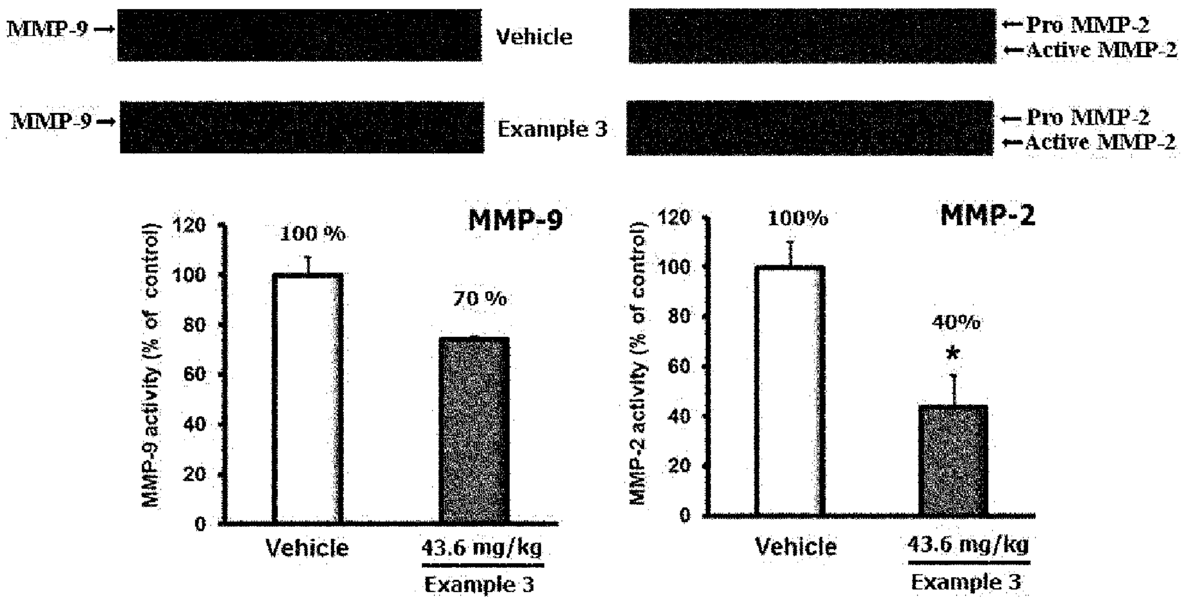


Data represents the mean ± S.E. (n = 5–10).
*: significantly different from vehicle group ($p < 0.05$).

【Figure 14a】

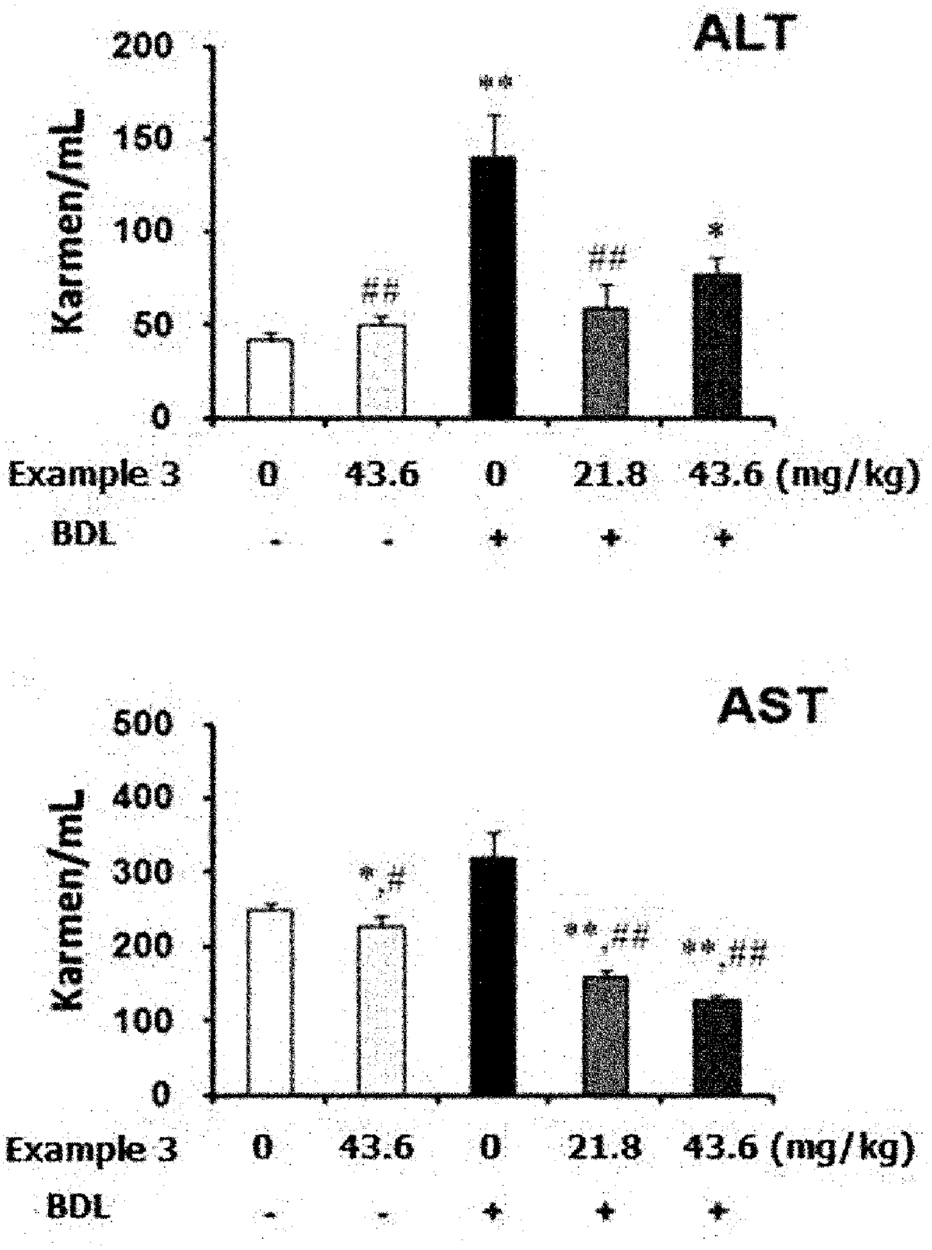


Data represents the mean ± S.E. (n = 5−8).
*: $p < 0.05$ vs. sham. **: $p < 0.01$ vs. sham.
#: $p < 0.05$ vs. BDL. ##: $p < 0.01$ vs. BDL.

【Figure 14b】

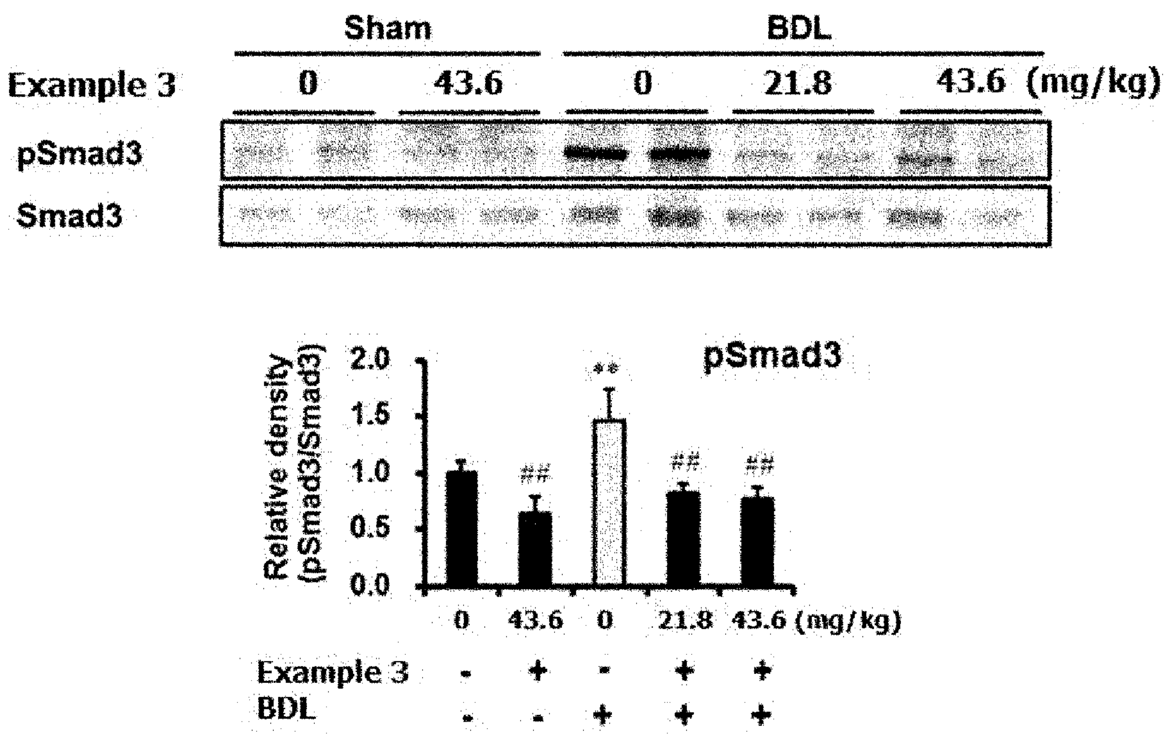


Data represents the mean ± S.E. (n = 5).
**: $p < 0.01$ vs. sham. ##: $p < 0.01$ vs. BDL.

【Figure 14c】

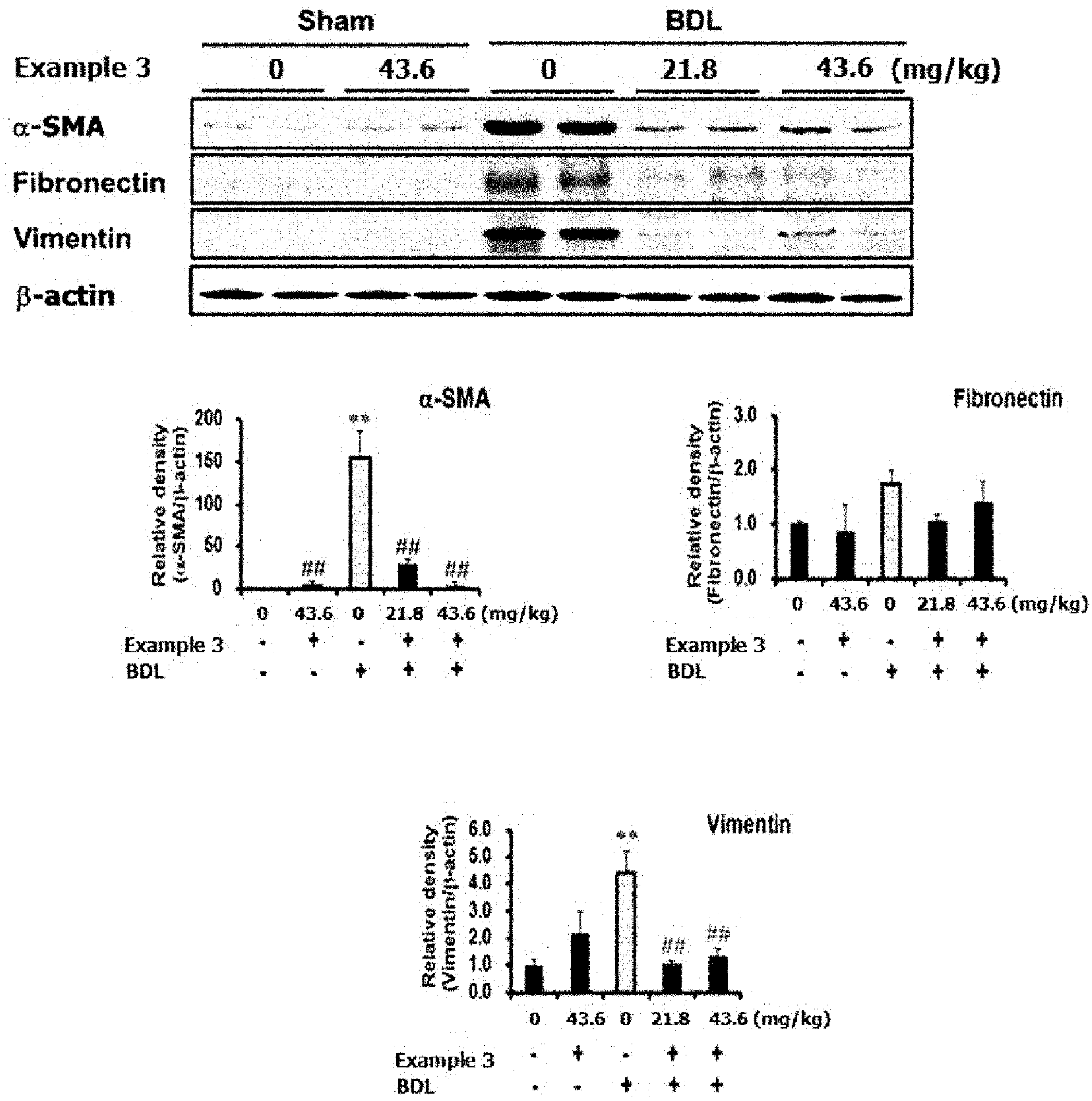


Data represents the mean ± S.E. (n = 5).
**: $p < 0.01$ vs. sham. ##: $p < 0.01$ vs. BDL.

【Figure 14d】

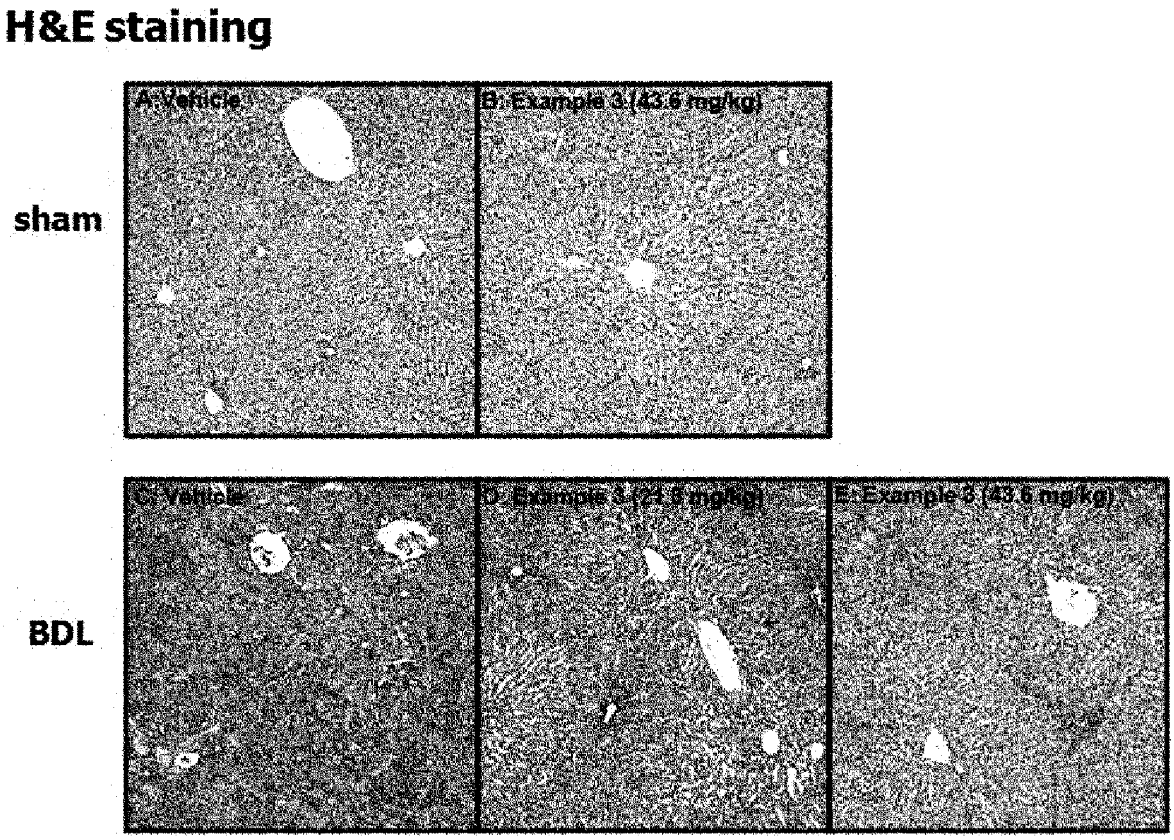
H&E staining
sham
BDL
A: Vehicle
B: Example 3 (43.6 mg/kg)
C: Vehicle
D: Example 3 (21.8 mg/kg)
E: Example 3 (43.6 mg/kg)

【Figure 15a】

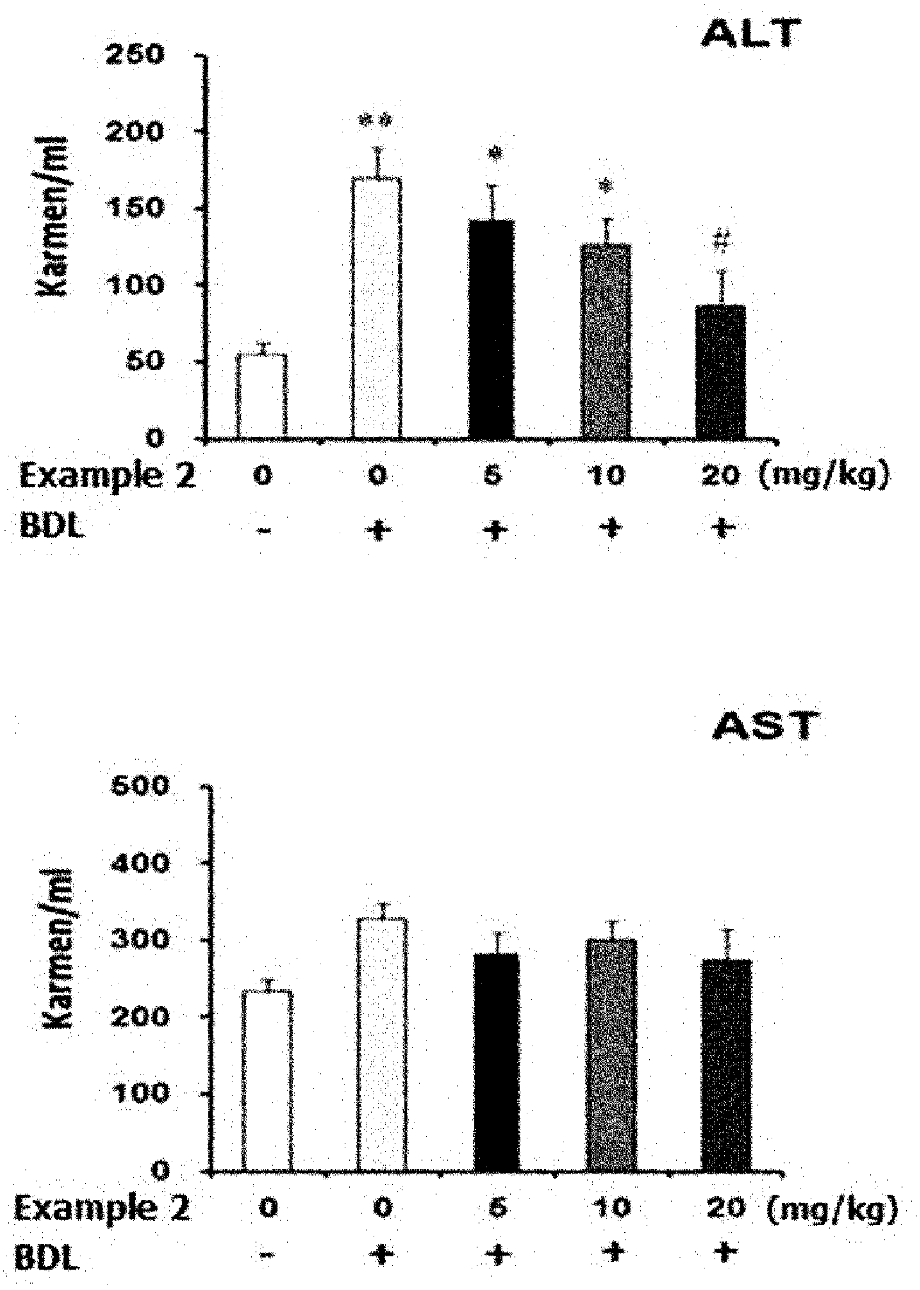


Data represents the mean ± S.E. (n = 5−8).
*: $p$ < 0.05 vs. sham. **: $p$ < 0.01 vs. sham.
#: $p$ < 0.05 vs. BDL.

【Figure 15b】

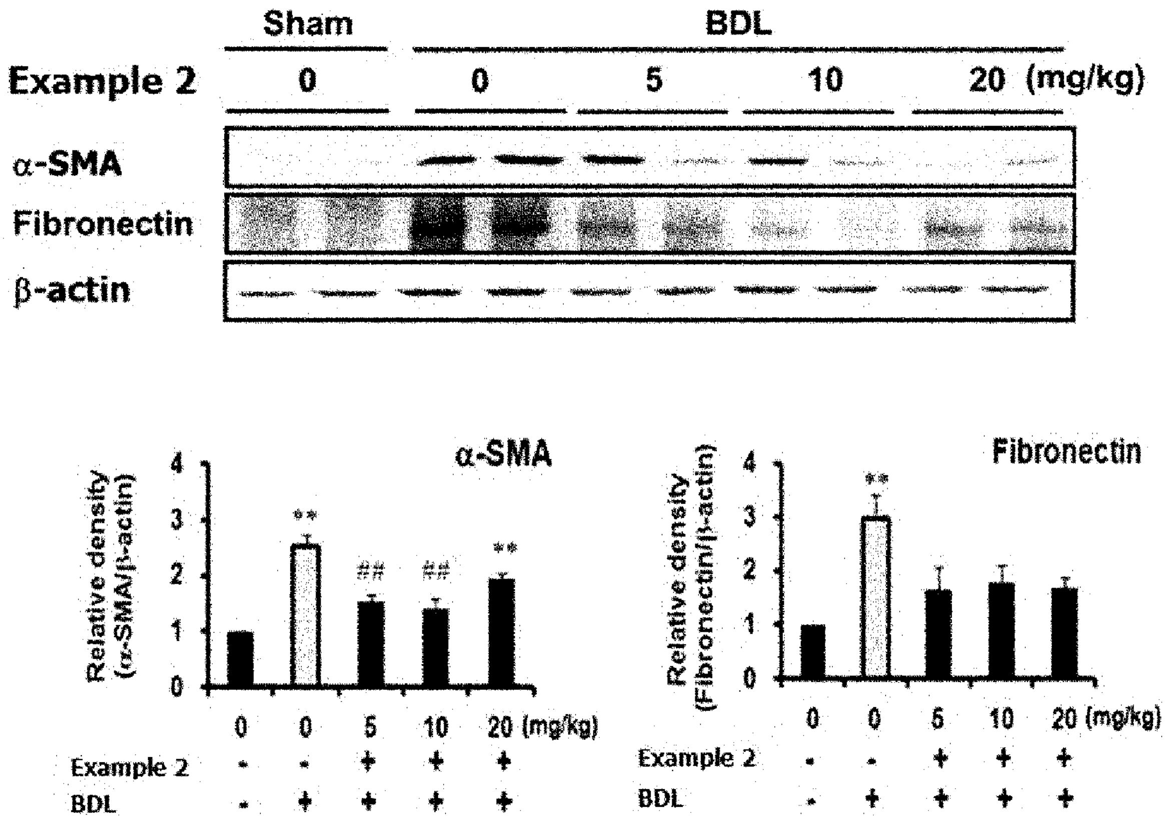


Data represents the mean ± S.E. (n = 5).
**: $p < 0.01$ vs. sham. ##: $p < 0.01$ vs. BDL.

【Figure 15c】

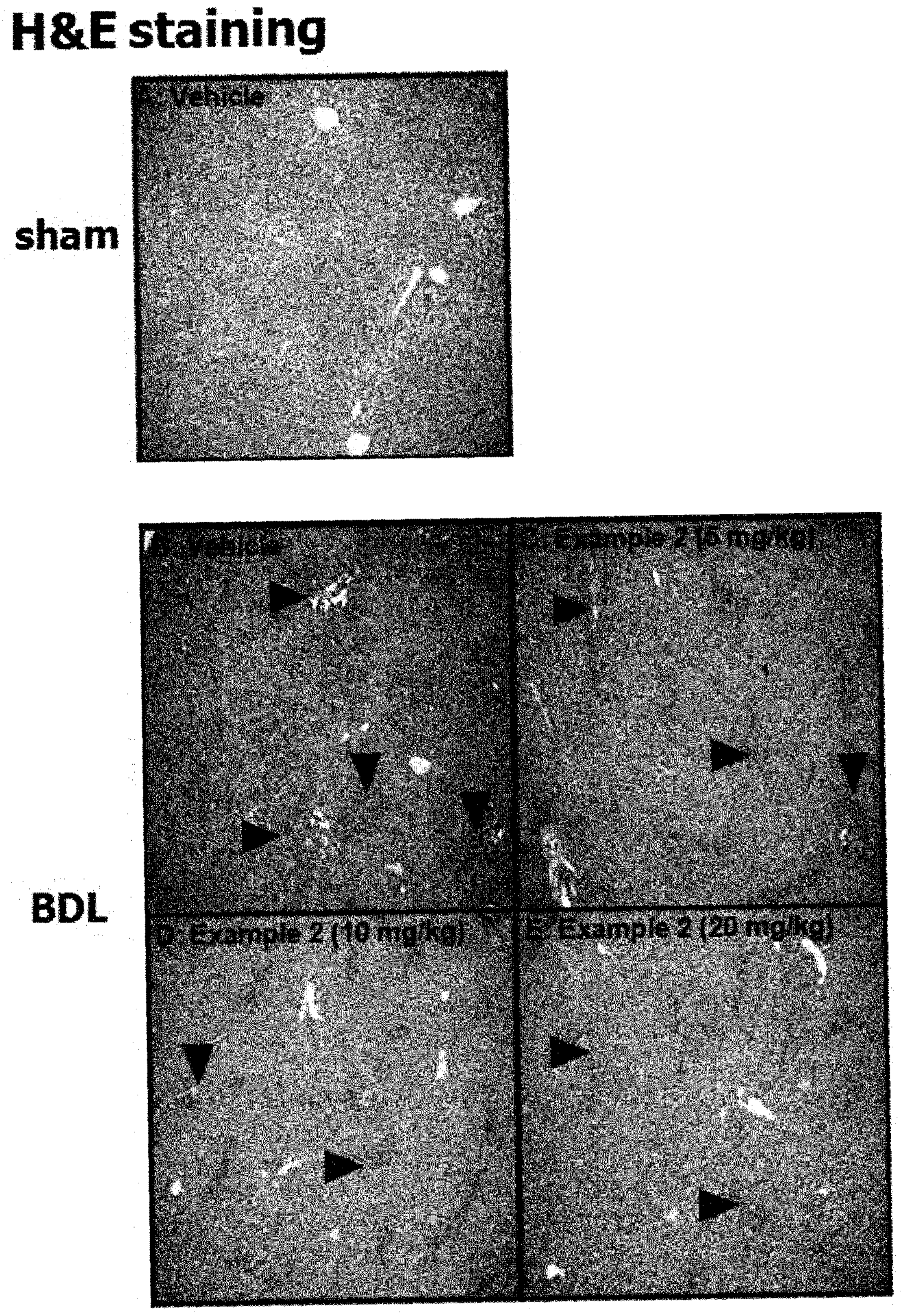
H&E staining
sham
Vehicle
BDL
Vehicle
Example 2 (5 mg/kg)
D: Example 2 (10 mg/kg)
E: Example 2 (20 mg/kg)

【Figure 16a】

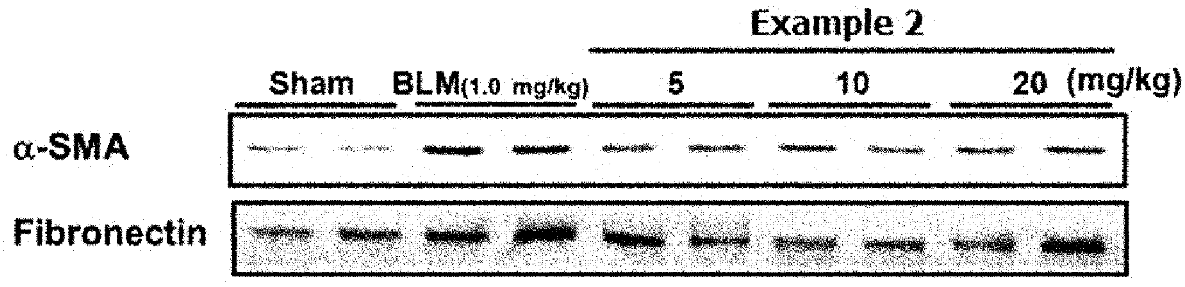

【Figure 16b】

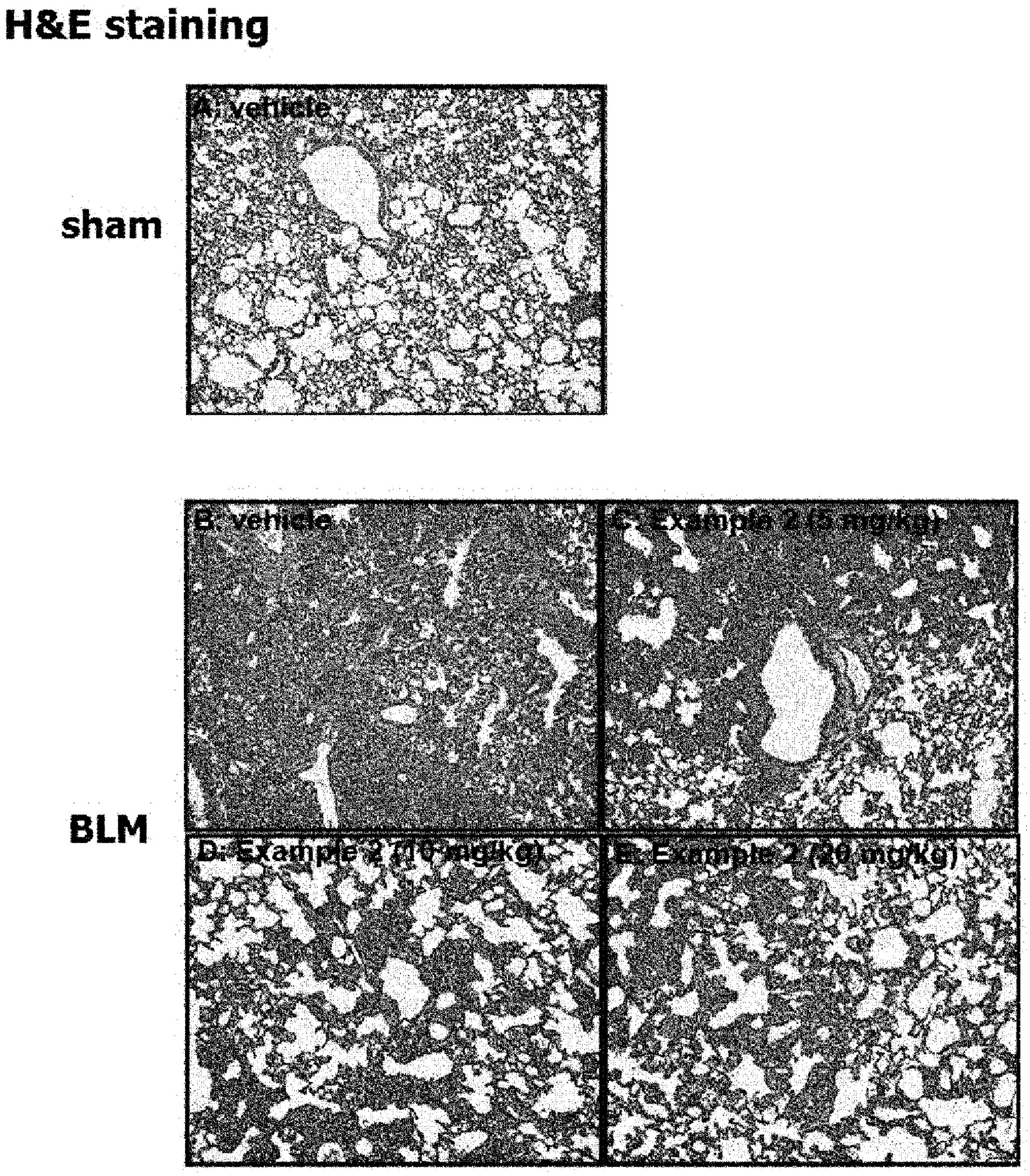
H&E staining
sham
A. vehicle
BLM
B. vehicle
C. Example 2 (5 mg/kg)
D. Example 2 (10 mg/kg)
E. Example 2 (20 mg/kg)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070142376 A1
- WO 0061576 A
- US 20030149277 A1
- WO 0162756 A1
- WO 02055077 A1
- WO 03087304 A2
- WO 2005103028 A1
- US 7407958 B2
- US 20080319012 A1
- WO 2005103028 A
- WO 2009150547 A
- WO 07024945 A1
- WO 02096875 A1


### Non-patent literature cited in the description


- **BITZER, M. et al.** *Kidney Blood Press. Res.,* 1998, vol. 21, 1-12
- **BORDER, W. A. et al.** *Nature,* 1990, vol. 346, 371-374
- **WAHAB, N. A. et al.** *Biochem. J.,* 1996, vol. 316, 985-992
- **ROCCO, M. V. et al.** *Kidney Int.,* 1992, vol. 41, 107-114
- **YOSHIOKA, K. et al.** *Lab. Invest.,* 1993, vol. 68, 154-163
- **EDDY, A. A.** *J. Am. Soc. Nephrol.,* 1996, vol. 7, 2495-2508
- **PHAN, S. H. ; KUNKEL, S. L.** *Exp. Lung Res.,* 1992, vol. 18, 29-43
- **WILLIAMS, A. O. et al.** *Am. J. Pathol.,* 1993, vol. 142, 1831-1840
- **RUBE, C. E. et al.** *Int. J. Radiat. Oncol. Biol. Phys.,* 2000, vol. 47, 1033-1042
- **BROEKELMANN, T. J. et al.** *Proc. Natl. Acad. Sci. USA,* 1991, vol. 88, 6642-6646
- **KHALIL, N. et al.** *Am. J. Respir. Cell. Mol. Biol.,* 1996, vol. 14, 131-138
- **JAGIRDAR, J. et al.** *Environ. Health Perspect.,* 1997, vol. 105, 1197-1203
- **GIRI, S. N. et al.** *Thorax,* 1993, vol. 48, 959-966
- **WANG, Q. et al.** *Thorax,* 1999, vol. 54, 805-812
- **WRIGHT, J. M. et al.** *Am. Rev. Respir. Dis.,* 1992, vol. 146, 240-262
- **TAKIZAWA, H.** *Int. J. Mol. Med.,* 1998, vol. 1, 367-378
- **NING, W. et al.** *Proc. Natl. Acad. Sci. USA,* 2004, vol. 101, 14895-14900
- **FRIEDMAN, S. L.** *Prog. Liver Dis.,* 1996, vol. 14, 101-130
- **PIETRANGELO, A.** *Semin. Liver Dis.,* 1996, vol. 16, 13-30
- **SANDERSON, N. et al.** *Proc. Natl. Acad. Sci. USA,* 1995, vol. 92, 2572-2576
- **SALTIS, J. et al.** *Clin. Exp. Pharmacol. Physiol.,* 1996, vol. 23, 193-200
- **MCCAFFREY, T. A. et al.** *J. Clin. Invest.,* 1995, vol. 96, 2667-2675
- **SHAH, M.** *J. Cell. Sci.,* 1995, vol. 108, 985-1002
- **MOLLER-PEDERSEN, T.** *Curr. Eye Res.,* 1998, vol. 17, 736-747
- **ERNST, H.** *Gut,* 1996, vol. 39, 172-175
- **MARTIN, M. et al.** *Int. J. Radiat. Oncol. Biol. Phys.,* 2000, vol. 47, 277-290
- **EL-GAMEL, A. et al.** *Eur. J. Cardiothorac. Surg.,* 1998, vol. 13, 424-430
- **SHIHAB, F. S. et al.** *J. Am. Soc. Nephrol.,* 1995, vol. 6, 286-294
- **SAED, G. M. et al.** *Wound Repair Regen.,* 1999, vol. 7, 504-510
- **PICHT, G. et al.** *Graefes Arch. Clin. Exp. Ophthalmol.,* 2001, vol. 239, 199-207
- **KOTTLER, U. B. et al.** *Exp. Eye Res.,* 2005, vol. 80, 121-134
- **HOJO, M. et al.** *Nature,* 1999, vol. 397, 530-534
- **MAEHARA, Y et al.** *J. Clin. Oncol.,* 1999, vol. 17, 607-614
- **PICON, A. et al.** *Cancer Epidemiol. Biomarkers Prev.,* 1998, vol. 7, 497-504
- **VAUGHAN, D. E.** *J. Invest. Med.,* 1998, vol. 46, 370-376
- **DENNLER, S. et al.** *EMBO J.,* 1998, vol. 17, 3091-3100
- **MATSUSE, T. et al.** *Am. J. Respir. Cell Mol. Biol.,* 1995, vol. 13, 17-24
- **INOUE, S. et al.** *Biochem. Biophys. Res. Comm.,* 1994, vol. 205, 441-448

- **MATSUSE, T. et al.** *Am. J. Pathol.,* 1996, vol. 148, 707-713
- **DE BLESER et al.** *Hepatology,* 1997, vol. 26, 905-912
- **PAWLOWSKI, J. E. et al.** *J. Clin. Invest.,* 1997, vol. 100, 639-648
- **SUGIYAMA, M. et al.** *Gastroenterology,* 1998, vol. 114, 550-558
- **MUNZ, B. et al.** *EMBO J.,* 1999, vol. 18, 5205-5215
- **ROSENDAHL, A. et al.** *Am. J. Respir. Cell Mol. Biol.,* 2001, vol. 25, 60-68
- **MATZUK, M. M. et al.** *Proc. Natl. Acd. Sci. USA,* 1994, vol. 91, 8817-8821
- **COERVER, K. A. et al.** *Mol. Endocrinol.,* 1996, vol. 10, 534-543
- **CIPRIANO, S. C. et al.** *Endocrinology,* 2000, vol. 141, 2319-2327
- **LOGAN, A. et al.** *Eur. J. Neurosci.,* 1999, vol. 11, 2367-2374
- **LOGAN, A. et al.** *Exp. Neurol.,* 1999, vol. 159, 504-510
- **MASLIAH, E. et al.** *Neurochem. Int.,* 2001, vol. 39, 393-400
- **DE GROOT, C. J. A. et al.** *J. Neuropathol. Exp. Neurol.,* 1999, vol. 58, 174-187
- **JOHN, G. R. et al.** *Nat. Med.,* 2002, vol. 8, 1115-1121
- **DAHLY, A. J. et al.** *Am. J. Physiol. Regul. Integr. Comp. Physiol.,* 2002, vol. 283, R757-767
- **MOON, J.-A. et al.** *Kidney Int.,* 2006, vol. 70, 1234-1243
- **LUO, J. et al.** *J. Clin. Invest.,* 2007, vol. 117, 3306-3315
- **RYU, J.-K. et al.** *J. Sex. Med.,* 2009, vol. 6, 1284-1296
- **LEE, G. T. et al.** *J. Urol.,* 2008, vol. 180, 2660-2667
- **LONG, L. et al.** *Circulation,* 2009, vol. 119, 566-576
- **KIM, J. H. et al.** *Radiology,* 2010, vol. 255, 75-82
- **KIM et al.** synthesis and biological evaluation of benzenesulfonamide-substituted 4-(6-alkylpyridon-2-yl)-5-(quioxalin-6-yl)imidazoles as transforming growth factor-beta type 1 receptor kinase inhibitors. *Eur.J. Med. Chem.,* 2009, vol. 44 (2), 568-576
- *J. Org. Chem.,* 2003, vol. 60, 5091-5103
- *Tetrahedron,* 1984, vol. 40, 1863-1868
- *J. Org. Chem.,* 1990, vol. 55, 1040-1043